# EUROPEAN PATENT APPLICATION

(11) **EP 3 904 568 A1**
(43) Date of publication of application: **03.11.2021**
(21) Application number: 19901650.2
(22) Date of filing: 26.12.2019
(51) Int. Cl.: C40B 40/06, C40B 40/10, C12N 15/09, C12P 21/02

(54) **MUTATED TRNA FOR CODON EXPANSION**

(30) Priority: 26.12.2018 JP 2018243478
(71) Applicant: CHUGAI SEIYAKU KABUSHIKI KAISHA, Tokyo 115-8543 (JP)
(72) Inventor: SHINOHARA, Shojiro, Kamakura-shi, Kanagawa 247-8530 (JP); TANIGUCHI, Takaaki, Synapse, 138623 (SG); MISAIZU, Miki, Kamakura-shi, Kanagawa 247-8530 (JP); NISHIMURA, Kaori, Kamakura-shi, Kanagawa 247-8530 (JP); IWAHASHI, Mana, Kamakura-shi, Kanagawa 247-8530 (JP); EMURA, Takashi, Gotemba-shi, Shizuoka 412-8513 (JP); NAKANO, Kazuhiko, Kamakura-shi, Kanagawa 247-8530 (JP); TANAKA, Masahiko, Kamakura-shi, Kanagawa 247-8530 (JP); OHDAKE, Takamichi, Kamakura-shi, Kanagawa 247-8530 (JP); OHTA, Atsushi, Kamakura-shi, Kanagawa 247-8530 (JP)
(74) Representative: Vossius & Partner Patentanwälte Rechtsanwälte mbB
(86) International application number: PCT/JP2019/051241
(87) International publication number: WO 2020/138336

(57) **Abstract**

In some embodiments, the present disclosure relates to mutated tRNAs in which the first letter of the anticodon has been substituted to lysidine or agmatidine, and translation systems containing the mutated tRNAs. In a specific embodiment, the present disclosure provides mutated tRNAs capable of selectively translating codon NNA. In another embodiment, the present disclosure provides translation systems capable of translating two or three types of amino acids from a single codon box. In still another embodiment, the present disclosure provides novel methods for synthesizing lysidine diphosphate, agmatidine diphosphate, and derivatives thereof.

## Description

### [Technical Field]

The present disclosure relates to tRNAs and translation systems, and methods of their use.

### [Background Art]

Display library is a very useful technology by which molecules binding to a target protein can be obtained efficiently in an evolutionary engineering manner. In order to use a display library to obtain a molecule that exhibits high binding ability to an arbitrary target molecule, or to obtain many molecules each of which respectively bind to different epitopes, panning of a highly diverse library is required. To construct a highly diverse library, the number or variety of building blocks of the library may be increased; however, when there is a limit on the molecular weight from the viewpoint of membrane permeability, the number of building blocks will also be limited. Therefore, the strategy of increasing the variety of building blocks is important for increasing library diversity.

In reconstituted cell-free translation systems such as PURESYSTEM (Non-Patent Literature (NPL) 1), natural codon-amino acid correspondences can be altered because the concentrations of components such as amino acids, tRNAs, and aminoacyl-tRNA synthetases (ARSs) can be adjusted. The use of such translation systems has enabled construction of display libraries into which 20 or more different arbitrary building blocks are introduced. However, in the *Escherichia coli* translation system using three-base codons, only up to 32 different building blocks may be introduced in principle, because of the wobble rule. To give a more specific explanation, there is some "play" in the pairing of the third letter of codons and the first letter of anticodons, and this allows pairing between G and U, called a wobble base pair, in addition to Watson-Crick base pairs. Therefore, the anticodon GNN decodes the NNU and NNC codons, and the anticodon UNN decodes the NNA and NNG codons. Thus, the discrimination between these codons is not possible, limiting the maximum number of different amino acids that can be introduced into one codon box to two (NPL 2).

On the other hand, in nature, there are means to enable discrimination between the AUA and AUG codons. One such example is lysidine modification introduced into *E. coli* tRNA Ile2 at position 34 (the first letter of the anticodon). This modification is known to let tRNA Ile2 decode only the AUA codon and not the AUG codon (NPL 3). This modification is introduced by isoleucine tRNA-lysidine synthetase (tRNA^{Ile}-lysidine synthetase; TilS) (NPL 4). Since its substrate tRNA is only tRNA Ile2, it is not easy to introduce lysidine into other tRNAs (NPL 5).

### [Citation List]

### [Non-patent Literature]

[NPL 1] Shimizu et al., Nat Biotechnol. 2001 Aug; 19(8): 751-755
[NPL 2] Iwane et al., Nat Chem. 2016 Apr; 8(4): 317-325
[NPL 3] Grosjean et al., Trends Biochem Sci. 2004 Apr; 29(4): 165-168
[NPL 4] Suzuki T et al., FEBS Lett. 2010 Jan 21; 584(2): 272-277
[NPL 5] Lajoie et al., J Mol Biol. 2016 Feb 27; 428(5 Pt B): 1004-1021

### [Summary of Invention]

### [Technical Problem]

As mentioned above, introduction of lysidine into tRNA Ile2 at position 34 (the first letter of the anticodon) enables discrimination of the AUA and AUG codons. However, there are no other tRNAs modified with lysidine in nature. In addition, no artificial means to discriminate the NNA and NNG codons have been reported. The present invention was achieved in view of such circumstances. An objective of the present disclosure is to provide novel means for enabling discrimination of the NNA and NNG codons.

### [Solution to Problem]

Here, the present inventors linked chemically synthesized tRNA fragments with lysidine (also known as 2-lysylcitidine) by an enzymatic reaction to prepare tRNAs into which lysidine is introduced at position 34, and which have various sequences at positions 35 and 36 (second and third letters of the anticodon). When translation systems containing these tRNAs were reconstituted and amino acid translations were performed, it was found that any of these translation systems enabled discrimination of the NNA and NNG codons. In addition, it was found in these examination processes that although tRNAs having the UNN anticodon decode not only the NNA and NNG codons but also the NNU codon, this misreading of the NNU codon was significantly reduced with lysidine-introduced tRNA.

The present disclosure is based on such findings, and specifically encompasses the embodiments exemplified below:
[1] a mutated tRNA produced by engineering a tRNA, wherein the engineering comprises a engineering, such that, in its anticodon represented by N₁N₂N₃, the first letter nucleoside N₁ after the engineering is any one of lysidine (k2C), a lysidine derivative, agmatidine (agm2C), and an agmatidine derivative, wherein N₂ and N₃ are arbitrary nucleosides for the second letter and the third letter of the anticodon, respectively;
[2] the mutated tRNA of [1], wherein N₁ prior to the engineering is cytidine (C), and the engineering from this cytidine (C) to lysidine (k2C) cannot be catalyzed by a lysidine synthetase (tRNA^{Ile}-lysidine synthetase; TilS) having the amino acid sequence of SEQ ID NO: 51;
[3] the mutated tRNA of [1], wherein N₁ prior to the engineering is cytidine (C), and the engineering from this cytidine (C) to agmatidine (agm2C) cannot be catalyzed by an agmatidine synthetase (tRNA^{Ile}-agmatidine synthetase; TiaS) having the amino acid sequence of SEQ ID NO: 52;
[4] the mutated tRNA of any one of [1] to [3], comprising an anticodon complementary to the codon represented by M₁M₂A (wherein M₁ and M₂ represent nucleosides for the first and second letters of the codon respectively; each of M₁ and M₂ is selected from any of adenosine (A), guanosine (G), cytidine (C), and uridine (U); and the nucleoside of the third letter corresponds to adenosine);
[5] the mutated tRNA of [4], wherein the anticodon is represented by k2CN₂N₃ or agm2CN₂N₃ (wherein the nucleoside of the first letter of the anticodon is lysidine (k2C) or agmatidine (agm2C), and the nucleoside of the second letter (N₂) and the nucleoside of the third letter (N₃) are complementary to M₂ and M₁, respectively);
[6] the mutated tRNA of [5], wherein each of N₂ and N₃ is selected from any of adenosine (A), guanosine (G), cytidine (C), and uridine (U);
[7] the mutated tRNA of any one of [1] to [6], wherein the tRNA is an initiator tRNA or an elongator tRNA;
[8] the mutated tRNA of any one of [1] to [7], wherein the tRNA is derived from a prokaryote or a eukaryote;
[9] the mutated tRNA of any one of [4] to [8], wherein M₁ and M₂ are selected from codons that constitute a codon box in which a codon with the third letter nucleoside being A and a codon with the third letter nucleoside being G both encode the same amino acid in the natural genetic code table;
[10] the mutated tRNA of any one of [4] to [8], wherein M₁ and M₂ are selected from codons that constitute a codon box in which a codon with the third letter nucleoside being U and a codon with the third letter nucleoside being A both encode the same amino acid in the natural genetic code table;
[11] the mutated tRNA of any one of [4] to [8], wherein M₁ and M₂ are selected from codons that constitute a codon box in which a codon with the third letter nucleoside being U, a codon with the third letter nucleoside being C, a codon with the third letter nucleoside being A, and a codon with the third letter nucleoside being G all encode the same amino acid in the natural genetic code table;
[12] the mutated tRNA of any one of [4] to [8], wherein M₁ and M₂ are selected from codons that constitute a codon box in which a codon with the third letter nucleoside being A and a codon with the third letter nucleoside being G encode different amino acids from each other in the natural genetic code table;
[13] the mutated tRNA of any one of [4] to [8], wherein M₁ and M₂ are selected from codons that constitute a codon box in which a codon with the third letter nucleoside being A and/or a codon with the third letter nucleoside being G are stop codons in the natural genetic code table;
[14] the mutated tRNA of any one of [4] to [8], wherein M₁ is uridine (U) and M₂ is cytidine (C);
[15] the mutated tRNA of any one of [4] to [8], wherein M₁ is cytidine (C) and M₂ is uridine (U);
[16] the mutated tRNA of any one of [4] to [8], wherein M₁ is cytidine (C) and M₂ is cytidine (C);
[17] the mutated tRNA of any one of [4] to [8], wherein M₁ is cytidine (C) and M₂ is guanosine (G);
[18] the mutated tRNA of any one of [4] to [8], wherein M₁ is adenosine (A) and M₂ is uridine (U);
[19] the mutated tRNA of any one of [4] to [8], wherein M₁ is guanosine (G) and M₂ is uridine (U);
[20] the mutated tRNA of any one of [4] to [8], wherein M₁ is guanosine (G) and M₂ is cytidine (C);
[21] the mutated tRNA of any one of [4] to [8], wherein M₁ is guanosine (G) and M₂ is guanosine (G);
[22] the mutated tRNA of [14], wherein N₂ is guanosine (G) and N₃ is adenosine (A);
[23] the mutated tRNA of [15], wherein N₂ is adenosine (A) and N₃ is guanosine (G);
[24] the mutated tRNA of [16], wherein N₂ is guanosine (G) and N₃ is guanosine (G);
[25] the mutated tRNA of [17], wherein N₂ is cytidine (C) and N₃ is guanosine (G);
[26] the mutated tRNA of [18], wherein N₂ is adenosine (A) and N₃ is uridine (U);
[27] the mutated tRNA of [19], wherein N₂ is adenosine (A) and N₃ is cytidine (C);
[28] the mutated tRNA of [20], wherein N₂ is guanosine (G) and N₃ is cytidine (C);
[29] the mutated tRNA of [21], wherein N₂ is cytidine (C) and N₃ is cytidine (C);
[30] the mutated tRNA of any one of [1] to [29], wherein an amino acid or an amino acid analog is attached to the 3' end;
[31] the mutated tRNA of [30], wherein the amino acid is a natural amino acid or an unnatural amino acid;
[32] the mutated tRNA of [31], wherein the natural amino acid is selected from the group consisting of glycine (Gly), alanine (Ala), serine (Ser), threonine (Thr), valine (Val), leucine (Leu), isoleucine (Ile), phenylalanine (Phe), tyrosine (Tyr), tryptophan (Trp), histidine (His), glutamic acid (Glu), aspartic acid (Asp), glutamine (Gln), asparagine (Asn), cysteine (Cys), methionine (Met), lysine (Lys), arginine (Arg), and proline (Pro);
[33] the mutated tRNA of [32], wherein the natural amino acid is selected from the group consisting of glycine (Gly), alanine (Ala), serine (Ser), threonine (Thr), valine (Val), leucine (Leu), phenylalanine (Phe), tyrosine (Tyr), tryptophan (Trp), histidine (His), glutamic acid (Glu), aspartic acid (Asp), glutamine (Gln), asparagine (Asn), cysteine (Cys), lysine (Lys), arginine (Arg), and proline (Pro);
[34] a translation system comprising a plurality of different tRNAs, wherein the system comprises the mutated tRNA of any one of [1] to [33];
[35] the translation system of [34], wherein a codon represented by M₁M₂A can be translated more selectively by the mutated tRNA than a codon different from the codon represented by M₁M₂A, and the mutated tRNA can translate the codon represented by M₁M₂A more selectively than a tRNA other than the mutated tRNA;
[36] the translation system of [34] or [35], comprising (a) the mutated tRNA of any one of [1] to [33], and (b) a tRNA comprising an anticodon complementary to a codon represented by M₁M₂G;
[37] the translation system of [36], wherein the anticodon of the tRNA according to [36](b) is CN₂N₃, ac4CN₂N₃, or CmN₂N₃ (wherein ac4C represents N4-acetylcytidine and Cm represents 2' -O-methylcytidine);
[38] the translation system of [36] or [37], wherein a codon represented by M₁M₂G can be translated more selectively by the tRNA of [36](b) than a codon different from the codon represented by M₁M₂G, and the tRNA of [36](b) can translate the codon represented by M₁M₂G more selectively than a tRNA other than the tRNA of [36](b);
[39] the translation system of any one of [36] to [38], wherein the amino acids or amino acid analogs attached to the tRNAs of [36](a) and [36](b) are different from each other;
[40] the translation system of [39], wherein two amino acids can be translated from the M₁M₂A and M₁M₂G codons;
[41] the translation system of [39], wherein the M₁M₂A and M₁M₂G codons may encode amino acids or amino acid analogs that are different from each other;
[42] the translation system of any one of [34] to [41], further comprising (c) a tRNA comprising an anticodon complementary to a codon represented by M₁M₂U or M₁M₂C;
[43] the translation system of [42], wherein an anticodon of the tRNA of [42](c) is selected from a group consisting of AN₂N₃, GN₂N₃, QN₂N₃, and GluQN₂N₃ (wherein Q represents queuosine, and GluQ represents glutamyl-queuosine);
[44] the translation system of [42] or [43], wherein a codon represented by M₁M₂U or M₁M₂C can be translated more selectively by the tRNA of [42](c) than a codon different from the codon represented by M₁M₂U or M₁M₂C, and the tRNA of [42](c) can translate the codon represented by M₁M₂U or M₁M₂C more selectively than a tRNA other than the tRNA of [42](c);
[45] the translation system of any one of [42] to [44], wherein the amino acids or amino acid analogs attached to the tRNAs of [36](a), [36](b), and [42](c) are all different from each other;
[46] the translation system of [45], wherein three amino acids can be translated from a codon box composed of M₁M₂U, M₁M₂C, M₁M₂A, and M₁M₂G;
[47] the translation system of [45], wherein in the codon box composed of M₁M₂U, M₁M₂C, M₁M₂A, and M₁M₂G,
   (i) M₁M₂A, M₁M₂G, and M₁M₂U may encode amino acids or amino acid analogs that are different from each other, or
   (ii) M₁M₂A, M₁M₂G, and M₁M₂C may encode amino acids or amino acid analogs that are different from each other;
[48] the translation system of any one of [45] to [47], wherein an unnatural amino acid is attached to at least one of the tRNAs of [36](a), [36](b), and [42](c);
[49] the translation system of any one of [34] to [48], which can translate more than 20 amino acids;
[50] the translation system of any one of [34] to [49], which is a cell-free translation system;
[51] the translation system of [50], which is a reconstituted cell-free translation system;
[52] the translation system of [50] or [51], comprising an *Escherichia coli*-derived ribosome;
[53] a method for producing a peptide, comprising translating a nucleic acid using the translation system of any one of [34] to [52];
[54] the method of [53], wherein the peptide has a cyclic portion;
[55] a peptide produced by the method of [53] or [54];
[56] a method for producing a peptide library, comprising translating a nucleic acid library using the translation system of any one of [34] to [52];
[57] a peptide library produced by the method of [56];
[58] a method for identifying a peptide having binding activity to a target molecule, comprising contacting the target molecule with the peptide library of [57];
[59] a nucleic acid-peptide complex comprising a peptide and a nucleic acid encoding the peptide, wherein the nucleic acid encoding the peptide comprises the three codons of either (A) or (B) below:
   (A) M₁M₂U, M₁M₂A, and M₁M₂G;
   (B) M₁M₂C, M₁M₂A, and M₁M₂G;
   and wherein the amino acids corresponding to the three codons are all different on the peptide.
[60] a library comprising the nucleic acid-peptide complex of [59];
[61] the following compound or a salt thereof: 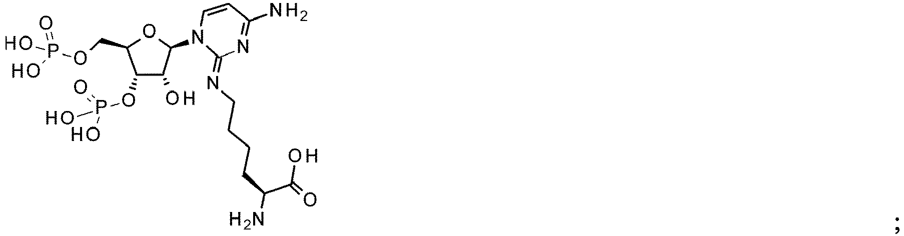
[62] a method for producing a mutated tRNA having lysidine at position 34 according to the tRNA numbering rule, comprising ligating the compound of [61] and a nucleic acid fragment constituting the tRNA by an enzymatic reaction;
[63] a method for producing a mutated tRNA having lysidine at position 34 according to the tRNA numbering rule and having an amino acid or an amino acid analog attached to the 3' end, comprising ligating the compound of [61], one or more nucleic acid fragments constituting the tRNA, and an amino acid or an amino acid analog, by an enzymatic reaction;
[64] the following compound or a salt thereof: 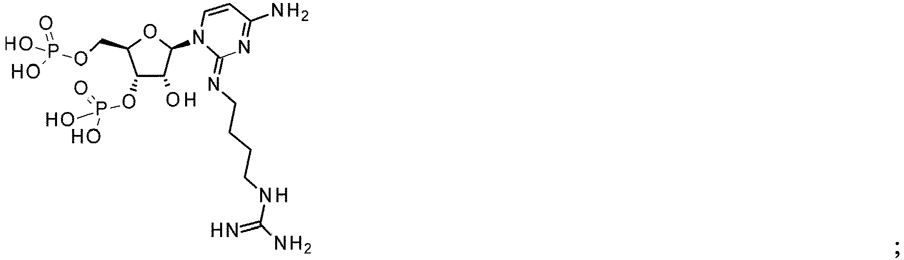
[65] a method for producing a mutated tRNA having agmatidine at position 34 according to the tRNA numbering rule, comprising ligating the compound of [64] and a nucleic acid fragment constituting the tRNA by an enzymatic reaction;
[66] a method for producing a mutated tRNA having agmatidine at position 34 according to the tRNA numbering rule and having an amino acid or an amino acid analog attached to the 3' end, comprising ligating the compound of [64], one or more nucleic acid fragments constituting the tRNA, and an amino acid or an amino acid analog, by an enzymatic reaction;
[67] the method of [63] or [66], wherein the amino acid is an amino acid other than methionine (Met) and isoleucine (Ile);
[68] a mutated tRNA produced by the method of [62] or [65];
[69] a mutated tRNA having an amino acid or an amino acid analog attached to the 3' end, which is produced by the method of [63], [66], or [67];
[70] a translation system comprising the mutated tRNA of [68] and/or [69];
[71] a method for producing a peptide, comprising translating a nucleic acid using the translation system of [70];
[72] a method for producing lysidine diphosphate or a derivative thereof, or agmatidine diphosphate or a derivative thereof, which is represented by the following formula A: 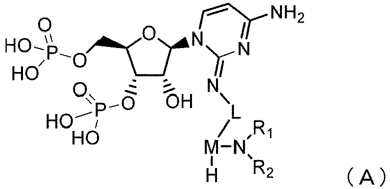 (wherein,
   R₁ and R₂ are each independently H or C₁-C₃ alkyl,
   L is a C₂-C₆ straight chain alkylene or a C₂-C₆ straight chain alkenylene optionally substituted with one or more substituents selected from the group consisting of a hydroxy and C₁-C₃ alkyl, wherein a carbon atom of the C₂-C₆ straight chain alkylene is optionally substituted with one oxygen atom or sulfur atom,
   M is a single bond 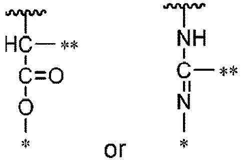
   wherein the wavy line indicates the point of attachment to the carbon atom, * indicates the point of attachment to the hydrogen atom, and ** indicates the point of attachment to the nitrogen atom, provided that when M is a single bond, H attached to M is not present), the method comprising the steps of:
      intramolecularly cyclizing a compound represented by the following formula B1: 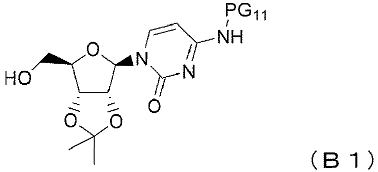
   (wherein, PG₁₁ is a protecting group for an amino group) to obtain a compound represented by the following formula C1: 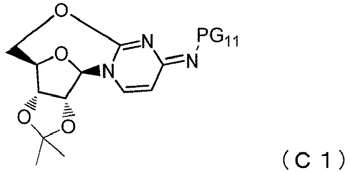 (wherein, PG₁₁ is the same as above);
   introducing an amine represented by the following formula D1: 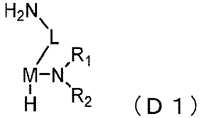 (wherein, R₁, R₂, L, and M are the same as above)
   or a salt thereof to the compound represented by the formula C1 to obtain a compound represented by the following formula E1: 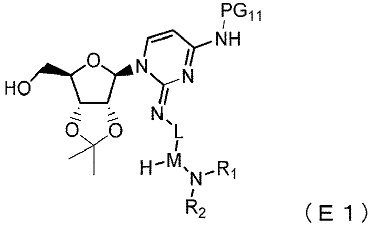 (wherein, R₁, R₂, L, M, and PG₁₁ are the same as above);
   introducing PG₁₂ and/or PG₁₃ to the compound represented by the formula E1 to obtain a compound represented by the following formula F1A or F1B: 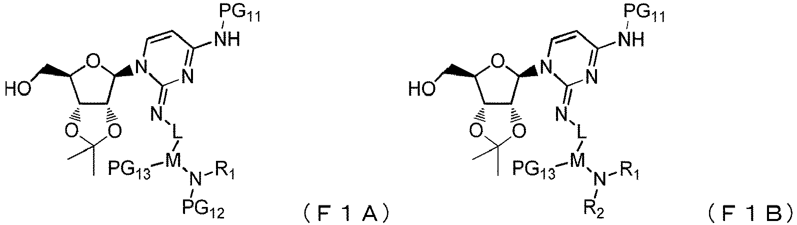 (wherein,
      R₂ is C₁-C₃ alkyl,
      PG₁₂ is a protecting group for an amino group,
      PG₁₃ is a protecting group for a carboxyl group or an imino group, and
      R₁, L, M, and PG₁₁ are the same as above,
      provided that when M is a single bond, PG₁₃ is not present);
   removing acetonide from the compound represented by the formula F1A or FIB, and introducing PG₁₄ and PG₁₅, to obtain a compound represented by the following formula G1A or GIB: 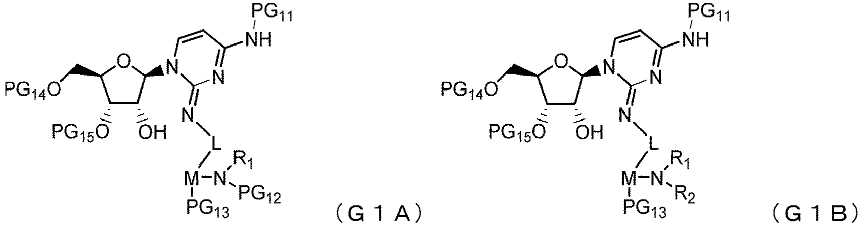 (wherein,
      R₂ is C₁-C₃ alkyl,
      PG₁₄ is a protecting group for a hydroxy group,
      PG₁₅ is a protecting group for a hydroxy group, and
      R₁, L, M, PG₁₁, PG₁₂, and PG₁₃ are the same as above);
   introducing PG₁₆ to the compound represented by the formula G1A or G1B to obtain a compound represented by the following formula H1A or H1B: 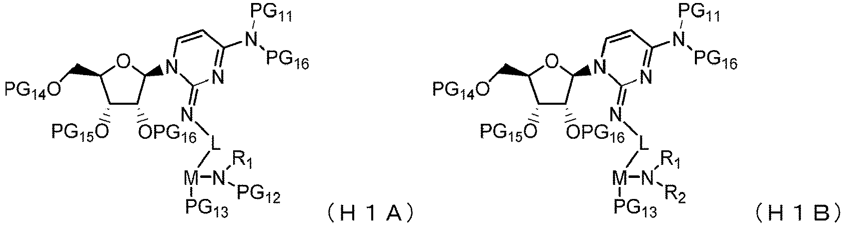 (wherein,
      R₂ is C₁-C₃ alkyl,
      PG₁₆ is a protecting group for a hydroxy group and/or an amino group, and
      R₁, L, M, PG₁₁, PG₁₂, PG₁₃, PG₁₄, and PG₁₅ are the same as above);
   removing PG₁₄ and PG₁₅ from the compound represented by the formula H1A or H1B to obtain a compound represented by the formula I1A or I1B: 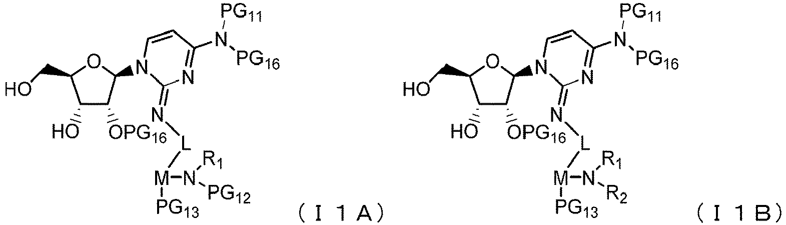 (wherein,
      R₂ is C₁-C₃ alkyl, and
      R₁, L, M, PG₁₁, PG₁₂, PG₁₃, and PG₁₆ are the same as above);
   phosphite-esterifying the compound represented by the formula I1A or I1B and then oxidizing it, to obtain a compound represented by the following formula J1A or J1B: 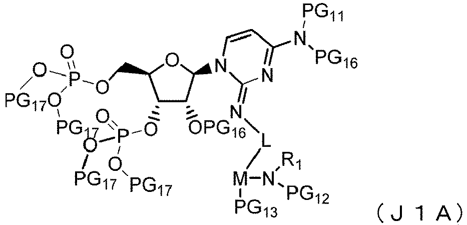 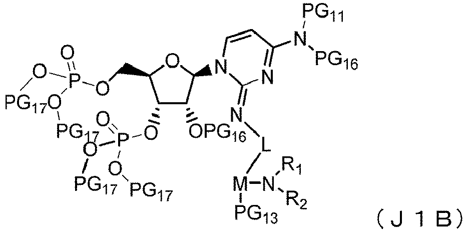 (wherein,
      R₂ is C₁-C₃ alkyl,
      PG₁₇ is a protecting group for a hydroxy group, and
      R₁, L, M, PG₁₁, PG₁₂, PG₁₃, and PG₁₆ are the same as above);
   removing PG₁₁, PG₁₂, PG₁₃, and PG₁₇ from the compound represented by the formula J1A, or removing PG₁₁, PG₁₃, and PG₁₇ from the compound represented by the formula JIB, to obtain a compound represented by the following formula K1: 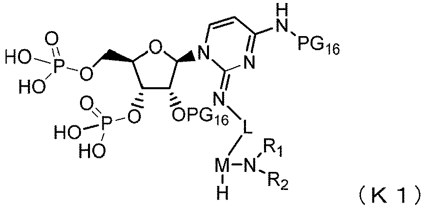 (wherein,
      R₂ is H or C₁-C₃ alkyl, and
      R₁, R₂, L, M, and PG₁₆ are the same as above); and
   removing PG₁₆ from the compound represented by the formula K1 to obtain the compound represented by the formula A;
[73] the method of [72], wherein the compound represented by the formula A is lysidine diphosphate: 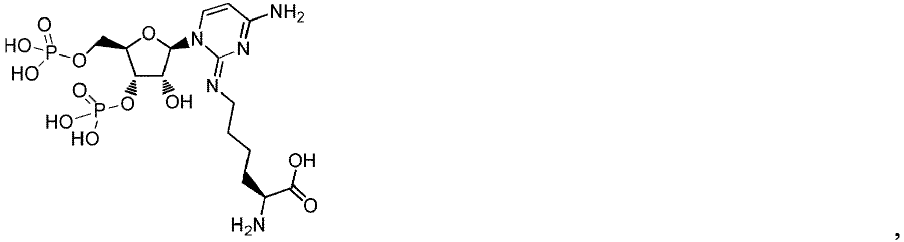 or agmatidine diphosphate: 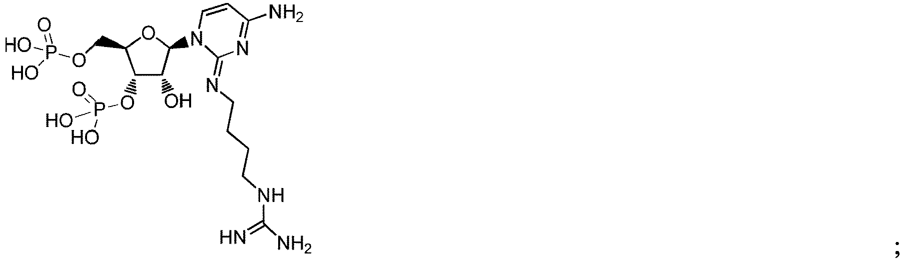
[74] the method of [72], wherein PG₁₁ is p-bromobenzoyl, an optionally substituted benzoyl, pyridinecarbonyl, or acetyl;
[75] the method of [72], wherein PG₁₂ is Fmoc;
[76] the method of [72], wherein when M is 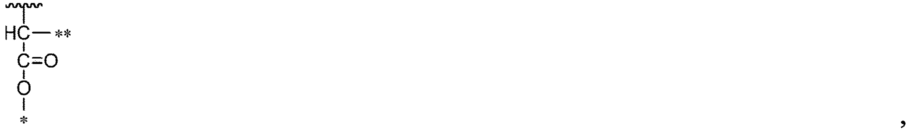 PG₁₃ is methyl, ethyl, or an optionally substituted benzyl, and when M is 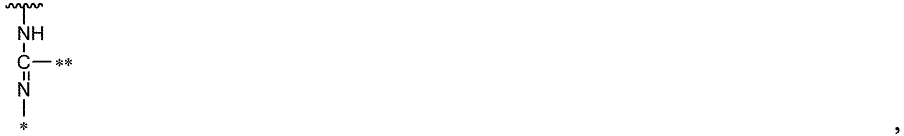 PG₁₃ is an optionally substituted benzyl, Cbz, or an optionally substituted benzyloxycarbonyl;
[77] the method of [72], wherein PG₁₄ and PG₁₅ are taken together to form di-tert-butylsilyl;
[78] the method of [72], wherein PG₁₆ is TOM;
[79] the method of [72], wherein PG₁₇ is cyanoethyl;
[80] the method of [72], wherein the intramolecular cyclization is carried out in the presence of diisopropyl azodicarboxylate and triphenylphosphine;
[81] the method of [72], wherein the introduction of the amine represented by the formula D1 or a salt thereof is carried out in the presence of lithium chloride and DBU;
[82] the method of [72], wherein PG₁₂ is Fmoc, and reagents used for introducing PG₁₂ are (2,5-dioxopyrrolidin-1-yl)(9H-fluoren-9-yl)methyl carbonate and sodium carbonate;
[83] the method of [72], wherein PG₁₃ is methyl, and reagents used to introduce PG₁₃ are N,N'-diisopropylcarbodiimide, methanol, and N,N-dimethyl-4-aminopyridine;
[84] the method of [72], wherein a reagent used for removing acetonide is TFA;
[85] the method of [72], wherein PG₁₄ and PG₁₅ are taken together to form di-tert-butylsilyl, and a reagent used to introduce di-tert-butylsilyl is di-tert-butylsilyl bis(trifluoromethanesulfonate);
[86] the method of [72], wherein PG₁₆ is TOM, and reagents used to introduce PG₁₆ are DIPEA and (triisopropylsiloxy)methyl chloride;
[87] the method of [72], wherein a reagent used to remove PG₁₄ and PG₁₅ is hydrogen fluoride pyridine complex;
[88] the method of [72], wherein a reagent used for t phosphite-esterification is bis(2-cyanoethyl)-N,N-diisopropylaminophosphoramidite;
[89] the method of [72], wherein a reagent used for oxidation is tert-butylhydroperoxide;
[90] the method of [72], wherein reagents used for removing PG₁₁, PG₁₂, PG₁₃, and PG₁₇ are bis-(trimethylsilyl)acetamide and DBU;
[91] the method of [72], wherein a reagent used for removing PG₁₆ is ammonium fluoride;
[92] the method of [72], wherein R₁ is H;
[93] the method of [72], wherein R₂ is H;
[94] the method of [72], wherein the C₂-C₆ straight chain alkylene or a C₂-C₆ straight chain alkenylene is C₄-C₅ straight chain alkylene or a C₄-C₅ straight chain alkenylene;
[95] the method of [72], wherein L is -(CH₂)₃-, -(CH₂)₄-, -(CH₂)₅, -(CH₂)₂-O-CH₂-, -(CH₂)₂-S-CH₂-, -CH₂CH(OH)(CH₂)₂-, or -CH₂CH=CH- (cis or trans);
[96] a method for producing lysidine diphosphate or a derivative thereof, or agmatidine diphosphate or a derivative thereof, which is represented by the following formula A: 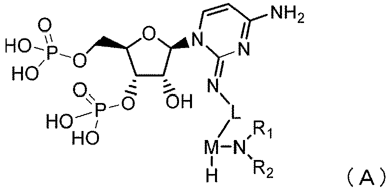 (wherein,
   R₁ and R₂ are each independently H or C₁-C₃ alkyl,
   L is a C₂-C₆ straight chain alkylene or a C₂-C₆ straight chain alkenylene optionally substituted with one or more substituents selected from the group consisting of a hydroxy and C₁-C₃ alkyl, wherein a carbon atom of the C₂-C₆ straight chain alkylene may be substituted with one oxygen atom or sulfur atom,
   M is a single bond, 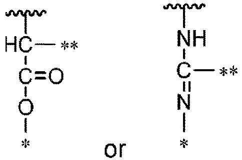
   wherein the wavy line indicates the point of attachment to the carbon atom, * indicates the point of attachment to the hydrogen atom, and ** indicates the point of attachment to the nitrogen atom, provided that when M is a single bond, H attached to M is not present), the method comprising the steps of:
      intramolecularly cyclizing a compound represented by the following formula B2: 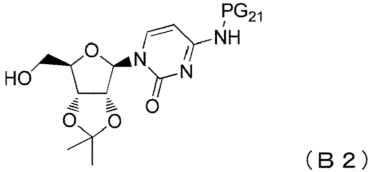 (wherein, PG₂₁ is a protecting group for an amino group)
   to obtain a compound represented by the following formula C2: 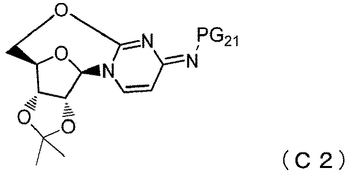 (wherein, PG₂₁ is the same as above);
   introducing an amine represented by the following formula D2A or D2B: 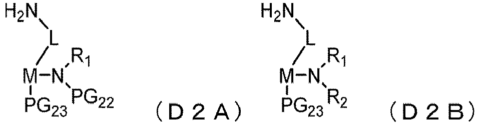 (wherein,
      R₂ is C₁-C₃ alkyl,
      PG₂₂ is a protecting group for an amino group,
      PG₂₃ is a protecting group for a carboxyl group or an imino group, and
      R₁, L, and M are the same as above,
      provided that when M is a single bond, PG₁₃ is not present);
   or a salt thereof to the compound represented by the formula C2, to obtain a compound represented by the following formula E2A or E2B: 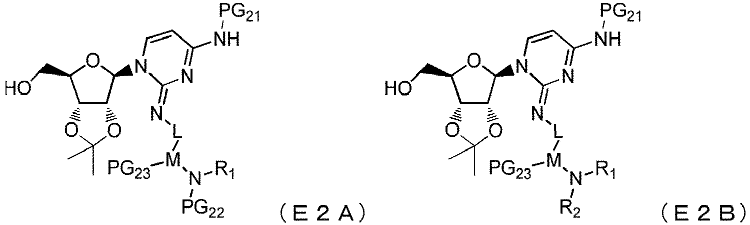 (wherein,
      R₂ is C₁-C₃ alkyl, and
      R₁, L, M, PG₂₁, PG₂₂, and PG₂₃ are the same as above);
   removing acetonide from the compound represented by the formula E2A or E2B, and introducing PG₂₄ and PG₂₅, to obtain a compound represented by the following formula F2A or F2B: 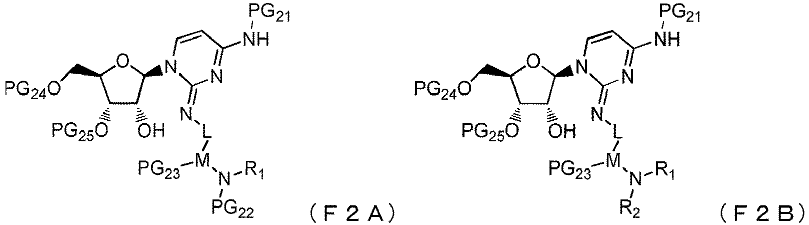 (wherein,
      R₂ is C₁-C₃ alkyl,
      PG₂₄ is a protecting group for a hydroxy group,
      PG₂₅ is a protecting group for a hydroxy group, and
      R₁, R₂, L, M, PG₂₁, PG₂₂, and PG₂₃ are the same as above);
   introducing PG₂₆ to the compound represented by the formula F2A or F2B to obtain a compound represented by the following formula G2A or G2B: 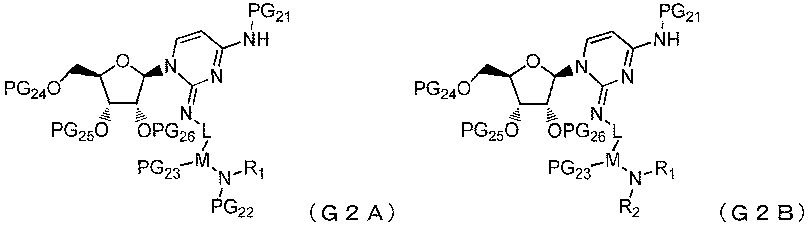 (wherein,
      R₂ is C₁-C₃ alkyl,
      PG₂₆ is a protecting group for a hydroxy group, and
      R₁, R₂, L, M, PG₂₁, PG₂₂, PG₂₃, PG₂₄, and PG₂₅ are the same as above);
   removing PG₂₄ and PG₂₅ from the compound represented by the formula G2A or G2B to obtain a compound represented by the formula H2A or H2B: 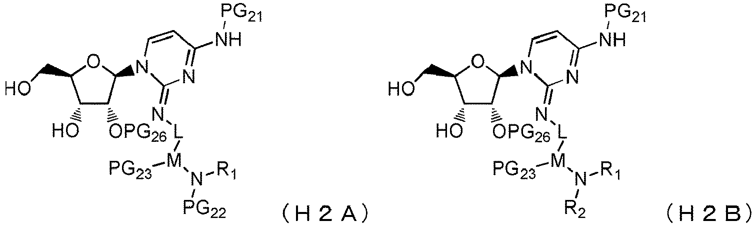 (wherein,
      R₂ is C₁-C₃ alkyl, and
      R₁, L, M, PG₂₁, PG₂₂, PG₂₃, and PG₂₆ are the same as above);
   phosphite-esterifying the compound represented by the formula H2A or H2B and then oxidized, to obtain a compound represented by the following formula I2A or I2B: 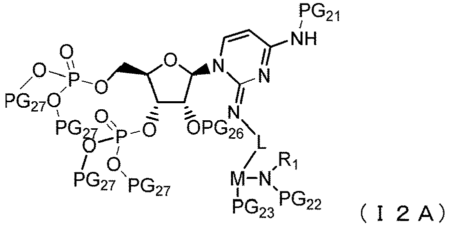 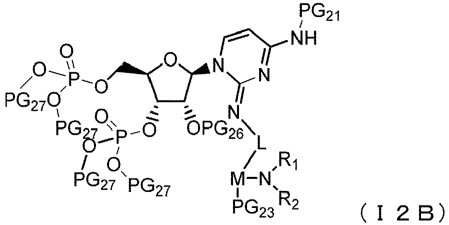 (wherein,
      R₂ is C₁-C₃ alkyl,
      PG₂₇ is a protecting group for a hydroxy group, and
      R₁, L, M, PG₂₁, PG₂₂, PG₂₃, and PG₂₆ are the same as above);
   removing PG₂₁, PG₂₂, PG₂₃, and PG₂₇ from the compound represented by the formula I2A, or removing PG₂₁, PG₂₃, and PG₂₇ from the compound represented by the formula I2B, to obtain a compound represented by the following formula J2: 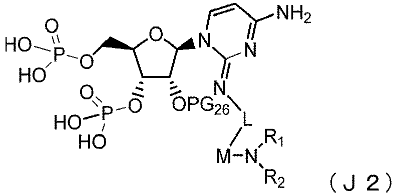 (wherein,
      R₂ is H or C₁-C₃ alkyl, and
      R₁, L, M, and PG₂₆ are the same as above); and
   removing PG₂₆ from the compound represented by the formula J2 to obtain the compound represented by the formula A;
[97] the method of [96], wherein the compound represented by the formula A is lysidine diphosphate: 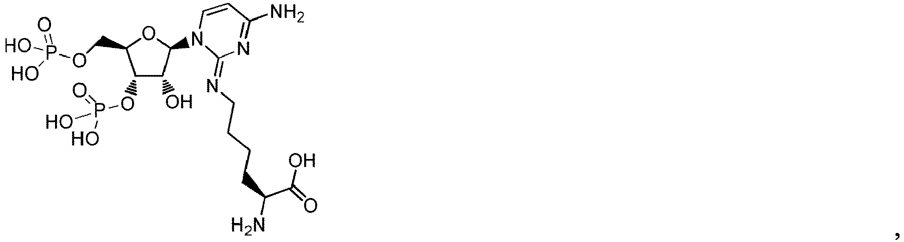 or agmatidine diphosphate: 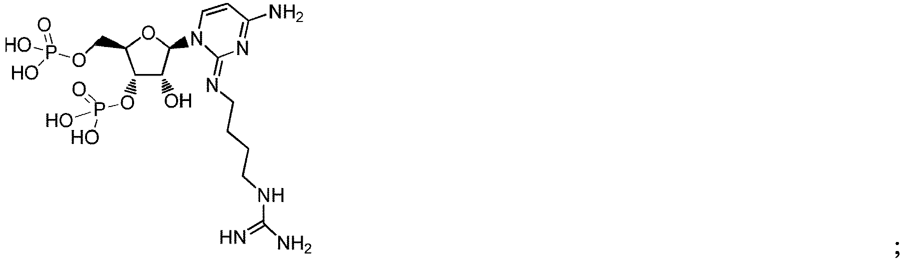
[98] the method of [96], wherein PG₂₁ is Cbz, an optionally substituted benzyloxycarbonyl, or an optionally substituted benzyl;
[99] the method of [96], wherein PG₂₂ is Cbz, an optionally substituted benzyloxycarbonyl, or an optionally substituted benzyl;
[100] the method of [96], wherein when M is 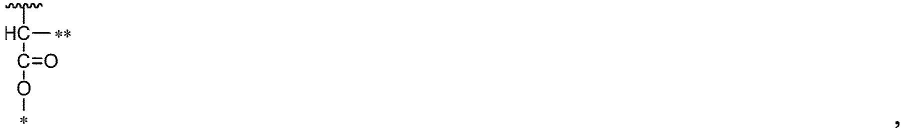 PG₂₃ is an optionally substituted benzyl, and when M is 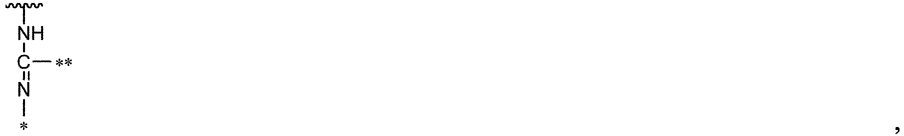 PG₂₃ is an optionally substituted benzyl, Cbz, or an optionally substituted benzyloxycarbonyl;
[101] the method of [96], wherein PG₂₄ and PG₂₅ are taken together to form di-tert-butylsilyl;
[102] the method of [96], wherein PG₂₆ is tetrahydropyranyl, tetrahydrofuranyl, or methoxymethyl;
[103] the method of [96], wherein PG₂₇ is benzyl;
[104] the method of [96], wherein the intramolecular cyclization is carried out in the presence of diisopropyl azodicarboxylate and triphenylphosphine;
[105] the method of [96], wherein the introduction of the amine represented by the formula D2 or a salt thereof is carried out in the presence of lithium chloride and DBU;
[106] the method of [96], wherein a reagent used for removing acetonide is TFA;
[107] the method of [96], wherein PG₂₄ and PG₂₅ are taken together to form di-tert-butylsilyl, and a reagent used to introduce di-tert-butylsilyl is di-tert-butylsilyl bis(trifluoromethanesulfonate);
[108] the method of [96], wherein PG₂₆ is tetrahydropyranyl, and reagents used to introduce PG₂₆ are TFA and 3,4-dihydro-2H-pyran;
[109] the method of [96], wherein a reagent used to remove PG₂₄ and PG₂₅ is tetrabutylammonium fluoride;
[110] the method of [96], wherein a reagent used for phosphite-esterification is dibenzyl N,N-diisopropylphosphoramidite;
[111] the method of [96], wherein a reagent used for oxidation is Dess-Martin periodinane;
[112] the method of [96], wherein PG₂₁, PG₂₂, PG₂₃, and PG₂₇ are removed by catalytic hydrogenation;
[113] the method of [96], wherein a reagent used for removing PG₂₆ is hydrochloric acid;
[114] the method of [96], wherein R₁ is H;
[115] the method of [96], wherein R₂ is H;
[116] the method of [96], wherein the C₂-C₆ straight chain alkylene or the C₂-C₆ straight chain alkenylene is a C₄-C₅ straight chain alkylene or a C₄-C₅ straight chain alkenylene; and
[117] the method of [96], wherein L is -(CH₂)₃-, -(CH₂)₄-, -(CH₂)₅, -(CH₂)₂-O-CH₂-, -(CH₂)₂-S-CH₂-, -CH₂CH(OH)(CH₂)₂-, or -CH₂CH=CH- (cis or trans).

### [Brief Description of Drawings]

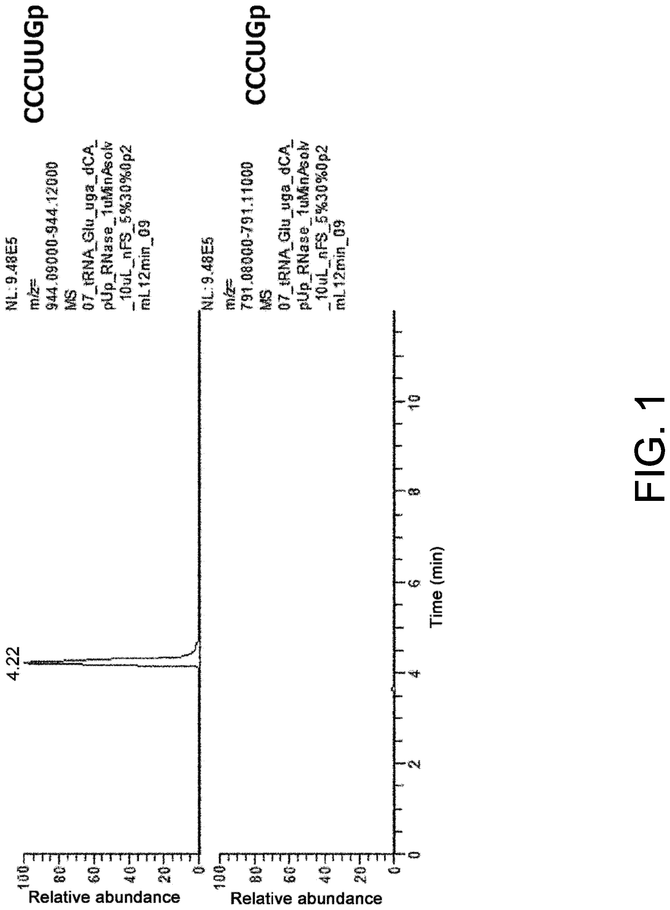

Fig. 1 shows mass chromatograms of products formed by RNase fragmentation of tRNA(Glu)uga-CA(UR-1) prepared by using a ligation reaction, as described in Example 10. The upper graph shows the result from the fragment having the CCCUUGp sequence, and the lower graph shows the result from the fragment having the CCCUGp sequence.

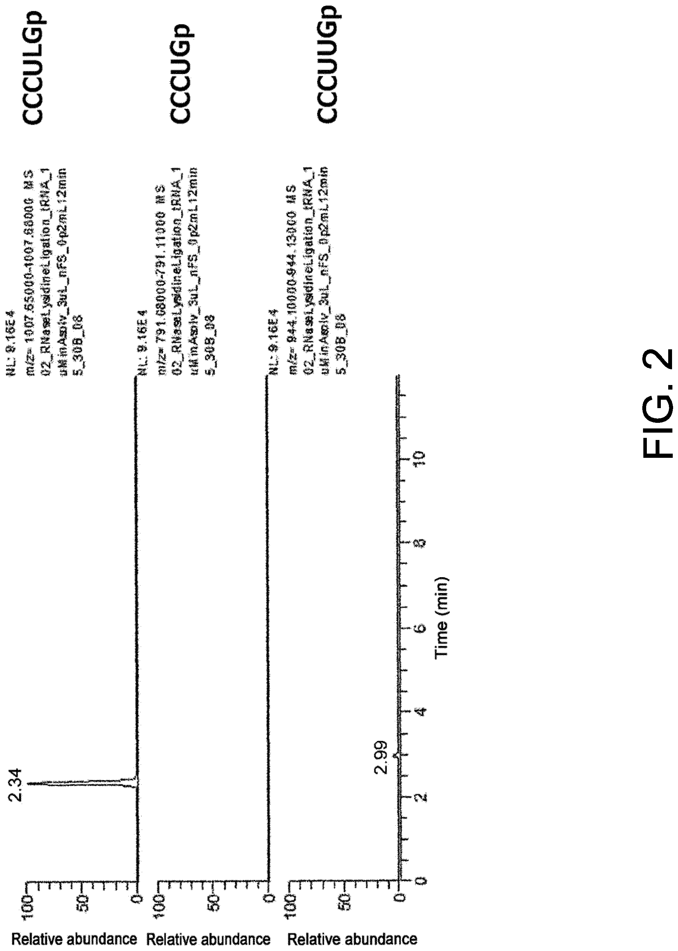

Fig. 2 shows mass chromatograms of products formed by RNase fragmentation of tRNA(Glu)Lga-CA(LR-1) prepared by using a ligation reaction, as described in Example 10. The upper graph shows the result from the fragment having the CCCULGp sequence, the middle graph shows the result from the fragment having the CCCUGp sequence, and the lower graph shows the result from the fragment having the CCCUUGp sequence.

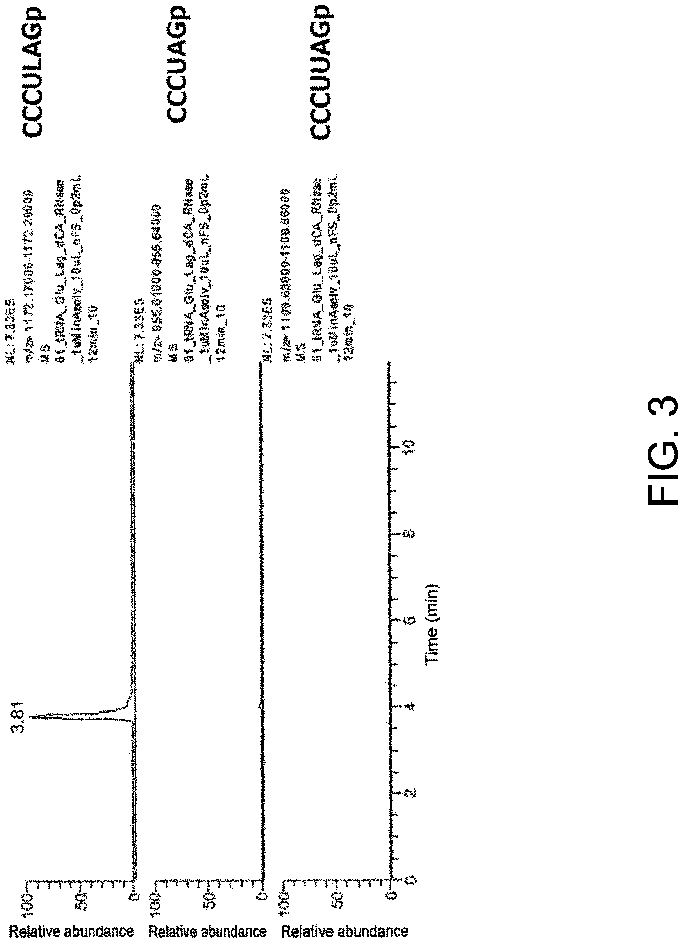

Fig. 3 shows mass chromatograms of products formed by RNase fragmentation of tRNA(Glu)Lag-CA(LR-2) prepared by using a ligation reaction, as described in Example 10. The upper graph shows the result from the fragment having the CCCULAGp sequence, the middle graph shows the result from the fragment having the CCCUAGp sequence, and the lower graph shows the result from the fragment having the CCCUUAGp sequence.

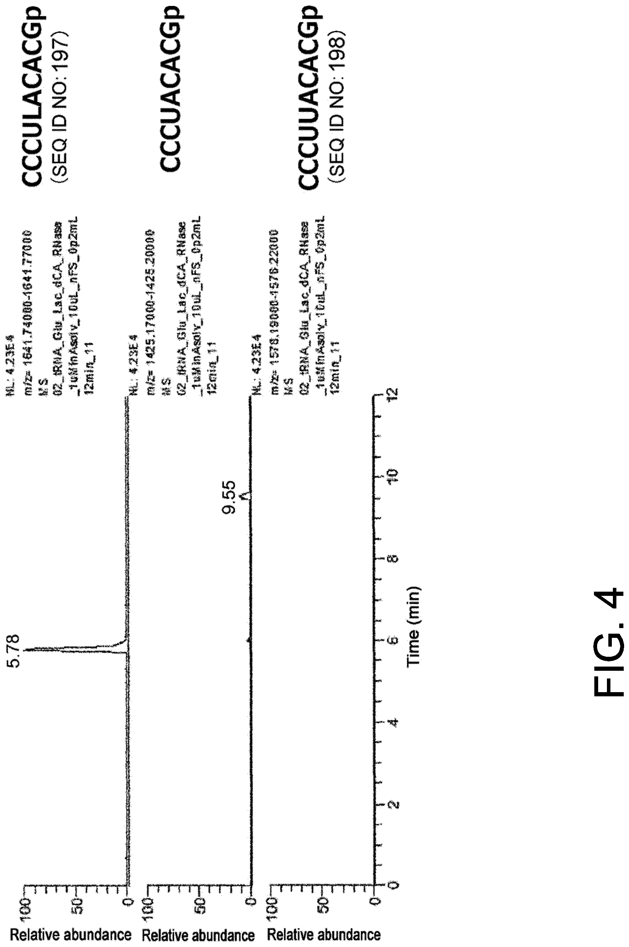

Fig. 4 shows mass chromatograms of products formed by RNase fragmentation of tRNA(Glu)Lac-CA(LR-3) prepared by using a ligation reaction, as described in Example 10. The upper graph shows the result from the fragment having the CCCULACACGp (SEQ ID NO: 197) sequence, the middle graph shows the result from the fragment having the CCCUACACGp sequence, and the lower graph shows the result from the fragment having the CCCUUACACGp (SEQ ID NO: 198) sequence.

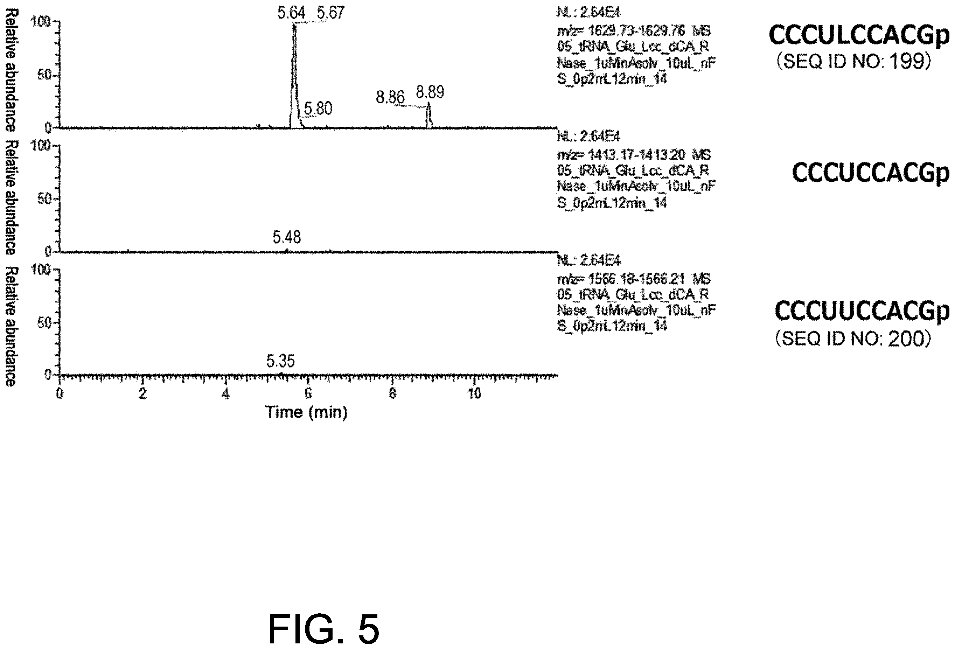

Fig. 5 shows mass chromatograms of products formed by RNase fragmentation of tRNA(Glu)Lcc-CA(LR-4) prepared by using a ligation reaction, as described in Example 10. The upper graph shows the result from the fragment having the CCCULCCACGp (SEQ ID NO: 199) sequence, the middle graph shows the result from the fragment having the CCCUCCACGp sequence, and the lower graph shows the result from the fragment having the CCCUUCCACGp (SEQ ID NO: 200) sequence.

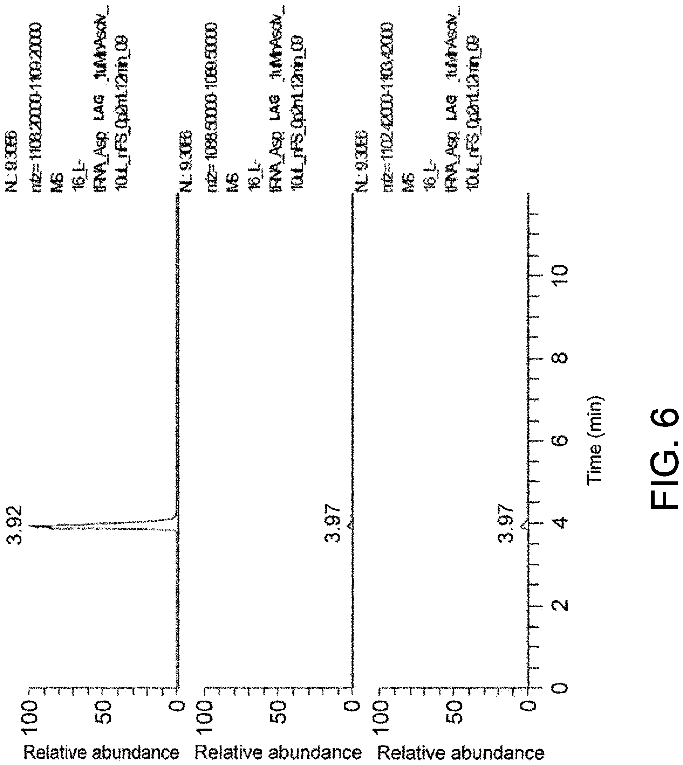

Fig. 6 shows mass chromatograms of tRNA(Asp)Lag-CA (LR-5) prepared by using a ligation reaction, as described in Example 10. The upper graph shows the result from the nucleic acid having the sequence
   pGGAGCGGUAGUUCAGUCGGUUAGAAUACCUGCUULAGGUGCAGGGGGUCGCGGG UUCGAGUCCCGUCCGUUCCGC (SEQ ID NO: 134) (substance of interest), the middle graph shows the result from the nucleic acid having the sequence
   pGGAGCGGUAGUUCAGUCGGUUAGAAUACCUGCUUAGGUGCAGGGGGUCGCGGG UUCGAGUCCCGUCCGUUCCGC (SEQ ID NO: 201) (by-product formed when pLp is not ligated), and the lower graph shows the result from the nucleic acid having the sequence
   pGGAGCGGUAGUUCAGUCGGUUAGAAUACCUGCUUUAGGUGCAGGGGGUCGCGG GUUCGAGUCCCGUCCGUUCCGC (SEQ ID NO: 154) (by-product formed when pUp is ligated instead of pLp).

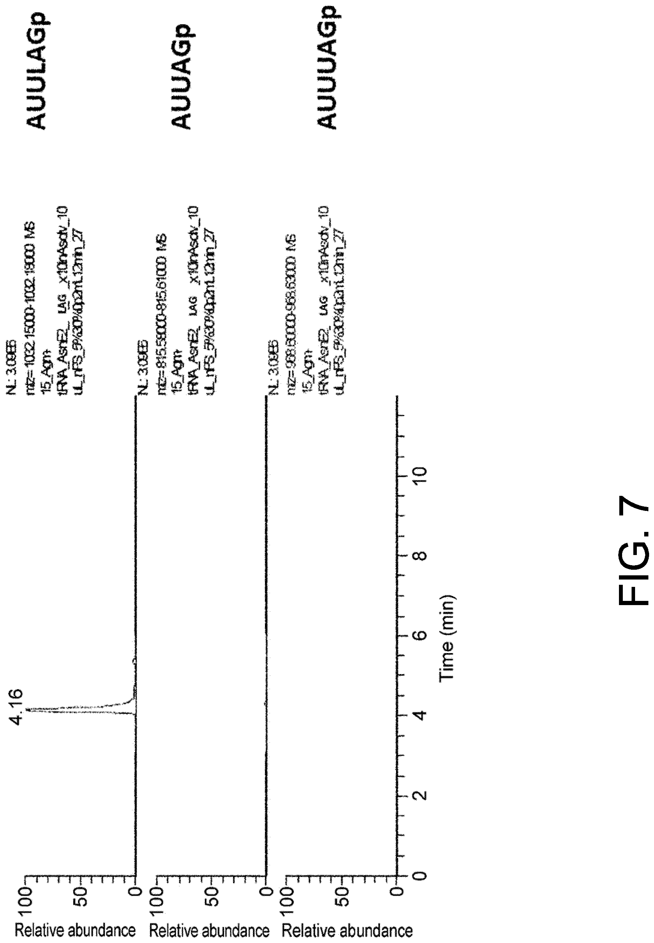

Fig. 7 shows mass chromatograms of products formed by RNase fragmentation of tRNA(AsnE2)Lag-CA (LR-6) prepared by using a ligation reaction, as described in Example 10. The upper graph shows the result from the fragment having the AUULAGp sequence, the middle graph shows the result from the fragment having the AUUAGp sequence, and the lower graph shows the result from the fragment having the AUUUAGp sequence.

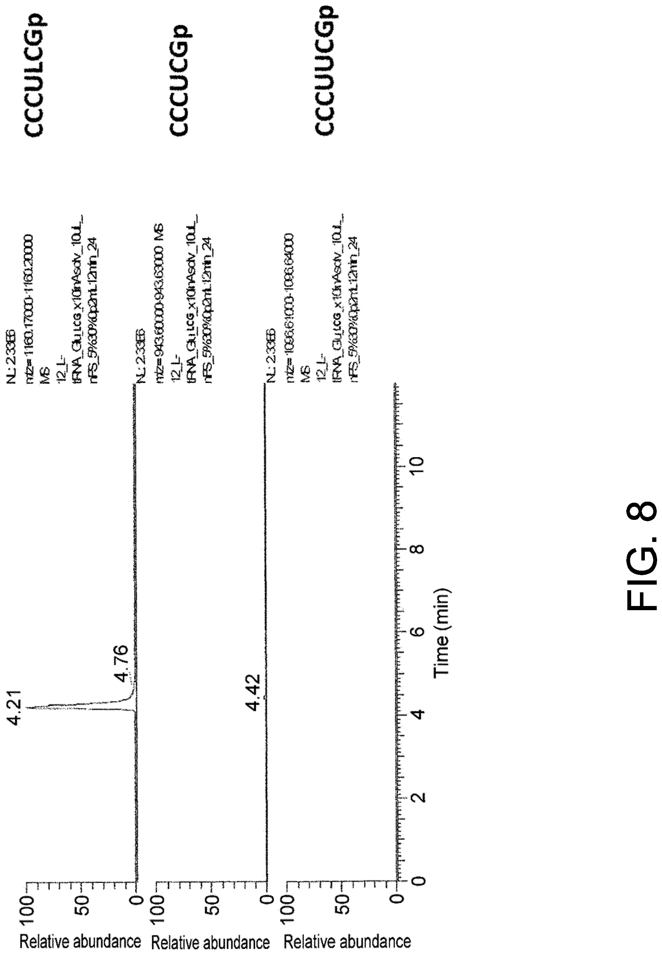

Fig. 8 shows mass chromatograms of products formed by RNase fragmentation of tRNA(Glu)Lcg-CA (LR-7) prepared by using a ligation reaction, as described in Example 10. The upper graph shows the result from the fragment having the CCCULCGp sequence, the middle graph shows the result from the fragment having the CCCUCGp sequence, and the lower graph shows the result from the fragment having the CCCUUCGp sequence.

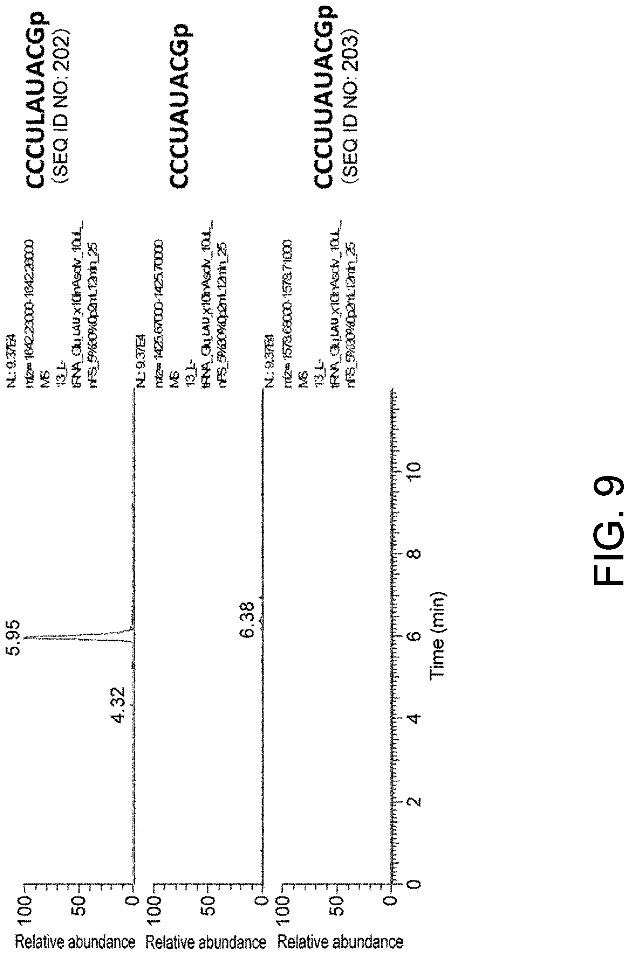

Fig. 9 shows mass chromatograms of products formed by RNase fragmentation of tRNA(Glu)Lau-CA (LR-8) prepared by using a ligation reaction, as described in Example 10. The upper graph shows the result from the fragment having the CCCULAUACGp (SEQ ID NO: 202) sequence, the middle graph shows the result from the fragment having the CCCUAUACGp sequence, and the lower graph shows the result from the fragment having the CCCUUAUACGp (SEQ ID NO: 203) sequence.

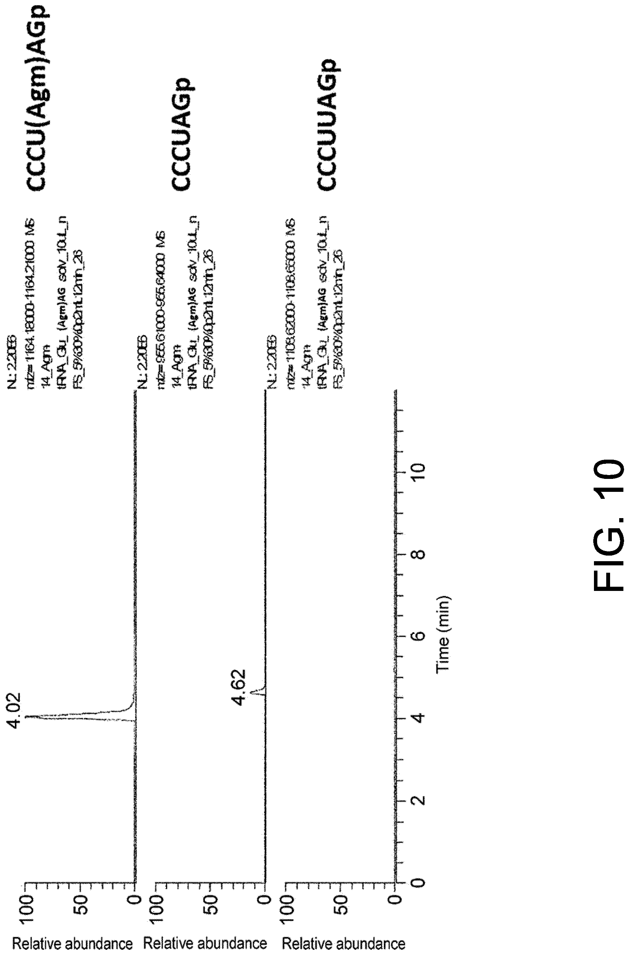

Fig. 10 shows mass chromatograms of products formed by RNase fragmentation of tRNA(Glu)(Agm)ag-CA (AR-1) prepared by using a ligation reaction, as described in Example 10. The upper graph shows the result from the fragment having the CCCU(Agm)AGp sequence, the middle graph shows the result from the fragment having the CCCUAGp sequence, and the lower graph shows the result from the fragment having the CCCUUAGp sequence.

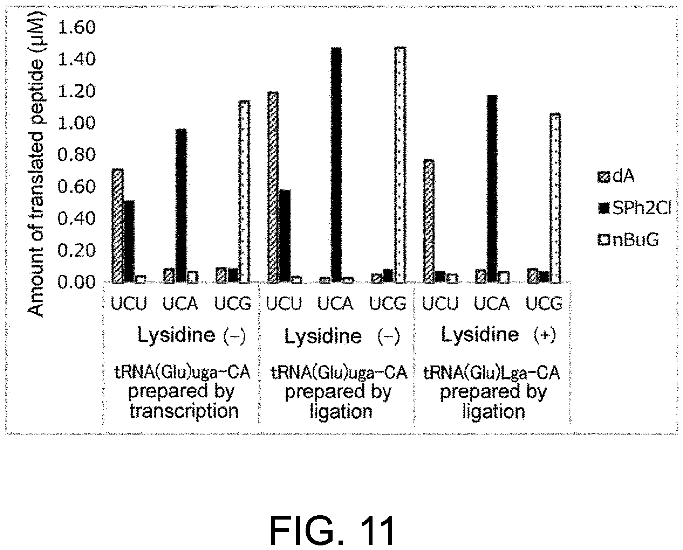

Fig. 11 is a graph showing the results of evaluating the effects of the presence or absence of lysidine modification on translation that discriminates three amino acids in a single codon box, as described in Examples 12 to 13. The codons evaluated are UCU, UCA, and UCG. The vertical axis of the graph shows the amount of translated peptide when the translation was performed using each combination of the tRNAs and the mRNAs described below (see Table 12 for specific measurement values).
   (left section of the graph)
      tRNA:
         Compound AAtR-1 (anticodon: aga; amino acid: dA)
         Compound AAtR-2 (anticodon: uga; amino acid: SPh2Cl)
         Compound AAtR-5 (anticodon: cga; amino acid: nBuG)
      mRNA:
         mR-1 (containing the UCU codon)
         mR-2 (containing the UCA codon)
         mR-3 (containing the UCG codon)
   (middle section of the graph)
      tRNA:
         Compound AAtR-1 (anticodon: aga; amino acid: dA)
         Compound AAtR-3 (anticodon: uga; amino acid: SPh2Cl)
         Compound AAtR-5 (anticodon: cga; amino acid: nBuG)
      mRNA:
         mR-1 (containing the UCU codon)
         mR-2 (containing the UCA codon)
         mR-3 (containing the UCG codon)
   (right section of the graph)
      tRNA:
         Compound AAtR-1 (anticodon: aga; amino acid: dA)
         Compound AAtR-4 (anticodon: Lga; amino acid: SPh2Cl)
         Compound AAtR-5 (anticodon: cga; amino acid: nBuG)
      mRNA:
         mR-1 (containing the UCU codon)
         mR-2 (containing the UCA codon)
         mR-3 (containing the UCG codon)

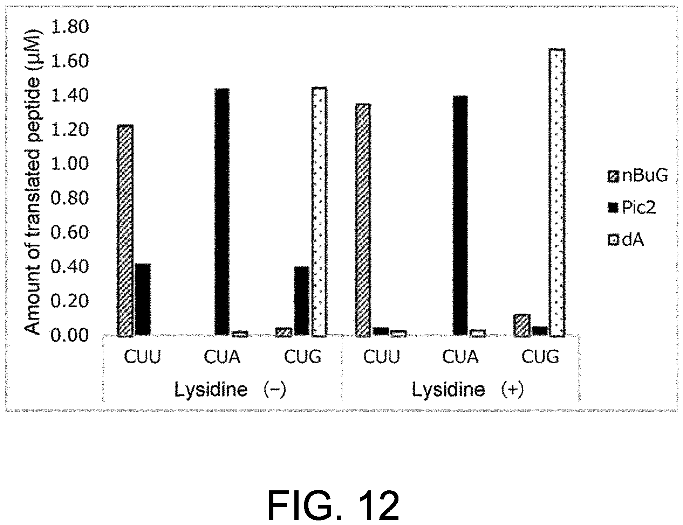

Fig. 12 is a graph showing the results of evaluating the effects of the presence or absence of lysidine modification on translation that discriminates three amino acids in a single codon box, as described in Examples 12 to 13. The codons evaluated are CUU, CUA, and CUG. The vertical axis of the graph shows the amount of translated peptide when the translation was performed using each combination of the tRNAs and the mRNAs described below (see Table 13 for specific measurement values).
   (left section of the graph)
      tRNA:
         Compound AAtR-6 (anticodon: aag; amino acid: nBuG)
         Compound AAtR-7 (anticodon: uag; amino acid: Pic2)
         Compound AAtR-9 (anticodon: cag; amino acid: dA)
      mRNA:
         mR-4 (containing the CUU codon)
         mR-5 (containing the CUA codon)
         mR-6 (containing the CUG codon)
   (right section of the graph)
      tRNA:
         Compound AAtR-6 (anticodon: aag; amino acid: nBuG)
         Compound AAtR-8 (anticodon: Lag; amino acid: Pic2)
         Compound AAtR-9 (anticodon: cag; amino acid: dA)
      mRNA:
         mR-4 (containing the CUU codon)
         mR-5 (containing the CUA codon)
         mR-6 (containing the CUG codon)

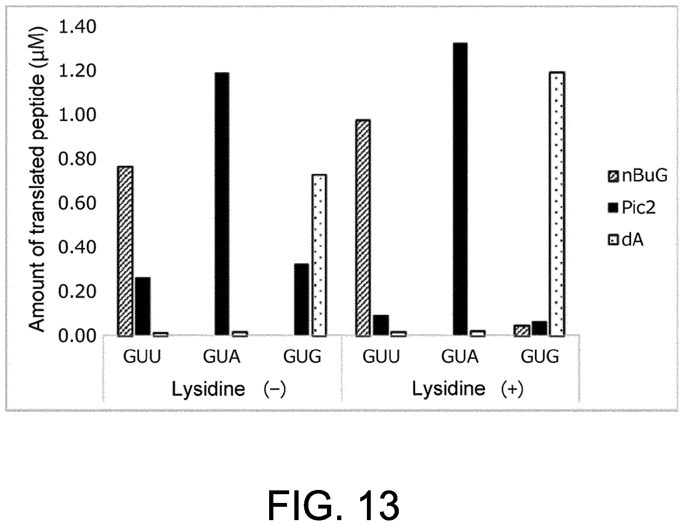

Fig. 13 is a graph showing the results of evaluating the effects of the presence or absence of lysidine modification on translation that discriminates three amino acids in a single codon box, as described in Examples 12 to 13. The codons evaluated are GUU, GUA, and GUG. The vertical axis of the graph shows the amount of translated peptide when the translation was performed using each combination of the tRNAs and the mRNAs described below (see Table 14 for specific measurement values).
   (left section of the graph)
      tRNA:
         Compound AAtR-10 (anticodon: aac; amino acid: nBuG)
         Compound AAtR-11 (anticodon: uac; amino acid: Pic2)
         Compound AAtR-13 (anticodon: cac; amino acid: dA)
      mRNA:
         mR-7 (containing the GUU codon)
         mR-8 (containing the GUA codon)
         mR-9 (containing the GUG codon)
   (right section of the graph)
      tRNA:
         Compound AAtR-10 (anticodon: aac; amino acid: nBuG)
         Compound AAtR-12 (anticodon: Lac; amino acid: Pic2)
         Compound AAtR-13 (anticodon: cac; amino acid: dA)
      mRNA:
         mR-7 (containing the GUU codon)
         mR-8 (containing the GUA codon)
         mR-9 (containing the GUG codon)

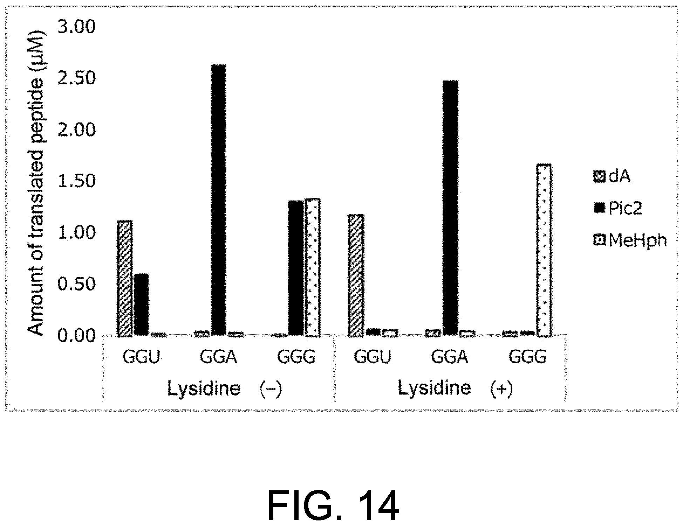

Fig. 14 is a graph showing the results of evaluating the effects of the presence or absence of lysidine modification on translation that discriminates three amino acids in a single codon box, as described in Examples 12 to 13. The codons evaluated are GGU, GGA, and GGG. The vertical axis of the graph shows the amount of translated peptide when the translation was performed using each combination of the tRNAs and the mRNAs described below (see Table 15 for specific measurement values).
   (left section of the graph)
      tRNA:
         Compound AAtR-14 (anticodon: gcc; amino acid: dA)
         Compound AAtR-15 (anticodon: ucc; amino acid: Pic2)
         Compound AAtR-17 (anticodon: ccc; amino acid: MeHph)
      mRNA:
         mR-10 (containing the GGU codon)
         mR-11 (containing the GGA codon)
         mR-12 (containing the GGG codon)
   (right section of the graph)
      tRNA:
         Compound AAtR-14 (anticodon: gcc; amino acid: dA)
         Compound AAtR-16 (anticodon: Lcc; amino acid: Pic2)
         Compound AAtR-17 (anticodon: ccc; amino acid: MeHph)
      mRNA:
         mR-10 (containing the GGU codon)
         mR-11 (containing the GGA codon)
         mR-12 (containing the GGG codon)

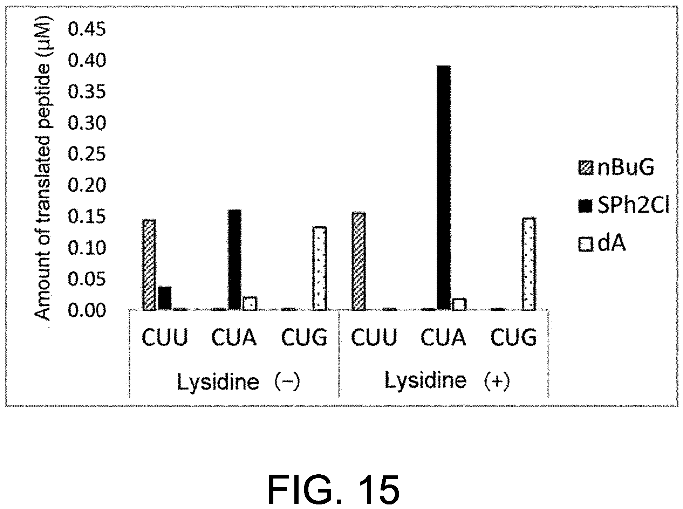

Fig. 15 is a graph showing the results of evaluating the effects of the presence or absence of lysidine modification on translation that discriminates three amino acids in a single codon box, as described in Examples 12 to 13. The codons evaluated are CUU, CUA, and CUG. The vertical axis of the graph shows the amount of translated peptide when the translation was performed using each combination of the tRNAs and the mRNAs described below (see Table 16 for specific measurement values).
   (left section of the graph)
      tRNA:
         Compound AAtR-19 (anticodon: aag; amino acid: nBuG)
         Compound AAtR-20 (anticodon: uag; amino acid: SPh2Cl)
         Compound AAtR-22 (anticodon: cag; amino acid: dA)
      mRNA:
         mR-4 (containing the CUU codon)
         mR-5 (containing the CUA codon)
         mR-6 (containing the CUG codon)
   (right section of the graph)
      tRNA:
         Compound AAtR-19 (anticodon: aag; amino acid: nBuG)
         Compound AAtR-21 (anticodon: Lag; amino acid: SPh2Cl)
         Compound AAtR-22 (anticodon: cag; amino acid: dA)
      mRNA:
         mR-4 (containing the CUU codon)
         mR-5 (containing the CUA codon)
         mR-6 (containing the CUG codon)

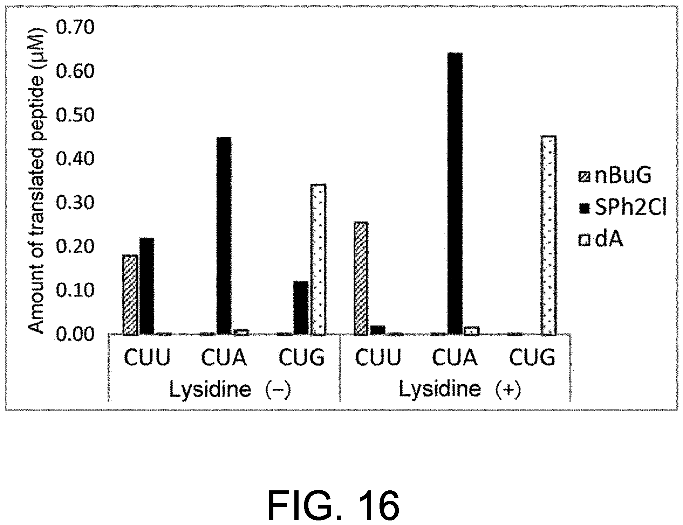

Fig. 16 is a graph showing the results of evaluating the effects of the presence or absence of lysidine modification on translation that discriminates three amino acids in a single codon box, as described in Examples 12 to 13. The codons evaluated are CUU, CUA, and CUG. The vertical axis of the graph shows the amount of translated peptide when the translation was performed using each combination of the tRNAs and the mRNAs described below (see Table 17 for specific measurement values).
   (left section of the graph)
      tRNA:
         Compound AAtR-23 (anticodon: aag; amino acid: nBuG)
         Compound AAtR-24 (anticodon: uag; amino acid: SPh2Cl)
         Compound AAtR-26 (anticodon: cag; amino acid: dA)
      mRNA:
         mR-4 (containing the CUU codon)
         mR-5 (containing the CUA codon)
         mR-6 (containing the CUG codon)
   (right section of the graph)
      tRNA:
         Compound AAtR-23 (anticodon: aag; amino acid: nBuG)
         Compound AAtR-25 (anticodon: Lag; amino acid: SPh2Cl)
         Compound AAtR-26 (anticodon: cag; amino acid: dA)
      mRNA:
         mR-4 (containing the CUU codon)
         mR-5 (containing the CUA codon)
         mR-6 (containing the CUG codon)

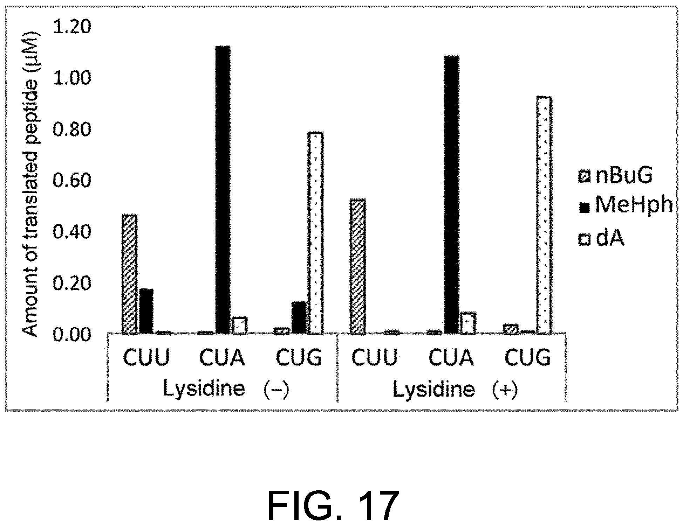

Fig. 17 is a graph showing the results of evaluating the effects of the presence or absence of lysidine modification on translation that discriminates three amino acids in a single codon box, as described in Examples 12 to 13. The codons evaluated are CUU, CUA, and CUG. The vertical axis of the graph shows the amount of translated peptide when the translation was performed using each combination of the tRNAs and the mRNAs described below (see Table 18 for specific measurement values).
   (left section of the graph)
      tRNA:
         Compound AAtR-6 (anticodon: aag; amino acid: nBuG)
         Compound AAtR-27 (anticodon: uag; amino acid: MeHph)
         Compound AAtR-9 (anticodon: cag; amino acid: dA)
      mRNA:
         mR-4 (containing the CUU codon)
         mR-5 (containing the CUA codon)
         mR-6 (containing the CUG codon)
   (right section of the graph)
      tRNA:
         Compound AAtR-6 (anticodon: aag; amino acid: nBuG)
         Compound AAtR-28 (anticodon: Lag; amino acid: MeHph)
         Compound AAtR-9 (anticodon: cag; amino acid: dA)
      mRNA:
         mR-4 (containing the CUU codon)
         mR-5 (containing the CUA codon)
         mR-6 (containing the CUG codon)

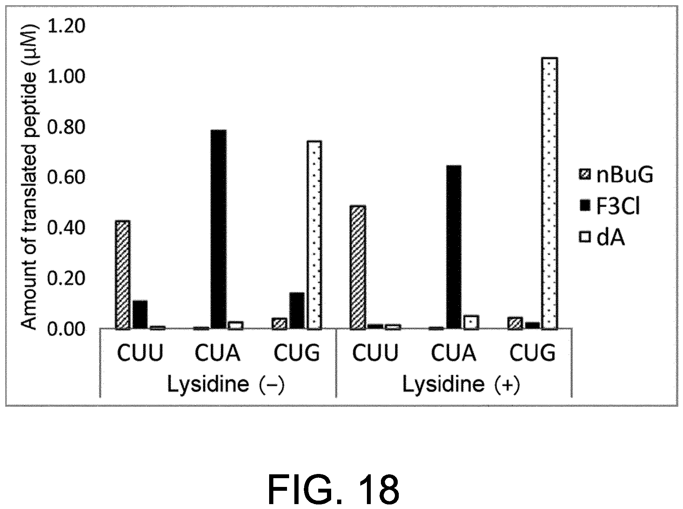

Fig. 18 is a graph showing the results of evaluating the effects of the presence or absence of lysidine modification on translation that discriminates three amino acids in a single codon box, as described in Examples 12 to 13. The codons evaluated are CUU, CUA, and CUG. The vertical axis of the graph shows the amount of translated peptide when the translation was performed using each combination of the tRNAs and the mRNAs described below (see Table 19 for specific measurement values).
   (left section of the graph)
      tRNA:
         Compound AAtR-6 (anticodon: aag; amino acid: nBuG)
         Compound AAtR-29 (anticodon: uag; amino acid: F3C1)
         Compound AAtR-9 (anticodon: cag; amino acid: dA)
      mRNA:
         mR-4 (containing the CUU codon)
         mR-5 (containing the CUA codon)
         mR-6 (containing the CUG codon)
   (right section of the graph)
      tRNA:
         Compound AAtR-6 (anticodon: aag; amino acid: nBuG)
         Compound AAtR-30 (anticodon: Lag; amino acid: F3C1)
         Compound AAtR-9 (anticodon: cag; amino acid: dA)
      mRNA:
         mR-4 (containing the CUU codon)
         mR-5 (containing the CUA codon)
         mR-6 (containing the CUG codon)

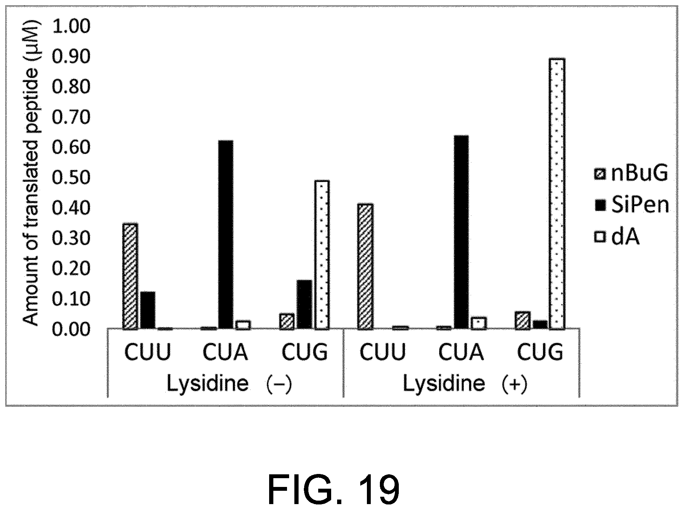

Fig. 19 is a graph showing the results of evaluating the effects of the presence or absence of lysidine modification on translation that discriminates three amino acids in a single codon box, as described in Examples 12 to 13. The codons evaluated are CUU, CUA, and CUG. The vertical axis of the graph shows the amount of translated peptide when the translation was performed using each combination of the tRNAs and the mRNAs described below (see Table 20 for specific measurement values).
   (left section of the graph)
      tRNA:
         Compound AAtR-6 (anticodon: aag; amino acid: nBuG)
         Compound AAtR-31 (anticodon: uag; amino acid: SiPen)
         Compound AAtR-9 (anticodon: cag; amino acid: dA)
      mRNA:
         mR-4 (containing the CUU codon)
         mR-5 (containing the CUA codon)
         mR-6 (containing the CUG codon)
   (right section of the graph)
      tRNA:
         Compound AAtR-6 (anticodon: aag; amino acid: nBuG)
         Compound AAtR-32 (anticodon: Lag; amino acid: SiPen)
         Compound AAtR-9 (anticodon: cag; amino acid: dA)
      mRNA:
         mR-4 (containing the CUU codon)
         mR-5 (containing the CUA codon)
         mR-6 (containing the CUG codon)

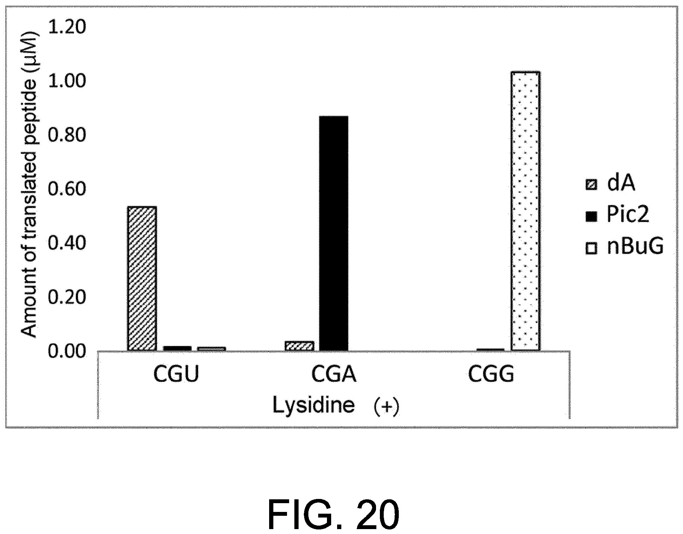

Fig. 20 is a graph showing the results of evaluating the effects of lysidine modification on translation that discriminates three amino acids in a single codon box, as described in Examples 12 to 13. The codons evaluated are CGU, CGA, and CGG. The vertical axis of the graph shows the amount of translated peptide when the translation was performed using each combination of the tRNAs and the mRNAs described below (see Table 21 for specific measurement values).
   tRNA:
      Compound AAtR-33 (anticodon: gcg; amino acid: dA)
      Compound AAtR-34 (anticodon: Lcg; amino acid: Pic2)
      Compound AAtR-35 (anticodon: ccg; amino acid: nBuG)
   mRNA:
      mR-13 (containing the CGU codon)
      mR-14 (containing the CGA codon)
      mR-15 (containing the CGG codon)

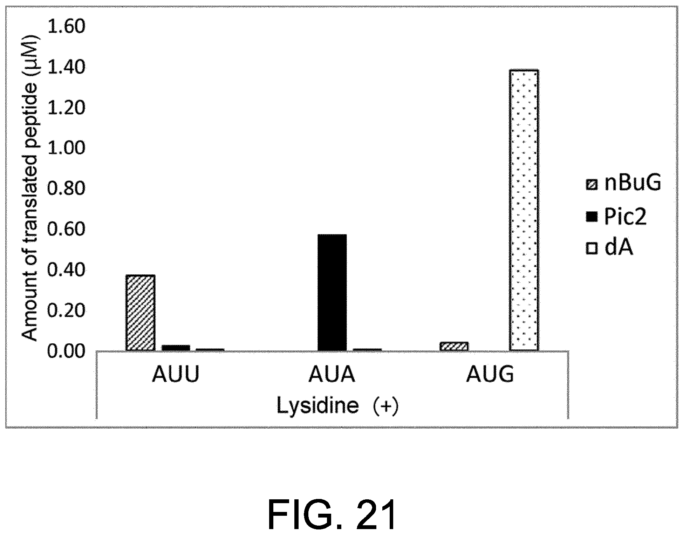

Fig. 21 is a graph showing the results of evaluating the effects of lysidine modification on translation that discriminates three amino acids in a single codon box, as described in Examples 12 to 13. The codons evaluated are AUU, AUA, and AUG. The vertical axis of the graph shows the amount of translated peptide when the translation was performed using each combination of the tRNAs and the mRNAs described below (see Table 22 for specific measurement values).
   tRNA:
      Compound AAtR-36 (anticodon: aau; amino acid: nBuG)
      Compound AAtR-37 (anticodon: Lau; amino acid: Pic2)
      Compound AAtR-38 (anticodon: cau; amino acid: dA)
   mRNA:
      mR-16 (containing the AUU codon)
      mR-17 (containing the AUA codon)
      mR-18 (containing the AUG codon)

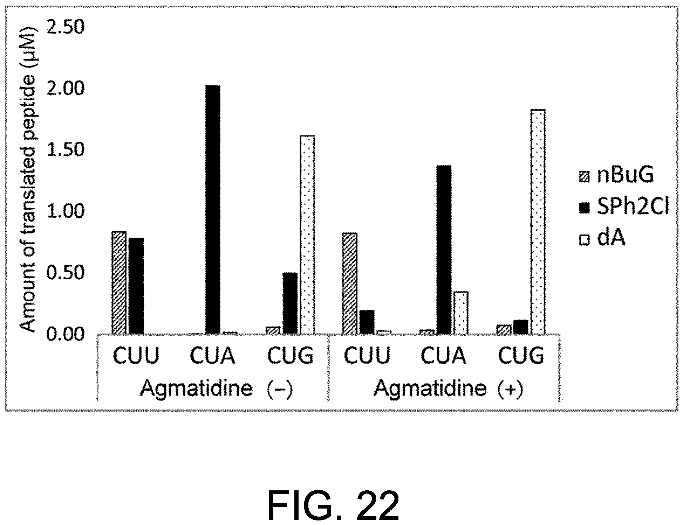

Fig. 22 is a graph showing the results of evaluating the effects of the presence or absence of agmatidine modification on translation that discriminates three amino acids in a single codon box, as described in Examples 12 to 13. The codons evaluated are CUU, CUA, and CUG. The vertical axis of the graph shows the amount of translated peptide when the translation was performed using each combination of the tRNAs and the mRNAs described below (see Table 23 for specific measurement values).
   (left section of the graph)
      tRNA:
         Compound AAtR-6 (anticodon: aag; amino acid: nBuG)
         Compound AAtR-39 (anticodon: uag; amino acid: SPh2Cl)
         Compound AAtR-9 (anticodon: cag; amino acid: dA)
      mRNA:
         mR-4 (containing the CUU codon)
         mR-5 (containing the CUA codon)
         mR-6 (containing the CUG codon)
   (right section of the graph)
      tRNA:
         Compound AAtR-6 (anticodon: aag; amino acid: nBuG)
         Compound AAtR-40 (anticodon: (Agm)ag; amino acid: SPh2Cl)
         Compound AAtR-9 (anticodon: cag; amino acid: dA)
      mRNA:
         mR-4 (containing the CUU codon)
         mR-5 (containing the CUA codon)
         mR-6 (containing the CUG codon)

### [Description of Embodiments]

### I. Definition

For the purpose of interpreting this specification, the following definitions will apply and whenever applicable, terms used in the singular will also include the plural, and vice versa. It is to be understood that the terminology used herein is for the purpose of describing particular embodiments only and is not intended to be limiting. If any of the following definitions conflict with any document incorporated herein by reference, the following definitions shall control.

"Codon" refers to a set of three nucleosides (triplet) that corresponds to each amino acid, when genetic information in a living body is translated to a protein. For DNA, four bases, adenine (A), guanine (G), cytosine (C), and thymine (T), are used. For mRNA, four bases, adenine (A), guanine (G), cytosine (C) and uracil (U), are used. The table showing the correspondence between each codon and amino acid is called the genetic code table or codon table, and 20 amino acids are assigned to 61 codons excluding the stop codon (Table 1). The genetic code table shown in Table 1 is used commonly for almost all eukaryote and prokaryote (eubacteria and archaea); therefore, it is called the standard genetic code table or the universal genetic code table. In the present disclosure, a genetic code table used for naturally-occurring organisms is referred to as the natural genetic code table, and it is distinguished from an artificially reprogrammed genetic code table (the correspondence between codons and amino acids is engineered). In the genetic code table, generally, four codons which are the same in the first and second letters and which differ only in the third letter are grouped into one box, and this group is called a codon box.

**[Table 1]**

| | U | | C | | A | | G | | |
|---|---|---|---|---|---|---|---|---|---|
| U | UUU | Phe | UCU | Ser | UAU | Tyr | UGU | Cys | U C A G |
| | UUC | | UCC | | UAC | | UGC | | |
| | UUA | Leu | UCA | | UAA | Stop | UGA | Stop | |
| | UUG | | UCG | | UAG | | UGG | Trp | |
| C | CUU | Leu | CCU | Pro | CAU | His | CGU | Arg | U C A G |
| | CUC | | CCC | | CAC | | CGC | | |
| | CUA | | CCA | | CAA | Gln | CGA | | |
| | CUG | | CCG | | CAG | | CGG | | |
| A | AUU | Ile | ACU | Thr | AAU | Asn | AGU | Ser | U C A G |
| | AUC | | ACC | | AAC | | AGC | | |
| | AUA | | ACA | | AAA | Lys | AGA | Arg | |
| | AUG | Met | ACG | | AAG | | AGG | | |
| G | GUU | Val | GCU | Ala | GAU | Asp | GGU | Gly | U C A G |
| | GUC | | GCC | | GAC | | GGC | | |
| | GUA | | GCA | | GAA | Glu | GGA | | |
| | GUG | | GCG | | GAG | | GGG | | |

In the present disclosure, a codon in mRNA may be expressed as "M₁M₂M₃". Here, M₁, M₂, and M₃ represent the nucleosides for the first letter, the second letter, and the third letter of the codon, respectively.

"Anticodon" refers to three consecutive nucleosides on tRNA that correspond to a codon on the mRNA. Similar to mRNA, four bases, adenine (A), guanine (G), cytosine (C), and uracil (U), are used for the anticodon. Furthermore, modified bases obtained by modifying these bases may be used. When the codon is specifically recognized by the anticodon, the genetic information on the mRNA is read and translated into a protein. The codon sequence on the mRNA in the 5' to 3' direction and the anticodon sequence on the tRNA in the 5' to 3' direction bind complementarily; therefore, complementary nucleotide pairs are formed between the nucleosides for the first, second, and third letters of the codon, and the nucleosides for the third, second, and first letters of the anticodon, respectively.

In the present disclosure, an anticodon in tRNA may be represented by "N₁N₂N₃". Here, N₁, N₂, and N₃ represent the nucleosides for the first letter, second letter, and third letter of the anticodon, respectively. According to the tRNA numbering rule described below, N₁, N₂, and N₃ are numbered as positions 34, 35, and 36 of tRNA, respectively.

In the present disclosure, a combination of nucleic acids capable of forming thermodynamically stable base pairs is said to be "complementary" to each other. In addition to Watson-Crick base pairs such as adenosine and uridine (A-U) and guanosine and cytidine (G-C), combinations of nucleic acids forming non-Watson-Crick base pairs such as guanosine and uridine (G-U), inosine and uridine (I-U), inosine and adenosine (I-A), and inosine and cytidine (I-C), may also be included in the "complementary" nucleic acid combinations in the present disclosure. In particular, only Watson-Crick base pair formation is allowed between the first letter of the codon and the third letter of the anticodon, and between the second letter of the codon and the second letter of the anticodon, whereas there is some fluctuation in space (wobble) between the third letter of the codon and the first letter of the anticodon; therefore, formation of non-Watson-Crick base pair, such as those described above, may be permitted (wobble hypothesis).

"Messenger RNA (mRNA)" refers to an RNA that carries genetic information that can be translated into a protein. Genetic information is coded on mRNA as codons, and each of these codons corresponds to one among all 20 different amino acids. Protein translation begins at the initiation codon and ends at the stop codon. In principle, the initiation codon in eukaryotes is AUG, but in prokaryotes (eubacteria and archaea), GUG and UUG may also be used as initiation codons in addition to AUG. AUG is a codon that encodes methionine (Met), and in eukaryotes and archaea, translation is initiated directly from methionine. On the other hand, in eubacteria, only the initiation codon AUG corresponds to N-formylmethionine (fMet); therefore, translation is initiated from formylmethionine. There are three stop codons: UAA (ochre), UAG (amber), and UGA (opal). When the stop codon is recognized by a protein called a translation termination factor (release factor (RF)), the peptide chain synthesized up to that point is dissociated from the tRNA, and the translation process ends.

"Transfer RNA (tRNA)" refers to a short RNA of 100 bases or less that mediates peptide synthesis using mRNA as a template. In terms of secondary structure, it has a cloverleaf-like structure consisting of three stem loops (the D arm, the anticodon arm, and the T arm) and one stem (the acceptor stem). Depending on the tRNA, an additional variable loop may be included. The anticodon arm has a region consisting of three consecutive nucleosides called an anticodon, and the codon is recognized when the anticodon forms a base pair with the codon on the mRNA. Meanwhile, a nucleic acid sequence (CCA sequence) consisting of cytidine-cytidine-adenosine exists at the 3' end of tRNA, and an amino acid is added to the adenosine residue at the end (specifically, the hydroxyl group at position 2 or position 3 of the ribose of the adenosine residue and the carboxyl group of the amino acid form an ester bond). A tRNA to which an amino acid is added is called an aminoacyl tRNA. In the present disclosure, aminoacyl tRNA is also included in the definition of tRNA. Further, as described later, a method is known in which two terminal residues (C and A) are removed from the CCA sequence of tRNA and then this is used for the synthesis of aminoacyl-tRNA. Such a tRNA from which the CA sequence at the 3' end has been removed is also included in the definition of tRNA in the present disclosure. Addition of amino acids to tRNA is carried out by an enzyme called aminoacyl-tRNA synthetase (aaRS or ARS), in vivo. Usually, there is one aminoacyl-tRNA synthetase for each amino acid, and each aminoacyl-tRNA synthetase specifically recognizes only a specific tRNA as a substrate from multiple tRNAs; accordingly, correspondence between tRNAs and amino acids is strictly controlled.

Each nucleoside in tRNA is numbered according to the tRNA numbering rule (Sprinzl et al., Nucleic Acids Res (1998) 26: 148-153). For example, an anticodon is numbered as positions 34 to 36 and the CCA sequence is numbered as positions 74 to 76.

"Initiator tRNA" is a specific tRNA used at the start of mRNA translation. The initiator tRNA attached to the initiator amino acid is catalyzed by a translation initiation factor (IF), introduced into the ribosome, and binds to the initiation codon on the mRNA, thereby translation is initiated. Since AUG, which is a methionine codon, is generally used as an initiation codon, the initiator tRNA has an anticodon corresponding to AUG, and has methionine (formylmethyonine for prokaryotes) attached to it as the initiator amino acid. Examples of the initiator tRNA include tRNA fMet (SEQ ID NOs: 10 and 11).

"Elongator tRNA" is tRNA used in the elongation reaction of the peptide chain in the translation process. In peptide synthesis, amino-acid-attached elongator tRNA is sequentially transported to the ribosome by the GTP-bound translation elongation factor (EF) EF-Tu/eEF-1, and this promotes the peptide chain elongation reaction. Examples of the elongator tRNA include tRNAs corresponding to various amino acids (SEQ ID NOs: 1 to 9 and 12 to 50).

"Lysidine" is a type of modified nucleoside and is also described as 2-lysylcytidine (k2C or L). Lysidine is used as the first letter nucleoside of the anticodon in tRNA corresponding to isoleucine (tRNA Ile2) in eubacteria. tRNA Ile 2 is synthesized in the precursor state carrying the anticodon CAU, and then the cytidine (C) of the first letter of the anticodon is engineeried (converted) to lysidine (k2C) by an enzyme called tRNA Ile-lysidine synthetase (TilS). As a result, tRNA Ile2 carrying the anticodon k2CAU is provided (Muramatsu et al., J Biol Chem (1988) 263: 9261-9267; and Suzuki et al., FEBS Lett (2010) 584: 272-277). It is known that the anticodon k2CAU specifically recognizes only the AUA codon of isoleucine. Moreover, it is believed that isoleucyl-tRNA synthetase recognizes tRNA Ile2 as a substrate and aminoacylation of (addition of isoleucine to) tRNA Ile2 occurs only when the anticodon is engineered to k2CAU. The amino acid sequence of *E. coli* TilS is shown in SEQ ID NO: 51.

"Agmatidine" is a type of modified nucleoside and is also referred to as 2-agmatinylcytidine (agm2C or Agm). Agmatidine is used as the first letter nucleoside of the anticodon in tRNA corresponding to isoleucine (tRNA Ile2) in archaea. tRNA Ile2 is synthesized in the precursor state carrying the anticodon CAU, and then the cytidine (C) of the first letter of the anticodon is engineered (converted) to agmatidine (agm2C) by an enzyme called tRNA Ile-agmatidine synthetase (TiaS). As a result, tRNAIle2 carrying the anticodon agm2CAU is provided (Ikeuchi et al., Nat Chem Biol (2010) 6(4): 277-282). It is known that the anticodon agm2CAU specifically recognizes only the AUA codon of isoleucine. Moreover, it is believed that isoleucyl-tRNA synthetase recognizes tRNA Ile2 as a substrate, and aminoacylation of (addition of isoleucine to) tRNAIle2 occurs only when the anticodon is engineered to agm2CAU. The amino acid sequence of TiaS of the archaea *Methanosarcina acetivorans* is shown in SEQ ID NO: 52.

### <Definition of substituents and the like>

In the present disclosure, "alkyl" is a monovalent group derived from an aliphatic hydrocarbon by removing one arbitrary hydrogen atom; it does not contain a hetero atom or an unsaturated carbon-carbon bond in the skeleton; and it has a subset of hydrocarbyl or hydrocarbon-group structures containing hydrogen and carbon atoms. The length of the carbon chain length, n, is in the range of 1 to 20. The examples of alkyl include C₂-C₁₀ alkyl, C₁-C₆ alkyl, and C₁-C₃ alkyl, and specific examples include methyl, ethyl, propyl, butyl, pentyl, hexyl, isopropyl, t-butyl, sec-butyl, 1-methylpropyl, 1,1-dimethylpropyl, 2,2-dimethylpropyl, 1,2-dimethylpropyl, 1,1,2-trimethylpropyl, 1,2,2-trimethylpropyl, 1,1,2,2-tetramethylpropyl, 1-methylbutyl, 2-methylbutyl, 3-methylbutyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, isopentyl, and neopentyl.

In the present disclosure, "cycloalkyl" means a saturated or partially saturated cyclic monovalent aliphatic hydrocarbon group, and includes a monocyclic ring, a bicyclic ring, and a spiro ring. Examples of cycloalkyl include C₃-C₁₀ cycloalkyl, and specific examples include cyclopropyl, cyclobutyl, cyclopentyl, cyclohexyl, cycloheptyl, cyclooctyl, and bicyclo[2.2.1]heptyl.

In the present disclosure, "alkenyl" is a monovalent group having at least one double bond (two adjacent SP2 carbon atoms). Depending on the arrangement of double bonds and substituents (if present), the geometric configuration of the double bond can be entgegen (E) or zusammen (Z), and cis or trans configurations. It can be a straight chain or branched chain alkenyl, and includes a straight chain alkenyl containing an internal olefin. Examples of the alkenyl include C₂-C₁₀ alkenyl and C₂-C₆ alkenyl, and specific examples include vinyl, allyl, 1-propenyl, 2-propenyl, 1-butenyl, 2-butenyl (including cis and trans), 3-butenyl, pentenyl, and hexenyl.

In the present disclosure, "alkynyl" is a monovalent group having at least one triple bond (two adjacent SP carbon atoms). It can be a straight or branched chain alkynyl, and includes an internal alkylene. Examples of the alkynyl include C₂-C₁₀ alkynyl and C₂-C₆ alkynyl, and specific examples include ethynyl, 1-propynyl, propargyl, 3-butynyl, pentynyl, hexynyl, 3-phenyl-2-propinyl, 3-(2'-fluorophenyl)-2-propynyl, 2-hydroxy-2-propynyl, 3-(3-fluorophenyl)-2-propynyl, and 3-methyl-(5-phenyl)-4-pentynyl.

In the present disclosure, "aryl" means a monovalent aromatic hydrocarbon ring. Examples of the aryl include C₆-C₁₀ aryl, and specific examples include phenyl and naphthyl (such as 1-naphthyl and 2-naphthyl).

In the present disclosure, "heteroaryl" means a monovalent aromatic ring group containing a hetero atom in the atoms constituting the ring, and may be partially saturated. The ring may be a monocyclic ring or a fused bicyclic ring (for example, a bicyclic heteroaryl formed by fusing with benzene or a monocyclic heteroaryl). The number of atoms constituting the ring is, for example, five to ten (5- to 10-membered heteroaryl). The number of heteroatoms contained in the ring-constituting atoms is, for example, one to five. Specific examples of the heteroaryl include furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyrimidyl, pyridazinyl, pyrazinyl, triazinyl, benzofuranyl, benzothienyl, benzothiadiazolyl, benzothiazolyl, benzoxazolyl, benzooxadiazolyl, benzimidazolyl, indolyl, isoindolyl, indazolyl, quinolyl, isoquinolyl, cinnolinyl, quinazolinyl, quinoxalinyl, benzodioxolyl, indolizinyl, and imidazopyridyl.

In the present disclosure, "arylalkyl (aralkyl)" is a group containing both aryl and alkyl, and means, for example, a group in which at least one hydrogen atom of the above-mentioned alkyl is substituted with aryl. Examples of the aralkyl include C₅-C₁₀ aryl C₁-C₆ alkyl, and specific examples include benzyl.

In the present disclosure, "alkylene" means a divalent group derived by further removing one arbitrary hydrogen atom from the above-mentioned "alkyl", and may be linear or branched. Examples of the straight chain alkylene include C₂-C₆ straight chain alkylene, C₄-C₅ straight chain alkylene and the like. Specific examples include -CH₂-, -(CH₂)₂-, -(CH₂)₃-, - (CH₂)₄-, -(CH₂)₅-, and -(CH₂)₆-. Examples of the branched alkylene include C₂-C₆ branched alkylene and C₄-C₅ branched alkylene. Specific examples include -CH(CH₃)CH₂-, -C(CH₃)₂-, - CH(CH₃)CH₂CH₂-, -C(CH₃)₂CH₂-, -CH₂CH(CH₃)CH₂-, CH₂C(CH₃)₂-, and -CH₂CH₂CH(CH₃)-.

In the present disclosure, "alkenylene" means a divalent group derived by further removing one arbitrary hydrogen atom from the above-mentioned "alkenyl", and may be linear or branched. Depending on the arrangement of double bonds and substituents (if present), it can take the form of entgegen (E) or zusammen (Z), and cis or trans configurations. Examples of the straight chain alkenylene include C₂-C₆ straight chain alkenylene and C₄-C₅ straight chain alkenylene. Specific examples include -CH=CH-, -CH=CHCH₂-, -CH₂CH=CH-, - CH=CHCH₂CH₂-, -CH₂CH=CHCH₂-, -CH₂CH₂CH=CH-, -CH=CHCH₂CH₂CH₂-, - CH₂CH=CHCH₂CH₂-, -CH₂CH₂CH=CHCH₂-, and -CH₂CH₂CH₂CH=CH-.

In the present disclosure, "arylene" means a divalent group derived by further removing one arbitrary hydrogen atom from the above-mentioned aryl. The ring may be a monocyclic ring or a fused ring. The number of atoms constituting the ring is not particularly limited, but is, for example, six to ten (C₆-C₁₀ arylene). Specific examples of arylene include phenylene and naphthylene.

In the present disclosure, "heteroarylene" means a divalent group derived by further removing one arbitrary hydrogen atom from the above-mentioned heteroaryl. The ring may be a monocyclic ring or a fused ring. The number of atoms constituting the ring is not particularly limited, but is, for example, five to ten (5- to 10-membered heteroarylene). As the heteroarylene, specific examples include pyrrolediyl, imidazoldiyl, pyrazolediyl, pyridinediyl, pyridazinediyl, pyrimidinediyl, pyrazinediyl, triazolediyl, triazinediyl, isoxazolediyl, oxazolediyl, oxadiazolediyl, isothiazolediyl, thiazolediyl, thiadiazolediyl, furandiyl, and thiophenediyl.

"Translation system" in the present disclosure is defined as a concept including both a method for translating a peptide and a kit for translating a peptide. The translation system usually contains as constituent components, ribosomes, translation factors, tRNAs, amino acids, aminoacyl-tRNA synthetase (aaRS), and factors necessary for peptide translation reactions such as ATP and GTP. The main types of translation systems include translation systems that utilize living cells and translation systems that utilize cell extract solutions (cell-free translation systems). As the translation system utilizing living cells, a known example is a system in which a desired aminoacyl-tRNA and mRNA are introduced into living cells such as Xenopus oocytes and mammalian cells by microinjection method or lipofection method to perform peptide translation (Nowak et al., Science (1995) 268: 439-442). Known examples of cell-free translation systems include translation systems that utilize extract solutions from *E. coli* (Chen et al., Methods Enzymol (1983) 101: 674-690), yeast (Gasior et al., J Biol Chem (1979) 254: 3965-3969), wheat germ (Erickson et al., Methods Enzymol (1983) 96: 38-50), rabbit reticulocytes (Jackson et al., Methods Enzymol (1983)96: 50-74), HeLa cells (Barton et al., Methods Enzymol (1996) 275: 35-57), or insect cells (Swerdel et al., Comp Biochem Physiol B (1989) 93: 803-806), etc. Such a translation system can be appropriately prepared by a method known to those skilled in the art or a similar method. The cell-free translation system also includes a translation system constructed by isolating and purifying each of the factors required for peptide translation and reconstituting them (reconstituted cell-free translation system) (Shimizu et al., Nat Biotech (2001) 19: 751-755). Reconstituted cell-free translation systems may usually include ribosomes, amino acids, tRNAs, aminoacyl-tRNA synthetases (aaRS), translation initiation factors (for example, IF1, IF2, and IF3), translation elongation factors (for example, EF-Tu, EF-Ts, and EF-G), translation termination factors (for example, RF1, RF2, and RF3), ribosome recycling factors (RRF), NTPs as energy sources, energy regeneration systems, and other factors required for translation. When the transcription reaction from DNA is also performed, RNA polymerase and the like may be further included. Various factors contained in the cell-free translation system can be isolated and purified by methods well known to those skilled in the art, and a reconstituted cell-free translation system can be appropriately constructed using them. Alternatively, a commercially available reconstituted cell-free translation system such as PUREfrex®from Gene Frontier or PURExpress®from New England BioLabs can be used. For a reconstituted cell-free translation system, a desired translation system can be constructed by reconstituting only the necessary components from among the translation system components.

An aminoacyl-tRNA is synthesized by a specific combination of amino acid, tRNA, and aminoacyl-tRNA synthetase, and it is used for peptide translation. Instead of the above-mentioned combination, aminoacyl-tRNA can be directly used as a constituent component of the translation system. In particular, when an amino acid that is difficult to aminoacylate with an aminoacyl-tRNA synthetase, such as an unnatural amino acid, is used for translation, it is desirable to use a tRNA which is aminoacylated in advance with an unnatural amino acid, as a constituent component.

The translation is started by adding mRNA to the translation system. An mRNA usually contains a sequence that encodes the peptide of interest, and may further include a sequence for increasing the efficiency of translation reaction (for example, a Shine-Dalgarno (SD) sequence in prokaryotes, or a Kozac sequence in eukaryotes). Pre-transcribed mRNA may be added directly to the system, or instead of mRNA, a template DNA containing a promoter and an RNA polymerase appropriate for the DNA (for example, T7 promoter and T7 RNA polymerase) can be added to the system, so that mRNA will be transcribed from the template DNA.

### II. Compositions and methods

### <Mutated tRNA>

In one aspect, the present disclosure provides engineered tRNAs. Specifically, the present invention provides mutated tRNAs produced by engineering tRNAs. The tRNAs to be engineered may be natural tRNAs derived from any organism (for example, *E. coli*), or non-natural tRNAs obtained by artificially synthesizing sequences different from the natural tRNA sequences. Alternatively, they may be tRNAs obtained by artificially synthesizing the same sequences as the natural tRNA sequences. In the present disclosure, any engineering introduced into tRNA is an artificial engineering, and any mutated tRNA produced by the engineering has a nucleic acid sequence that does not exist in nature.

In some embodiments, engineering of tRNA in the present disclosure means introducing at least one engineering selected from the following group into one or more nucleosides constituting a tRNA: (i) addition (adding any new nucleoside to an existing tRNA), (ii) deletion (deleting any nucleoside from an existing tRNA), (iii) substitution (substituting any nucleoside in an existing tRNA with another arbitrary nucleoside), (iv) insertion (adding a new arbitrary nucleoside between any two nucleosides in an existing tRNA), and (v) modification (changing a part of the structure (for example, the nucleotide or sugar portion) of any nucleoside in an existing tRNA to another structure). Engineer may be made to any structure of a tRNA (for example, the D arm, anticodon arm, T arm, acceptor stem, variable loop, and such). In certain embodiments, tRNA engineerings in the present disclosure are made to anticodons contained in anticodon arms. In a further embodiment, tRNA engineerings in the present disclosure are made to at least one of the nucleosides for the first, second, and third letters of the anticodon. According to the nucleoside numbering rule in tRNA, nucleosides for the first, second, and third letters of the anticodon correspond to positions 34, 35, and 36 of tRNA, respectively. Herein, the nucleosides for the first, second, and third letters of the anticodon may be represented as N₁, N₂, and N₃, respectively. In certain embodiments, tRNA engineerings in the present disclosure include engineerings made to the nucleoside of the first letter of the anticodon. The number of nucleosides engineeredin the tRNA of the present disclosure can be any number not less than one. In some embodiments, the number of nucleosides engineered in the tRNA of the present disclosure is 20 or less, 15 or less, 10 or less, 9 or less, 8 or less, 7 or less, 6 or less, 5 or less, 4 or less, 3 or less, 2 or less, or 1. In another embodiment, the nucleic acid sequence of the engineered tRNA has sequence identity of 80% or more, 85% or more, 90% or more, 91% or more, 92% or more, 93% or more, 94% or more, 95% or more, 96% or more, 97% or more, 98% or more, or 99% or more, as compared to the nucleic acid sequence before the engineering.

In a specific embodiment, engineering of tRNA in the present disclosure means substitution of one or more nucleosides constituting a tRNA. Regarding the types of nucleosides, a substituted nucleoside may be any nucleoside present in natural tRNAs or any nucleoside not present in natural tRNAs (an artificially synthesized nucleoside). In addition to the four typical nucleosides, adenosine, guanosine, cytidine and uridine, natural tRNAs include engineered forms obtained by modifying these four nucleosides (modified nucleosides). In some embodiments, the nucleoside present in natural tRNAs can be selected from among the following nucleosides: adenosine (A); cytidine (C); guanosine (G); uridine (U); 1-methyladenosine (m1A); 2-methyladenosine (m2A); N6-isopentenyladenosine (i6A); 2-methylthio-N6-isopentenyladenosine (ms2i6A); N6-methyladenosine (m6A); N6-threonylcarbamoyladenosine (t6A); N6-methyl-N6-threonylcarbamoyladenosine (m6t6A); 2-methylthio-N6-threonylcarbamoyladenosine (ms2t6A); 2'-O-methyladenosine (Am); inosine (I); 1-methylinosine (m1I); 2'-O-ribosyladenosine (phosphate) (Ar(p)); N6-(cis-hydroxyisopentenyl)adenosine (io6A); 2-thiocytidine (s2C); 2'-O-methylcytidine (Cm); N4-acetylcytidine (ac4C); 5-methylcytidine (m5C); 3-methylcytidine (m3C); lysidine (k2C); 5-formylcytidine (f5C); 2'-O-methyl-5-formylcytidine (f5Cm); agmatidine (agm2C); 2'-O-ribosylguanosine (phosphate) (Gr(p)); 1-methylguanosine (m1G); N2-methylguanosine (m2G); 2'-O-methylguanosine (Gm); N2, N2-dimethylguanosine(m22G); N2, N2, 2'-O-trimethylguanosine (m22Gm); 7-methylguanosine (m7G); archaeosine (G*); queuosine (Q); mannosylqueuosine (manQ); galactosylqueuosine (galQ); wybutosine (yW); peroxywybutosine (o2yW); 5-methylaminomethyluridine (mnm5U); 2-thiouridine (s2U); 2'-O-methyluridine (Um); 4-thiouridine (s4U); 5-carbamoylmethyluridine (ncm5U); 5-methoxycarbonylmethyluridine (mcm5U); 5-methylaminomethyl-2-thiouridine (mnm5s2U); 5-methoxycarbonylmethyl-2-thiouridine (mcm5s2U); uridine 5-oxyacetic acid (cmo5U); 5-methoxyuridine (mo5U); 5-carboxymethylaminomethyluridine (cmnm5U); 5-carboxymethylaminomethyl-2-thiouridine (cmnm5s2U); 3-(3-amino-3-carboxypropyl)uridine (acp3U); 5-(carboxyhydroxymethyl)uridinemethyl ester (mchm5U); 5-carboxymethylaminomethyl-2'-O-methyluridine (cmnm5Um); 5-carbamoylmethyl-2'-O-methyluridine (ncm5Um); dihydrouridine (D); pseudouridine (Ψ); 1-methylpseudouridine (m1Ψ); 2'-O-methylpseudouridine (Ψm); 5-methyluridine (m5U); 5-methyl-2-thiouridine (m5s2U); and 5, 2'-O-dimethyluridine (m5Um). In certain embodiments, one or more nucleosides that constitute the tRNAs of the present disclosure are replaced with lysidine or agmatidine. A nucleoside derivative obtained by modifying a part (for example, the nucleotide portion) of the structure of a nucleoside existing in natural tRNAs, described above, can also be used for substitution. In certain embodiments, one or more nucleosides constituting the tRNAs of the present disclosure are replaced with lysidine derivatives or agmatidine derivatives.

The tRNA engineered in the present disclosure can be appropriately selected from tRNAs having an arbitrary nucleic acid sequence. In some embodiments, the tRNA is any one of tRNA Ala, tRNA Arg, tRNA Asn, tRNA Asp, tRNA Cys, tRNA Gln, tRNA Glu, tRNA Gly, tRNA His, tRNA Ile, tRNA Leu, tRNA Lys, tRNA Met, tRNA Phe, tRNA Pro, tRNA Ser, tRNA Thr, tRNA Trp, tRNA Tyr, and tRNA Val. In addition to the above-mentioned 20 tRNAs, tRNA fMet, tRNA Sec (selenocysteine), tRNA Pyl (pyrrolysine), tRNA AsnE2 and the like may be used. In a particular embodiment, the tRNA is any one of tRNA Glu, tRNA Asp, tRNA AsnE2. For some tRNAs, exemplary nucleic acid sequences are shown in SEQ ID NOs: 1 to 50. The term "tRNA body" is sometimes used to refer to the main part of tRNA (the main part of the structure, which is composed of nucleic acids).

In addition, in the present disclosure, tRNA may be expressed as follows.
- "tRNA Xxx" or "tRNA(Xxx)"... indicates a tRNA (full length) corresponding to the amino acid Xxx (for example, tRNA Glu or tRNA(Glu)).
- "tRNA(Xxx)nnn"... indicates a tRNA corresponding to the amino acid Xxx, which is a tRNA (full length) having an anticodon sequence of nnn (for example, tRNA(Glu)uga or tRNA(Glu)Lga).
- "tRNA(Xxx)nnn-CA"... indicates a tRNA corresponding to the amino acid Xxx, which is a tRNA (the CA sequence at the 3' end has been removed) having an anticodon sequence of nnn (for example, tRNA(Glu)uga-CA and tRNA(Glu)Lga-CA).

In certain embodiments, tRNA engineerings in the present disclosure include engineerings that substitute the nucleoside of the first letter (N₁) of the anticodon with any one of lysidine, a lysidine derivative, agmatidine, or an agmatidine derivative. Here, a lysidine derivative means a molecule produced by modifying a part of the structure of lysidine (for example, the nucleotide portion), and when used as a part of an anticodon, it has the same codon discrimination ability (ability to form complementary base pairs) as that of lysidine. Furthermore, an agmatidine derivative means a molecule produced by modifying a part of the structure of agmatidine (for example, the nucleotide portion), and when used as a part of an anticodon, it has the same codon discrimination ability (ability to form complementary base pairs) as that of agmatidine.

Lysidine in natural tRNA is synthesized by the action of an enzyme called tRNA Ile-lysidine synthetase (TilS). TilS has the activity of specifically recognizing tRNA corresponding to isoleucine (tRNA Ile2) as a substrate, and engineering (converting) cytidine (C) at the first letter (N₁) of its anticodon to lysidine (k2C). The lysidine in the tRNA of the present disclosure may be lysidine synthesized with or without the mediation of TilS.

In the former case (when lysidine was synthesized via TilS), the tRNA of the present disclosure may be recognized by TilS as a substrate. That is, when N₁ in the tRNA before engineering is cytidine, the cytidine may be engineered to lysidine by TilS. Whether or not cytidine at N₁ of a tRNA can be engineered to lysidine by TilS, can be confirmed, for example, by preparing TilS by genetic recombination technique or extracting TilS from a biological material, reacting it with the tRNA in which N₁ is cytidine under appropriate conditions, and then detecting lysidine in the reaction product (see, for example, Suzuki et al., FEBS Lett (2010) 584: 272-277). Alternatively, this confirmation can be carried out by introducing a tRNA in which N₁ is cytidine into cells that endogenously express TilS or into cells made to express TilS by a genetic recombination technique, reacting the introduced tRNA with the intracellular TilS under appropriate conditions, and then detecting lysidine contained in the tRNA. In one embodiment of the present disclosure, when N₁ in the tRNA before engineering is cytidine, the engineering of the cytidine to lysidine may be catalyzed by TilS.

On the other hand, in the latter case (when lysidine is synthesized without the mediation of TilS), the tRNA of the present disclosure cannot be recognized as a substrate by TilS. That is, even if N₁ in the tRNA before engineered is cytidine, the cytidine cannot be engineered to lysidine by TilS. In that case, lysidine and the tRNA containing lysidine can be synthesized by a method that does not use TilS (for example, a chemical synthesis method). An example of such a synthesis method is shown in the Examples described later. In one embodiment of the present disclosure, if N₁ in the tRNA before engineering is cytidine, the engineering of the cytidine to lysidine cannot be catalyzed by TilS. The condition in which engineering of cytidine to lysidine cannot be catalyzed by TilS, can be represented as the following condition: when 10 µg/mL TilS is reacted with 1 µM tRNA at 37°C for 2 hours, in 100 mM Hepes-KOH (pH 8.0), 10 mM KCl, 10 mM MgCl₂, 2 mM DTT, 2 mM ATP, and 100 µM lysine, if the activity to engineer cytidine of the natural substrate tRNA Ile2 to lysidine is 1, the activity of TilS to engineer the cytidine of the target tRNA to lysidine is reduced by 10 times or more, 20 times or more, 40 times or more, 100 times or more, 200 times or more, or 400 times or more. When the catalytic activity by TilS is reduced, only a low-purity target product containing a large amount of unengineered tRNA in which N₁ remains cytidine can be obtained as a result; therefore, synthesizing lysidine by a method without using TilS (for example, a chemical synthesis method) rather than by the method using TilS may be more advantageous. In a particular embodiment, TilS is TilS from *E. coli.* In a further embodiment, TilS is wild type TilS from *E. coli* having the amino acid sequence of SEQ ID NO: 51.

In addition, TilS has been reported to maintain a certain amount of lysidine synthesizing ability for tRNA even after some nucleosides in tRNA Ile2 have been engineered to other nucleosides (Ikeuchi et al., Mol Cell (2005) 19: 235-246).

Agmatidine in natural tRNA is synthesized by the action of an enzyme called tRNA Ile-agmatidine synthetase (TiaS). TiaS specifically recognizes tRNA corresponding to isoleucine (tRNA Ile2) as a substrate, and has an activity of engineering (converting) cytidine (C) in the first letter (N₁) of its anticodon to agmatidine (agm2C). Agmatidine in the tRNA of the present disclosure may be agmatidine synthesized with or without the mediation of TiaS.

In the former case (when agmatidine is synthesized via TiaS), the tRNA of the present disclosure may be recognized by TiaS as a substrate. That is, when N₁ in the tRNA before engineering is cytidine, the cytidine may be engineered to agmatidine by TiaS. Whether cytidine at N₁ of a tRNA can be engineered to agmatidine by TiaS, can be confirmed for example, by preparing TiaS by a genetic recombination technique, or extracting TiaS from a biological material, reacting the TiaS with a tRNA in which N₁ is cytidine under appropriate conditions, and then detecting agmatidine in the reaction product (see for example, Ikeuchi et al., Nat Chem Biol (2010) 6(4): 277-282). Alternatively, this confirmation can be carried out by introducing a tRNA in which N₁ is cytidine into cells that endogenously express TiaS or into cells made to express TiaS by a genetic recombination technique, reacting the introduced tRNA with the intracellular TiaS under appropriate conditions, and then detecting agmatidine contained in the tRNA. In one embodiment of the present disclosure, when N₁ in the tRNA before engineering is cytidine, the engineering of the cytidine to agmatidine may be catalyzed by TiaS.

On the other hand, in the latter case (when agmatidine is synthesized without the mediation of TiaS), the tRNA of the present disclosure cannot be recognized as a substrate by TiaS. That is, even if N₁ in the tRNA before engineering is cytidine, the cytidine cannot be engineered to agmatidine by TiaS. In that case, agmatidine and the tRNA containing agmatidine can be synthesized by a method that does not use TiaS (for example, a chemical synthesis method). In one embodiment of the present disclosure, if N₁ in the tRNA before engineering is cytidine, the engineering of the cytidine to agmatidine cannot be catalyzed by TiaS. The condition in which engineering of cytidine to agmatidine cannot be catalyzed by TiaS, can be represented as the following condition: when the activity of TiaS to engineer cytidine of the natural substrate tRNA Ile2 to agmatidine is 1, the activity of TiaS to engineer the cytidine of the target tRNA to agmatidine is reduced by 10 times or more, 20 times or more, 40 times or more, 100 times or more, 200 times or more, or 400 times or more. When the catalytic activity by TiaS is reduced, only a low-purity target product containing a large amount of unengineered tRNA in which N₁ remains cytidine can be obtained as a result; therefore, synthesizing agmatidine by a method without using TiaS (for example, a chemical synthesis method) rather than by the method using TiaS may be more advantageous. In a particular embodiment, TiaS is TiaS from archaea. In a further embodiment, TiaS is wild type TiaS from the archaea *Methanosarcina acetivorans* having the amino acid sequence of SEQ ID NO: 52.

In addition, TiaS has been reported to maintain a certain amount of agmatidine synthesizing ability for tRNA even after some nucleosides in tRNA Ile2 have been engineered to other nucleosides (Osawa et al., Nat Struct Mol Biol (2011) 18: 1275-1280).

In some embodiments, the mutated tRNA of the present disclosure is an initiator tRNA or an elongator tRNA. The mutated tRNA may be produced by engineering the initiator tRNA or the elongator tRNA, or the mutated tRNA produced by the engineering may have a function as the initiator tRNA or the elongator tRNA. Whether or not a certain tRNA has a function as an initiator tRNA can be judged by observing whether the tRNA (i) is introduced into the ribosome via IF2, and (ii) whether the amino acid attached to the tRNA can be used as the initiator amino acid to start the peptide translation, when the tRNA is used in a translation system. Furthermore, whether or not a certain tRNA has a function as an elongator tRNA can be determined by observing whether the tRNA (i) is introduced into the ribosome via EF-Tu, and (ii) whether or not the amino acid attached to the tRNA can be incorporated into the peptide chain to extend the peptide chain, when the tRNA is used in a translation system.

In some embodiments, the mutated tRNA of the present disclosure is a prokaryote-derived tRNA or a eukaryote-derived tRNA. A mutated tRNA may be produced by engineering a prokaryote-derived tRNA or a eukaryote-derived tRNA, and the mutated tRNA produced by the engineering may have the highest nucleic acid sequence identity with the prokaryote-derived tRNA or the eukaryote-derived tRNA. Eukaryotes are further classified into animals, plants, fungi, and protists. The mutated tRNA of the present disclosure may be, for example, a human-derived tRNA. Prokaryotes are further classified into eubacteria and archaea. Examples of eubacteria include *E. coli, Bacillus subtilis,* lactic acid bacteria, and *Desulfitobacterium hafniense.* Examples of archaea include extreme halophile, thermophile, or methane bacteria (for example, *Methanosarcina mazei, Methanosarcina barkeri,* and *Methanocaldococcus jannaschii*). The mutated tRNA of the present disclosure may be, for example, tRNA derived from *E. coli, Desulfitobacterium hafniense,* or *Methanosarcina mazei.*

In some embodiments, the mutated tRNA of the present disclosure, can translate codons represented by M₁M₂A. Here, the nucleoside of the first letter (Mi) and the nucleoside of the second letter (M₂) of the codon are each independently selected from any of adenosine (A), guanosine (G), cytidine (C), or uridine (U), and the nucleoside of the third letter is adenosine. In another embodiment, the mutated tRNA of the present disclosure has an anticodon complementary to the specific codon represented by M₁M₂A. In certain embodiments, the mutated tRNA of the present disclosure has an anticodon represented by k2CN₂N₃ or agm2CN₂N₃. Here, the nucleoside of the first letter of the anticodon is lysidine (k2C) or agmatidine (agm2C), and the nucleoside of the second letter (N₂) and the third nucleoside of the third letter (N₃) are nucleosides complementary to the above-mentioned M₁ and M₂, respectively. Lysidine and agmatidine are both known as nucleosides that complementarily bind to adenosine. In a further embodiment, each of N₂ and N₃ may be independently selected from any of adenosine (A), guanosine (G), cytidine (C), and uridine (U). Specifically, when M₂ (or M₁) is adenosine, N₂ (or N₃) is uridine. When M₂ (or M₁) is guanosine, N₂ (or N₃) is cytidine. When M₂ (or M₁) is cytidine, N₂ (or N₃) is guanosine. When M₂ (or M₁) is uridine, N₂ (or N₃) is adenosine.

In the context of the present disclosure, the embodiment "a certain tRNA is capable of translating a specific codon" essentially includes the embodiment "a certain tRNA has an anticodon complementary to the specific codon," and as long as one the sequence of the anticodon on the tRNA is referred to, these expressions can be used interchangeably.

The nucleoside of the first letter (M₁) and the nucleoside of the second letter (M₂) of the codon translatable by the mutated tRNA of the present disclosure can be selected from the nucleoside of the first letter (M₁) and the nucleoside of the second letter (M₂) of codons constituting a specific codon box in the genetic code table, respectively. In a particular embodiment, the genetic code table is a standard genetic code table. In another embodiment, the genetic code table is the natural genetic code table.

In one embodiment, M₁ and M₂ may be selected from M₁ and M₂, respectively, in codons constituting a codon box in which a codon having A as the third letter and a codon having G as the third letter encode the same amino acid. As an example, in the codon box whose codons are represented by UUN, the codon having A as the third letter (UUA) and the codon having G as the third letter (UUG) both encode the same amino acid (Leu); therefore, the nucleoside of the first letter (U) and the nucleoside of the second letter (U) in the codons constituting this codon box can be selected as M₁ and M₂, respectively.

In one embodiment, M₁ and M₂ may be selected from M₁ and M₂, respectively, in codons constituting a codon box in which a codon having U as the third letter and a codon having A as the third letter both encode the same amino acid. As an example, in the codon box whose codons are represented by AUN, the codon having U as the third letter (AUU) and the codon having A as the third letter (AUA) both encode the same amino acid (Ile); therefore, the nucleoside of the first letter (A) and the nucleoside of the second letter (U) in the codons constituting this codon box can be selected as M₁ and M₂, respectively.

In one embodiment, M₁ and M₂ may be selected from M₁ and M₂, respectively, in codons constituting a codon box in which a codon having U, a codon having C as the third letter, a codon having A as the third letter, and a codon having G as the third letter all encode the same amino acid. As an example, in the codon box whose codons are represented by UCN, the codon having U as the third letter (UCU), the codon having C as the third letter (UCC), the codon having A as the third letter (UCA), and the codon having G as the third letter (UCG) all encode the same amino acid (Ser); therefore, the nucleoside of the first letter (U) and the nucleoside of the second letter (C) in the codons constituting this codon box can be selected as M₁ and M₂, respectively.

In one embodiment, M₁ and M₂ may be selected from M₁ and M₂, respectively, in codons constituting a codon box in which a codon having A as the third letter and a codon having G as the third letter encode different amino acids from each other. As an example, in the codon box whose codons are represented by AUN, the codon having A as the third letter (AUA) and the codon having G as the third letter (AUG) encode different amino acids from each other (Ile and Met); therefore, the nucleoside of the first letter (A) and the nucleoside of the second letter (U) in the codons constituting this codon box can be selected as M₁ and M₂, respectively.

In one embodiment, M₁ and M₂ may be selected from M₁ and M₂, respectively, in codons constituting a codon box in which a codon having A as the third letter and/or a codon having G as the third letter are stop codons. As an example, in the codon box whose codons are represented by UGN, the codon having A as the third letter (UGA) is a stop codon (opal); therefore, the nucleoside of the first letter (U) and the nucleoside of the second letter (G) in the codons constituting this codon box can be selected as M₁ and M₂, respectively.

In further embodiments, M₁ and M₂ may be selected from M₁ and M₂, respectively, in codons constituting a codon box whose codons are represented by UNN. Specifically, the nucleoside of the first letter (U) and the nucleoside of the second letter (U) in the codons can be selected as M₁ and M₂, respectively.

In further embodiments, M₁ and M₂ may be selected from M₁ and M₂, respectively, in codons constituting a codon box whose codons are represented by UCN. Specifically, the nucleoside of the first letter (U) and the nucleoside of the second letter (C) in the codons can be selected as M₁ and M₂, respectively.

In further embodiments, M₁ and M₂ may be selected from M₁ and M₂, respectively, in codons constituting a codon box whose codons are represented by UAN. Specifically, the nucleoside of the first letter (U) and the nucleoside of the second letter (A) in the codons can be selected as M₁ and M₂, respectively.

In further embodiments, M₁ and M₂ may be selected from M₁ and M₂, respectively, in codons constituting a codon box whose codons are represented by UGN. Specifically, the nucleoside of the first letter (U) and the nucleoside of the second letter (G) in the codons can be selected as M₁ and M₂, respectively.

In further embodiments, M₁ and M₂ may be selected from M₁ and M₂, respectively, in codons constituting a codon box whose codons are represented by CUN. Specifically, the nucleoside of the first letter (C) and the nucleoside of the second letter (U) in the codons can be selected as M₁ and M₂, respectively.

In further embodiments, M₁ and M₂ may be selected from M₁ and M₂, respectively, in codons constituting a codon box whose codons are represented by CCN. Specifically, the nucleoside of the first letter (C) and the nucleoside of the second letter (C) in the codons can be selected as M₁ and M₂, respectively.

In further embodiments, M₁ and M₂ may be selected from M₁ and M₂, respectively, in codons constituting a codon box whose codons are represented by CAN. Specifically, the nucleoside of the first letter (C) and the nucleoside of the second letter (A) in the codons can be selected as M₁ and M₂, respectively.

In further embodiments, M₁ and M₂ may be selected from M₁ and M₂, respectively, in codons constituting a codon box whose codons are represented by CGN. Specifically, the nucleoside of the first letter (C) and the nucleoside of the second letter (G) in the codons can be selected as M₁ and M₂, respectively.

In further embodiments, M₁ and M₂ may be selected from M₁ and M₂, respectively, in codons constituting a codon box whose codons are represented by AUN. Specifically, the nucleoside of the first letter (A) and the nucleoside of the second letter (U) in the codons can be selected as M₁ and M₂, respectively.

In further embodiments, M₁ and M₂ may be selected from M₁ and M₂, respectively, in codons constituting a codon box whose codons are represented by ACN. Specifically, the nucleoside of the first letter (A) and the nucleoside of the second letter (C) in the codons can be selected as M₁ and M₂, respectively.

In further embodiments, M₁ and M₂ may be selected from M₁ and M₂, respectively, in codons constituting a codon box whose codons are represented by AAN. Specifically, the nucleoside of the first letter (A) and the nucleoside of the second letter (A) in the codons can be selected as M₁ and M₂, respectively.

In further embodiments, M₁ and M₂ may be selected from M₁ and M₂, respectively, in codons constituting a codon box whose codons are represented by AGN. Specifically, the nucleoside of the first letter (A) and the nucleoside of the second letter (G) in the codons can be selected as M₁ and M₂, respectively.

In further embodiments, M₁ and M₂ may be selected from M₁ and M₂, respectively, in codons constituting a codon box whose codons are represented by GUN. Specifically, the nucleoside of the first letter (G) and the nucleoside of the second letter (U) in the codons can be selected as M₁ and M₂, respectively.

In further embodiments, M₁ and M₂ may be selected from M₁ and M₂, respectively, in codons constituting a codon box whose codons are represented by GCN. Specifically, the nucleoside of the first letter (G) and the nucleoside of the second letter (C) in the codons can be selected as M₁ and M₂, respectively.

In further embodiments, M₁ and M₂ may be selected from M₁ and M₂, respectively, in codons constituting a codon box whose codons are represented by GAN. Specifically, the nucleoside of the first letter (G) and the nucleoside of the second letter (A) in the codons can be selected as M₁ and M₂, respectively.

In further embodiments, M₁ and M₂ may be selected from M₁ and M₂, respectively, in codons constituting a codon box whose codons are represented by GGN. Specifically, the nucleoside of the first letter (G) and the nucleoside of the second letter (G) in the codons can be selected as M₁ and M₂, respectively.

The nucleoside of the third letter (N₃) and the nucleoside of the second letter (N₂) of the anticodon in the mutated tRNA of the present disclosure may be selected as nucleosides complementary to M₁ and M₂, respectively.

In one embodiment, N₃ and N₂ may be selected as nucleosides complementary to the nucleoside of the first letter (U) and the nucleoside of the second letter (U), respectively, in codons constituting a codon box whose codons are represented by UUN. Specifically, A can be selected as N₃ and A can be selected as N₂.

In one embodiment, N₃ and N₂ may be selected as nucleosides complementary to the nucleoside of the first letter (U) and the nucleoside of the second letter (C), respectively, in codons constituting a codon box whose codons are represented by UCN. Specifically, A can be selected as N₃ and G can be selected as N₂.

In one embodiment, N₃ and N₂ may be selected as nucleosides complementary to the nucleoside of the first letter (U) and the nucleoside of the second letter (A), respectively, in codons constituting a codon box whose codons are represented by UAN. Specifically, A can be selected as N₃ and U can be selected as N₂.

In one embodiment, N₃ and N₂ may be selected as nucleosides complementary to the nucleoside of the first letter (U) and the nucleoside of the second letter (G), respectively, in codons constituting a codon box whose codons are represented by UGN. Specifically, A can be selected as N₃ and C can be selected as N₂.

In one embodiment, N₃ and N₂ may be selected as nucleosides complementary to the nucleoside of the first letter (C) and the nucleoside of the second letter (U), respectively, in codons constituting a codon box whose codons are represented by CUN. Specifically, G can be selected as N₃ and A can be selected as N₂.

In one embodiment, N₃ and N₂ may be selected as nucleosides complementary to the nucleoside of the first letter (C) and the nucleoside of the second letter (C), respectively, in codons constituting a codon box whose codons are represented by CCN. Specifically, G can be selected as N₃ and G can be selected as N₂.

In one embodiment, N₃ and N₂ may be selected as nucleosides complementary to the nucleoside of the first letter (C) and the nucleoside of the second letter (A), respectively, in codons constituting a codon box whose codons are represented by CAN. Specifically, G can be selected as N₃ and U can be selected as N₂.

In one embodiment, N₃ and N₂ may be selected as nucleosides complementary to the nucleoside of the first letter (C) and the nucleoside of the second letter (G), respectively, in codons constituting a codon box whose codons are represented by CGN. Specifically, G can be selected as N₃ and C can be selected as N₂.

In one embodiment, N₃ and N₂ may be selected as nucleosides complementary to the nucleoside of the first letter (A) and the nucleoside of the second letter (U), respectively, in codons constituting a codon box whose codons are represented by AUN. Specifically, U can be selected as N₃ and A can be selected as N₂.

In one embodiment, N₃ and N₂ may be selected as nucleosides complementary to the nucleoside of the first letter (A) and the nucleoside of the second letter (C), respectively, in codons constituting a codon box whose codons are represented by ACN. Specifically, U can be selected as N₃ and G can be selected as N₂.

In one embodiment, N₃ and N₂ may be selected as nucleosides complementary to the nucleoside of the first letter (A) and the nucleoside of the second letter (A), respectively, in codons constituting a codon box whose codons are represented by AAN. Specifically, U can be selected as N₃ and U can be selected as N₂.

In one embodiment, N₃ and N₂ may be selected as nucleosides complementary to the nucleoside of the first letter (A) and the nucleoside of the second letter (G), respectively, in codons constituting a codon box whose codons are represented by AGN. Specifically, U can be selected as N₃ and C can be selected as N₂.

In one embodiment, N₃ and N₂ may be selected as nucleosides complementary to the nucleoside of the first letter (G) and the nucleoside of the second letter (U), respectively, in codons constituting a codon box whose codons are represented by GUN. Specifically, C can be selected as N₃ and A can be selected as N₂.

In one embodiment, N₃ and N₂ may be selected as nucleosides complementary to the nucleoside of the first letter (G) and the nucleoside of the second letter (C), respectively, in codons constituting a codon box whose codons are represented by GCN. Specifically, C can be selected as N₃ and G can be selected as N₂.

In one embodiment, N₃ and N₂ may be selected as nucleosides complementary to the nucleoside of the first letter (G) and the nucleoside of the second letter (A), respectively, in codons constituting a codon box whose codons are represented by GAN. Specifically, C can be selected as N₃ and U can be selected as N₂.

In one embodiment, N₃ and N₂ may be selected as nucleosides complementary to the nucleoside of the first letter (G) and the nucleoside of the second letter (G), respectively, in codons constituting a codon box whose codons are represented by GGN. Specifically, C can be selected as N₃ and C can be selected as N₂.

In some embodiments, an amino acid or amino acid analog is attached to the mutated tRNA of the present disclosure. The amino acid or amino acid analog is usually attached to the 3' end of the tRNA, or more specifically, to the adenosine residue of the CCA sequence at the 3' end. The specific type of the amino acid or amino acid analog attached to the mutated tRNA can be appropriately selected from the following amino acids or amino acid analogs.

The amino acids in the present disclosure include α-amino acids, β-amino acids, and γ-amino acids. Regarding three-dimensional structures, both L-type amino acids and D-type amino acids are included. Furthermore, amino acids in the present disclosure include natural and unnatural amino acids. In a particular embodiment, the natural amino acids consist of the following 20α-amino acids: glycine (Gly), alanine (Ala), serine (Ser), threonine (Thr), valine (Val), leucine (Leu), isoleucine (Ile), phenylalanine (Phe), tyrosine (Tyr), tryptophan (Trp), histidine (His), glutamic acid (Glu), aspartic acid (Asp), glutamine (Gln), asparagine (Asn), cysteine (Cys), methionine (Met), lysine (Lys), arginine (Arg), and proline (Pro). Alternatively, the natural amino acids in the present disclosure may be those obtained by removing any one or more amino acids from the above-mentioned 20 amino acids. In one embodiment, the natural amino acids consist of 19 amino acids, excluding isoleucine. In one embodiment, the natural amino acids consist of 19 amino acids, excluding methionine. In a further embodiment, the natural amino acids consist of 18 amino acids, excluding isoleucine and methionine. Natural amino acids are usually L-type amino acids.

In the present disclosure, unnatural amino acids refer to all amino acids excluding the above-mentioned natural amino acids consisting of 20α-amino acids. Examples of unnatural amino acids include β-amino acids, γ-amino acids, D-type amino acids, α-amino acids whose side chains differ from natural amino acids, α,α-disubstituted amino acids, and amino acids whose main chain amino group has a substituent (N-substituted amino acids). The side chain of the unnatural amino acid is not particularly limited, but may have, for example, alkyl, alkenyl, alkynyl, aryl, heteroaryl, aralkyl, and cycloalkyl, in addition to the hydrogen atom. Further, in the case of an α,α-disubstituted amino acid, two side chains may form a ring. Furthermore, these side chains may have one or more substituents. In a particular embodiment, the substituents can be selected from any functional group containing a halogen atom, O atom, S atom, N atom, B atom, Si atom, or P atom. For example, in the present disclosure, "C₁-C₆ alkyl having halogen as a substituent" means a "C₁-C₆ alkyl" in which at least one hydrogen atom in an alkyl is substituted with a halogen atom, and specific examples include, trifluoromethyl, difluoromethyl, fluoromethyl, pentafluoroethyl, tetrafluoroethyl, trifluoroethyl, difluoroethyl, fluoroethyl, trichloromethyl, dichloromethyl, chloromethyl, pentachloroethyl, tetrachloroethyl, trichloroethyl, dichloroethyl, and chloroethyl. In addition, for example, "C₅-C₁₀ aryl C₁-C₆ alkyl having a substituent" means "C₅-C₁₀ aryl C₁-C₆ alkyl" in which at least one hydrogen atom in aryl and/or alkyl is substituted with a substituent. Furthermore, the meaning of the phrase "having two or more substituents" includes having a certain functional group (for example, a functional group containing an S atom) as a substituent, and the functional group has another substituent (for example, a substituent such as amino or halogen). For specific examples of unnatural amino acids, one can refer to WO2013/100132, WO2018/143145, and such.

The amino group of the main chain of the unnatural amino acid may be an unsubstituted amino group (NH₂ group) or a substituted amino group (NHR group). Here, R indicates an alkyl, alkenyl, alkynyl, aryl, heteroaryl, aralkyl, or cycloalkyl which optionally has a substituent. Further, like proline, the carbon chain attached to the N atom of the main chain amino group and the α-position carbon atom may form a ring. The substituent can be selected from any functional group containing a halogen atom, O atom, S atom, N atom, B atom, Si atom, or P atom. Examples of alkyl substitution of an amino group include N-methylation, N-ethylation, N-propylation, and N-butylation, and example of aralkyl substitution of an amino group include N-benzylation. Specific examples of an N-methylamino acid include N-methylalanine, N-methylglycine, N-methylphenylalanine, N-methyltyrosine, N-methyl-3-chlorophenylalanine, N-methyl-4-chlorophenylalanine, N-methyl-4-methoxyphenylalanine, N-methyl-4-thiazolealanine, N-methylhistidine, N-methylserine and N-methylaspartic acid.

Examples of a substituent containing a halogen atom include fluoro (-F), chloro (-Cl), bromo (-Br), and iodo (-I).

Examples of a substituent containing an O atom include hydroxyl (-OH), oxy (-OR), carbonyl (-C=O-R), carboxyl (-CO₂H), oxycarbonyl (-C=O-OR), carbonyloxy (-O-C=O-R), thiocarbonyl (-C=O-SR), carbonylthio (-S-C=O-R), aminocarbonyl (-C=O-NHR), carbonyl amino (-NH-C=O-R), oxycarbonyl amino (-NH-C=O-OR), sulfonyl amino (-NH-SO₂-R), aminosulfonyl (-S₂-NHR), sulfamoyl amino (-NH-SO₂-NHR), thiocarboxyl (-C(=O)-SH), carboxyl carbonyl (-C(=O)-CO₂H).

Examples of oxy (-OR) include alkoxy, cycloalkoxy, alkenyloxy, alkynyloxy, aryloxy, heteroaryloxy, and aralkyloxy.

Examples of carbonyl (-C=O-R) include formyl (-C=O-H), alkylcarbonyl, cycloalkylcarbonyl, alkenylcarbonyl, alkynylcarbonyl, arylcarbonyl, heteroarylcarbonyl, and aralkylcarbonyl.

Examples of oxycarbonyl (-C=O-OR) include alkyloxycarbonyl, cycloalkyloxycarbonyl, alkenyloxycarbonyl, alkynyloxycarbonyl, aryloxycarbonyl, heteroaryloxycarbonyl, and aralkyloxycarbonyl.

Examples of carbonyloxy (-O-C=O-R) include alkylcarbonyloxy, cycloalkylcarbonyloxy, alkenylcarbonyloxy, alkynylcarbonyloxy, arylcarbonyloxy, heteroarylcarbonyloxy, and aralkylcarbonyloxy.

Examples of thiocarbonyl (-C=O-SR) include alkylthiocarbonyl, cycloalkylthiocarbonyl, alkenylthiocarbonyl, alkynylthiocarbonyl, arylthiocarbonyl, heteroarylthiocarbonyl, and aralkylthiocarbonyl.

Examples of carbonylthio (-S-C=O-R) include alkylcarbonylthio, cycloalkylcarbonylthio, alkenylcarbonylthio, alkynylcarbonylthio, arylcarbonylthio, heteroarylcarbonylthio, and aralkylcarbonylthio.

Examples of aminocarbonyl (-C=O-NHR) include alkylaminocarbonyl, cycloalkylaminocarbonyl, alkenylaminocarbonyl, alkynylaminocarbonyl, arylaminocarbonyl, heteroarylaminocarbonyl, and aralkylaminocarbonyl. Furthermore, the H atom attached to the N atom in -C=O-NHR may be substituted with a substituent selected from the group consisting of alkyl, cycloalkyl, alkenyl, alkynyl, aryl, heteroaryl, and aralkyl.

Examples of carbonylamino (-NH-C=O-R) include alkylcarbonylamino, cycloalkylcarbonylamino, alkenylcarbonylamino, alkynylcarbonylamino, arylcarbonylamino, heteroarylcarbonylamino, and aralkylcarbonylamino. Furthermore, the H atom attached to the N atom in -NH-C=0-R may be substituted with a substituent selected from the group consisting of alkyl, cycloalkyl, alkenyl, alkynyl, aryl, heteroaryl, and aralkyl.

Examples of oxycarbonylamino (-NH-C=O-OR) include alkoxycarbonylamino, cycloalkoxycarbonylamino, alkenyloxycarbonylamino, alkynyloxycarbonylamino, aryloxycarbonylamino, heteroaryloxycarbonylamino, and aralkyloxycarbonylamino. Furthermore, the H atom attached to the N atom in -NH-C=O-OR may be substituted with a substituent selected from the group consisting of alkyl, cycloalkyl, alkenyl, alkynyl, aryl, heteroaryl, and aralkyl.

Examples of sulfonylamino (-NH-SO₂-R) include alkylsulfonylamino, cycloalkylsulfonylamino, alkenylsulfonylamino, alkynylsulfonylamino, arylsulfonylamino, heteroarylsulfonylamino, and aralkylsulfonylamino. Furthermore, the H atom attached to the N atom in -NH-SO₂-R may be substituted with a substituent selected from the group consisting of alkyl, cycloalkyl, alkenyl, alkynyl, aryl, heteroaryl, and aralkyl.

Examples of aminosulfonyl (-SO₂-NHR) include alkylaminosulfonyl, cycloalkylaminosulfonyl, alkenylaminosulfonyl, alkynylaminosulfonyl, arylaminosulfonyl, heteroarylaminosulfonyl, and aralkylaminosulfonyl. Furthermore, the H atom attached to the N atom in -SO₂-NHR may be substituted with a substituent selected from the group consisting of alkyl, cycloalkyl, alkenyl, alkynyl, aryl, heteroaryl, and aralkyl.

Examples of sulfamoylamino (-NH-SO₂-NHR) include alkylsulfamoylamino, cycloalkylsulfamoylamino, alkenylsulfamoylamino, alkynylsulfamoylamino, arylsulfamoylamino, heteroarylsulfamoylamino, and aralkylsulfamoylamino. Furthermore, at least one of the two H atoms attached to the N atoms in -NH-SO₂-NHR may be substituted with a substituent selected from the group consisting of alkyl, cycloalkyl, alkenyl, alkynyl, aryl, heteroaryl, and aralkyl. When the two H atoms are both substituted, a substituent may each be independently selected, or these two substituents may form a ring.

Examples of a substituent containing an S atom include thiol (-SH), thio (-S-R), sulfinyl (-S=O-R), sulfonyl (-S(O)₂-R), and sulfo (-SO₃H).

Examples of thio (-S-R) include alkylthio, cycloalkylthio, alkenylthio, alkynylthio, arylthiol, heteroarylthio, and aralkylthio.

Examples of sulfinyl (-S=O-R) include alkylsulfinyl, cycloalkylsulfinyl, alkenylsulfinyl, alkynylsulfinyl, arylsulfinyl, heteroarylsulfinyl, and aralkylsulfinyl.

Examples of sulfonyl (-S(O)₂-R) include alkylsulfonyl, cycloalkylsulfonyl, alkenylsulfonyl, alkynylsulfonyl, arylsulfonyl, heteroarylsulfonyl, and aralkylsulfonyl.

Examples of a substituent containing an N atom include azide (-N₃), cyano (-CN), primary amino (-NH₂), secondary amino (-NH-R), tertiary amino (-NR(R')), amidino (-C(=NH)-NH₂), substituted amidino (-C(=NR)-NR'R"), guanidino (-NH=C(=NH)-NH₂), substituted guanidino (-NR-C(=NR"')-NR'R"), and aminocarbonylamino (-NR-CO-NR'R").

Examples of the secondary amino (-NH-R) include alkylamino, cycloalkylamino, alkenylamino, alkynylamino, arylamino, heteroarylamino, and aralkylamino.

The two substituents R and R' on the N atom in the tertiary amino (-NR(R')) can each be independently selected from the group consisting of alkyl, cycloalkyl, alkenyl, alkynyl, aryl, heteroaryl, and aralkyl. Examples of the tertiary amino include, for example, alkyl(aralkyl)amino. These two substituents may form a ring.

The three substituents R, R', and R" on the N atom in the substituted amidino (-C(=NR)-NR'R") can each be independently selected from the group consisting of a hydrogen atom, alkyl, cycloalkyl, alkenyl, alkynyl, aryl, heteroaryl, and aralkyl. Examples of the substituted amidino include alkyl(aralkyl)(aryl)amidino. These substituents may together form a ring.

The four substituents R, R', R", and R'" on the N atom in the substituted guanidino (-NR-C(=NR"')-NR'R") can each be independently selected from the group consisting of a hydrogen atom, alkyl, cycloalkyl, alkenyl, alkynyl, aryl, heteroaryl, and aralkyl. These substituents may together form a ring.

The three substituents R, R', and R" on the N atom in the aminocarbonylamino (-NR-CO-NR'R") can each be independently selected from the group consisting of a hydrogen atom, alkyl, cycloalkyl, alkenyl, alkynyl, aryl, heteroaryl, and aralkyl. These substituents may together form a ring.

Examples of a substituent containing a B atom include boryl (-BR(R')) and dioxyboryl (-B(OR)(OR')). The two substituents R and R' on the B atom can each be independently selected from the group consisting of a hydrogen atom, alkyl, cycloalkyl, alkenyl, alkynyl, aryl, heteroaryl, and aralkyl. These substituents may together form a ring.

Examples of amino acid analogs in the present disclosure include hydroxycarboxylic acid (hydroxy acid). The hydroxycarboxylic acid includes α-hydroxycarboxylic acid, β-hydroxycarboxylic acid, and γ-hydroxycarboxylic acid. A side chain other than a hydrogen atom may be attached to the carbon at the α-position in the hydroxycarboxylic acid, as with amino acids. Regarding three-dimensional structures, both the L-type and D-type can be included. The structure of the side chain can be defined similarly to the side chain of the above-mentioned natural amino acid or unnatural amino acid. Examples of hydroxycarboxylic acids include hydroxyacetic acid, lactic acid, and phenyllactic acid.

The amino acid in the present disclosure may be a translatable amino acid, and the amino acid analog may be a translatable amino acid analog. As used herein, a "translatable" amino acid or amino acid analog (may be collectively referred to as an amino acid or the like) means amino acids and the like that can be incorporated into a peptide by translational synthesis (for example, using the translation system described in this disclosure). Whether a certain amino acid or the like is translatable can be confirmed by a translation synthesis experiment using a tRNA to which the amino acid or the like is attached. A reconstituted cell-free translation system may be used in the translation synthesis experiment (see for example, WO2013100132).

The unnatural amino acid or amino acid analog according to the present disclosure can be prepared by a conventionally known chemical synthesis method, a synthesis method described in the later-discussed Examples, or a synthesis method similar thereto.

### <Preparation of tRNA>

A tRNA can be synthesized, for example, by preparing a DNA encoding a desired tRNA gene, then placing an appropriate promoter such as T7, T3, or SP6 upstream of the DNA, and performing a transcription reaction with the DNA as a template using an RNA polymerase adapted to each promoter. Furthermore, tRNA can also be prepared by purification from biological materials. For example, tRNA can be recovered by preparing an extract solution from a material containing tRNA such as cells, and adding thereto a probe containing a sequence complementary to the nucleic acid sequence of tRNA. In this case, the material for the preparation may be cells transformed with an expression vector capable of expressing a desired tRNA. Usually, tRNAs synthesized by in vitro transcription only contain four typical nucleosides: adenosine, guanosine, cytidine, and uridine. On the other hand, tRNAs synthesized in cells may contain modified nucleosides resulting from modification of the typical nucleosides. It is considered that a modified nucleoside (for example, lysidine) in a natural tRNA is specifically introduced into that tRNA by the action of an enzyme for that modification (for example, TilS) after the tRNA is synthesized by transcription. Alternatively, tRNA can also be prepared by a method in which fragments synthesized by transcription or chemically synthesized fragments or such as described in the Examples below are ligated by an enzymatic reaction.

Aminoacyl-tRNAs can also be prepared by chemical and/or biological synthesis methods. For example, an aminoacyl-tRNA can be synthesized using an aminoacyl-tRNA synthetase (ARS) to attach an amino acid to a tRNA. The amino acid may be either natural amino acid or unnatural amino acid as long as it can serve as a substrate for ARS. Alternatively, a natural amino acid may be attached to a tRNA and then chemically modified. Furthermore, as there are many reports that introducing an amino acid mutation into ARSs enhanced their action on unnatural amino acids (see for example, WO2006/135096, WO2007/061136, WO2007/103307, WO2008/001947, WO2010/141851, and WO2015/120287), such mutated ARSs may be used to attach an amino acid to tRNA. In addition to the method using ARSs, aminoacyl-tRNAs can be synthesized by, for example, removing the CA sequence from the 3' end of tRNA, and ligating an aminoacylated pdCpA (a dinucleotide composed of deoxycytidine and adenosine) to it using RNA ligase (pdCpA method; Hecht et al., J Biol Chem (1978) 253: 4517-4520). A method using pCpA (a dinucleotide composed of cytidine and adenosine) instead of pdCpA is also known (pCpA method; Wang et al., ACS Chem Biol (2015)10: 2187-2192). Furthermore, aminoacyl-tRNAs can also be synthesized by attaching an unnatural amino acid previously activated by esterification to a tRNA, using an artificial RNA catalyst (flexizyme) (WO2007/066627).

### <Translation system>

In one aspect, the present disclosure provides a set of tRNAs suitable for peptide translation. A set of tRNAs contains a plurality of different tRNAs, and a plurality of different amino acids can be translated from those tRNAs. In one aspect, the present disclosure provides compositions comprising a plurality of different tRNAs suitable for peptide translation. In another aspect, the present disclosure provides methods of translating a peptide, comprising providing a plurality of different tRNAs suitable for peptide translation. In one aspect, the present disclosure provides translation systems comprising a plurality of different tRNAs suitable for peptide translation. In certain aspects, the plurality of different tRNAs mentioned above include a mutated tRNA of the present disclosure. The following description relates to these tRNAs, compositions, translation methods, and translation systems suitable for peptide translation.

In one embodiment, the mutated tRNA in the present disclosure has any one of lysidine (k2C), a lysidine derivative, agmatidine (agm2C), or an agmatidine derivative at the first letter (N₁) of the anticodon. Since lysidine and agmatidine form complementary base pairs with adenosine (A), their role in the codon may correspond to that of uridine (U). In some embodiments, the mutated tRNA of the present disclosure can translate a codon represented by M₁M₂A selectively over other codons. The other codons may be codons different from the codon represented by M₁M₂A; for example, a codon represented by M₁M₂U, M₁M₂C, or M₁M₂G. In certain embodiments, the mutated tRNA of the present disclosure can translate a codon represented by M₁M₂A selectively over all of the codons represented by M₁M₂U, M₁M₂C, and M₁M₂G.

In one embodiment of the present disclosure, "a mutated tRNA can translate the M₁M₂A codon selectively" means that [the amount of translation on the M₁M₂A codon by the tRNA] is, for example, not less than twice, not less than 3 times, not less than 4 times, not less than 5 times, not less than 6 times, not less than 7 times, not less than 8 times, not less than 9 times, not less than 10 times, not less than 15 times, not less than 20 times, not less than 30 times, not less than 40 times, not less than 50 times, not less than 60 times, not less than 70 times, not less than 80 times, not less than 90 times, or not less than 100 times [the amount of translation on another codon by the tRNA]. As an example, whether or not a certain mutated tRNA can selectively translate the codon represented by CUA can be judged by whether [the amount of translation on the CUA codon by the tRNA] is, for example, not less than twice, not less than 3 times, not less than 4 times, not less than 5 times, not less than 6 times, not less than 7 times, not less than 8 times, not less than 9 times, not less than 10 times, not less than 15 times, not less than 20 times, not less than 30 times, not less than 40 times, not less than 50 times, not less than 60 times, not less than 70 times, not less than 80 times, not less than 90 times, or not less than 100 times [the amount of translation on the CUG codon by the tRNA].

Comparing the amount of translation of a specific codon (for example, M₁M₂A) and the amount of translation of another codon (for example, M₁M₂G) can be carried out by, for example, preparing a peptide-encoding mRNA that contains a M₁M₂A codon and another mRNA having the same nucleic acid sequence as the aforementioned mRNA except that the M₁M₂A codon has been replaced with a M₁M₂G codon, translating those two mRNAs under the same conditions, and comparing the amounts of two synthesized peptides obtained.

In another embodiment of the present disclosure, "a mutated tRNA is capable of selectively translating the M₁M₂A codon" means that [the amount of translation on the codon other than M₁M₂A by the tRNA] is decreased to, for example, not more than 1/2, not more than 1/3, not more than 1/4, not more than 1/5, not more than 1/6, not more than 1/7, not more than 1/8, not more than 1/9, not more than 1/10, not more than 1/15, not more than 1/20, not more than 1/30, not more than 1/40, not more than 1/50, not more than 1/60, not more than 1/70, not more than 1/80, not more than 1/90, or not more than 1/100 [the amount of translation on the codon other than M₁M₂A by a tRNA having a UN₂N₃ anticodon]. Here, the UN₂N₃ anticodon represents an anticodon in which the first letter (N₁) of the anticodon is uridine, and the second letter (N₂) and the third letter (N₃) of the anticodon are nucleosides complementary to M₂ and M₁, respectively. Since the roles of lysidine and agmatidine in anticodons correspond to uridine, uridine is selected here for comparison. Furthermore, the codon other than M₁M₂A can be any one of the codons represented by M₁M₂U, M₁M₂C, or M₁M₂G. As an example, whether or not a certain mutated tRNA can selectively translate the codon represented by CUA can be judged by whether [the amount of translation on the CUG codon by the tRNA] is decreased to, for example, not more than 1/2, not more than 1/3, not more than 1/4, not more than 1/5, not more than 1/6, not more than 1/7, not more than 1/8, not more than 1/9, not more than 1/10, not more than 1/15, not more than 1/20, not more than 1/30, not more than 1/40, not more than 1/50, not more than 1/60, not more than 1/70, not more than 1/80, not more than 1/90, or not more than 1/100 [the amount of translation on the CUG codon by a tRNA having the UN₂N₃ anticodon].

In another embodiment, a codon represented by M₁M₂A may be translated more selectively by a mutated tRNA of the present disclosure than by other tRNA. The other tRNA may be a tRNA capable of translating a codon different from the codon represented by M₁M₂A, for example, a tRNA capable of translating the M₁M₂U, M₁M₂C, or M₁M₂G codon. In a particular embodiment, the codon represented by M₁M₂A may be selectively translated by the mutated tRNA of the present disclosure than by all of the tRNAs capable of translating the M₁M₂U codon, the tRNAs capable of translating the M₁M₂C codon, and the tRNAs capable of translating the M₁M₂G codon.

In one embodiment of the present disclosure, "a codon represented by M₁M₂A may be selectively translated by a mutated tRNA" means that [the amount of translation on the M₁M₂A codon by the tRNA] is, for example, not less than twice, not less than 3 times, not less than 4 times, not less than 5 times, not less than 6 times, not less than 7 times, not less than 8 times, not less than 9 times, not less than 10 times, not less than 15 times, not less than 20 times, not less than 30 times, not less than 40 times, not less than 50 times, not less than 60 times, not less than 70 times, not less than 80 times, not less than 90 times, or not less than 100 times [the amount of translation on the M₁M₂A codon by other tRNAs]. As an example, whether or not the codon represented by CUA can be selectively translated by a certain mutated tRNA can be judged by whether [the amount of translation on the CUA codon by the tRNA] is, for example, not less than twice, not less than 3 times, not less than 4 times, not less than 5 times, not less than 6 times, not less than 7 times, not less than 8 times, not less than 9 times, not less than 10 times, not less than 15 times, not less than 20 times, not less than 30 times, not less than 40 times, not less than 50 times, not less than 60 times, not less than 70 times, not less than 80 times, not less than 90 times, or not less than 100 times [the amount of translation on the CUA codon by a tRNA capable of translating the CUG codon (for example, a tRNA having the CAG anticodon].

A translation system comprising the mutated tRNA of the present disclosure may have both of the above two characteristics. That is, in a particular embodiment, in the translation system of the present disclosure, (i) the mutated tRNA can translate the codon represented by M₁M₂A selectively over other codons, and (ii) the codon represented by M₁M₂A may be translated by the mutated tRNA of the present disclosure selectively over other tRNAs. When such a relationship is established, in the translation system of the present disclosure, the peptide translation using the mutated tRNA of the present disclosure and the peptide translation using other tRNAs are in an independent relationship where they do not interact with each other; in other words, an orthogonal relationship. The translation system of the organisms in nature essentially has strict correspondences established between codons and amino acids; therefore, addition of a non-orthogonal mutated tRNA to it may disturb these correspondences, and lead to a fatal effect on the function of the translation system. Therefore, in the translation system of the present disclosure, the orthogonality established between the mutated tRNA of the present disclosure and other tRNAs may be one of the important features.

In one embodiment, the translation system in the present disclosure further comprises a tRNA having an anticodon complementary to the codon represented by M₁M₂G (hereinafter, this tRNA is also referred to as "tRNA-G"). In some embodiments, the translation system in this disclosure comprises at least two tRNAs: (a) a mutated tRNA described in this disclosure and (b) a tRNA-G described in this disclosure. In a particular embodiment, the anticodon complementary to the codon represented by M₁M₂G is, for example, CN₂N₃, ac4CN₂N₃, or CmN₂N₃. Here, the nucleoside of the first letter of each anticodon is cytidine (C), N4-acetylcytidine (ac4C), or 2'-O-methylcytidine (Cm), and the nucleoside of the second letter (N₂) and the nucleoside of the third letter (N₃) are nucleosides complementary to the above-described M₂ and Mi, respectively. The mutated tRNA and tRNA-G described in the present disclosure may have the same nucleic acid sequence except for the anticodon, or may have different nucleic acid sequences. When the nucleic acid sequences other than the anticodon are the same, the physicochemical properties of these two tRNAs may be similar to each other; therefore, a translation system with more homogeneous and stable reactivity may be constructed.

In some embodiments, tRNA-G of the present disclosure can selectively translate the codons represented by M₁M₂G over other codons. The other codons may be codons different from the codons represented by M₁M₂G; for example, codons represented by M₁M₂U, M₁M₂C, or M₁M₂A. In certain embodiments, tRNA-G of the present disclosure can selectively translate a codon represented by M₁M₂G over any of the codons represented by M₁M₂U, M₁M₂C, and M₁M₂A.

In one embodiment of the present disclosure, a certain tRNA can selectively translate the M₁M₂G codon means that [the amount of translation on the M₁M₂G codon by the tRNA] is, for example, not less than twice, not less than 3 times, not less than 4 times, not less than 5 times, not less than 6 times, not less than 7 times, not less than 8 times, not less than 9 times, not less than 10 times, not less than 15 times, not less than 20 times, not less than 30 times, not less than 40 times, not less than 50 times, not less than 60 times, not less than 70 times, not less than 80 times, not less than 90 times, or not less than 100 times [the amount of translation on the other codons by the tRNA]. As an example, whether or not a certain mutated tRNA can selectively translate the codon represented by CUG can be judged by observing whether [the amount of translation on the CUG codon by the tRNA] is, for example, not less than twice, not less than 3 times, not less than 4 times, not less than 5 times, not less than 6 times, not less than 7 times, not less than 8 times, not less than 9 times, not less than 10 times, not less than 15 times, not less than 20 times, not less than 30 times, not less than 40 times, not less than 50 times, not less than 60 times, not less than 70 times, not less than 80 times, not less than 90 times, or not less than 100 times [the amount of translation on the CUA codon by the tRNA].

In another embodiment, the codon represented by M₁M₂G may be translated more selectively by a tRNA-G of the present disclosure than by other tRNAs. Other tRNAs may be tRNAs capable of translating codons different from the codons represented by M₁M₂G, for example, tRNAs capable of translating any one of the M₁M₂U, M₁M₂C, or M₁M₂A codons. In a particular embodiment, the codon represented by M₁M₂G may be selectively translated by the tRNA-Gs of the present disclosure than by any one of the tRNAs capable of translating the M₁M₂U codons, the tRNAs capable of translating the M₁M₂C codons, and the tRNAs capable of translating the M₁M₂A codons.

In one embodiment of the present disclosure, the codon represented by M₁M₂G may be selectively translated by a certain tRNA means that [the amount of translation on the M₁M₂G codon by the tRNA] is, for example, not less than twice, not less than 3 times, not less than 4 times, not less than 5 times, not less than 6 times, not less than 7 times, not less than 8 times, not less than 9 times, not less than 10 times, not less than 15 times, not less than 20 times, not less than 30 times, not less than 40 times, not less than 50 times, not less than 60 times, not less than 70 times, not less than 80 times, not less than 90 times, or not less than 100 times [the amount of translation on the M₁M₂G codon by other tRNAs]. As an example, whether or not the codon represented by CUG can be selectively translated by a certain tRNA can be judged by observing whether [the amount of translation on the CUG codon by the tRNA] is, for example, not less than twice, not less than 3 times, not less than 4 times, not less than 5 times, not less than 6 times, not less than 7 times, not less than 8 times, not less than 9 times, not less than 10 times, not less than 15 times, not less than 20 times, not less than 30 times, not less than 40 times, not less than 50 times, not less than 60 times, not less than 70 times, not less than 80 times, not less than 90 times, or not less than 100 times [the amount of translation on the CUG codon by a tRNA capable of translating the CUA codon (for example, a tRNA that has the k2CAG anticodon] .

A translation system comprising tRNA-G of the present disclosure may have the above two characteristics in combination. That is, in a particular embodiment, in the translation system of the present disclosure, (i) tRNA-G can selectively translate the codon represented by M₁M₂G over other codons, and (ii) the codon represented by M₁M₂G may be selectively translated by tRNA-G of the present disclosure over other tRNAs. When such a relationship is established, in the translation system of the present disclosure, the peptide translation using tRNA-G and the peptide translation using other tRNAs are independent and do not interact with each other; in other words, they have an orthogonal relationship. In the translation system of the present disclosure, establishment of orthogonality between tRNA-G and other tRNAs may be one of the important features.

In a further embodiment, an amino acid attached to the mutated tRNA (hereinafter, this amino acid is also referred to as "amino acid-A") and an amino acid attached to tRNA-G (hereinafter, this amino acid is also referred to as "amino acid-G") of the present disclosure may be different from one another. Regarding the mutated tRNA and tRNA-G in the present disclosure, when the above-mentioned orthogonal relationship is established, the M₁M₂A codon and amino acid-A, and the M₁M₂G codon and amino acid-G each have a one-to-one correspondence in the present translation system. That is, in the translation system of the present disclosure, two different amino acids can be translated from two codons, (i) M₁M₂A and (ii) M₁M₂G, in the same codon box.

In one embodiment, the translation system in the present disclosure further comprises a tRNA having an anticodon complementary to the codon represented by M₁M₂U or M₁M₂C (hereinafter, this tRNA is also referred to as "tRNA-U/C"). In some embodiments, the translation system in the present disclosure comprises at least three tRNAs, which are (a) a mutated tRNA described in this disclosure, (b) a tRNA-G described in this disclosure, and (c) a tRNA-U/C described in this disclosure. In a particular embodiment, the anticodon complementary to a codon represented by M₁M₂U is, for example, AN₂N₃, GN₂N₃, QN₂N₃, or GluQN₂N₃. Here, the nucleoside of the first letter of each anticodon is adenosine (A), guanosine (G), queuosine (Q), or glutamylqueuosine (GluQ), and the nucleoside of the second letter (N₂) and the nucleoside of the third letter (N₃) are nucleosides complementary to the above-described M₂ and Mi, respectively. In another embodiment, the anticodon complementary to a codon represented by M₁M₂C is, for example, GN₂N₃, QN₂N₃, or GluQN₂N₃. Since many of the anticodons complementary to the M₁M₂U and M₁M₂C codons overlap with each other, these two codons may be treated as a single codon in the present disclosure. In a particular embodiment, the anticodon complementary to the codon represented by M₁M₂U or M₁M₂C is, for example, AN₂N₃, GN₂N₃, QN₂N₃, or GluQN₂N₃. The mutated tRNA, tRNA-G, and tRNA-U/C described in the present disclosure may have the same nucleic acid sequence except for the anticodon, or they may have different nucleic acid sequences from each other. When the nucleic acid sequences other than the anticodon are the same, the physicochemical properties of these three tRNAs may be similar to each other; therefore, a translation system with more homogeneous and stable reactivity may be constructed.

In some embodiments, tRNA-U/C of the present disclosure can selectively translate the codons represented by M₁M₂U or M₁M₂C over other codons. The other codons may be codons different from the codons represented by M₁M₂U or M₁M₂C; for example, they may be codons represented by M₁M₂A or M₁M₂G. In specific embodiments, tRNA-U/C of the present disclosure can selectively translate a codon represented by M₁M₂U or M₁M₂C over the codons represented by M₁M₂A and M₁M₂G.

In one embodiment of the present disclosure, a certain tRNA can selectively translate the M₁M₂U or M₁M₂C codon means that [the amount of translation on the M₁M₂U or M₁M₂C codon by the tRNA] is, for example, not less than twice, not less than 3 times, not less than 4 times, not less than 5 times, not less than 6 times, not less than 7 times, not less than 8 times, not less than 9 times, not less than 10 times, not less than 15 times, not less than 20 times, not less than 30 times, not less than 40 times, not less than 50 times, not less than 60 times, not less than 70 times, not less than 80 times, not less than 90 times, or not less than 100 times [the amount of translation on the other codons by the tRNA]. As an example, whether or not a certain tRNA can selectively translate the codon represented by CUU or CUC can be judged by observing whether [the amount of translation on the CUU or CUC codon by the tRNA] is, for example, not less than twice, not less than 3 times, not less than 4 times, not less than 5 times, not less than 6 times, not less than 7 times, not less than 8 times, not less than 9 times, not less than 10 times, not less than 15 times, not less than 20 times, not less than 30 times, not less than 40 times, not less than 50 times, not less than 60 times, not less than 70 times, not less than 80 times, not less than 90 times, or not less than 100 times [the amount of translation on the CUA codon by the tRNA].

In another embodiment, the codon represented by M₁M₂U or M₁M₂C may be translated more selectively by a tRNA-U/C of the present disclosure than by other tRNAs. Other tRNAs may be tRNAs capable of translating codons different from the codons represented by M₁M₂U or M₁M₂C, for example, tRNAs capable of translating any one of the M₁M₂A or M₁M₂G codons. In a particular embodiment, the codon represented by M₁M₂U or M₁M₂C may be selectively translated by the tRNA-U/Cs of the present disclosure than by any of the tRNAs capable of translating the M₁M₂A codons and the tRNAs capable of translating the M₁M₂G codons.

In one embodiment of the present disclosure, the codon represented by M₁M₂U or M₁M₂C may be selectively translated by a certain tRNA means that [the amount of translation on the M₁M₂U or M₁M₂C codon by the tRNA] is, for example, not less than twice, not less than 3 times, not less than 4 times, not less than 5 times, not less than 6 times, not less than 7 times, not less than 8 times, not less than 9 times, not less than 10 times, not less than 15 times, not less than 20 times, not less than 30 times, not less than 40 times, not less than 50 times, not less than 60 times, not less than 70 times, not less than 80 times, not less than 90 times, or not less than 100 times [the amount of translation on the M₁M₂U or M₁M₂C codon by other tRNAs]. As an example, whether or not the codon represented by CUU or CUC can be selectively translated by a certain tRNA can be judged by observing whether [the amount of translation on the CUU or CUC codon by the tRNA] is, for example, not less than twice, not less than 3 times, not less than 4 times, not less than 5 times, not less than 6 times, not less than 7 times, not less than 8 times, not less than 9 times, not less than 10 times, not less than 15 times, not less than 20 times, not less than 30 times, not less than 40 times, not less than 50 times, not less than 60 times, not less than 70 times, not less than 80 times, not less than 90 times, or not less than 100 times [the amount of translation on the CUU or CUC codon by a tRNA capable of translating the CUA codon (for example, a tRNA that has the k2CAG anticodon].

A translation system comprising tRNA-U/C of the present disclosure may have the above two characteristics in combination. That is, in a particular embodiment, in the translation system of the present disclosure, (i) tRNA-U/C can selectively translate the codon represented by M₁M₂U or M₁M₂C over other codons, and (ii) the codon represented by M₁M₂U or M₁M₂C may be selectively translated by tRNA-U/C of the present disclosure over other tRNAs. When such a relationship is established, in the translation system of the present disclosure, the peptide translation using tRNA-U/C and the peptide translation using other tRNAs are independent and do not interact with each other; in other words, they have an orthogonal relationship. In the translation system of the present disclosure, establishment of orthogonality between tRNA-U/C and other tRNAs may be one of the important features.

In a further embodiment, an amino acid attached to the mutated tRNA ("amino acid-A"), an amino acid attached to tRNA-G ("amino acid-G"), and an amino acid attached to tRNA-U/C (hereinafter, this amino acid is referred to as "amino acid-U/C") of the present disclosure may be different from one another. Regarding the mutated tRNA, tRNA-G, and tRNA-U/C in the present disclosure, when the above-mentioned orthogonal relationship is established, the M₁M₂A codon and amino acid-A, the M₁M₂G codon and amino acid-G, and the M₁M₂U or M₁M₂C codon and amino acid-U/C each have a one-to-one correspondence in the present translation system. That is, in the translation system of the present disclosure, three different amino acids can be translated from three codons, (i) M₁M₂A, (ii) M₁M₂G, and (iii) M₁M₂U or M₁M₂C in the same codon box. Alternatively, in the translation system of the present disclosure, three different amino acids can be translated from a codon box composed of M₁M₂U, M₁M₂C, M₁M₂A, and M₁M₂G.

In some embodiments, an unnatural amino acid may be attached to at least one of the mutated tRNA, tRNA-G, and tRNA-U/C of the present disclosure.

In some embodiments, the mutated tRNAs of the present disclosure may be assigned to codons that constitute at least one codon box in the genetic code table. In a further embodiment, the mutated tRNAs of the present disclosure may be assigned to codons that constitute multiple codon boxes in the genetic code table. The multiple codon boxes may be, for example, 2, 3, 4, 5, 6, 7, 8, 9, 10, 11, 12, 13, 14, 15, or 16 codon boxes. In addition to the mutated tRNA, tRNA-G may be assigned to other codons (a codon different from the codon to which the mutated tRNA is assigned) that constitute the same codon box, or tRNA-U/C may be assigned to other codons (codons different from the codon to which the mutated tRNA is assigned and the codon to which tRNA-G is assigned) that constitute the same codon box. To which codon box-constituting codon each tRNA will be assigned is determined by the nucleoside of the second letter (N₂) and the nucleoside of the third letter (N₃) of the anticodon carried by the tRNA. The tRNAs assigned to codons that constitute different codon boxes have different N₂ and N₃. Further, the tRNAs assigned to the codons constituting different codon boxes may have the same nucleic acid sequence except for the anticodon, or they may have different nucleic acid sequences from each other. When the nucleic acid sequences other than the anticodon are the same, the physicochemical properties of these tRNAs may be similar to each other; therefore, a translation system with more homogeneous and stable reactivity may be constructed.

In some embodiments, one, two, three, four, five, six, seven, eight, nine, ten, eleven, twelve, thirteen, 14, 15, 16, 17, 18, 19, or 20 kinds of amino acids can be translated from the translation system of the present disclosure. Alternatively, more than 20 amino acids can be translated by discriminating the M₁M₂A and M₁M₂G codons in a single codon box using the mutated tRNA of the present disclosure. In a further embodiment, for example, 21, 22, 23, 24, 25, 26, 27, 28, 29, 30, 31, 32, 33, 34, 35, 36, 37, 38, 39, 40, 41, 42, 43, 44, 45, 46, 47, or 48 amino acids can be translated from the translation system of the present disclosure.

In some embodiments, the translation system of the present disclosure is a cell-free translation system. In a further embodiment, the translation system of the present disclosure is a reconstituted cell-free translation system. As the cell extract solution in the cell-free translation system and the factors required for peptide translation (for example, ribosome), those derived from various biological materials can be used. Examples of such biological materials include E. *coli*, yeast, wheat germ, rabbit reticulocytes, HeLa cells, and insect cells.

In one aspect, the present disclosure provides a method for producing a peptide, comprising translating a nucleic acid using the translation system described in the present disclosure. The peptides of this disclosure may include compounds in which two or more amino acids are linked by an amide bond in. In addition, the peptides of this disclosure may also include a compound in which amino acid analogs such as hydroxycarboxylic acid instead of amino acids are linked by an ester bond. The number of amino acids or amino acid analogs contained in the peptide is not particularly limited as long as it is 2 or more, for example, 2 or more, 3 or more, 4 or more, 5 or more, 6 or more, 7 or more, 8 or more, 9 or more, 10 or more, or 11 or more, and also 100 or less, 80 or less, 50 or less, 30 or less, 25 or less, 20 or less, 19 or less, 18 or less, 17 or less, 16 or less, 15 or less, 14 or less, 13 or less, or 12 or less. Alternatively, the number can be selected from 9, 10, 11, and 12.

In one aspect, the peptide of the present disclosure may contain N-substituted amino acids, and the number of N-substituted amino acids contained in the peptide may be, for example, 2, 3, 4, 5, 6, 6, 7, 8, 9, or 10. In another embodiment, the peptide of the present disclosure may contain amino acids that are not N-substituted, and the number of N-unsubstituted amino acids may be, for example, 1, 2, 3, or 4. In a further embodiment, peptides of the present disclosure may contain both N-substituted and N-unsubstituted amino acids.

In some embodiments, the peptide of the present disclosure may be a linear peptide or a peptide comprising a cyclic portion. A peptide comprising a cyclic portion means a peptide in which the main chain or side chain of an amino acid or amino acid analog existing on a peptide chain is attached to the main chain or side chain of another amino acid or amino acid analog existing on the same peptide chain to form a cyclic structure in the molecule. The peptide having a cyclic portion may be composed of only a cyclic portion, or may contain both a cyclic portion and a linear portion. The number of amino acids or amino acid analogs contained in the cyclic portion is, for example, 4 or more, 5 or more, 6 or more, 7 or more, 8 or more, or 9 or more, and 14 or less, 13 or less, 12 or less, or 11 or less. Alternatively, the number can be selected from 9, 10, and 11. The number of amino acids or amino acid analogs contained in the linear portion is, for example, 0 or more, and may be 8 or less, 7 or less, 6 or less, 5 or less, or 4 or less. Alternatively, the number can be selected from 0, 1, 2, and 3.

As the bond for forming the cyclic portion, for example, a peptide bond formed from an amino group and a carboxyl group can be used. In addition, an amide bond, disulfide bond, ether bond, thioether bond, ester bond, thioester bond, carbon-carbon bond, alkyl bond, alkenyl bond, phosphonate ether bond, azo bond, amine bond, C=N-C bond, lactam bridge, carbamoyl bond, urea bond, thiourea bond, thioamide bond, sulfinyl bond, sulfonyl bond, triazole bond, benzoxazole bond, and such formed from a combination of appropriate functional groups can be used. The carbon-carbon bond can be formed by a transition metal-catalyzed reaction such as a Suzuki reaction, a Heck reaction, and a Sonogashira reaction. In one embodiment, the peptides of the present disclosure contain at least one set of functional groups capable of forming the above-mentioned bond in the molecule. The formation of the cyclic portion may be performed by producing a linear peptide using the translation system of the present disclosure and then separately performing a reaction for linking the above-mentioned functional groups with each other. Regarding the synthesis of the peptide having a cyclic portion, one can refer to WO2013/100132, WO2012/026566, WO2012/033154, WO2012/074130, WO2015/030014, WO2018/052002, Comb Chem High Throughput Screen (2010)13: 75-87, Nat Chem Biol (2009) 5: 502-507, Nat Chem Biol (2009) 5: 888-90, Bioconjug Chem (2007) 18: 469-476, Chem Bio Chem (2009) 10: 787-798, Chem. Commun. (Camb) (2011) 47: 9946-9958, and such.

In some embodiments, the nucleic acid translated in the translation system of the present disclosure is mRNA. A peptide having a desired amino acid sequence may be encoded in an mRNA. By adding an mRNA to the translation system of the present disclosure, the mRNA can be translated into a peptide. On the other hand, when an RNA polymerase for transcribing DNA into mRNA is contained in the translation system, by adding the DNA to the translation system of the present disclosure, transcription of the DNA into mRNA can be performed in conjunction with translation of the mRNA into a peptide.

Methionine is usually present at the N-terminal of the translated peptide as an initiator amino acid, but some methods for introducing an amino acid other than methionine to the N-terminus have been reported. They may be used in combination with the methods for producing a peptide described in the present disclosure. Examples of such a method include a method of translating a peptide starting from a desired amino acid, using an initiator tRNA which is aminoacylated with an amino acid other than methionine (initiation suppression). Particularly, the degree to which an exogenous amino acid may be tolerated is higher at the time of translation initiation than at the time of peptide chain elongation; therefore, at the N-terminal, even an amino acid having a structure largely different from that of a natural amino acid may be used (Goto & Suga, J Am Chem Soc (2009)131(14):5040-5041). Another method includes, for example, a method of translating a peptide starting from the second or subsequent codon by removing the initiator methionyl tRNA from the translation system or by replacing the initiator amino acid with an amino acid having low translation efficiency other than methionine (initiation read-through; skipping the start codon). Another method includes, for example, removing methionine at the N-terminus of the peptide by allowing enzymes such as peptide deformylase and methionine aminopeptidase to act (Meinnel et al., Biochimie (1993) 75: 1061-1075). A library of peptides starting from methionine is prepared, and the above enzyme is made to act on the peptide library to prepare a library of peptides starting from a random amino acid at N-terminus.

In another aspect, the present disclosure provides a peptide produced by the method for producing a peptide described in the present disclosure. Peptides obtained by further chemically modifying the peptide produced by the method described in the present disclosure are also included in the peptides provided by the present disclosure.

In one aspect, the present disclosure provides a method for producing a peptide library, comprising translating a nucleic acid library using the translation system described in the present disclosure. By preparing a plurality of nucleic acid molecules each encoding a peptide and rich in nucleic acid sequence diversity, and then translating each of them into a peptide, a plurality of peptide molecules rich in amino acid sequence diversity can be produced. The size of the library is not particularly limited, and may be, for example, 10⁶ or more, 10⁷ or more, 10⁸ or more, 10⁹ or more, 10¹⁰ or more, 10¹¹ or more, 10¹² or more, 10¹³ or more, or 10¹⁴ or more. The nucleic acid may be DNA or RNA. RNA is usually mRNA. DNA is translated into a peptide via transcription into mRNA. Such a nucleic acid library can be prepared by a method known to those skilled in the art or a similar method. By using a mixed base at a desired position when synthesizing a nucleic acid library, a plurality of nucleic acid molecules rich in nucleic acid sequence diversity can be easily prepared. Examples of codons using mixed bases are, for example, NNN (where N represents a mixture of 4 bases, A, T, G, and C), NNW (where W represents a mixture of 2 bases, A and T), NNM (where W represents a mixture of two bases, A and C), NNK (where K represents a mixture of two bases, G and T), and NNS (where S represents a mixture of two bases, C and G). Alternatively, by limiting the base used in the third letter of the codon to any one of A, T, G, and C, a nucleic acid library in which only some specific amino acids are encoded can be synthesized. Furthermore, when a codon containing mixed bases is prepared, it is possible to arbitrarily adjust the appearance frequency of amino acids obtainable from the codon by mixing a plurality of bases at different ratios rather than in equal proportions. By taking a codon such as that mentioned above as one unit to prepare a plurality of different codon units, and then linking them in the desired order, a library in which the appearance position and appearance frequency of the contained amino acids are controlled can be designed.

In some embodiments, the peptide library described in the present disclosure is a library in which peptides are displayed on nucleic acids (nucleic acid display library, or simply, display library). A display library is a library in which a phenotype and a genotype are associated with each other as a result of formation of a single complex by linking a peptide to a nucleic acid encoding that peptide. Examples of major display libraries include libraries prepared by the mRNA display method (Roberts and Szostak, Proc Natl Acad Sci USA (1997) 94: 12297-12302), in vitro virus method (Nemoto et al., FEBS Lett (1997) 414: 405-408), cDNA display method (Yamaguchi et al., Nucleic Acids Res (2009) 37: e108), ribosome display method (Mattheakis et al, Proc Natl Acad Sci USA (1994) 91: 9022-9026), covalent display method (Reiersen et.al., Nucleic Acids Res (2005) 33: e10), CIS display method (Odegrip et.al., Proc Natl Acad Sci USA (2004) 101: 2806-2810), and such. Alternatively, a library prepared by using the in vitro compartmentalization method (Tawfik and Griffiths, Nat Biotechnol (1998) 16: 652-656) can be mentioned as one embodiment of the display library.

In another aspect, the present disclosure provides a peptide library produced by the method for producing a peptide library described in the present disclosure.

In one aspect, the present disclosure provides a method for identifying a peptide having binding activity to a target molecule, which comprises contacting the target molecule with a peptide library described in the present disclosure. The target molecule is not particularly limited and can be appropriately selected from, for example, low molecular weight compounds, high molecular weight compounds, nucleic acids, peptides, proteins, sugars, and lipids. The target molecule may be a molecule existing outside the cell or a molecule existing inside the cell. Alternatively, it may be a molecule existing in the cell membrane, in which case any of the extracellular domain, the transmembrane domain, and the intracellular domain may be the target. In the step of contacting the target molecule with the peptide library, the target molecule is usually immobilized on some kind of solid-phase carrier (for example, a microtiter plate or microbeads). Then, by removing the peptides not attached to the target molecule and recovering only the peptides attached to the target molecule, the peptides having binding activity to the target molecule can be selectively concentrated (panning method). When the peptide library used is a nucleic acid display library, the recovered peptides have the nucleic acid encoding their respective genetic information attached to them; therefore, the nucleic acid sequence encoding the recovered peptide and the amino acid sequence can be readily identified by isolating and analyzing them. Furthermore, based on the obtained nucleic acid sequence or amino acid sequence, the identified peptides can be individually produced by chemical synthesis or gene recombination techniques.

In one aspect, the present disclosure provides a nucleic acid-peptide complex comprising a peptide and a nucleic acid encoding the peptide, wherein the complex has the following features:
(i) the nucleic acid sequence encoding the peptide comprises two codons, M₁M₂A and M₁M₂G; and
(ii) in the amino acid sequence of the peptide, the amino acids corresponding to the M₁M₂A codon and the amino acids corresponding to the M₁M₂G codon are different from one another.

Here, M₁ and M₂ represent the first and the second letters of a specific codon, respectively (however, the codons in which M₁ is A and M₂ is U are excluded).

In a further aspect, the present disclosure provides a nucleic acid-peptide complex comprising a peptide and a nucleic acid encoding the peptide, wherein the complex has the following features:
(i) the nucleic acid sequence encoding the peptide comprises three codons, M₁M₂U, M₁M₂A, and M₁M₂G; and
(ii) in the amino acid sequence of the peptide, the amino acid corresponding to the M₁M₂U codon, the amino acid corresponding to the M₁M₂A codon, and the amino acid corresponding to the M₁M₂G codon are all different from each other.

Here, M₁ and M₂ represent the first and second letters of a specific codon, respectively.

In another aspect, the present disclosure provides a nucleic acid-peptide complex comprising a peptide and a nucleic acid encoding the peptide, wherein the complex has the following features:
(i) the nucleic acid sequence encoding the peptide comprises three codons, M₁M₂C, M₁M₂A, and M₁M₂G; and
(ii) in the amino acid sequence of the peptide, the amino acid corresponding to the M₁M₂C codon, the amino acid corresponding to the M₁M₂A codon, and the amino acid corresponding to the M₁M₂G codon are all different from each other.

Here, M₁ and M₂ represent the first and second letters of a specific codon, respectively.

In some embodiments, the nucleic acid-peptide complex described above may be contained in a peptide library as one of the elements constituting the library (particularly a nucleic acid display library). In one aspect, the present disclosure provides a library (a peptide library or a nucleic acid display library) comprising the nucleic acid-peptide complex described in the present disclosure. In certain embodiments, the nucleic acid-peptide complexes and libraries described above may be prepared using the mutated tRNA described in this disclosure or the translation system described in this disclosure.

In one aspect, the present disclosure provides the following compounds, *i.e*., lysidine-diphosphate (pLp), or salts thereof.

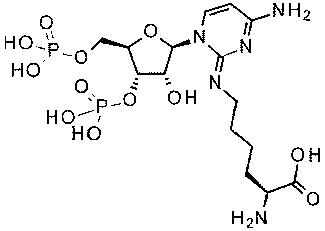

Such a compound can be used for preparing a mutated tRNA into which lysidine is introduced. Accordingly, the present disclosure relates to a method for producing a mutated tRNA into which lysidine is introduced using lysidine-diphosphate, and a mutated tRNA produced by the method. The present disclosure also relates to a method for producing a mutated tRNA into which a lysidine is introduced using lysidine-diphosphate, wherein the mutated tRNA has an amino acid or an amino acid analog attached to it (aminoacyl mutated tRNA), and an aminoacyl mutated tRNA produced by the method. Such mutated tRNA and/or aminoacyl mutated tRNA can be used in the translation system in the present disclosure. Accordingly, the present disclosure relates to translation systems comprising such mutated tRNAs and/or aminoacyl mutated tRNAs. The present disclosure also provides methods for producing peptides or peptide libraries using the translation system. The present disclosure also provides peptides or peptide libraries produced by the method.

In the present disclosure, lysidine may be introduced at position 34 of tRNA (based on tRNA numbering rules). In one embodiment, a mutated tRNA in which lysidine is introduced at position 34 according to the tRNA numbering rule can be obtained by preparing one or more (for example, 2, 3, 4, 5, or more) tRNA nucleic acid fragments and lysidine-diphosphate, and ligating them by a method known to those skilled in the art. Specifically, as an example, a nucleic acid fragment consisting of bases at positions 1 to 33 of tRNA, lysidine-diphosphate, and the nucleic acid fragment consisting of bases at positions 35 to 76 of tRNA (or positions 35 to 75 of tRNA, or positions 35 to 74 of tRNA) are ligated in this order from the 5' side. The CA sequence at the 3' end may be removed.

In one aspect, the present disclosure provides the following compound, *i.e*., agmatidine-diphosphate (p(Agm)p), or salts thereof.

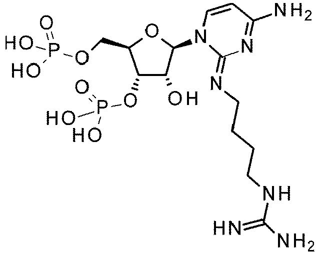

Such a compound can be used for preparing a mutated tRNA into which agmatidine is introduced. Accordingly, the present disclosure relates to a method for producing a mutated tRNA into which agmatidine is introduced using agmatidine-diphosphate, and a mutated tRNA produced by the method. The present disclosure also relates to a method for producing an agmatidine-introduced mutated tRNA using agmatidine-diphosphate, wherein the mutated tRNA has an amino acid or an amino acid analog attached to it (aminoacyl mutated tRNA), and an aminoacyl mutated tRNA produced by the method. Such mutated tRNA and/or aminoacyl mutated tRNA can be used in the translation system in the present disclosure. Accordingly, the present disclosure relates to translation systems comprising such mutated tRNAs and/or aminoacyl mutated tRNAs. The present disclosure also provides methods for producing peptides or peptide libraries using the translation system. The present disclosure also provides peptides or peptide libraries produced by the method.

In the present disclosure, agmatidine may be introduced at position 34 of tRNA (based on tRNA numbering rules). In one embodiment, a mutated tRNA in which agmatidine is introduced at position 34 according to the tRNA numbering rule can be obtained by preparing one or more (for example, 2, 3, 4, 5, or more) tRNA nucleic acid fragments and agmatidine-diphosphate, and ligating them by a method known to those skilled in the art. Specifically, as an example, a nucleic acid fragment consisting of bases at positions 1 to 33 of tRNA, agmatidine-diphosphate, and the nucleic acid fragment consisting of bases at positions 35 to 76 of tRNA (or positions 35 to 75 of tRNA, or positions 35 to 74 of tRNA) are ligated in this order from the 5' side. The CA sequence at the 3' end may be removed.

The compound of the present disclosure can be a free body or a salt. Examples of the salts of compounds of the present disclosure include the following: hydrochloride; hydrobromide; hydroiodide; phosphate; phosphonate; sulfate; sulfonates such as methanesulfonate, and p-toluenesulfonate; carboxylates such as acetate, citrate, malate, tartrate, succinate, and salicylate; alkali metal salts such as sodium salt and potassium salt; alkaline earth metal salts such as magnesium salt and calcium salt; and ammonium salts such as ammonium salt, alkylammonium salt, dialkylammonium salt, trialkylammonium salt, and tetraalkylammonium salt. The salt of the compound of the present disclosure is produced, for example, by contacting the compound of the present disclosure with an acid or a base. The compounds of the present disclosure may be hydrates, and such hydrates are also included in the salts of the compounds of the present disclosure. In addition, the compounds of the present disclosure may be solvates, and such solvates are also included in the salts of the compounds of the present disclosure.

In one aspect, the present invention relates to a method for producing lysidine diphosphate represented by the following formula A or a derivative thereof, or agmatidine diphosphate or a derivative thereof.

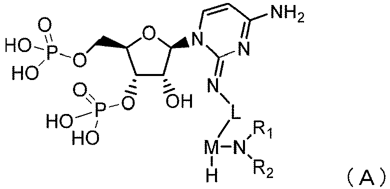

In formula A, R₁ and R₂ are each independently H or C₁-C₃ alkyl, and it is preferred that both R₁ and R₂ are H.

In formula A, L is a C₂-C₆ straight chain alkylene or a C₂-C₆ straight chain alkenylene, optionally substituted with one or more substituents selected from the group consisting of hydroxy and C₁-C₃ alkyl, wherein the carbon atom of the C₂-C₆ straight chain alkylene is optionally substituted with one oxygen atom or sulfur atom. The C₂-C₆ straight chain alkylene is preferably C₄-C₅ straight chain alkylene, and the C₂-C₆ straight chain alkenylene is preferably C₄-C₅ straight chain alkenylene. Specific examples of such L include -(CH₂)₃-, -(CH₂)₄-, - (CH₂)₅, -(CH₂)₂-O-CH₂-, -(CH₂)₂-S-CH₂- -CH₂CH(OH)(CH₂)₂- and -CH₂CH=CH- (cis or trans).

In formula A, M is a single bond, 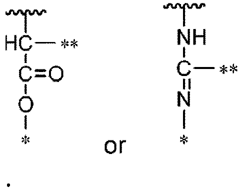 The wavy line indicates the point of attachment to the carbon atom, * indicates the point of attachment to the hydrogen atom, and ** indicates the point of attachment to the nitrogen atom. When M is a single bond, H attached to M does not exist. For example, when M is 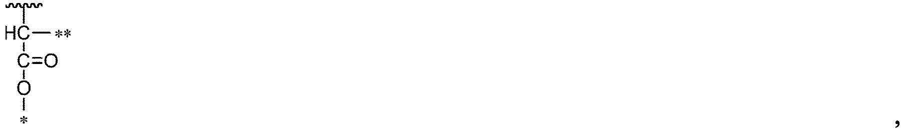 the compound of formula A can be represented as follows: 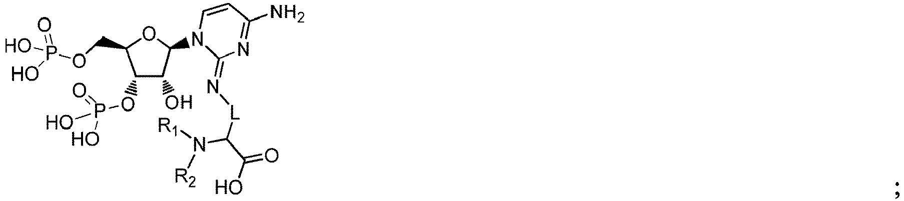 when M is 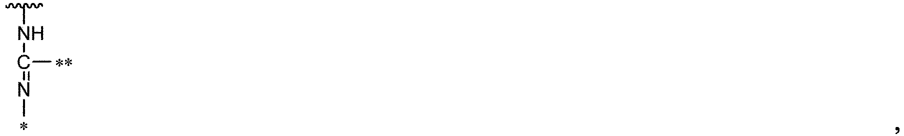 the compound of formula A can be represented as follows: 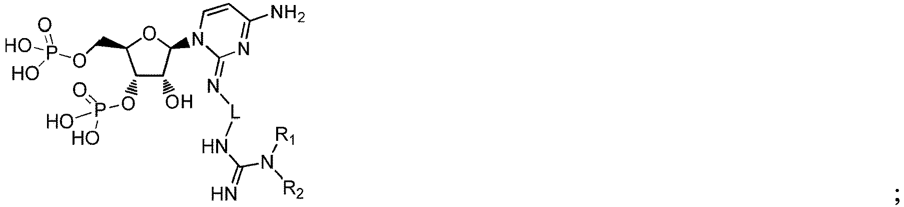 and when M is a single bond, the compound of formula A can be represented as follows:

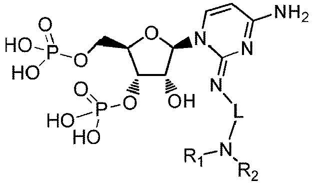

The compound represented by formula A is preferably lysidine diphosphate, agmatidine diphosphate, or a salt thereof.

In some embodiments, compounds of formula A can be produced according to Scheme 1 shown below.

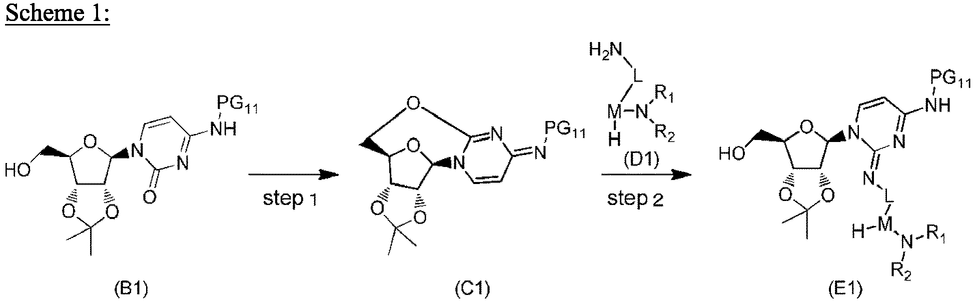

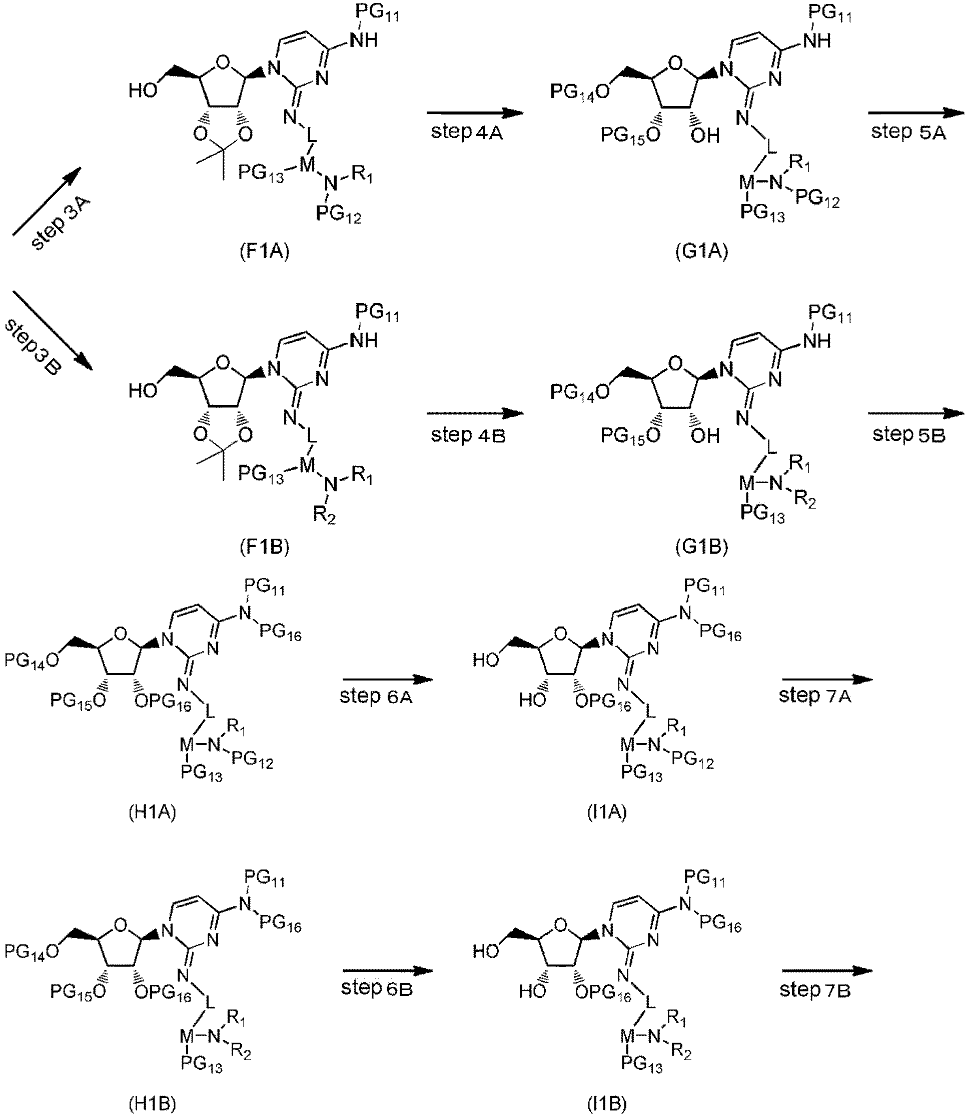

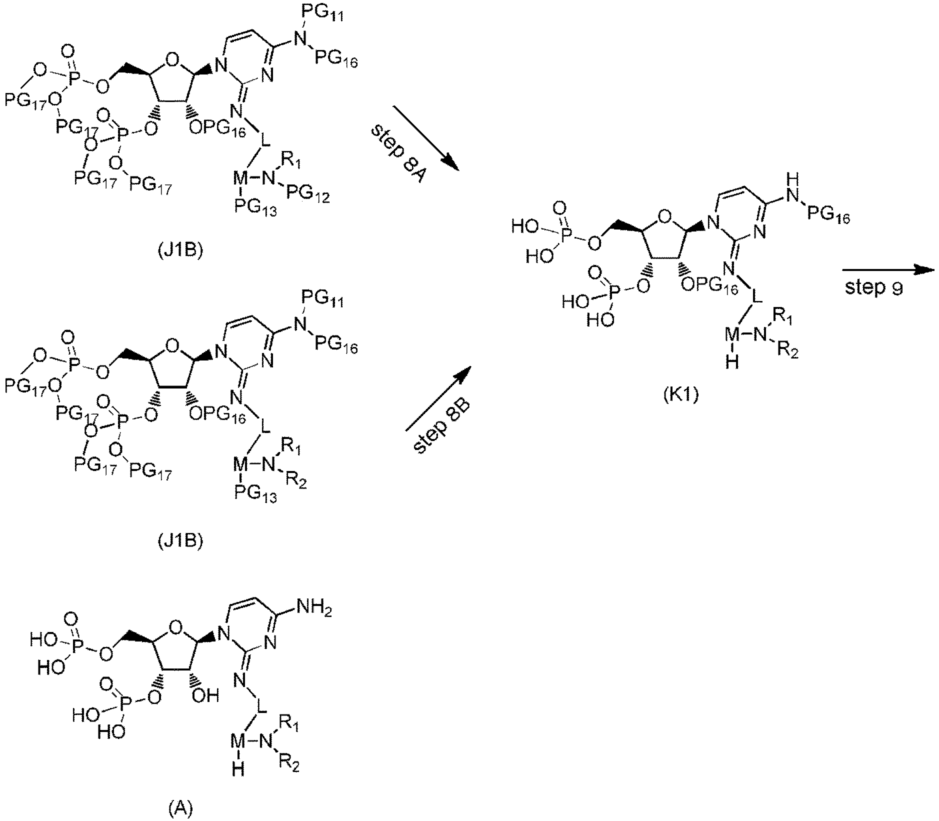

Step 1 of Scheme 1 is a step of intramolecularly cyclizing the compound represented by formula B1 to obtain a compound represented by formula C1. This step can be carried out by stirring the reaction mixture for 15 minutes to 48 hours in the presence of an intramolecular cyclization reagent in a solvent at a temperature from -20°C to around the boiling point of the solvent, preferably 0°C to 180°C.

Compounds represented by formula B1 can be obtained from commercial suppliers, or they can be produced using methods known in the literature. PG₁₁ in formula B1 is a protecting group for an amino group, and any protecting group can be used as long as it does not interfere with the progress of the reaction according to the above-mentioned Scheme 1; for example, protecting groups that are not deprotected by an acid or a fluoride ion are preferred. Specific examples of PG₁₁ include p-bromobenzoyl, optionally substituted benzoyl, pyridinecarbonyl, and acetyl.

The intramolecular cyclization reagent is not particularly limited, but diisopropyl azodicarboxylate and triphenylphosphine can be preferably used.

Examples of the solvent include halogenated solvents, ether solvents, benzene solvents, ester solvents, and ketone solvents, and dichloromethane can be preferably used.

Step 2 of Scheme 1 is a step of introducing the amine represented by formula D1 into the compound represented by formula C1 to obtain the compound represented by formula E1. This step can be performed by stirring the reaction mixture for 15 minutes to 48 hours in the presence of a reagent for introducing amine in a solvent at a temperature from -20°C to around the boiling point of the solvent, preferably 0°C to 180°C.

The amine-introducing reagent is not particularly limited, but lithium chloride and DBU can be preferably used.

Examples of the solvent include halogenated solvents, ether solvents, benzene solvents, ester solvents, and ketone solvents, and tetrahydrofuran is preferably used in this step.

Steps 3A and 3B of Scheme 1 are steps of introducing PG₁₂ and/or PG₁₃ into the compound represented by formula E1 to obtain the compound represented by formula F1A or F1B. When R₂ of formula E1 is alkyl, only PG₁₃ is introduced to give formula F1A; and when R₂ of formula E1 is hydrogen, PG₁₂ and PG₁₃ are introduced to give formula F1B. This step can be performed by stirring the reaction mixture for 15 minutes to 48 hours in the presence of a reagent for introducing a protecting group in a solvent at a temperature from -20°C to around the boiling point of the solvent, preferably at 0°C to 180°C.

PG₁₂ is a protecting group for an amino group, and PG₁₃ is a protecting group for a carboxyl group or an imino group. Any protecting group can be used for these protecting groups, as long as it does not interfere with the progress of the reaction according to the above-mentioned Scheme 1; for example, protecting groups that are not deprotected by an acid or a fluoride ion are preferred. Fmoc is preferably used as PG₁₂; and when M is 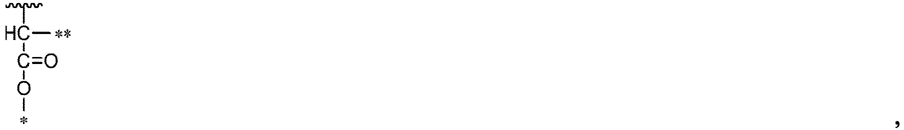 a methyl, an ethyl, or an optionally substituted benzyl is preferably used as PG₁₃, and when M is 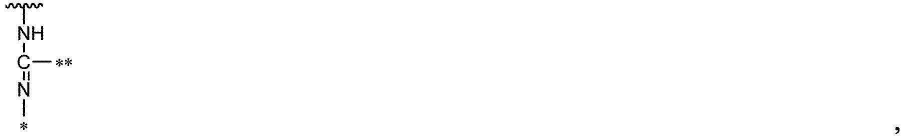 an optionally substituted benzyl, Cbz, or an optionally substituted benzyloxycarbonyl is preferably used as PG₁₃. PG₁₂ and PG₁₃ may be introduced simultaneously or sequentially. When they are introduced sequentially, either PG₁₂ or PG₁₃ may be introduced first, but it is preferred to introduce PG₁₂ at first and then PG₁₃. For the introduction of a protecting group, for example, a method described in "Greene's, "Protective Groups in Organic Synthesis" (5th edition, John Wiley & Sons 2014)" can be used; and, when PG₁₂ is Fmoc, the Fmoc is preferably introduced using (2,5-dioxopyrrolidin-1-yl)(9H-fluoren-9-yl)methyl carbonate and sodium carbonate, and when PG₁₃ is methyl, the methyl is preferably introduced using N,N'-diisopropylcarbodiimide, methanol, and N,N-dimethyl-4-aminopyridine.

Examples of the solvent include halogenated solvents, ether solvents, benzene solvents, ester solvents, and ketone solvents. Dioxane is preferably used when introducing an Fmoc, and dichloromethane is preferably used when introducing a methyl.

Steps 4A and 4B of Scheme 1 are steps of removing acetonide from the compound represented by formula F1A or F1B and introducing PG₁₄ and PG₁₅ to obtain the compound represented by formula G1A or G1B. Acetonide can be removed in the presence of an acid, and the protecting group can be introduced in the presence of a reagent for introducing a protecting group by stirring the reaction mixture for 15 minutes to 48 hours in a solvent at a temperature from -20°C to around the boiling point of the solvent, preferably 0°C to 180°C.

PG₁₄ and PG₁₅ are each independently a protecting group for a hydroxy group, and any protecting group can be used as long as it does not interfere with the progress of the reaction according to the above-mentioned Scheme 1; for example, silyl protecting groups that are deprotected by a fluoride ion are preferably used. It is preferable that PG₁₄ and PG₁₅ together form a divalent protecting group, and specific examples of such a protecting group include di-tert-butylsilyl. For removal of acetonide and introduction of a protecting group, for example, a method described in "Greene's, "Protective Groups in Organic Synthesis" (5th edition, John Wiley & Sons 2014)" can be used; and the acid used for acetonide removal is preferably TFA. When PG₁₄ and PG₁₅ together form a di-tert-butylsilyl, the di-tert-butylsilyl is introduced preferably by using di-tert-butylsilyl bis(trifluoromethanesulfonate).

As the solvent used for removing acetonide, examples include water and carboxylic acid solvents, and a mixed solvent of water and TFA can be preferably used. Furthermore, as the solvent used for introducing PG₁₄ and PG₁₅, examples include halogenated solvents, ether solvents, benzene solvents, ester solvents, ketone solvents, and amide solvents, and DMF is preferably used.

Steps 5A and 5B of Scheme 1 are steps of introducing PG₁₆ into the compound represented by formula G1A or G1B to obtain the compound represented by formula H1A or H1B. PG₁₆ can be introduced by stirring the reaction mixture for 15 minutes to 48 hours in the presence of a reagent for introducing the protecting group in a solvent at a temperature from -20°C to around the boiling point of the solvent, preferably 0°C to 180°C.

PG₁₆ is a protecting group for a hydroxy group and/or an amino group, and any protecting group can be used as long as it does not interfere with the progress of the reaction according to the above-mentioned Scheme 1; for example, protecting groups that are not deprotected by a fluoride ion are preferably used. TOM is preferred for PG₁₆. For the introduction of a protecting group, for example, a method described in "Greene's, "Protective Groups in Organic Synthesis" (5th edition, John Wiley & Sons 2014)" can be used; and when PG₁₆ is TOM, TOM is preferably introduced using DIPEA and (triisopropylsiloxy)methyl chloride.

Examples of the solvent include halogenated solvents, ether solvents, benzene solvents, ester solvents, ketone solvents, and amide solvents, and dichloromethane is preferably used.

Steps 6A and 6B of Scheme 1 are steps of removing PG₁₄ and PG₁₅ from the compound represented by formula G1A or G1B to obtain the compound represented by formula I1A or I1B. PG₁₄ and PG₁₅ can be removed by stirring the reaction mixture for 15 minutes to 48 hours in the presence of a deprotecting reagent in a solvent at a temperature from -20°C to around the boiling point of the solvent, preferably at 0°C to 180°C.

Any reagent can be used for the deprotecting reagent as long as it can selectively remove only PG₁₄ and PG₁₅; however, when PG₁₄ and PG₁₅ together form a di-tert-butylsilyl, it is preferably removed using a reagent that produces fluoride ion, or more specifically, for example, a hydrogen fluoride pyridine complex.

Examples of the solvent include halogenated solvents, ether solvents, benzene solvents, ester solvents, ketone solvents, and amide solvents, and THF is preferably used.

Steps 7A and 7B of Scheme 1 are steps of phosphite esterification of a compound represented by formula I1A or I1B and subsequent oxidation to obtain a compound represented by formula J1A or J1B. The phosphite esterification can be carried out by stirring the reaction mixture for 15 minutes to 48 hours in the presence of a phosphite esterification reagent in a solvent at a temperature from -20°C to around the boiling point of the solvent, preferably 0°C to 180°C. The oxidation can be carried out by stirring the reaction mixture for 15 minutes to 48 hours in the presence of an oxidizing reagent in a solvent at a temperature from -20°C to around the boiling point of the solvent, preferably 0°C to 180°C. The compound may be isolated after the phosphite esterification, but it is preferable to carry out the phosphite esterification reaction and the oxidation reaction in one pot.

In formula J1A or J1B, PG₁₇ is a protecting group for a hydroxy group, and any protecting group can be used as long as it does not interfere with the progress of the reaction according to the above-mentioned Scheme 1; for example, protecting groups that can be deprotected simultaneously with PG₁₁, PG₁₂, and PG₁₃ are preferred. Specific examples of PG₁₇ include cyanoethyl. A phosphite esterification reagent having a hydroxy group protected by a protecting group may be used, or an unprotected phosphite esterification reagent may be used and then a protecting group may be introduced to the hydroxy group. For the introduction of a protecting group, for example, a method described in "Greene's, "Protective Groups in Organic Synthesis" (5th edition, John Wiley & Sons 2014)" can be used. When using a phosphite esterification reagent having a hydroxy group protected by a cyanoethyl group, bis(2-cyanoethyl)-N,N-diisopropylaminophosphoramidite is preferably used as the phosphite esterification reagent. The oxidizing agent used in the oxidation subsequent to phosphite esterification is not particularly limited, but tert-butyl hydroperoxide can be preferably used.

Examples of the solvent include halogenated solvents, ether solvents, benzene solvents, ester solvents, ketone solvents, and nitrile solvents, and acetonitrile is preferably used.

Steps 8A and 8B of Scheme 1 are steps of removing PG₁₁, PG₁₂, PG₁₃, and PG₁₇ from the compound represented by formula J1A, or removing PG₁₁, PG₁₃, and PG₁₇ from the compound represented by formula J1B, to obtain the compound represented by formula K1. These protecting groups can be removed by stirring the reaction mixture for 15 minutes to 48 hours in the presence of a deprotecting reagent in a solvent at a temperature from -20°C to around the boiling point of the solvent, preferably at 0°C to 180°C.

Any reagent can be used for the deprotecting reagent as long as it can selectively remove the above-mentioned protecting groups. Specific examples of such a reagent include the use of bis-(trimethylsilyl)acetamide and DBU in combination.

Examples of the solvent include halogenated solvents, ether solvents, benzene solvents, ester solvents, ketone solvents, nitrile solvents, and amine solvents, and pyridine is preferably used.

Step 9 of Scheme 1 is a step of removing PG₁₆ from the compound represented by formula K1 to obtain the compound represented by formula A. PG₁₆ can be removed by stirring the reaction mixture for 15 minutes to 48 hours in the presence of a deprotecting reagent in a solvent at a temperature from -20°C to around the boiling point of the solvent, preferably at 0°C to 180°C.

Any reagent can be used for the deprotecting reagent as long as it can selectively remove only PG₁₆, and ammonium fluoride is preferably used.

Examples of the solvent include water, halogenated solvents, ether solvents, benzene solvents, ester solvents, ketone solvents, and nitrile solvents, and a combined solvent consisting of water and acetonitrile can be preferably used.

In a certain embodiment, compounds of formula A can be prepared according to Scheme 2 shown below.

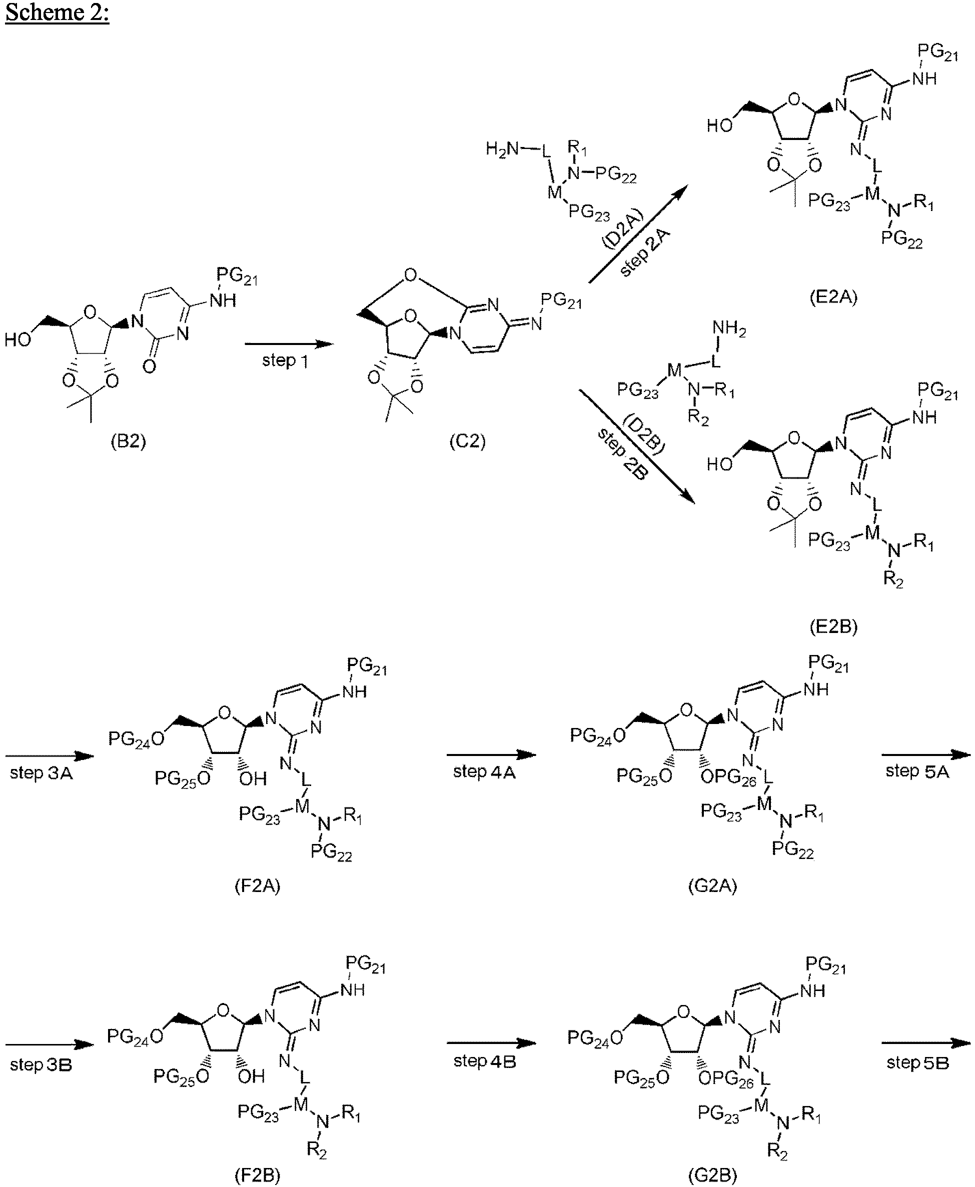

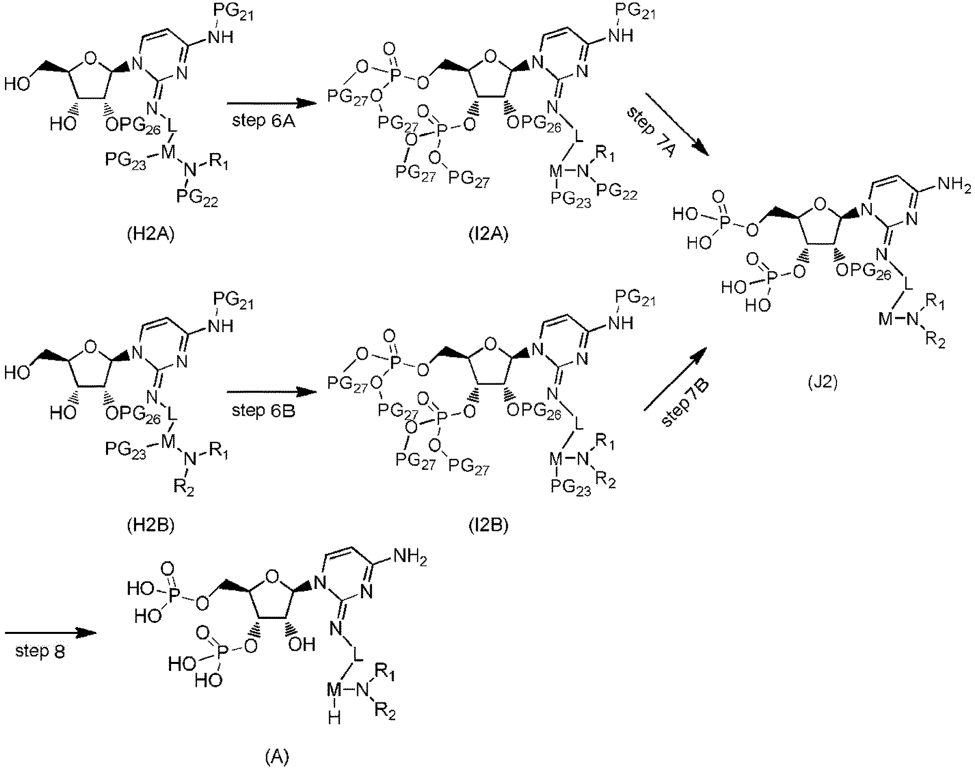

Step 1 of Scheme 2 is a step of intramolecularly cyclizing the compound represented by formula B2 to obtain a compound represented by formula C2. This step can be carried out by stirring the reaction mixture for 15 minutes to 48 hours in the presence of an intramolecular cyclization reagent in a solvent at a temperature from -20°C to around the boiling point of the solvent, preferably 0°C to 180°C.

Compounds represented by formula B2 can be obtained from commercial suppliers, or they can be produced using methods known in the literature. PG₂₁ in formula B2 is a protecting group for an amino group, and any protecting group can be used as long as it does not interfere with the progress of the reaction according to the above-mentioned Scheme 2; for example, protecting groups that are not deprotected by an acid or a fluoride ion are preferred. Specific examples of PG₂₁ include Cbz, optionally substituted benzyloxycarbonyl, and optionally substituted benzyl.

The intramolecular cyclization reagent is not particularly limited, but diisopropyl azodicarboxylate and triphenylphosphine can be preferably used.

Examples of the solvent include halogenated solvents, ether solvents, benzene solvents, ester solvents, and ketone solvents, and dichloromethane can be preferably used.

Step 2 of Scheme 2 is a step of introducing the amine represented by formula D2A or D2B into the compound represented by formula C2 to obtain the compound represented by formula E2A or E2B. This step can be performed by stirring the reaction mixture for 15 minutes to 48 hours in the presence of an amine-introducing reagent in a solvent at a temperature from -20°C to around the boiling point of the solvent, preferably 0°C to 180°C.

The amine-introducing reagent is not particularly limited, but lithium chloride and DBU can be preferably used.

Examples of the solvent include halogenated solvents, ether solvents, benzene solvents, ester solvents, and ketone solvents, and THF is preferably used in this step.

Steps 3A and 3B of Scheme 2 are steps of removing acetonide from the compound represented by formula E2A or E2B, and introducing PG₂₄ and PG₂₅, to obtain the compound represented by formula F2A or F2B. Acetonide can be removed in the presence of an acid, and the protecting group can be introduced by stirring the reaction mixture for 15 minutes to 48 hours in the presence of a reagent for introducing a protecting group in a solvent at a temperature from -20°C to around the boiling point of the solvent, preferably 0°C to 180°C.

PG₂₄ and PG₂₅ are each independently a protecting group for a hydroxy group, and any protecting group can be used as long as it does not interfere with the progress of the reaction according to the above-mentioned Scheme 2; for example, silyl protecting groups that are deprotected by a fluoride ion are preferably used. It is preferable that PG₂₄ and PG₂₅ together form a divalent protecting group, and specific examples of such a protecting group include di-tert-butylsilyl. For removal of acetonide and introduction of a protecting group, for example, a method described in "Greene's, "Protective Groups in Organic Synthesis" (5th edition, John Wiley & Sons 2014)" can be used; and the acid used for acetonide removal is preferably TFA. When PG₂₄ and PG₂₅ together form a di-tert-butylsilyl, the di-tert-butylsilyl is introduced preferably by using di-tert-butylsilyl bis(trifluoromethanesulfonate).

As the solvent used for removing acetonide, examples include water and carboxylic acid solvents, and a mixed solvent of water and TFA can be preferably used. As the solvent used for introducing PG₂₄ and PG₂₅, examples include halogenated solvents, ether solvents, benzene solvents, ester solvents, ketone solvents, and amide solvents, and DMF is preferably used.

Steps 4A and 4B of Scheme 2 are steps of introducing PG₂₆ into the compound represented by formula F2A or F2B to obtain the compound represented by formula G2A or G2B. PG₂₆ can be introduced by stirring the reaction mixture for 15 minutes to 48 hours in the presence of a reagent for introducing the protecting group in a solvent at a temperature from -20°C to around the boiling point of the solvent, preferably 0°C to 180°C.

PG₂₆ is a protecting group for a hydroxy group, and any protecting group can be used as long as it does not interfere with the progress of the reaction according to the above-mentioned Scheme 2; for example, protecting groups that are not deprotected by a fluoride ion are preferred. Tetrahydropyranyl, tetrahydrofuranyl, or methoxymethyl is preferred for PG₂₆. For the introduction of a protecting group, for example, a method described in "Greene's, "Protective Groups in Organic Synthesis" (5th edition, John Wiley & Sons 2014)" can be used; and when PG₁₆ is tetrahydropyranyl, the tetrahydropyranyl is preferably introduced using TFA and 3,4-dihydro-2H-pyran.

Examples of the solvent include halogenated solvents, ether solvents, benzene solvents, ester solvents, ketone solvents, and amide solvents, and dichloromethane is preferably used.

Steps 5A and 5B of Scheme 2 are steps of removing PG₂₄ and PG₂₅ from the compound represented by formula G2A or G2B to obtain the compound represented by formula H2A or H2B. PG₂₄ and PG₂₅ can be removed by stirring the reaction mixture for 15 minutes to 48 hours in the presence of a deprotecting reagent in a solvent at a temperature from -20°C to around the boiling point of the solvent, preferably 0°C to 180°C.

Any reagent can be used for the deprotecting reagent as long as it can selectively remove only PG₂₄ and PG₂₅; however, when PG₂₄ and PG₂₅ together form a di-tert-butylsilyl, it is preferably removed using a reagent that produces fluoride ion, or more specifically, for example, a tetrabutylammonium fluoride.

Examples of the solvent include halogenated solvents, ether solvents, benzene solvents, ester solvents, ketone solvents, and amide solvents, and THF is preferably used.

Steps 6A and 6B of Scheme 2 are steps of phosphite esterification of a compound represented by formula H2A or H2B and subsequent oxidation to obtain a compound represented by formula I2A or I2B. The phosphite esterification can be carried out by stirring the reaction mixture for 15 minutes to 48 hours in the presence of a phosphite esterification reagent in a solvent at a temperature from -20°C to around the boiling point of the solvent, preferably 0°C to 180°C. The oxidation can be carried out by stirring the reaction mixture for 15 minutes to 48 hours in the presence of an oxidizing reagent in a solvent at a temperature from -20°C to around the boiling point of the solvent, preferably 0°C to 180°C. The compound may be isolated after the phosphite esterification, but it is preferable to carry out the phosphite esterification reaction and the oxidation reaction in one pot.

In formula I2A or I2B, PG₂₇ is a protecting group for a hydroxy group, and any protecting group can be used as long as it does not interfere with the progress of the reaction according to the above-mentioned Scheme 2, and it is preferably a protecting group that can be deprotected simultaneously with PG₂₁, PG₂₂, and PG₂₃. Specific examples of PG₂₇ include benzyl. A phosphite esterification reagent having a hydroxy group protected by a protecting group may be used, or an unprotected phosphite esterification reagent may be used and then a protecting group may be introduced to the hydroxy group. For the introduction of a protecting group, for example, a method described in "Greene's, "Protective Groups in Organic Synthesis" (5th edition, John Wiley & Sons 2014)" can be used. When using a phosphite esterification reagent having a hydroxy group protected by a benzyl, dibenzyl-N,N-diisopropylphosphoramidite is preferably used as the phosphite esterification reagent. The oxidizing agent used in the oxidation subsequent to phosphite esterification is not particularly limited, but Dess-Martin periodinane can be preferably used.

Examples of the solvent include halogenated solvents, ether solvents, benzene solvents, ester solvents, ketone solvents, and nitrile solvents, and acetonitrile is preferably used.

Steps 7A and 7B of Scheme 2 are steps of removing PG₂₁, PG₂₂, PG₂₃, and PG₂₇ from the compound represented by formula I2A, or removing PG₂₁, PG₂₃, and PG₂₇ from the compound represented by formula I2B, to obtain the compound represented by formula J2. These protecting groups can be removed by stirring the reaction mixture for 15 minutes to 48 hours in the presence of a deprotecting reagent in a solvent at a temperature from -20°C to around the boiling point of the solvent, preferably 0°C to 180°C.

Any method can be used for the deprotection as long as the above-mentioned protecting groups can be selectively removed. Specific examples of such a method include catalytic hydrogenation. For catalytic hydrogenation, Pd catalysts such as palladium-carbon can be preferably used.

Examples of the solvent include water, alcohol solvents, halogenated solvents, ether solvents, benzene solvents, ester solvents, ketone solvents, nitrile solvents, and amine solvents, and a combined solvent consisting of water and methanol is preferably used.

Step 8 of Scheme 2 is a step of removing PG₂₆ from the compound represented by formula J2 to obtain the compound represented by formula A. PG₂₆ can be removed by stirring the reaction mixture for 15 minutes to 48 hours in the presence of a deprotecting reagent in a solvent at a temperature from -20°C to around the boiling point of the solvent, preferably 0°C to 180°C.

Any reagent can be used for the deprotecting reagent as long as it can selectively remove PG₂₆, and hydrochloric acid is preferably used.

Examples of the solvent include water, halogenated solvents, ether solvents, benzene solvents, ester solvents, ketone solvents, and nitrile solvents, and water can be preferably used.

All prior art literatures cited in the present specification are incorporated herein by reference.

### [Examples]

The present invention is further illustrated by the following examples, but is not limited thereto.

The following abbreviations were used in the Examples.
AA: ammonium acetate
CH₂CN: cyanomethyl group
DBU: 1,8-diazabicyclo[5.4.0]-7-undecene
DCM: dichloromethane
DIC: N,N-diisopropylcarbodiimide
DIPEA: N,N-diisopropylethylamine
DMF: dimethylformamide
DMSO: dimethyl sulfoxide
FA: formic acid
Fmoc: 9-fluorenylmethyloxycarbonyl group
F-Pnaz: 4-(2-(4-fluorophenyl)acetamido)benzyloxycarbonyl group 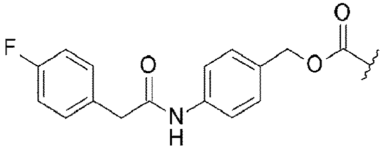
HFIP: 1,1,1,3,3,3-hexafluoro-2-propanol
MeCN: acetonitrile
NMP: N-methyl-2-pyrrolidone
TEA: triethylamine
TFA: trifluoroacetic acid
TFE: 2,2,2-trifluoroethanol
THF: tetrahydrofuran

The following abbreviations were used in this Example: Gly or G (glycine), Ile or I (isoleucine), Leu or L (leucine), Phe or F (phenylalanine), Pro or P (proline), Thr or T (threonine). In addition to these, the abbreviations shown in Table 2 were used.

**[Table 2]**

| Bio code | Structure |
|---|---|
| dA | 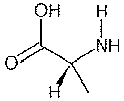 |
| MeHph | 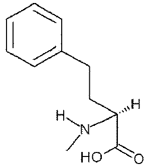 |
| nBuG | 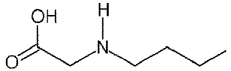 |
| F3Cl | 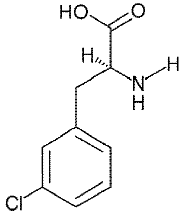 |
| Pic2 | 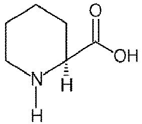 |
| SPh2CI | 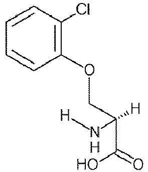 |
| BdpFL -Phe | 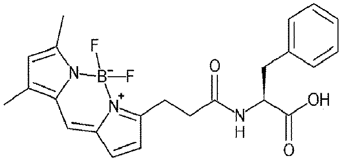 |
| Thr (THP) | 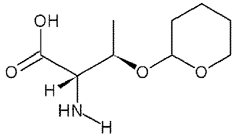 |
| SiPen | 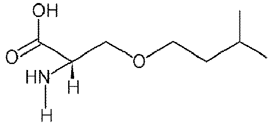 |

The LCMS analysis conditions are shown in Table 3 shown below.

**[Table 3]**

| Analysis condition | System | Column (I. D. x Length (mm)) | Mobile phase | Gradient (A/B) | Flow rate (ml/min) | Column temperature (°C) | Wave length |
|---|---|---|---|---|---|---|---|
| SQD | Acquity | Aldrich Ascentis Express C18 2. 7 *µ*m (2.1 x 50) | A) 0.1% FA, H20 | 95/5 => 0/100 (1.0 min) => 0/100 (0.4 min) | 1 | 35 | 210-400nm |
| FA05_01 | UPLC/SQD | | B) 0.1% FA CH3CN | | | | PDA total |
| SQD | Acquity | Aldrich Ascentis Express C18 2. 7 *µ*m (2.1 x 50) | A) 0.1% FA, H20 | 95/5 => 0/100 (1.0 min) => 0/100 (0.4 min) | 0.9 | 35 | 210-400nm |
| FA05_02 | UPLC/SQD2 | | B) 0.1% FA CH3CN | | | | PDA total |
| SQD | Acquity | Aldrich Ascentis Express C18 2. 7 *µ*m (2.1 x 50) | A) 0.1% FA, H20 | 50/50 => 0/100 (0.7 min) => 0/100 (0.7 min) | 0.9 | 35 | 210-400nm |
| FA50 | UPLC/SQD2 | | B) 0.1% FA CH3CN | | | | PDA total |
| SQD | Acquity | Aldrich Ascentis Express C18 2.7 *µ*m (2.1 x 50) | A) 0.1% FA, H20 | 95/5 => 0/100 (4.5 min) => 0/100 (0.5 min) | 0.9 | 35 | 210-400nm |
| FA05 long | UPLC/SQD2 | | B) 0.1% FA CH3CN | | | | PDA total |
| SQD | Acquity | Aldrich Ascent is Express C18 5.0 *µ*m (2.1 x 50) | A) 10mM Ac0NH4, H20 | 95/5 => 0/100 (4. 5 min) => 0/100 (0.5 min) | 0.9 | 35 | 210-400nm |
| AA05 long | UPLC/SQD2 | | B) MeOH | | | | PDA total |
| SQD | Acquity | Aldrich Ascentis Express C18 5. 0 *µ*m (2.1 x 50) | A) 10mM AcONH4, H20 | 50/50 => 0/100 (4.5 min) => 0/100 (0.5 min) | 0.9 | 35 | 210-400nm |
| AA50 long | UPLC/SQD2 | | B) MeOH | | | | PDA total |
| LTQ | Acquity | Waters ACQUITY UPLC BEH C18 1.7 *µ*m (2.1 x 50) | A) 15mM TEA, | 95/5 => 10/90 (9.0 min) => 10/90 (1.0 min) | 0.2 | 30 | 190-400nm |
| TEA/HFIP05_01 | UPLC/LTQ | | 400mM HFIP H20 | | | | PDA total |
| | Orbitrap XL | | B) 15mM TEA, 400mM HFIP MeOH | | | | |
| LTQ | Acquity | Waters ACQUITY UPLC BEH C18 1.7 *µ*m (2.1 x 50) | A) 15mM TEA, | 95/5 => 95/5 (8.0 min) => 10/90 (2.0 min) | 0.2 | 30 | 190-400nm |
| TEA/HFIP05_02 | UPLC/LTQ | | 400mM HFIP H20 | | | | PDA total |
| | Orbitrap XL | | B) 15mM TEA, 400mM HFIP MeOH | | | | |
| LTQ | Acquity | Waters ACQUITY UPLC BEH C18 1.7 *µ*m (2.1 x 50) | A) 15mM TEA, | 95/5 => 70/30 (9.0 min) => 10/90 (1.0 min) | 0.2 | 30 | 190-400nm |
| TEA/HFIP05_03 | UPLC/LTQ | | 400mM HFIP H20 | | | | PDA total |
| | Orbitrap XL | | B) 15mM TEA, 400mM HFIP MeOH | | | | |
| SMD | Shimadzu | Shim-Pack XR-ODS 1. 7 *µ*m (2.1 x 50) | A) 0.1% FA, H20 | 90/10 => 0/100 (1.1 min) => 0/100 (0.6 min) | 1.2 | 40 | 190-400nm |
| method 1 | LCMS-2020 | | B) 0.1% FA CH3CN | | | | PDA total |
| | LC-20AD | | | | | | |
| SMD | Shimadzu | CORTECS C18 2.7 *µ*m (2. 1 x 50) | A) 0.1% FA, H20 | 90/10 => 0/100 (1.2 min) => 0/100 (0.5 min) | 1.0 | 40 | 190-400nm |
| method 2 | LCMS-2020 | | B) 0.1% FA CH3CN | | | | PDA total |
| | LC-20ADXR | | | | | | |
| SMD | Shimadzu | kinetex 2.6u XB-C18 100A 2. 6 *µ*m (3.0 x 50) | A) 0.1% FA, H20 | 90/10 => 0/100 (1.2 min) => 0/100 (0.5 min) | 1.5 | 40 | 190-400nm |
| method 3 | LCMS-2020 | | B) 0.1% FA CH3CN | | | | PDA total |
| | LC-20ADXR | | | | | | |
| SMD | Shimadzu | kinetex 2.6u XB-C18 100A 2. 6 *µ*m (2. 1 x 50) | A) 0.1% FA, H20 | 90/10 => 0/100 (1.2 min) => 0/100 (0.5 min) | 0.8 | 40 | 190-400nm |
| method 4 | LCMS-2020 | | B) 0.1% FA CH3CN | | | | PDA total |
| | LC-20ADXR | | | | | | |

### Example 1. Synthesis of uridine-diphosphate for introducing a uridine unit at the 3' end of a tRNA fragment by a ligation method

To introduce a uridine unit at the 3' end of a tRNA fragment by a ligation method, uridine-diphosphate (SS01, pUp) was synthesized by referring to a method described in literature (Nucleic Acids Research 2003, 31 (22), e145).

### Synthesis of a mixture (Compound SS01, pUp) of ((2R,3S,4R,5R)-5-(2,4-dioxo-3,4-dihydropyrimidin-1(2H)-yl)-4-hydroxy-3-(phosphonooxy)tetrahydrofuran-2-yl)methyl dihydrogen phosphate (Compound SS02) and ((2R,3R,4R,5R)-5-(2,4-dioxo-3,4-dihydropyrimidin-1(2H)-yl)-3-hydroxy-4-(phosphonooxy)tetrahydrofuran-2-yl)methyl dihydrogen phosphate (Compound SS03)

1-((2R,3R,4S,5R)-3,4-dihydroxy-5-(hydroxymethyl)tetrahydrofuran-2-yl)pyrimidin-2,4(1H,3H)-dione (10 mg, 0.041 mmol) and pyrophosphoric acid tetrachloride (56.6 µL, 0.409 mmol) were mixed in an ice bath. After stirring the reaction mixture at 0°C for five hours, ice-cooled pure water (38 mL) and triethylammonium bicarbonate buffer (1 M, 2 mL) were added under ice cooling. The mixture was purified by DEAE-Sephadex A-25 column chromatography (0.05 M triethylammonium bicarbonate buffer → 1 M triethylammonium bicarbonate buffer), and the collected solution was concentrated under reduced pressure. The obtained residue was purified by reverse-phase silica gel column chromatography (aqueous solution of 15 mM TEA and 400 mM HFIP / methanol solution of 15 mM TEA and 400 mM HFIP) to obtain an aqueous solution of the mixture (Compound SS01, pUp) of ((2R,3S,4R,5R)-5-(2,4-dioxo-3,4-dihydropyrimidin-1(2H)-yl)-4-hydroxy-3-(phosphonooxy)tetrahydrofuran-2-yl)methyl dihydrogen phosphate (Compound SS02) and ((2R,3R,4R,5R)-5-(2,4-dioxo-3,4-dihydropyrimidin-1(2H)-yl)-3-hydroxy-4-(phosphonooxy)tetrahydrofuran-2-yl)methyl dihydrogen phosphate (Compound SS03) (100 µL, 40.30 mM).
LCMS (ESI) m/z = 403 (M-H)-
Retention time: 1.79 minutes, 1.89 minutes (analysis condition LTQTEA/HFIP05_01)

### Example 2. Synthesis of lysidine-diphosphate for introducing a lysidine unit at the 3' end of a tRNA fragment by a ligation method

To introduce a lysidine unit at the 3' end of a tRNA fragment by a ligation method, a diphosphate of lysidine was synthesized. More specifically, lysidine-diphosphate (SS04, pLp) was synthesized according to the following scheme.

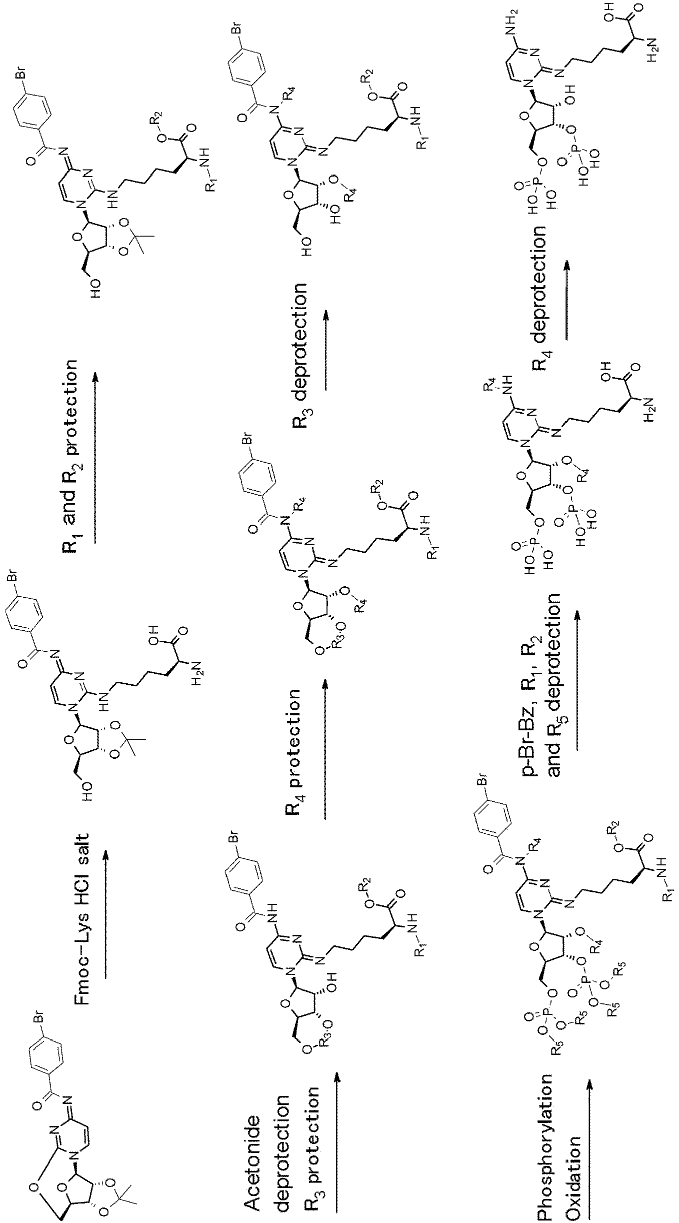

### Synthesis of N⁶-(4-(4-bromobenzamido)-1-((3aR,4R,6R,6aR)-6-(hydroxymethyl)-2,2-dimethyltetrahydrofuro[3,4-d][1,3]dioxol-4-yl)pyrimidin-2(1H)-ylidene)-L-lysine 2,2,2-trifluoroacetic acid salt (Compound SS05)

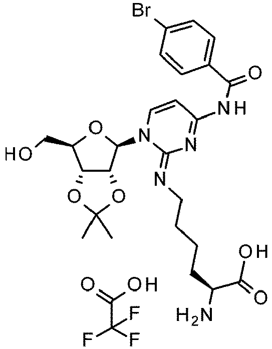

Under nitrogen atmosphere, THF (6.7 mL) was added to a mixture of (((9H-fluoren-9-yl)methoxy)carbonyl)-L-lysine hydrochloride (813 mg, 2.01 mmol), lithium chloride (213 mg, 5.02 mmol) and N⁴-p-bromobenzoyl-2',3'-O-isopropylidene-O2,5'-cyclocytidine (300 mg, 0.067 mmol) synthesized by the method described in literature (Org. Lett. 2012, 14(16), 4118-4121) at room temperature. After cooling the mixture in an ice bath, DBU (1.50 mL, 10.04 mmol) was added. The reaction mixture was stirred at 0°C for one hour, and then purified by reverse-phase silica gel column chromatography (0.1% aqueous FA solution/0.1% FA-acetonitrile solution) to obtain N⁶-(4-(4-bromobenzamido)-1-((3aR,4R,6R,6aR)-6-(hydroxymethyl)-2,2-dimethyltetrahydrofuro[3,4-d][1,3]dioxol-4-yl)pyrimidin-2(1H)-ylidene)-L-lysine 2,2,2-trifluoroacetic acid salt (Compound SS05) (335.6 mg, 71%).
LCMS (ESI) m/z = 594 (M+H)+
Retention time: 0.41 minutes (analysis condition SQDFA05_01)

### Synthesis of N²-(((9H-fluoren-9-yl)methoxy)carbonyl)-N⁶-(4-(4-bromobenzamido)-1-((3aR,4R,6R,6aR)-6-(hydroxymethyl)-2,2-dimethyltetrahydrofuro[3,4-d][1,3]dioxol-4-yl)pyrimidin-2(1H)-ylidene)-L-lysine 2,2,2-trifluoroacetic acid salt (Compound SS06)

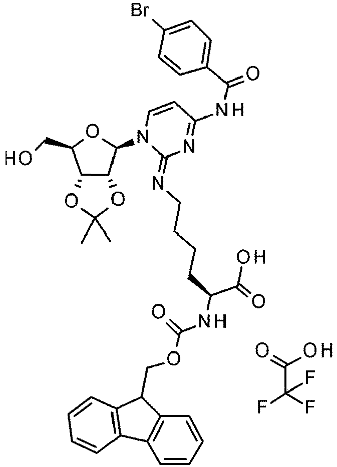

N⁶-(4-(4-bromobenzamido)-1-((3aR,4R,6R,6aR)-6-(hydroxymethyl)-2,2-dimethyltetrahydrofuro[3,4-d][1,3]dioxol-4-yl)pyrimidin-2(1H)-ylidene)-L-lysine 2,2,2-trifluoroacetic acid salt (Compound SS05) (311.12 mg, 0.44 mmol) and (2,5-dioxopyrrolidin-1-yl) (9H-fluoren-9-yl)methyl carbonate (148.09 mg, 0.44 mmol) were dissolved in a mixed solvent of 1,4-dioxane (2.75 mL) and ultrapure water (1.65 mL) at room temperature. After cooling the mixture using an ice bath, sodium carbonate (186.22 mg, 1.76 mmol) was added, and then the mixture was warmed to room temperature and stirred at room temperature for two hours. The reaction solution was concentrated, and the residue was purified by reverse-phase silica gel column chromatography (0.05% aqueous TFA solution/0.05% TFA-acetonitrile solution) to obtain N²-(((9H-fluoren-9-yl)methoxy)carbonyl)-N⁶-(4-(4-bromobenzamido)-1-((3aR,4R,6R,6aR)-6-(hydroxymethyl)-2,2-dimethyltetrahydrofuro[3,4-d][1,3]dioxol-4-yl)pyrimidin-2(1H)-ylidene)-L-lysine 2,2,2-trifluoroacetic acid salt (Compound SS06) (328.78 mg, 80%).
LCMS (ESI) m/z = 816 (M+H)+
Retention time: 0.68 minutes (analysis condition SQDFA05_01)

### Synthesis of methyl N²-(((9H-fluoren-9-yl)methoxy)carbonyl)-N⁶-(4-(4-bromobenzamido)-1-((3aR,4R,6R,6aR)-6-(hydroxymethyl)-2,2-dimethyltetrahydrofuro[3,4-d][1,3]dioxol-4-yl)pyrimidin-2(1H)-ylidene)-L-lysinate 2,2,2-trifluoroacetate (Compound SS07)

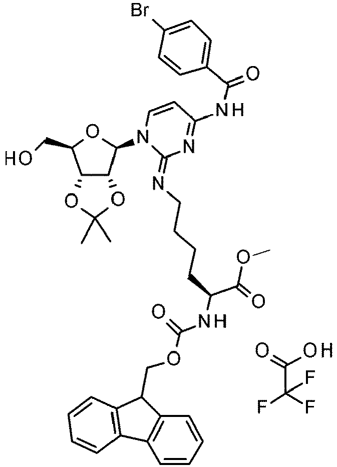

Under nitrogen atmosphere, N²-(((9H-fluoren-9-yl)methoxy)carbonyl)-N⁶-(4-(4-bromobenzamido)-1-((3aR,4R,6R,6aR)-6-(hydroxymethyl)-2,2-dimethyltetrahydrofuro[3,4-d][1,3]dioxol-4-yl)pyrimidin-2(1H)-ylidene)-L-lysine 2,2,2-trifluoroacetic acid salt (Compound SS06) (438.60 mg, 0.47 mmol) was dissolved in DCM (4.71 mL) at room temperature. After cooling the mixture in an ice bath, N,N'-diisopropylcarbodiimide (221.40 µL, 1.41 mmol), methanol (382.07 µL, 9.42 mmol), and N,N-dimethyl-4-aminopyridine (11.51 mg, 0.09 mmol) were added, and then the mixture was warmed to room temperature, and stirred at room temperature for two hours. The reaction solution was concentrated, and the residue was purified by reverse-phase silica gel column chromatography (0.05% aqueous TFA solution/0.05% TFA-acetonitrile solution) to obtain methyl N²-(((9H-fluoren-9-yl)methoxy)carbonyl)-N⁶-(4-(4-bromobenzamido)-1-((3aR,4R,6R,6aR)-6-(hydroxymethyl)-2,2-dimethyltetrahydrofuro[3,4-d][1,3]dioxol-4-yl)pyrimidin-2(1H)-ylidene)-L-lysinate 2,2,2-trifluoroacetate (Compound SS07) (405.00 mg, 91%).
LCMS (ESI) m/z = 828 (M-H)-
Retention time: 0.76 minutes (analysis condition SQDFA05_02)

### Synthesis of methyl N²-(((9H-fluoren-9-yl)methoxy)carbonyl)-N⁶-(4-(4-bromobenzamido)-1-((2R,3R,4S,5R)-3,4-dihydroxy-5-(hydroxymethyl)tetrahydrofuran-2-yl)pyrimidin-2(1H)-ylidene)-L-lysinate 2,2,2-trifluoroacetate (Compound SS08)

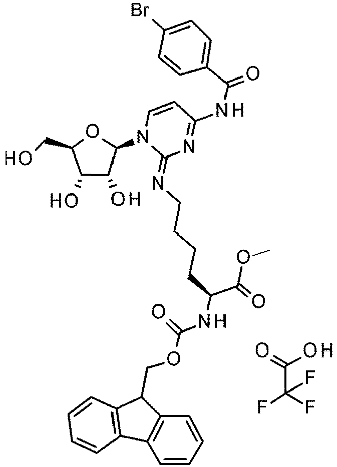

Methyl N²-(((9H-fluoren-9-yl)methoxy)carbonyl)-N⁶-(4-(4-bromobenzamido)-1-((3aR,4R,6R,6aR)-6-(hydroxymethyl)-2,2-dimethyltetrahydrofuro[3,4-d][1,3]dioxol-4-yl)pyrimidin-2(1H)-ylidene)-L-lysinate 2,2,2-trifluoroacetate (Compound SS07) (308.70 mg, 0.33 mmol) was dissolved in a mixed solvent of TFA (8.71 mL) and ultrapure water (4.36 mL) while cooling in an ice bath, and the mixture was stirred at room temperature for 45 minutes. The reaction solution was concentrated to obtain a crude product, methyl N²-(((9H-fluoren-9-yl)methoxy)carbonyl)-N⁶-(4-(4-bromobenzamido)-1-((2R,3R,4S,5R)-3,4-dihydroxy-5-(hydroxymethyl)tetrahydrofuran-2-yl)pyrimidin-2(1H)-ylidene)-L-lysinate 2,2,2-trifluoroacetate (Compound SS08) (296.00 mg). The obtained crude product, methyl N²-(((9H-fluoren-9-yl)methoxy)carbonyl)-N⁶-(4-(4-bromobenzamido)-1-((2R,3R,4S,5R)-3,4-dihydroxy-5-(hydroxymethyl)tetrahydrofuran-2-yl)pyrimidin-2(1H)-ylidene)-L-lysinate 2,2,2-trifluoroacetate (Compound SS08), was directly used in the next step.
LCMS (ESI) m/z = 790 (M+H)+
Retention time: 0.70 minutes (analysis condition SQDFA05_02)

### Synthesis of methyl N²-(((9H-fluoren-9-yl)methoxy)carbonyl)-N⁶-(4-(4-bromobenzamido)-1-((4aR,6R,7R,7aS)-2,2-di-tert-butyl-7-hydroxytetrahydro-4H-furo[3,2-d][1,3,2]dioxasilin-6-yl)pyrimidin-2(1H)-ylidene)-L-lysinate (Compound SS09)

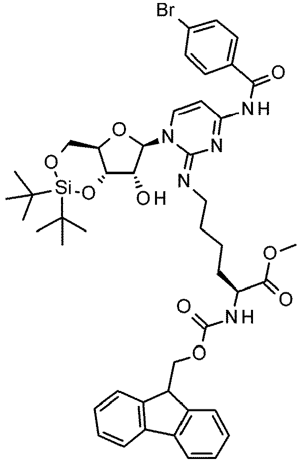

Under nitrogen atmosphere, the crude product obtained in the previous step, methyl N²-(((9H-fluoren-9-yl)methoxy)carbonyl)-N⁶-(4-(4-bromobenzamido)-1-((2R,3R,4S,5R)-3,4-dihydroxy-5-(hydroxymethyl)tetrahydrofuran-2-yl)pyrimidin-2(1H)-ylidene)-L-lysinate 2,2,2-trifluoroacetate (Compound SS08) (296.00 mg, 0.33 mmol), was dissolved in DMF (3.27 mL) at room temperature. After cooling the mixture in an ice bath, di-tert-butylsilyl bis(trifluoromethane sulfonate) (211.80 µL, 0.65 mmol) was added, and the mixture was stirred in an ice bath for one hour. Di-tert-butylsilyl bis(trifluoromethanesulfonate) (158.85 µL, 0.49 mmol) was further added, and the mixture was stirred in an ice bath for 30 minutes. A saturated aqueous sodium bicarbonate solution was added to the reaction solution, DCM was used to perform extraction operations on the obtained mixture, and the organic layer was washed with saturated brine. The obtained organic layer was dried over anhydrous sodium sulfate, filtered, and then concentrated under reduced pressure. The obtained residue was purified by normal phase silica gel column chromatography (normal hexane/ ethyl acetate, dichloromethane/ methanol) to obtain methyl N²-(((9H-fluoren-9-yl)methoxy)carbonyl)-N⁶-(4-(4-bromobenzamido)-1-((4aR,6R,7R,7aS)-2,2-di-tert-butyl-7-hydroxytetrahydro-4H-furo[3,2-d][1,3,2]dioxasilin-6-yl)pyrimidin-2(1H)-ylidene)-L-lysinate (Compound SS09) (273.80 mg, 90%, 2 steps).
LCMS (ESI) m/z = 928.5 (M-H)-
Retention time: 0.92 minutes (analysis condition SQDFA05_02)

### Synthesis of methyl N²-(((9H-fluoren-9-yl)methoxy)carbonyl)-N⁶-(4-(4-bromo-N-(((triisopropylsilyl)oxy)methyl)benzamido)-1-((4aR,6R,7R,7aR)-2,2-di-tert-butyl-7-(((triisopropylsilyl)oxy)methoxy)tetrahydro-4H-furo[3,2-d][1,3,2]dioxasilin-6-yl)pyrimidin-2(1H)-ylidene)-L-lysinate (Compound SS10)

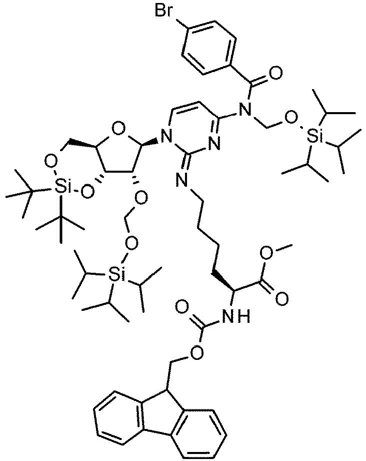

Under nitrogen atmosphere, methyl N²-(((9H-fluoren-9-yl)methoxy)carbonyl)-N⁶-(4-(4-bromobenzamido)-1-((4aR,6R,7R,7aS)-2,2-di-tert-butyl-7-hydroxytetrahydro-4H-furo[3,2-d][1,3,2]dioxasilin-6-yl)pyrimidin-2(1H)-ylidene)-L-lysinate (Compound SS09) (386.15 mg, 0.42 mmol) was dissolved in DCM (8.30 mL) at room temperature, and DIPEA (722.88 µL, 4.15 mmol) and (triisopropylsiloxy)methyl chloride (481.40 µL, 2.07 mmol) were added. The reaction mixture was stirred at 45°C for three hours and then returned to room temperature, DIPEA (722.88 µL, 4.15 mmol) and (triisopropylsiloxy)methyl chloride (481.40 µL, 2.07 mmol) were added, and the reaction mixture was stirred at 45°C for four hours. After returning the mixture to room temperature and adding DMSO, nitrogen was blown to remove DCM, and the obtained DMSO solution was purified by reverse-phase silica gel column chromatography (0.05% aqueous TFA solution/0.05% TFA-acetonitrile solution). The obtained fraction was neutralized with saturated sodium bicarbonate, and the compound of interest was extracted with ethyl acetate. The obtained organic layer was dried over anhydrous sodium sulfate, filtered, and then concentrated under reduced pressure to obtain methyl N²-(((9H-fluoren-9-yl)methoxy)carbonyl)-N⁶-(4-(4-bromo-N-(((triisopropylsilyl)oxy)methyl)benzamido)-1-((4aR,6R,7R,7aR)-2,2-di-tert-butyl-7-(((triisopropylsilyl)oxy)methoxy)tetrahydro-4H-furo[3,2-d][1,3,2]dioxasilin-6-yl)pyrimidin-2(1H)-ylidene)-L-lysinate (Compound SS10) (291.55 mg, 54%).
LCMS (ESI) m/z = 1303 (M+H)+
Retention time: 0.84 minutes (analysis condition SQDFA50)

### Synthesis of methyl N²-(((9H-fluoren-9-yl)methoxy)carbonyl)-N⁶-(4-(4-bromo-N-(((triisopropylsilyl)oxy)methyl)benzamido)-1-((2R,3R,4R,5R)-4-hydroxy-5-(hydroxymethyl)-3-(((triisopropylsilyl)oxy)methoxy)tetrahydrofuran-2-yl)pyrimidin-2(1H)-ylidene)-L-lysinate (Compound SS11)

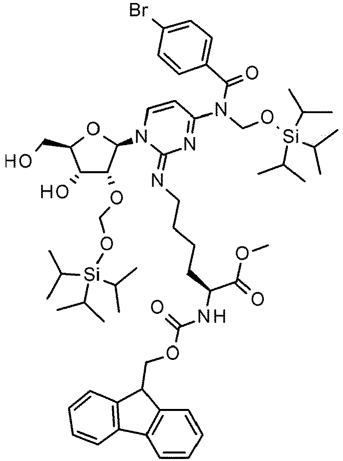

Under nitrogen atmosphere, methyl N²-(((9H-fluoren-9-yl)methoxy)carbonyl)-N⁶-(4-(4-bromo-N-(((triisopropylsilyl)oxy)methyl)benzamido)-1-((4aR,6R,7R,7aR)-2,2-di-tert-butyl-7-(((triisopropylsilyl)oxy)methoxy)tetrahydro-4H-furo[3,2-d][1,3,2]dioxasilin-6-yl)pyrimidin-2(1H)-ylidene)-L-lysinate (Compound SS10) (141.55 mg, 0.11 mmol) was dissolved in THF (2.17 mL) and cooled to -80°C. A hydrogen fluoride pyridine complex (approximately 30% pyridine, approximately 70% hydrogen fluoride) (9.85 µL) diluted with pyridine (134.41 µL) was added at -80°C, and the reaction mixture was stirred at -15°C for 15 minutes. After cooling to -80°C, methoxytrimethylsilane (7.0 mL) was added, and the obtained mixture was purified by reverse-phase silica gel column chromatography (0.05% aqueous TFA solution/0.05% TFA-acetonitrile solution). The obtained fraction was neutralized with saturated sodium bicarbonate, and the compound of interest was extracted with ethyl acetate. The obtained organic layer was dried over anhydrous sodium sulfate, filtered, and then toluene was added, and the mixture was concentrated under reduced pressure to obtain a crude product, methyl N²-(((9H-fluoren-9-yl)methoxy)carbonyl)-N⁶-(4-(4-bromo-N-(((triisopropylsilyl)oxy)methyl)benzamido)-1-((2R,3R,4R,5R)-4-hydroxy-5-(hydroxymethyl)-3-(((triisopropylsilyl)oxy)methoxy)tetrahydrofuran-2-yl)pyrimidin-2(1H)-ylidene)-L-lysinate (Compound SS11) (61.53 mg). The obtained crude product, methyl N²-(((9H-fluoren-9-yl)methoxy)carbonyl)-N⁶-(4-(4-bromo-N-(((triisopropylsilyl)oxy)methyl)benzamido)-1-((2R,3R,4R,5R)-4-hydroxy-5-(hydroxymethyl)-3-(((triisopropylsilyl)oxy)methoxy)tetrahydrofuran-2-yl)pyrimidin-2(1H)-ylidene)-L-lysinate (Compound SS11), was directly used in the next step.
LCMS (ESI) m/z = 1162.8 (M+H)+
Retention time: 3.69 minutes (analysis condition SQDFA05long)

### Synthesis of methyl N²-(((9H-fluoren-9-yl)methoxy)carbonyl)-N⁶-(1-((2R,3R,4R,5R)-4-((bis(2-cyanoethoxy)phosphoryl)oxy)-5-(((bis(2-cyanoethoxy)phosphoryl)oxy)methyl)-3-(((triisopropylsilyl)oxy)methoxy)tetrahydrofuran-2-yl)-4-(4-bromo-N-(((triisopropylsilyl)oxy)methyl)benzamido)pyrimidin-2(1H)-ylidene)-L-lysinate (Compound SS12)

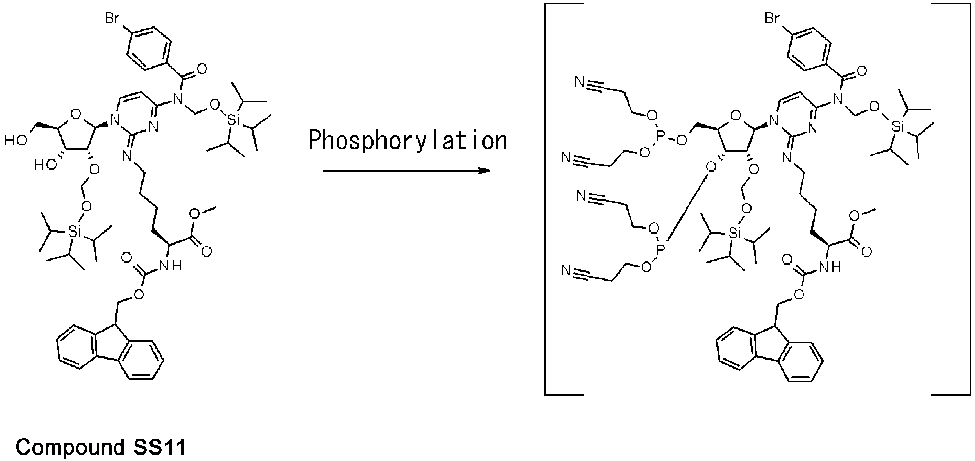

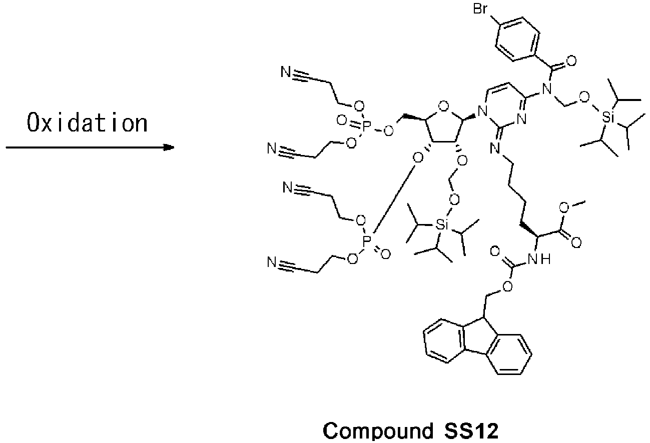

Under nitrogen atmosphere, the crude product, methyl N²-(((9H-fluoren-9-yl)methoxy)carbonyl)-N⁶-(4-(4-bromo-N-(((triisopropylsilyl)oxy)methyl)benzamido)-1-((2R,3R,4R,5R)-4-hydroxy-5-(hydroxymethyl)-3-(((triisopropylsilyl)oxy)methoxy)tetrahydrofuran-2-yl)pyrimidin-2(1H)-ylidene)-L-lysinate (Compound SS11) (61.53 mg, 0.053 mmol), and 1H-tetrazole (44.48 mg, 0.64 mmol) were dissolved in acetonitrile (3.53 mL) at room temperature. After cooling the mixture in an ice bath, bis(2-cyanoethyl)-N,N-diisopropylamino phosphoramidite (82.77 µL, 0.32 mmol) was added, and then the mixture was warmed to room temperature, and stirred at room temperature for three hours. After adding tert-butyl hydroperoxide 5-6 M in decane (303.92 µL, 3.17 mmol) at room temperature and stirring for ten minutes, the reaction solution was concentrated, and the obtained residue was purified by normal phase silica gel column chromatography (normal hexane/ ethyl acetate, dichloromethane/ methanol) to obtain a crude product, methyl N²-(((9H-fluoren-9-yl)methoxy)carbonyl)-N⁶-(1-((2R,3R,4R,5R)-4-((bis(2-cyanoethoxy)phosphoryl)oxy)-5-(((bis(2-cyanoethoxy)phosphoryl)oxy)methyl)-3-(((triisopropylsilyl)oxy)methoxy)tetrahydrofuran-2-yl)-4-(4-bromo-N-(((triisopropylsilyl)oxy)methyl)benzamido)pyrimidin-2(1H)-ylidene)-L-lysinate (Compound SS12) (54.33 mg). The obtained crude product, methyl N²-(((9H-fluoren-9-yl)methoxy)carbonyl)-N⁶-(1-((2R,3R,4R,5R)-4-((bis(2-cyanoethoxy)phosphoryl)oxy)-5-(((bis(2-cyanoethoxy)phosphoryl)oxy)methyl)-3-(((triisopropylsilyl)oxy)methoxy)tetrahydrofuran-2-yl)-4-(4-bromo-N-(((triisopropylsilyl)oxy)methyl)benzamido)pyrimidin-2(1H)-ylidene)-L-lysinate (Compound SS12), was directly used in the next step.
LCMS (ESI) m/z = 1534.9 (M+H)+
Retention time: 3.62 minutes (analysis condition SQDFA05long)

### Synthesis of N⁶-(1-((2R,3R,4R,5R)-4-(phosphonooxy-5-((phosphonooxy)methyl)-3-(((triisopropylsilyl)oxy)methoxy)tetrahydrofuran-2-yl)-4-((((triisopropylsilyl)oxy)methyl)amino)pyrimidin-2(1H)-ylidene)-L-lysine (Compound SS13)

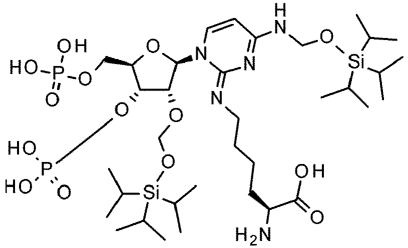

Under nitrogen atmosphere, the crude product, methyl N²-(((9H-fluoren-9-yl)methoxy)carbonyl)-N⁶-(1-((2R,3R,4R,5R)-4-((bis(2-cyanoethoxy)phosphoryl)oxy)-5-(((bis(2-cyanoethoxy)phosphoryl)oxy)methyl)-3-(((triisopropylsilyl)oxy)methoxy)tetrahydrofuran-2-yl)-4-(4-bromo-N-(((triisopropylsilyl)oxy)methyl)benzamido)pyrimidin-2(1H)-ylidene)-L-lysinate (Compound SS12) (54.33 mg, 0.035 mmol), was dissolved in pyridine (2.36 mL) at room temperature, and bis-(trimethylsilyl)acetamide (345.98 µL, 1.42 mmol) and DBU (84.64 µL, 0.57 mmol) were added, then the mixture was stirred at room temperature for 45 minutes. The reaction solution was added with ultrapure water, and washed with diethyl ether and normal hexane. The obtained aqueous layer was added with toluene and acetonitrile, and concentrated under reduced pressure to obtain a crude product, N⁶-(1-((2R,3R,4R,5R)-4-(phosphonooxy)-5-((phosphonooxy)methyl)-3-(((triisopropylsilyl)oxy)methoxy)tetrahydrofuran-2-yl)-4-((((triisopropylsilyl)oxy)methyl)amino)pyrimidin-2(1H)-ylidene)-L-lysine (Compound SS13). The obtained crude product, N⁶-(1-((2R,3R,4R,5R)-4-(phosphonooxy)-5-((phosphonooxy)methyl)-3-(((triisopropylsilyl)oxy)methoxy)tetrahydrofuran-2-yl)-4-((((triisopropylsilyl)oxy)methyl)amino)pyrimidin-2(1H)-ylidene)-L-lysine (Compound SS13), was directly used in the next step.
LCMS (ESI) m/z = 904.7 (M+H)+
Retention time: 0.79 minutes (analysis condition SQDFA05_02)

### Synthesis of N⁶-(4-amino-1-((2R,3R,4S,5R)-3-hydroxy-4-(phosphonooxy)-5-((phosphonooxy)methyl)tetrahydrofuran-2-yl)pyrimidin-2(1H)-ylidene)-L-lysine (Compound SS04, pLp)

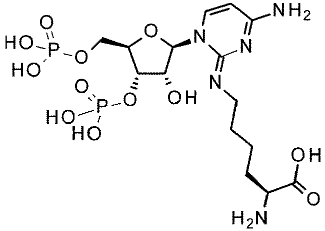

The crude product, methyl N⁶-(1-((2R,3R,4R,5R)-4-(phosphonooxy)-5-((phosphonooxy)methyl)-3-(((triisopropylsilyl)oxy)methoxy)tetrahydrofuran-2-yl)-4-((((triisopropylsilyl)oxy)methyl)amino)pyrimidin-2(1H)-ylidene)-L-lysine (Compound SS13), was dissolved in a mixed solvent of acetonitrile (885 µL) and ultrapure water (885 µL) at room temperature, ammonium fluoride (15.73 mg, 0.43 mmol) was added, and the mixture was stirred at 60°C for 2.5 hours. After returning the mixture to room temperature, an additional ammonium fluoride (15.73 mg, 0.43 mmol) was added, and the mixture was stirred at 60°C for one hour. After returning to room temperature, nitrogen was blown to remove acetonitrile, and the obtained aqueous solution was purified by reverse-phase silica gel column chromatography (aqueous solution of 15 mM TEA and 400 mM HFIP / methanol solution of 15 mM TEA and 400 mM HFIP) to obtain an aqueous solution of N⁶-(4-amino-1-((2R,3R,4S,5R)-3-hydroxy-4-(phosphonooxy)-5-((phosphonooxy)methyl)tetrahydrofuran-2-yl)pyrimidin-2(1H)-ylidene)-L-lysine (Compound SS04, pLp) (100 µL, 17.20 mM).
LCMS (ESI) m/z = 530 (M-H)-
Retention time: 1.64 minutes (analysis condition LTQTEA/HFIP05_02)

### Example 3. Synthesis of lysidine-diphosphate for introducing a lysidine unit at the 3' end of a tRNA fragment by a ligation method - an alternative method

The method for synthesizing the diphosphate of lysidine used for introducing a lysidine unit at the 3' end of a tRNA fragment by a ligation method was improved. More specifically, lysidine-diphosphate (SS04, pLp) was synthesized according to the following scheme.

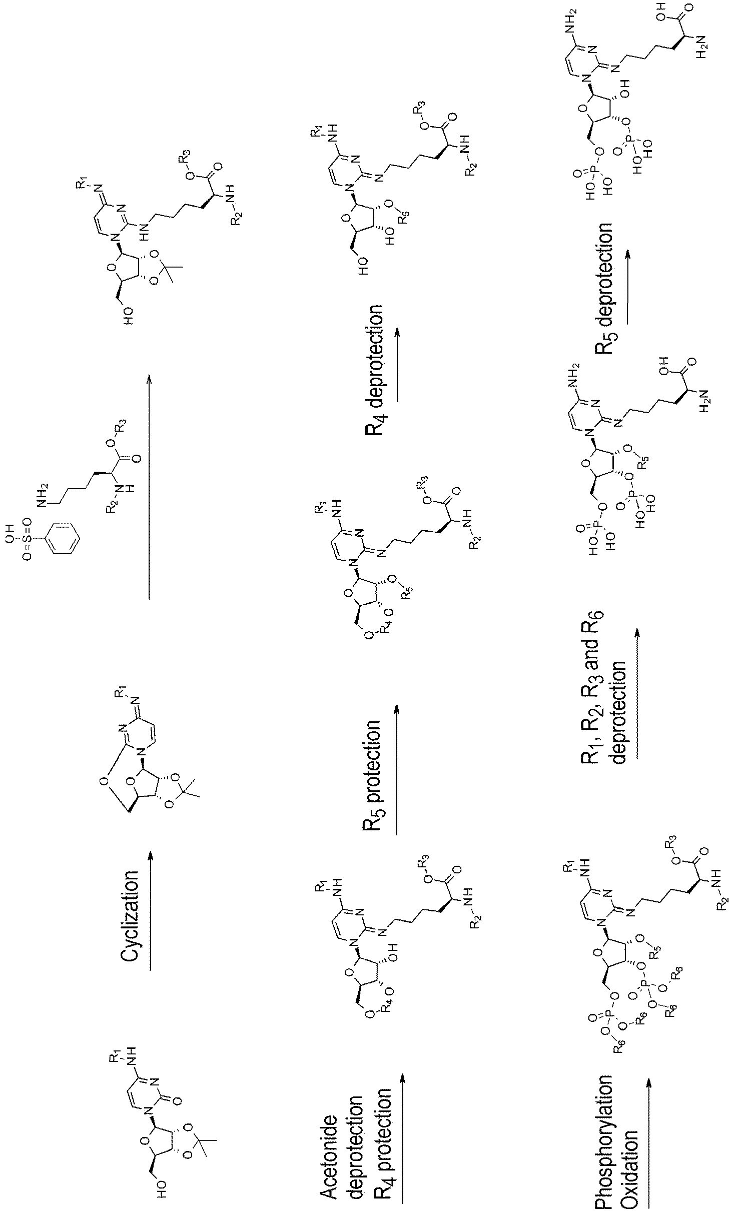

### Synthesis of benzyl ((3aR,4R,12R,12aR)-2,2-dimethyl-3a,4,12,12a-tetrahydro-5H,8H-4,12-epoxy[1,3]dioxolo[4,5-e]pyrimido[2,1-b][1,3]oxazocin-8-ylidene)carbamate (Compound SS24)

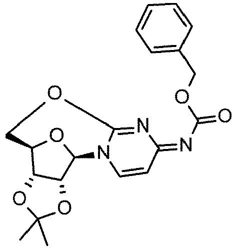

Under nitrogen atmosphere, DCM (17.2 mL) was added to a mixture of 2',3'-O-isopropylidene-4-N-(benzyl-oxy-carbonyl)-cytidine (718.2 mg, 1.72 mmol), which is a literature (Antiviral Chemistry & Chemotherapy, 2003, 14(4), 183-194)-known compound, and triphenylphosphine (474 mg, 1.81 mmol), at room temperature. After cooling the mixture in an ice bath, diisopropyl azodicarbonate (385 µL, 1.98 mmol) was added, then the mixture was warmed to room temperature, and stirred at room temperature for 1.5 hours. The reaction solution was concentrated, toluene (20 mL) was added, and then the produced precipitates were recovered by filtration. The obtained solid was washed three times using toluene to obtain benzyl ((3aR,4R,12R,12aR)-2,2-dimethyl-3a,4,12,12a-tetrahydro-5H,8H-4,12-epoxy[1,3]dioxolo[4,5-e]pyrimido[2,1-b][1,3]oxazocin-8-ylidene)carbamate (Compound SS24) (525.7 mg, 76%).
LCMS (ESI) m/z = 400.3 (M+H)+
Retention time: 0.48 minutes (analysis condition SQDFA05_02)

### Synthesis of benzyl (2S)-6-[[1-(3aR,4R,6R,6aR)-6-(hydroxymethyl)-2,2-dimethyl-3a,4,6,6a-tetrahydrofuro[3,4-d][1,3]dioxol-4-yl]-4-(benzyloxycarbonylamino)pyrimidin-2-ylidene]amino]-2-(benzyloxycarbonylamino)hexanoate; 2,2,2-trifluoroacetic acid (Compound SS25)

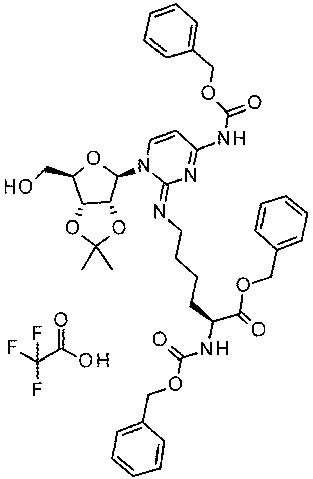

Under nitrogen atmosphere, THF (7.5) was added to a mixture of benzyl ((3aR,4R,12R,12aR)-2,2-dimethyl-3a,4,12,12a-tetrahydro-5H,8H-4,12-epoxy[1,3]dioxolo[4,5-e]pyrimido[2,1-b][1,3]oxazocin-8-ylidene)carbamate (Compound SS24) (300 mg, 0.75 mmol) and lithium chloride (159 mg, 3.76 mmol), at room temperature, and the mixture was cooled in an ice bath. To this mixture, a mixture of benzyl ((benzyloxy)carbonyl)-L-lysinate benzenesulfonate (813 mg, 2.01 mmol) and DBU (673 µL, 4.51 mmol)added with THF (7.5 mL) was added in an ice bath, and the reaction mixture was stirred at 0°C for 30 minutes. DMSO was added to the reaction solution in an ice bath, the mixture was warmed to room temperature, and then the reaction solution was concentrated to remove THF. The residue was purified by reverse-phase silica gel column chromatography (0.05% aqueous TFA solution/0.05% TFA-acetonitrile solution) to obtain benzyl (2S)-6-[[1-[(3aR,4R,6R,6aR)-6-(hydroxymethyl)-2,2-dimethyl-3a,4,6,6a-tetrahydrofuro[3,4-d][1,3]dioxol-4-yl]-4-(benzyloxycarbonylamino)pyrimidin-2-ylidene]amino]-2-(benzyloxycarbonylamino)hexanoate; 2,2,2-trifluoroacetic acid (Compound SS25) (726.6 mg) quantitatively.
LCMS (ESI) m/z = 768.6 (M-H)-
Retention time: 0.74 minutes (analysis condition SQDFA05_02)

### Synthesis of benzyl (2S)-2-(benzyloxycarbonylamino)-6-[[4-(benzyloxyarbonylamino)-1-[(2R,3R,4S,5R)-3,4-dihydroxy-5-(hydroxymethyl)tetrahydrofuran-2-yl]pyrimidin-2-ylidene]amino]hexanoate; 2,2,2-trifluoroacetic acid (Compound SS26)

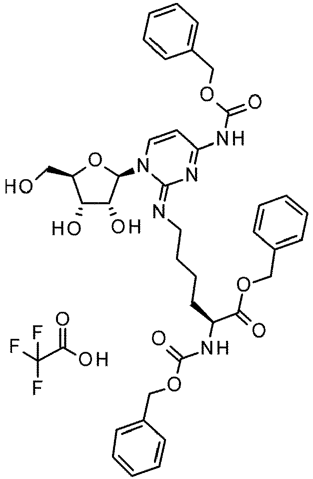

Benzyl (2S)-6-[[1-[(3aR,4R,6R,6aR)-6-(hydroxymethyl)-2,2-dimethyl-3a,4,6,6a-tetrahydrofuro [3,4-d] [1,3]dioxol-4-yl]-4-(benzyloxycarbonylamino)pyrimidin-2-ylidene]amino]-2-(benzyloxycarbonylamino)hexanoate; 2,2,2-trifluoroacetic acid (Compound SS25) (281.1 mg, 0.318 mmol) was dissolved in a mixed solvent of TFA (4.24 mL) and ultrapure water (2.12 mL) while cooling in an ice bath, and the mixture was stirred at room temperature for 50 minutes. Toluene and acetonitrile were added and the reaction solution was concentrated. This operation was repeated multiple times to distill off water and TFA to obtain a crude product, benzyl (2S)-2-(benzyloxycarbonylamino)-6-[[4-(benzyloxyarbonylamino)-1-[(2R,3R,4S,5R)-3,4-dihydroxy-5-(hydroxymethyl)tetrahydrofuran-2-yl]pyrimidin-2-ylidene]amino]hexanoate; 2,2,2-trifluoroacetic acid (Compound SS26) (272.6 mg). The obtained crude product, benzyl (2S)-2-(benzyloxycarbonylamino)-6-[[4-(benzyloxyarbonylamino)-1-[(2R,3R,4S,5R)-3,4-dihydroxy-5-(hydroxymethyl)tetrahydrofuran-2-yl]pyrimidin-2-ylidene]amino]hexanoate; 2,2,2-trifluoroacetic acid (Compound SS26), was directly used in the next step.
LCMS (ESI) m/z = 728.5 (M-H)-
Retention time: 0.69 minutes (analysis condition SQDFA05_02)

### Synthesis of benzyl(2S)-6-[[1-[(4aR,6R,7R,7aS)-2,2-ditert-butyl-7-hydroxy-4a,6,7,7a-tetrahydro-4H-furo[3,2-d][1,3,2]dioxasilin-6-yl]-4-(benzyloxycarbonylamino)pyrimidin-2-ylidene]amino]-2-(benzyloxycarbonylamino)hexanoate;2,2,2-trifluoroacetic acid (Compound SS27)

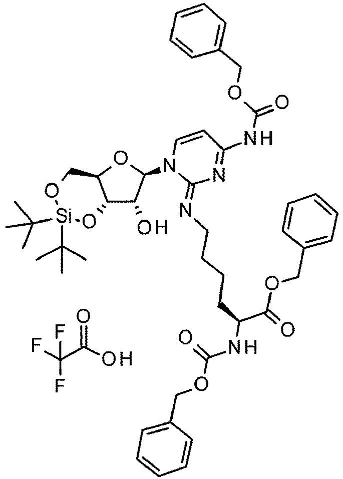

Under nitrogen atmosphere, the crude product obtained in the previous step, benzyl (2S)-2-(benzyloxycarbonylamino)-6-[[4-(benzyloxyarbonylamino)-1-[(2R,3R,4S,5R)-3,4-dihydroxy-5-(hydroxymethyl)tetrahydrofuran-2-yl]pyrimidin-2-ylidene]amino]hexanoate; 2,2,2-trifluoroacetic acid (Compound SS26) (258 mg, 0.306 mmol), was dissolved in DMF (3.06 mL). After the mixture was cooled in an ice bath, di-tert-butylsilyl bis(trifluoromethanesulfonate) (396 µL, 1.22 mmol) was added, and the mixture was stirred in an ice bath for two hours. In an ice bath, saturated aqueous sodium bicarbonate solution was added to the reaction solution, and the obtained mixture was purified by reverse-phase silica gel column chromatography (0.05% aqueous TFA solution/0.05% TFA-acetonitrile solution) to obtain benzyl (2S)-6-[[1-[(4aR,6R,7R,7aS)-2,2-ditert-butyl-7-hydroxy-4a,6,7,7a-tetrahydro-4H-furo[3,2-d][1,3,2]dioxasilin-6-yl]-4-(benzyloxycarbonylamino)pyrimidin-2-ylidene]amino]-2-(benzyloxycarbonylamino)hexanoate; 2,2,2-trifluoroacetic acid (Compound SS27) (234.0 mg, 78%, two steps).
LCMS (ESI) m/z = 868.8 (M-H)-
Retention time: 0.88 minutes (analysis condition SQDFA05_02)

### Synthesis of benzyl (2S)-6-[[1-[(4aR,6R,7R,7aR)-2,2-ditert-butyl-7-tetrahydropyran-2-ylox-4a,6,7,7a-tetrahydro-4H-furo[3,2-d][1,3,2]dioxacillin-6-yl]-4-(benzyloxycarbonylamino)pyrimidin-2-ylidene]amino]-2-(benzyloxycarbonylamino)hexanoate; 2,2,2-trifluoroacetic acid (Compound SS28)

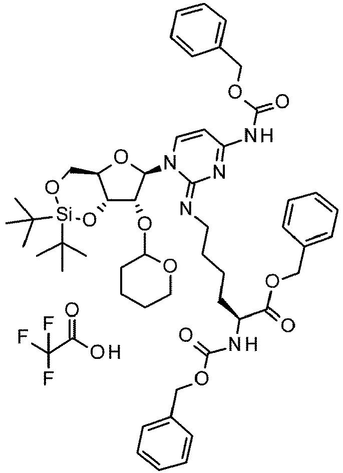

Under nitrogen atmosphere, benzyl (2S)-6-[[1-[(4aR,6R,7R,7aS)-2,2-ditert-butyl-7-hydroxy-4a,6,7,7a-tetrahydro-4H-furo[3,2-d] [1,3,2]dioxasilin-6-yl]-4-(benzyloxycarbonylamino)pyrimidin-2-ylidene]amino]-2-(benzyloxycarbonylamino)hexanoate; 2,2,2-trifluoroacetic acid (Compound SS27) (30 mg, 0.03 mmol) and TFA (6.98 µL, 0.09 mmol) were dissolved in DCM (610 µL) at room temperature, and 3,4-dihydro-2H-pyran (83 µL, 0.915 mmol) was added. After stirring the reaction mixture at room temperature for 13 hours, toluene was added, and the reaction solution was concentrated to obtain a crude product, benzyl (2S)-6-[[1-[(4aR,6R,7R,7aR)-2,2-ditert-butyl-7-tetrahydropyran-2-yloxy-4a,6,7,7a-tetrahydro-4H-furo[3,2-d][1,3,2]dioxacillin-6-yl]-4-(benzyloxycarbonylamino)pyrimidin-2-ylidene]amino]-2-(benzyloxycarbonylamino)hexanoate; 2,2,2-trifluoroacetic acid (Compound SS28), as a mixture of diastereomers derived from the asymmetric carbon on the THP protecting group. The obtained crude product, benzyl (2S)-6-[[1-[(4aR,6R,7R,7aR)-2,2-ditert-butyl-7-tetrahydropyran-2-yloxy-4a,6,7,7a-tetrahydro-4H-furo[3,2-d][1,3,2]dioxacillin-6-yl]-4-(benzyloxycarbonylamino)pyrimidin-2-ylidene]amino]-2-(benzyloxycarbonylamino)hexanoate; 2,2,2-trifluoroacetic acid (Compound SS28), was directly used in the next step.
LCMS (ESI) m/z = 952.8 (M-H)-
Retention time: 3.17 minutes, 3.38 minutes (analysis condition SQDAA05long)

### Synthesis of benzyl (2S)-2-(benzyloxycarbonylamino)-6-[[4-(benzyloxycarbonylamino)-1-[(2R,3R,4R,5R)-4-hydroxy-5-(hydroxymethyl)-3-tetrahydropyran-2-yloxy-tetrahydrofuran-2-yl]pyrimidin-2-ylidene]amino]hexanoate (Compound SS29)

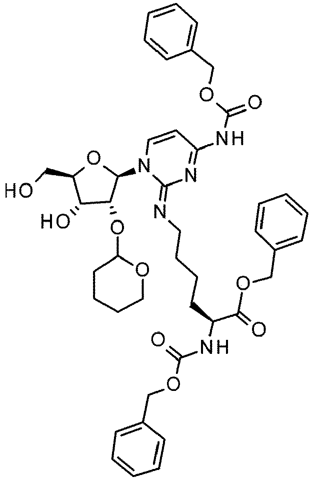

Under nitrogen atmosphere, the crude product obtained in the previous step, benzyl (2S)-6-[[1-[(4aR,6R,7R,7aR)-2,2-ditert-butyl-7-tetrahydropyran-2-yloxy-4a,6,7,7a-tetrahydro-4H-furo[3,2-d][1,3,2]dioxacillin-6-yl]-4-(benzyloxycarbonylamino)pyrimidin-2-ylidene]amino]-2-(benzyloxycarbonylamino)hexanoate; 2,2,2-trifluoroacetic acid (Compound SS28), was dissolved in THF (610 µL) at room temperature, then tetrabutylammonium fluoride (tetrahydrofuran solution of approximately 1 mol/L) (305 µL, approximately 0.305 mmol) was added at room temperature, and the reaction mixture was stirred at room temperature for 30 minutes. The reaction solution was added with DMSO, and then concentrated to distill off THF. The residue was purified by reverse-phase silica gel column chromatography (10 mM aqueous AA solution/ 10 mM AA-acetonitrile solution) to obtain benzyl (2S)-2-(benzyloxycarbonylamino)-6-[[4-(benzyloxycarbonylamino)-1-[(2R,3R,4R,5R)-4-hydroxy-5-(hydroxymethyl)-3-tetrahydropyran-2-yloxy-tetrahydrofuran-2-yl]pyrimidin-2-ylidene]amino]hexanoate (Compound SS29) (21.51 mg, 87%, two steps) as a mixture of diastereomers derived from the asymmetric carbon on the THP protecting group.
LCMS (ESI) m/z = 812.7 (M-H)-
Retention time: 1.74 minutes (analysis condition SQDAA05long)

### Synthesis of benzyl (2S)-2-(benzyloxycarbonylamino)-6-[[4-(benzyloxycarbonylamino)-1-[(2R,3R,4R,5R)-4-dibenzyloxyphosphoryloxy-5-(dibenzyloxyphosphoryloxymethyl)-3-tetrahydropyran-2-yloxy-tetrahydrofuran-2-yl[pyrimidin-2-ylidene]amino]hexanoate (Compound SS30)

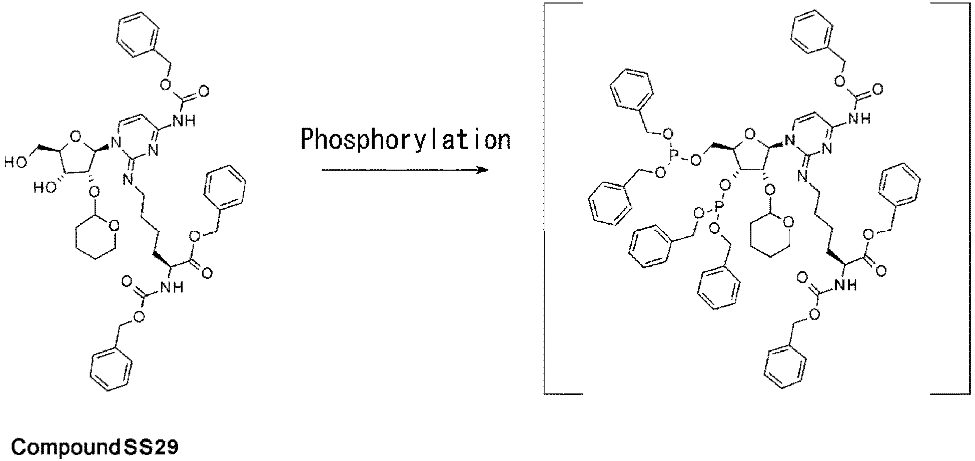

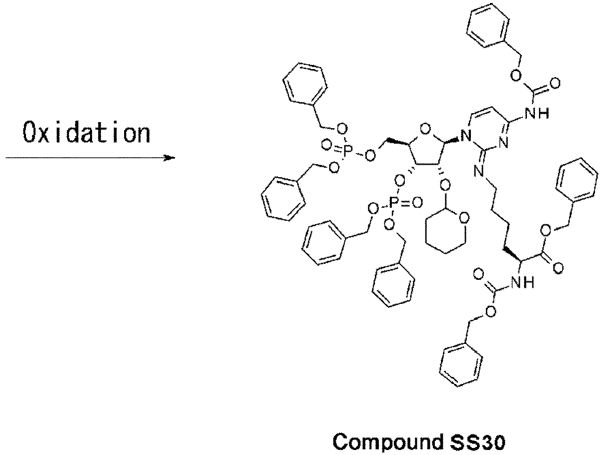

Under nitrogen atmosphere, benzyl (2S)-2-(benzyloxycarbonylamino)-6-[[4-(benzyloxycarbonylamino)-1-[(2R,3R,4R,5R)-4-hydroxy-5-(hydroxymethyl)-3-tetrahydropyran-2-yloxy-tetrahydrofuran-2-yl]pyrimidin-2-ylidene]amino]hexanoate (Compound SS29) (21.51 mg, 0.026 mmol) and 1H-tetrazole (22.22 mg, 0.317 mmol) were dissolved in acetonitrile (1.06 mL) at room temperature, dibenzyl N,N-diisopropylphosphoroamidite (53.2 µL, 0.159 mmol) was added, and the mixture was stirred at room temperature for one hour. The mixture was added with Dess-Martin Periodinane (135 mg, 0.317 mmol) and stirred at room temperature for 15 minutes, then the reaction solution was purified by reverse-phase silica gel column chromatography (10 mM aqueous AA solution / 10 mM AA solution in acetonitrile) to obtain benzyl (2S)-2-(benzyloxycarbonylamino)-6-[[4-(benzyloxycarbonylamino)-1-[(2R,3R,4R,5R)-4-dibenzyloxyphosphoryloxy-5-(dibenzyloxyphosphoryloxymethyl)-3-tetrahydropyran-2-yloxy-tetrahydrofuran-2-yl]pyrimidin-2-ylidene]amino]hexanoate (Compound SS30) (36.59 mg, two steps) quantitatively, as a mixture of diastereomers derived from the asymmetric carbon on the THP protecting group.
LCMS (ESI) m/z = 1332.8 (M-H)-
Retention time: 3.08 minutes, 3.11 minutes (analysis condition SQDAA05long)

### Synthesis of (2S)-2-amino-6-[[4-amino-1-[(2R,3R,4S,SR)-3-hydroxy-4-phosphonooxy-5-(phosphonooxymethyl)tetrahydrofuran-2-yl]pyrimidin-2-ylidene]amino]hexanoic acid (Compound SS04, pLp)

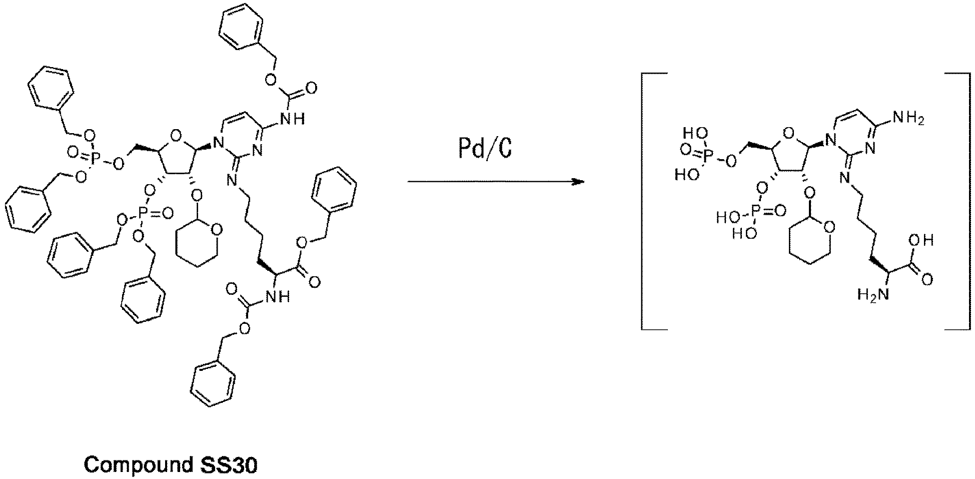

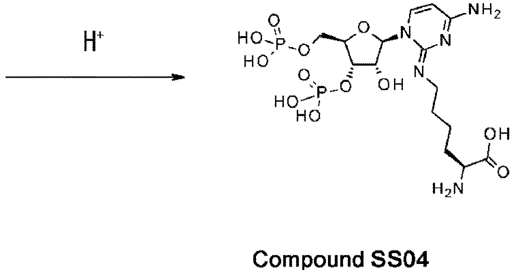

Benzyl (2S)-2-(benzyloxycarbonylamino)-6-[[4-(benzyloxycarbonylamino)-1-[(2R,3R,4R,5R)-4-dibenzyloxyphosphoryloxy-5-(dibenzyloxyphosphoryloxymethyl)-3-tetrahydropyran-2-yloxy-tetrahydrofuran-2-yl]pyrimidin-2-ylidene]amino]hexanoate (Compound SS30) (36.59 mg, 0.027 mmol) was dissolved in a mixed solvent of methanol (649 µL) and ultrapure water (152 µL) at room temperature, and palladium on carbon (10% Pd) (5.84 mg, 5.48 µmol) was added under nitrogen atmosphere. Under hydrogen atmosphere, this mixture was stirred at room temperature for 18 hours. The reaction solution was filtered through Celite, and washed several times using ultrapure water. To the obtained filtrate (24.66 mL), 1 mol/L hydrogen chloride (2.74 mL, 2.74 mmol) was added, and was left to stand at room temperature for one hour. The reaction solution was filtered through Celite, and washed several times using ultrapure water. After freeze-drying the filtrate, the obtained powder was redissolved using ultrapure water (1.52 mL), and then centrifugation was performed and the supernatant was recovered to obtain an aqueous solution of (2S)-2-amino-6-[[4-amino-1-[(2R,3R,4S,5R)-3-hydroxy-4-phosphonooxy-5-(phosphonooxymethyl)tetrahydrofuran-2-yl]pyrimidin-2-ylidene]amino]hexanoic acid (Compound SS04, pLp) (1.37mL, 17.47 mM, 87%, two steps).
LCMS (ESI) m/z = 530.1 (M-H)-
Retention time: 1.60 minutes (analysis condition LTQTEA/HFIP05_02)

Column exchange was performed during the time after analyzing Compound SS04 synthesized in Example 2 and before analyzing Compound SS04 synthesized in Example 3. Compound SS04 synthesized in Example 2 was analyzed again after column exchange, and was confirmed to be the same as Compound SS04 synthesized in Example 3. The results are shown below.
LCMS (ESI) m/z = 530.1 (M-H)-
Retention time: 1.60 minutes (analysis condition LTQTEA/HFIP05_02)

### Example 4. Synthesis of agmatidine-diphosphate for introducing an agmatidine unit at the 3' end of a tRNA fragment by a ligation method

To introduce an agmatidine unit at the 3' end of a tRNA fragment by a ligation method, a diphosphate of agmatidine was synthesized. More specifically, agmatidine-diphosphate (SS31, p(Agm)p) was synthesized according to the following scheme.

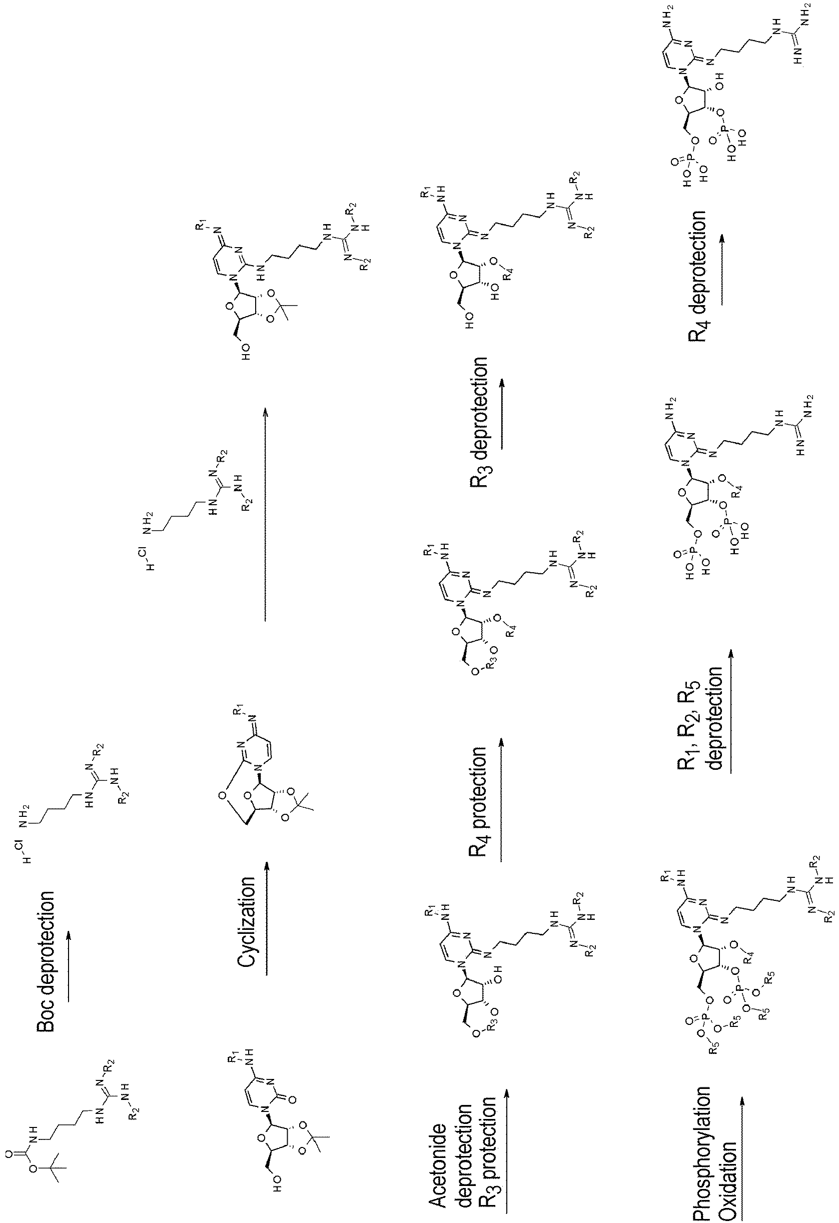

### Synthesis of benzyl N-[(4-aminobutylamino)-(benzyloxycarbonylamino)methylen]carbamate; hydrochloride salt (Compound SS32)

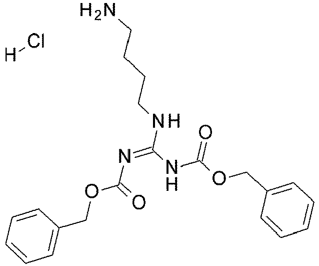

Under nitrogen atmosphere, benzyl N-[benzyloxycarbonylamino-[4-(tertbutoxycarbonylamino)butylamino]methylene]carbamate (241 mg, 0.483 mmol), which is a literature (Chemistry A European Journal, 2015, 21(26), 9370-9379)-known compound, was added with 4 N-HCl/1,4-Dioxane (3.63 mL) in an ice bath, warmed to room temperature, and then stirred for 20 minutes. After adding n-hexane, the reaction solution was concentrated, and benzyl N-[(4-aminobutylamino)-(benzyloxycarbonylamino)methylen]carbamate; hydrochloride salt (Compound SS32) (256.5 mg) was obtained quantitatively.
LCMS (ESI) m/z = 399.4 (M+H)+
Retention time: 0.61 minutes (analysis condition SQDFA05_02)

### Synthesis of benzyl N-[[4-[[1-[(3aR,4R,6R,6aR)-6-(hydroxymethyl)2,2-dimethyl-3a,4,6,6a-tetrahydrofuro[3,4-d][1,3]dioxol-4-yl]-4-(benzyloxycarbonylamino)pyrimidin-2-ylidene]amino]butylamino]-(benzyloxycarbonylamino)methylene]carbamate; 2,2,2-trifluoroacetic acid (Compound SS33)

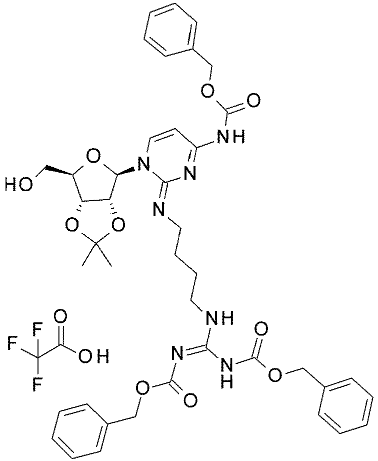

Under nitrogen atmosphere, THF (0.998 mL) was added to a mixture of benzyl N-[(4-aminobutylamino)-(benzyloxycarbonylamino)methylen]carbamate; hydrochloride salt (SS32) (65.1 mg, 0.150 mmol) and DBU (112 µL, 0.748 mmol) at room temperature, and then cooled in an ice bath. To this mixture, a mixture of benzyl ((3aR,4R,12R,12aR)-2,2-dimethyl-3a,4,12,12a-tetrahydro-5H,8H-4,12-epoxy[1,3]dioxolo[4,5-e]pyrimido[2,1-b][1,3]oxazocin-8-ylidene)carbamate (Compound SS24) (49.8 mg, 0.125 mmol) and lithium chloride (26.4 mg, 0.624 mmol) added with THF (1.497 mL) was added in an ice bath, the reaction mixture was pulverized with an ultrasonic cleaner, and then stirred in an ice bath for 60 minutes. DMSO was added to the reaction solution in an ice bath, warmed to room temperature, and then the reaction solution was concentrated to remove THF. The residue was purified by reverse-phase silica gel column chromatography (0.05% aqueous TFA solution/0.05% TFA-acetonitrile solution) to obtain benzyl N-[[4-[[1-[(3aR,4R,6R,6aR)-6-(hydroxymethyl)-2,2-dimethyl-3a,4,6,6a-tetrahydrofuro[3,4-d][1,3]dioxol-4-yl]-4-(benzyloxycarbonylamino)pyrimidin-2-ylidene]amino]butylamino]-(benzyloxycarbonylamino)methylene]carbamate; 2,2,2-trifluoroacetic acid (Compound SS33) (74.0 mg, 65%).
LCMS (ESI) m/z = 796.6 (M-H)-
Retention time: 0.78 minutes (analysis condition SQDFA05_02)

### Synthesis of benzyl N-[benzyloxycarbonylamino-[4-[[4-(benzyloxycarbonylamino)-1-[(2R,3R,4S,5R)-3,4-dihydroxy-5-(hydroxymethyl)tetrahydrofuran-2-yl]pyrimidin-2-ylidene]amino]butylamino]methylene]carbamate; 2,2,2-trifluoroacetic acid (Compound SS34)

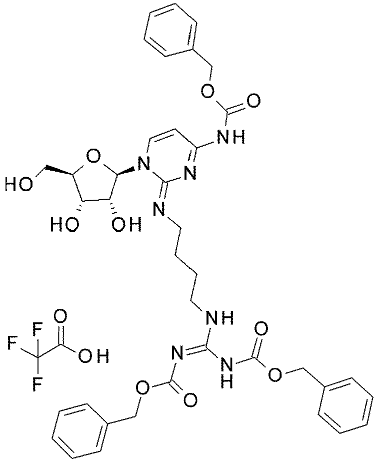

Benzyl N-[[4-[[1-[(3aR,4R,6R,6aR)-6-(hydroxymethyl)-2,2-dimethyl-3a,4,6,6a-tetrahydrofuro[3,4-d][1,3]dioxol-4-yl] -4-(benzyloxycarbonylamino)pyrimidin-2-ylidene]amino]butylamino]-(benzyloxycarbonylamino)methylene]carbamate; 2,2,2-trifluoroacetic acid (Compound SS33) (109.5 mg, 0.120 mmol) was dissolved in a mixed solvent of TFA (1.60 mL) and ultrapure water (0.80 mL) while cooling in an ice bath, and the mixture was stirred at room temperature for 45 minutes. Operation of adding toluene and concentrating the reaction solution was repeated several times to distill off water and TFA to obtain a crude product, benzyl N-[benzyloxycarbonylamino-[4-[[4-(benzyloxycarbonylamino)-1-[(2R,3R,4S,5R)-3,4-dihydroxy-5-(hydroxymethyl)tetrahydrofuran-2-yl]pyrimidin-2-ylidene]amino]butylamino]methylene]carbamate; 2,2,2-trifluoroacetic acid (Compound SS34) (105 mg). The obtained crude product, benzyl N-[benzyloxycarbonylamino-[4-[[4-(benzyloxycarbonylamino)-1-[(2R,3R,4S,5R)-3,4-dihydroxy-5-(hydroxymethyl)tetrahydrofuran-2-yl]pyrimidin-2-ylidene]amino]butylamino]methylene]carbamate; 2,2,2-trifluoroacetic acid (Compound SS34), was directly used in the next step.
LCMS (ESI) m/z = 756.5 (M-H)-
Retention time: 0.71 minutes (analysis condition SQDFA05_02)

### Synthesis of benzyl N-[[4-[[1-[(4aR,6R,7R,7aS)-2,2-ditert-butyl-7-hydroxy-4a,6,7,7a-tetrahydro-4H-furo[3,2-d][1,3,2]dioxacillin-6-yl]-4-(benzyloxycarbonylamino)pyrimidin-2-ylidene]amino]butylamino]-(benzyloxycarbonylamino)methylene]carbamate; 2,2,2-trifluoroacetic acid (Compound SS35)

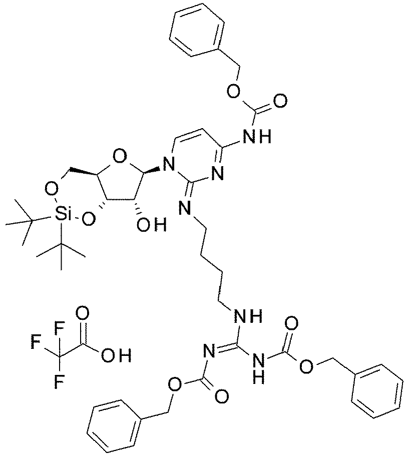

Under nitrogen atmosphere, the crude product obtained in the previous step, benzyl N-[benzyloxycarbonylamino-[4-[[4-(benzyloxycarbonylamino)-l-[(2R,3R,4S,5R)-3,4-dihydroxy-5-(hydroxymethyl)tetrahydrofuran-2-yl]pyrimidin-2-ylidene]amino]butylamino]methylene]carbamate; 2,2,2-trifluoroacetic acid (Compound SS34) (105 mg, 0.120 mmol), was dissolved in DMF (1.20 mL), the mixture was cooled in an ice bath, then added with di-tert-butylsilyl bis(trifluoromethanesulfonate) (78 µL, 0.241 mmol), and stirred in an ice bath for one hour. Additional di-tert-butylsilyl bis(trifluoromethanesulfonate) (78 µL, 0.241 mmol) was added, and was stirred in an ice bath for 30 minutes. Additional di-tert-butylsilyl bis(trifluoromethanesulfonate) (19.5 µL, 0.060 mmol) was further added, and was stirred in an ice bath for 15 minutes. In an ice bath, saturated aqueous sodium bicarbonate solution was added to the reaction solution, and the obtained mixture was purified by reverse-phase silica gel column chromatography (0.05% aqueous TFA solution/0.05% TFA-acetonitrile solution) to obtain benzyl N-[[4-[[1-[(4aR,6R,7R,7aS)-2,2-ditert-butyl-7-hydroxy-4a,6,7,7a-tetrahydro-4H-furo[3,2-d][1,3,2]dioxacillin-6-yl]-4-(benzyloxycarbonylamino)pyrimidin-2-ylidene]amino]butylamino]-(benzyloxycarbonylamino)methylene]carbamate; 2,2,2-trifluoroacetic acid (Compound SS35) (108.02 mg, 89%, two steps).
LCMS (ESI) m/z = 896.7 (M-H)-
Retention time: 0.91 minutes (analysis condition SQDFA05_02)

### Synthesis of benzyl N-[[4-[[1-[(4aR,6R,7R,7aR)-2,2-ditert-butyl-7-tetrahydropyran-2-yloxy-4a,6,7,7a-tetrahydro-4H-furo[3,2-d][1,3,2]dioxacillin-6-yl]-4-(benzyloxycarbonylamino)pyrimidin-2-ylidene]amino]butylamino]-(benzyloxycarbonylamino)methylene]carbamate; 2,2,2-trifluoroacetic acid (Compound SS36)

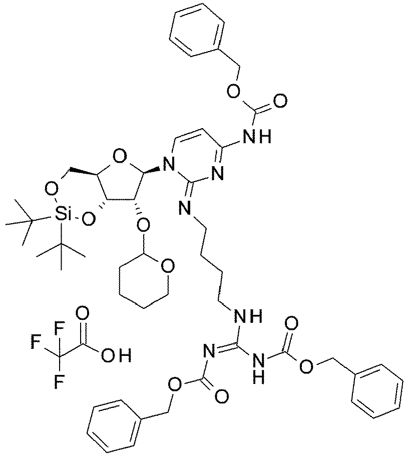

Under nitrogen atmosphere, benzyl N-[[4-[[1-[(4aR,6R,7R,7aS)-2,2-ditert-butyl-7-hydroxy-4a,6,7,7a-tetrahydro-4H-furo[3,2-d][1,3,2]dioxacillin-6-yl]-4-(benzyloxycarbonylamino)pyrimidin-2-ylidene]amino]butylamino]-(benzyloxycarbonylamino)methylene]carbamate; 2,2,2-trifluoroacetic acid (Compound SS35) (64.51 mg, 0.064 mmol) and 3,4-dihydro-2H-pyran (173 µL, 1.912 mmol) were dissolved in DCM (1.28 mL), and after cooling the mixture in an ice bath, TFA (14.60 µL, 0.191 mmol) was added to it. The reaction mixture was warmed to room temperature and stirred for 22.5 hours, then toluene was added, and the reaction solution was concentrated to obtain a crude product, benzyl N-[[4-[[1-[(4aR,6R,7R,7aR)-2,2-ditert-butyl-7-tetrahydropyran-2-yloxy-4a,6,7,7a-tetrahydro-4H-furo[3,2-d][1,3,2]dioxacillin-6-yl]-4-(benzyloxycarbonylamino)pyrimidin-2-ylidene]amino]butylamino]-(benzyloxycarbonylamino)methylene]carbamate; 2,2,2-trifluoroacetic acid (Compound SS36), as a mixture of diastereomers derived from the asymmetric carbon on the THP protecting group. The obtained crude product, benzyl N-[[4-[[1-[(4aR,6R,7R,7aR)-2,2-ditert-butyl-7-tetrahydropyran-2-yloxy-4a,6,7,7a-tetrahydro-4H-furo[3,2-d][1,3,2]dioxacillin-6-yl]-4-(benzyloxycarbonylamino)pyrimidin-2-ylidene]amino]butylamino]-(benzyloxycarbonylamino)methylene]carbamate; 2,2,2-trifluoroacetic acid (Compound SS36), was directly used in the next step.
LCMS (ESI) m/z = 980.9 (M-H)-
Retention time: 3.51 minutes, 3.72 minutes (analysis condition SQDAA50long)

### Synthesis of benzyl N-[benzyloxycarbonylamino-[4-[[4-(benzyloxycarbonylamino)-1-[(2R,3R,4R,5R)-4-hydroxy-5-(hydroxymethyl)-3-tetrahydropyran-2-yloxy-tetrahydrofuran-2-yl[pyrimidin-2-ylidene]amino]butylamino]methylene]carbamate (Compound SS37)

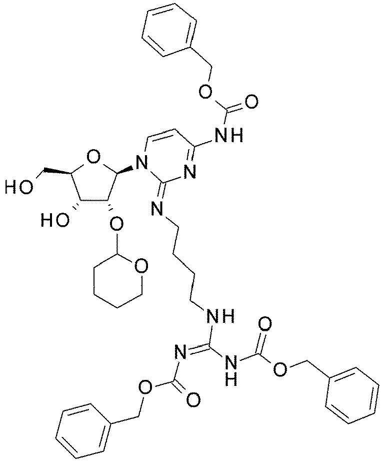

Under nitrogen atmosphere, the crude product obtained in the previous step, benzyl N-[[4-[[1-[(4aR,6R,7R,7aR)-2,2-ditert-butyl-7-tetrahydropyran-2-yloxy-4a,6,7,7a-tetrahydro-4H-furo[3,2-d][1,3,2]dioxacillin-6-yl]-4-(benzyloxycarbonylamino)pyrimidin-2-ylidene]amino]butylamino]-(benzyloxycarbonylamino)methylene]carbamate; 2,2,2-trifluoroacetic acid (Compound SS36), was dissolved in THF (1.28 mL) at room temperature, the mixture was cooled in an ice bath, and tetrabutylammonium fluoride (approximately 1 mol/L solution in tetrahydrofuran) (638 µL, approximately 0.638 mmol) was added. The reaction mixture was warmed to room temperature and stirred for 30 minutes, then DMSO was added to the reaction solution, and concentrated to distill off THF. The residue was purified by reverse-phase silica gel column chromatography (10 mM aqueous AA solution/ 10 mM AA-acetonitrile solution) to obtain benzyl N-[benzyloxycarbonylamino-[4-[[4-(benzyloxycarbonylamino)-1-[(2R,3R,4R,5R)-4-hydroxy-5-(hydroxymethyl)-3-tetrahydropyran-2-yloxy-tetrahydrofuran-2-yl]pyrimidin-2-ylidene]amino]butylamino]methylene]carbamate (Compound SS37) (40.62 mg, 76%, two steps) as a mixture of diastereomers derived from the asymmetric carbon on the THP protecting group.
LCMS (ESI) m/z = 840.7 (M-H)-
Retention time: 2.27 minutes (analysis condition SQDAA50long)

### Synthesis of benzyl N-[benzyloxycarbonylamino-[4-[[4-(benzyloxycarbonylamino)-1-[(2R.3R.4R,5R)-4-dibenzyloxyphosphoryloxy-5-(dibenzyloxyphosphoryloxymethyl)-3-tetrahydropyran-2-yloxy-tetrahydrofuran-2-yl]pyrimidin-2-ylidene]amino]butylamino]methylene]carbamate (Compound SS38)

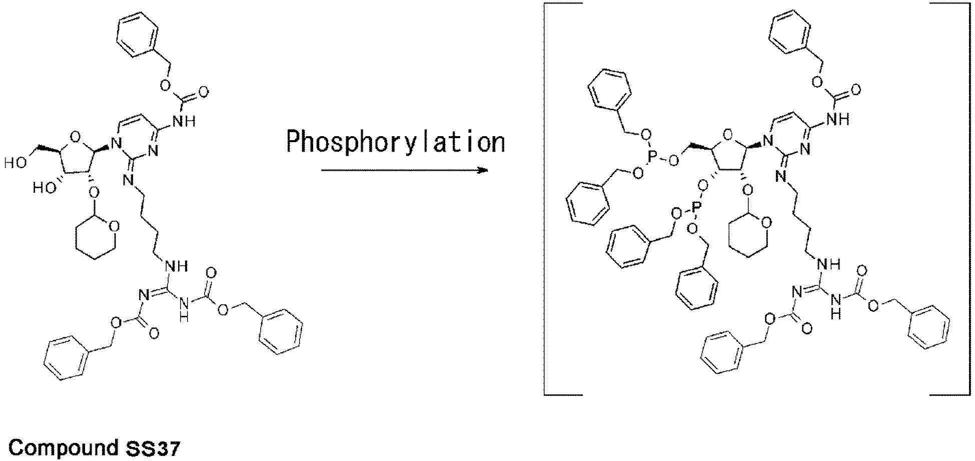

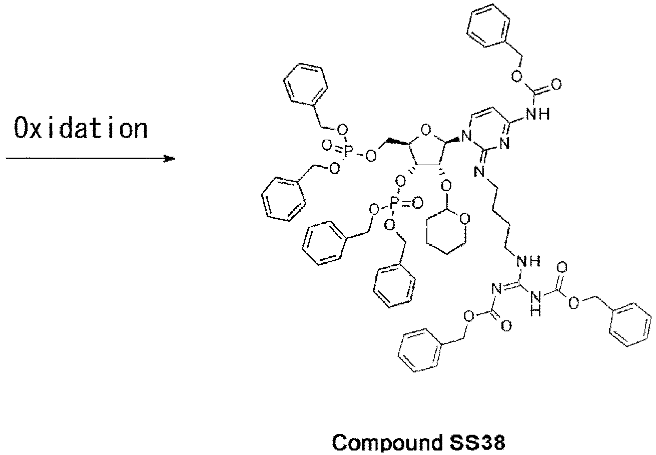

Under nitrogen atmosphere, benzyl N-[benzyloxycarbonylamino-[4-[[4-(benzyloxycarbonylamino)-1-[(2R,3R,4R,5R)-4-hydroxy-5-(hydroxymethyl)-3-tetrahydropyran-2-yloxy-tetrahydrofuran-2-yl]pyrimidin-2-ylidene]amino]butylamino]methylene]carbamate (Compound SS37) (40.62 mg, 0.048 mmol) and 1H-tetrazole (40.6 mg, 0.579 mmol) were dissolved in toluene. The residue was dissolved in acetonitrile (1.93 mL) at room temperature, the mixture was cooled in an ice bath, then dibenzyl N,N-diisopropylphosphoroamidite (97 µL, 0.289 mmol) was added, and the reaction mixture was warmed to room temperature and stirred for 2.5 hours. Dess-Martin Periodinane (246 mg, 0.579 mmol) was added, and stirred at room temperature for 15 minutes, then the reaction solution was purified by reverse-phase silica gel column chromatography (10 mM aqueous AA solution/ 10 mM AA-acetonitrile solution), and benzyl N-[benzyloxycarbonylamino-[4-[[4-(benzyloxycarbonylamino)-1-[(2R,3R,4R,5R)-4-dibenzyloxyphosphoryloxy-5-(dibenzyloxyphosphoryloxymethyl)-3-tetrahydropyran-2-yloxy-tetrahydrofuran-2-yl]pyrimidin-2-ylidene]amino]butylamino]methylene]carbamate (Compound SS38) (59.66 mg, 91%, two steps) was obtained as a mixture of diastereomers derived from the asymmetric carbon on the THP protecting group.
LCMS (ESI) m/z = 1363.0 (M+H)+
Retention time: 4.11 minutes, 4.14 minutes (analysis condition SQDAA05long)

### Synthesis of [(2R,3S,4R,5R)-5-[4-amino-2-(4-guanidinobutylimino)pyrimidin-1-yl]-4-hydroxy-2-(phosphonooxymethyl)tetrahydrofuran-3-yl] dihydrogen phosphate (Compound SS31, p(Agm)p)

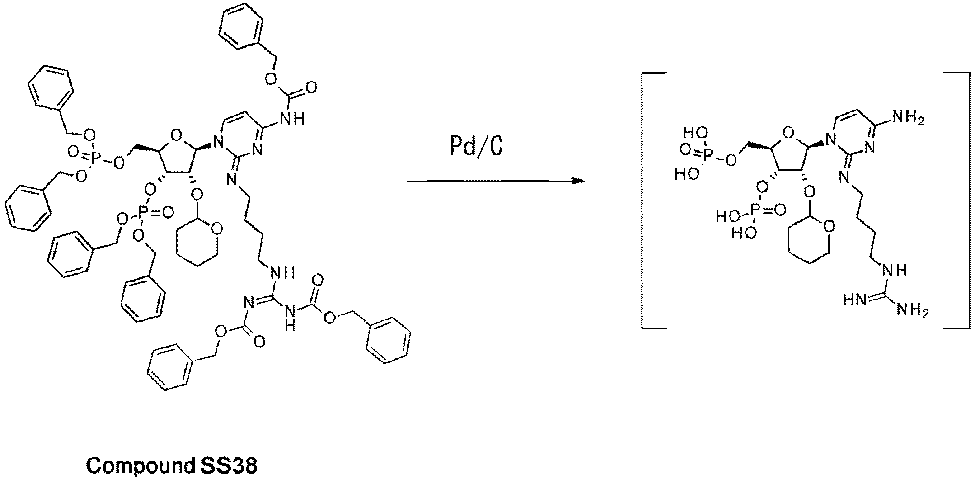

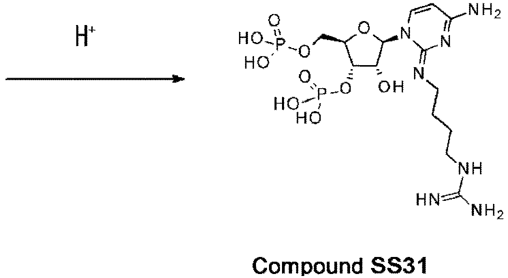

Benzyl N-[benzyloxycarbonylamino-[4-[[4-(benzyloxycarbonylamino)-1-[(2R,3R,4R,5R)-4-dibenzyloxyphosphoryloxy-5-(dibenzyloxyphosphoryloxymethyl)-3-tetrahydropyran-2-yloxy-tetrahydrofuran-2-yl]pyrimidin-2-ylidene]amino]butylamino]methylene]carbamate (Compound SS38) (30.59 mg, 0.022 mmol) was dissolved in a mixed solvent of methanol (727 µL) and ultrapure water (171 µL) at room temperature, and palladium on carbon (10% Pd) (4.78 mg, 4.49 µmol) was added under nitrogen atmosphere. Under hydrogen atmosphere, the mixture was stirred at room temperature for seven hours. The reaction solution was filtered through Celite, and washed several times using ultrapure water. 1 mol/L hydrogen chloride (2.78 mL, 2.78 mmol) was added to the obtained filtrate (25 mL), and was left to stand at room temperature for 45 minutes. The reaction solution was freeze-dried, then the obtained powder was dissolved using ultrapure water, filtered through celite, and washed several times using ultrapure water. After freeze-drying the filtrate, the obtained powder was dissolved using ultrapure water (1.7 mL), the solution was centrifuged and the supernatant was recovered to obtain [(2R,3S,4R,5R)-5-[4-amino-2-(4-guanidinobutylimino)pyrimidin-1-yl]-4-hydroxy-2-(phosphonooxymethyl)tetrahydrofuran-3-yl] dihydrogen phosphate (Compound SS31, p(Agm)p) as an aqueous solution (1.61 mL, 12.11 mM, 87%, two steps).
LCMS (ESI) m/z = 514.1 (M-H)-
Retention time: 1.58 minutes (analysis condition LTQTEA/HFIP05_02)

### Example 5. Synthesis of pCpA-amino acid to be used in a cell-free translation system

Aminoacylated pCpA (SS14, SS15, SS16, SS39, and SS40) was synthesized according to the following scheme.

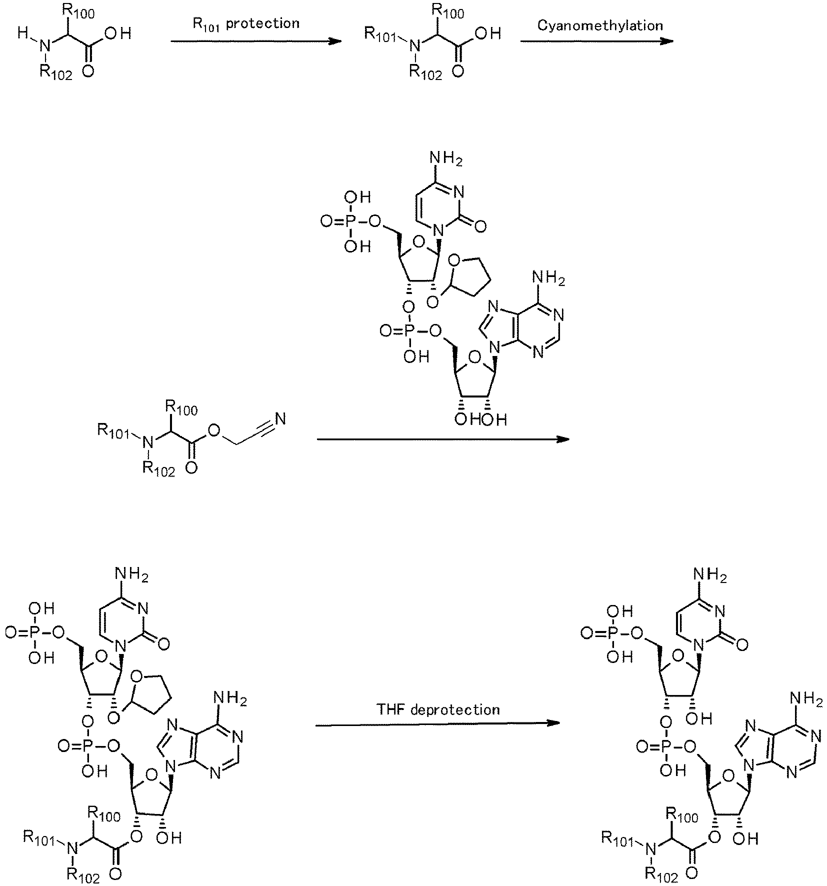

### Synthesis of (S)-1-(((4-(2-(4-fluorophenyl)acetamido)benzyl)oxy)carbonyl)piperidine-2-carboxylic acid (Compound SS17, F-Pnaz-Pic2-OH)

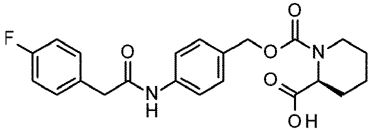

Under nitrogen atmosphere, DMF (330 µL) was added to a mixture of (S)-piperidine-2-carboxylic acid (42.6 mg, 0.33 mmol) and (4-nitrophenyl)-4-(2-(4-fluorophenyl)acetamido)benzyl carbonate (Compound ts11) (140 mg, 0.44 mmol) synthesized by the method of a patent literature (WO2018143145A1) at room temperature. After stirring this mixture at room temperature for five minutes, triethylamine (105.6 µL,2.25 mmol) was added at 0°C. The reaction mixture was stirred at room temperature for 30 minutes, and then purified by reverse-phase silica gel column chromatography (0.1% aqueous formic acid solution/0.1% formic acid-acetonitrile solution) to obtain (S)-1-(((4-(2-(4-fluorophenyl)acetamido)benzyl)oxy)carbonyl)piperidine-2-carboxylic acid (Compound SS17, F-Pnaz-Pic2-OH) (92 mg, 67%).
LCMS (ESI) m/z = 413 (M-H)⁻
Retention time: 0.70 minutes (analysis condition SQDFA05_01)

### Synthesis of 1-(4-(2-(4-fluorophenyl)acetamido)benzyl) 2-(cyanomethyl) (S)-piperidine-1,2-dicarboxylate (Compound SS18, F-Pnaz-Pic2-OCH₂CN)

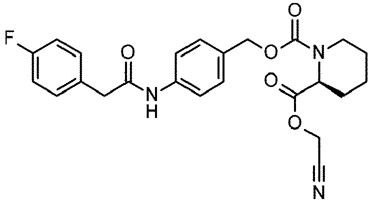

Under nitrogen atmosphere, (S)-1-(((4-(2-(4-fluorophenyl)acetamido)benzyl)oxy)carbonyl)piperidine-2-carboxylic acid (Compound SS17, F-Pnaz-Pic2-OH) (30 mg, 0.072 mmol) and N-ethyl-isopropylpropan-2-amine (DIPEA) (20.23 µL, 0.116 mmol) were dissolved in acetonitrile (90 µL), added with 2-bromoacetonitrile (5.34 µL, 0.080 mmol) at 0°C, and the mixture was stirred at room temperature for two hours. The reaction solution was concentrated to obtain a crude product, 1-(4-(2-(4-fluorophenyl)acetamido)benzyl) 2-(cyanomethyl) (S)-piperidine-1,2-dicarboxylate (Compound SS18, F-Pnaz-Pic2-OCH₂CN). The obtained crude product was dissolved in acetonitrile (2.00 mL), and was directly used in the next step.
LCMS (ESI) m/z = 452 (M-H)⁻
Retention time: 0.79 minutes (analysis condition SQDFA05_01)

### Synthesis of 1-(4-(2-(4-fluoro-ohenyl)acetamido)benal) 2-((2R,3S,4R,5R)-2-((((((2R,3S,4R,5R)-5-(4-amino-2-oxopyrimidin-1(2H)-yl-4-hydroxy-2-((phosphonooxy)methyl)tetrahydrofuran-3-yl)oxy)(hydroxy)phosphoryl)oxy)methyl)-5-(6-amino-9H -purin-9-yl)-4-hydroxytetrahydrofuran-3-yl) (2S)-piperidine-1,2-dicarboxylate (Compound SS14, F-Pnaz-Pic2-pCpA)

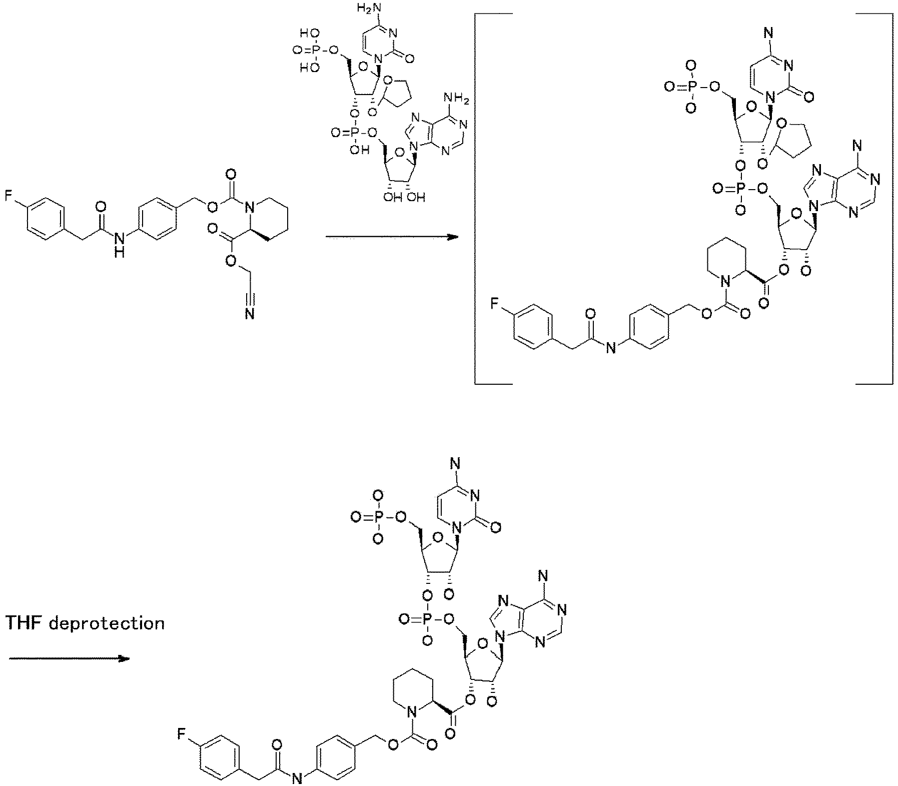

((2R,3R,4R,5R)-5-(4-amino-2-oxopyrimidin-1(2H)-yl)-3-(((((2R,3 S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogen phosphate (Compound pc01) (113 mg, 0.156 mmol) synthesized by a method described in a literature (Helv. Chim. Acta, 90, 297-310) was dissolved in Buffer A (40 mL), a solution of 1-(4-(2-(4-fluorophenyl)acetamido)benzyl) 2-(cyanomethyl) (S)-piperidine-1,2-dicarboxylate (Compound SS18, F-Pnaz-Pic2-OCH₂CN) (35.4 mg, 0.078 mmol) in acetonitrile (2.00 mL) was added, and the mixture was stirred at room temperature for 150 minutes. The reaction solution was cooled to 0°C, and then trifluoroacetic acid (2.00 mL) was added. The reaction solution was stirred at 0°C for 45 minutes, and then purified by reverse-phase silica gel column chromatography (0.05% aqueous trifluoroacetic acid solution/ 0.05% trifluoroacetic acid-acetonitrile) to obtain the title compound (Compound SS14, F-Pnaz-Pic2-pCpA) (6.0 mg, 7.3%).
LCMS (ESI) m/z = 1047.5 (M-H)-
Retention time: 0.50 minutes (analysis condition SQDFA05_01)

Buffer A was prepared as follows.

Acetic acid was added to an aqueous solution of N,N,N-trimethylhexadecan-1-aminium chloride (6.40 g, 20 mmol) and imidazole (6.81 g, 100 mmol) to give Buffer A (1L) of 20 mM N,N,N-trimethylhexadecan-1-aminium and 100 mM imidazole at pH8.

### Synthesis of O-(2-chloro-ohenyl)-N-(((4-(2-(4-fluorophenyl)acetamido)benzyl)oxy)carbonyl)-L-serine (Compound SS19, F-Pnaz-SPh2Cl-OH)

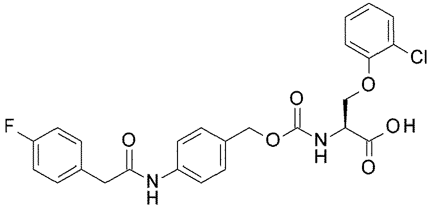

Under nitrogen atmosphere, DMSO (15 mL) and triethylamine (0.95 g, 9.42 mmol) were added to a mixture of O-(2-chlorophenyl)-L-serine (Compound aa63) (1.25 g, 5.80 mmol) synthesized by a method described in a patent literature (WO2018225864) and (4-nitrophenyl)-4-(2-(4-fluorophenyl)acetamido)benzyl carbonate (Compound ts11) (2 g, 4.71 mmol) synthesized by a method described in a patent literature (WO2018143145A1) at room temperature. The reaction mixture was stirred at room temperature for 16 hours and then purified by reverse-phase silica gel column chromatography (0.1% aqueous formic acid solution/0.1% formic acid-acetonitrile solution) to obtain O-(2-chlorophenyl)-N-(((4-(2-(4-fluorophenyl)acetamido)benzyl)oxy)carbonyl)-L-serine (Compound SS19, F-Pnaz-SPh2Cl-OH) (1.8 g, 73%).
LCMS (ESI) m/z = 523 (M+Na)+
Retention time: 1.26 minutes (analysis condition SMD method 1)

### Synthesis of cyanomethyl O-(2-chlorophenyl)-N-(((4-(2-(4-fluorophenyl)acetamido)benzyl)oxy)carbonyl)-L-serinate(CompoundSS20, F-Pnaz-SPh2Cl-OCH₂CN)

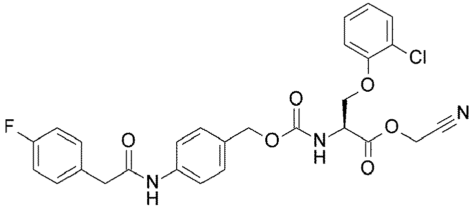

Under nitrogen atmosphere, O-(2-chlorophenyl)-N-(((4-(2-(4-fluorophenyl)acetamido)benzyl)oxy)carbonyl)-L-serine (Compound SS19, F-Pnaz-SPh2Cl-OH) (800 mg, 1.60 mmol) and N-ethyl-isopropylpropan-2-amine (DIPEA) (0.412 g, 3.19 mmol) were dissolved in DCM (15 mL), 2-bromoacetonitrile (760 mg, 6.34 mmol) was added at room temperature, and the mixture was stirred at room temperature for 16 hours. The reaction solution was concentrated and purified by reverse-phase silica gel column chromatography (0.1% aqueous formic acid solution/0.1% formic acid-acetonitrile solution) to obtain cyanomethyl O-(2-chlorophenyl)-N-(((4-(2-(4-fluorophenyl)acetamido)benzyl)oxy)carbonyl)-L-serinate (Compound SS20, F-Pnaz-SPh2Cl-OCH₂CN) (220 mg, 26%). The obtained product was dissolved in acetonitrile (5 mL), and used in the next step.
LCMS (ESI) m/z = 562 (M+Na)+
Retention time: 1.15 minutes (analysis condition SMD method 2)

### Synthesis of (2R,3S,4R,5R)-2-((((((2R,3S,4R,5R)-5-(4-amino-2-oxopyrimidin-1(2H)-yl-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl O-(2-chlorophenyl)-N-(((4-(2-(4-fluorophenyl)acetamido)benzyl)oxy)carbonyl)-L-serinate(Compound SS15, F-Pnaz-SPh2Cl-pCpA)

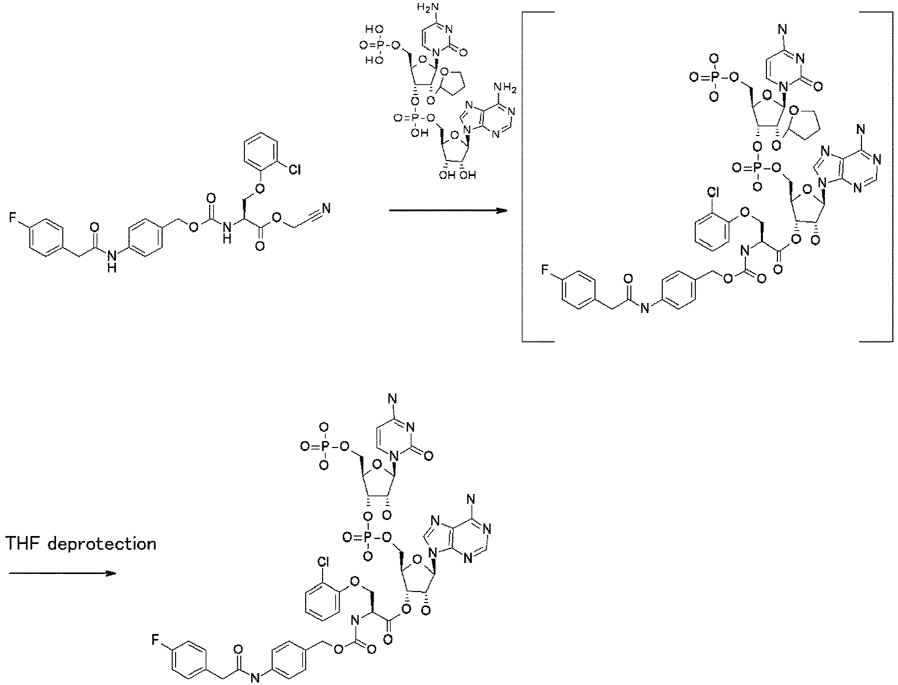

((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogen phosphate (Compound pc01) (400 mg, 0.55 mmol) was dissolved in Buffer A (100 mL), a solution of cyanomethyl O-(2-chlorophenyl)-N-(((4-(2-(4-fluorophenyl)acetamido)benzyl)oxy)carbonyl)-L-serinate (Compound SS20, F-Pnaz-SPh2Cl-OCH₂CN) (220 mg, 0.41 mmol) in acetonitrile (5 mL) was added to it dropwise over 15 minutes or longer using a syringe pump, and this was stirred at room temperature for five minutes. Next, trifluoroacetic acid (2.3 mL) was added to the reaction solution. The reaction solution was freeze-dried, and then purified by reverse-phase silica gel column chromatography (0.05% aqueous trifluoroacetic acid solution/ 0.05% trifluoroacetic acid-acetonitrile) to obtain the title compound (Compound SS15, F-Pnaz-SPh2Cl-pCpA) (20.7 mg, 2%).
LCMS (ESI) m/z = 1133.4 (M-H)-
Retention time: 0.55 minutes (analysis condition SQDFA05_01)

### Synthesis of ((S)-2-(methylamino)-4-phenylbutanoic acid (Compound SS21, MeHph-OH)

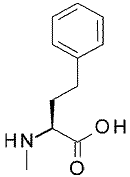

DCM (903 µL), water (903 µL), and piperidine (178 µL, 1.805 mmol) were added to (S)-2-((((9H-fluoren-9-yl)methoxy)carbonyl)(methyl)amino)-4-phenylbutanoic acid (Compound aa11) (150 mg, 0.361 mmol) synthesized by a method described in a patent literature (WO2018225864) at room temperature. The reaction mixture was stirred at room temperature for 30 minutes and then purified by reverse-phase silica gel column chromatography (0.1% aqueous formic acid solution/ 0.1% formic acid-acetonitrile solution) to obtain ((S)-2-(methylamino)-4-phenylbutanoic acid (Compound SS21, MeHph-OH) (55 mg, 79%).
LCMS (ESI) m/z = 192 (M-H)-
Retention time: 0.15 minutes (analysis condition SQDFA05_02)

### Synthesis of (S)-2-((((4-(2-(4-fluorophenyl)acetamido)benzyl)oxy)carbonyl)(methyl)amino)-4-phenylbutanoic acid (Compound SS22, F-Pnaz-MeHph-OH)

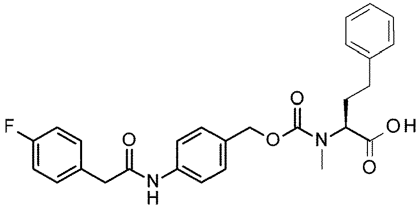

Under nitrogen atmosphere, DMSO (727 µL) was added to a mixture of ((S)-2-(methylamino)-4-phenylbutanoic acid (Compound SS21, MeHph-OH) (35.1 mg, 0.182 mmol) and (4-nitrophenyl)-4-(2-(4-fluorophenyl)acetamido)benzyl carbonate (Compound ts11) (85 mg, 0.20 mmol) synthesized a method described in by a patent literature (WO2018143145A1) at room temperature. Triethylamine (76 µL, 0.545 mmol) was added at 50°C. The reaction mixture was stirred at 40°C for 16 hours, and then purified by reverse-phase silica gel column chromatography (0.1% aqueous formic acid solution/ 0.1% formic acid-acetonitrile solution) to obtain (S)-2-((((4-(2-(4-fluorophenyl)acetamido)benzyl)oxy)carbonyl)(methyl)amino)-4-phenylbutanoic acid (Compound SS22, F-Pnaz-MeHph-OH) (80 mg, 92%).
LCMS (ESI) m/z = 477 (M-H)-
Retention time: 0.85 minutes (analysis condition SQDFA05_02)

### Synthesis of cyanomethyl (S)-2-((((4-(2-(4-fluorophenyl)acetamido)benzyl)oxy)carbonyl)(methyl)amino)-4-phenylbutanoate (Compound SS23, F-Pnaz-MeHph-OCH₂CN)

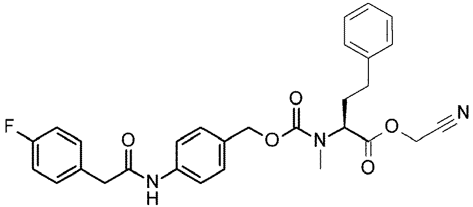

Under nitrogen atmosphere, acetonitrile (533 µL) was added to a mixture of (S)-2-((((4-(2-(4-fluorophenyl)acetamido)benzyl)oxy)carbonyl)(methyl)amino)-4-phenylbutanoic acid (Compound SS22, F-Pnaz-MeHph-OH) (77 mg, 0.16 mmol) and N-ethyl-isopropylpropan-2-amine (DIPEA) (31 µL,0.176 mmol) at room temperature. Then, 2-bromoacetonitrile (86 µL, 1.280 mmol) was added at room temperature, and the reaction mixture was stirred at 40°C for one hour. The reaction solution was concentrated to obtain a crude product, cyanomethyl (S)-2-((((4-(2-(4-fluorophenyl)acetamido)benzyl)oxy)carbonyl)(methyl)amino)-4-phenylbutanoate (Compound SS23, F-Pnaz-MeHph-OCH₂CN). The obtained crude product was dissolved in acetonitrile (5.00 mL) and was directly used in the next step.
LCMS (ESI) m/z = 516 (M-H)-
Retention time: 0.92 minutes (analysis condition SQDFA05_02)

### Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl (2S)-2-((((4-(2-(4-fluorophenyl)acetamido)benzyl)oxy)carbonyl)(methyl)amino)-4-phenylbutanoate (Compound SS16, F-Pnaz-MeHph-pCpA)

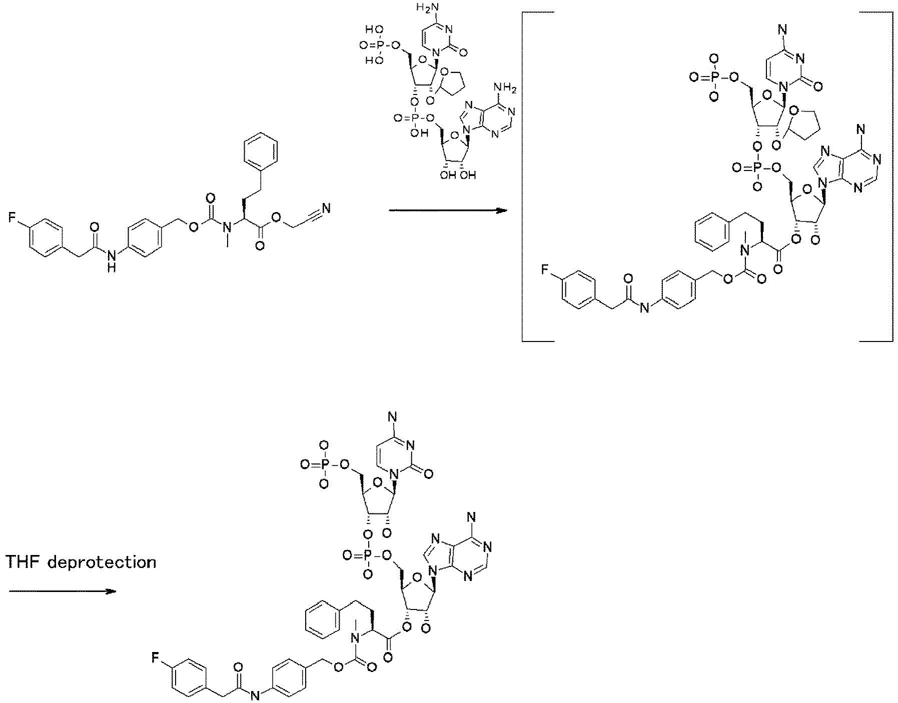

((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)hydroxy)phosphoryl)oxy)-4-((tetrahydrofuran-2-yl)oxy)tetrahydrofuran-2-yl)methyl dihydrogen phosphate (Compound pc01) (127 mg, 0.176 mmol) was dissolved in Buffer A (100 mL), a solution of cyanomethyl (S)-2-((((4-(2-(4-fluorophenyl)acetamido)benzyl)oxy)carbonyl)(methyl)amino)-4-phenylbutanoate (Compound SS23, F-Pnaz-MeHph-OCH₂CN) (83 mg, 0.16 mmol) in acetonitrile (5.00 mL) was added, and the mixture was stirred at room temperature for one hour. The reaction solution was cooled to 0°C, and then trifluoroacetic acid (5.00 mL) was added. The reaction solution was stirred at 0°C for one hour, and then purified by reverse-phase silica gel column chromatography (0.05% aqueous trifluoroacetic acid solution/ 0.05% trifluoroacetic acid-acetonitrile), and then further purified by reverse-phase silica gel column chromatography (0.1% aqueous formic acid solution/ 0.1% formic acid acetonitrile solution) to obtain the title compound (Compound SS16, F-Pnaz-MeHph-pCpA) (26 mg, 14.6%).
LCMS (ESI) m/z = 1111.5 (M-H)-
Retention time: 0.64 minutes (analysis condition SQDFA05_02)

### Synthesis of (S)-3-(3-chlorophenyl)-2-((((4-(2-(4-fluorophenyl)acetamido)benzyl)oxy)carbonyl)amino)propanoic acid (Compound SS41, F-Pnaz-F3Cl-OH)

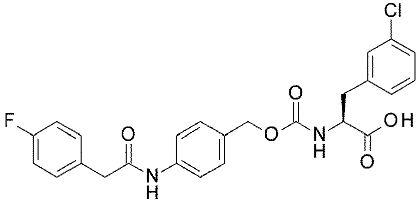

Under nitrogen atmosphere, DMSO (15 mL) and triethylamine (1.43 g, 14.13 mmol) were added to a mixture of (S)-2-amino-3-(3-chlorophenyl)propanoic acid (H-Phe(3-Cl)-OH) (2.17 g, 10.87 mmol) synthesized by a method described in a patent literature (WO2018225864) and (4-nitrophenyl)-4-(2-(4-fluorophenyl)acetamido)benzyl carbonate (Compound ts11) (3.0 g, 7.07 mmol) synthesized by a method described in a patent literature (WO2018143145A1) at room temperature. The reaction mixture was stirred at room temperature for 16 hours, and then purified by reverse-phase silica gel column chromatography (0.1% aqueous formic acid solution/ 0.1% formic acid-acetonitrile solution) to obtain (S)-3-(3-chlorophenyl)-2-((((4-(2-(4-fluorophenyl)acetamido)benzyl)oxy)carbonyl)amino)propanoic acid (Compound SS41, F-Pnaz-F3Cl-OH) (0.7 g, 20%).
LCMS (ESI) m/z = 507 (M+Na)+
Retention time: 1.06 minutes (analysis condition SMD method 3)

### Synthesis of cyanomethyl (S)-3-(3-chlorophenyl)-2-((((4-(2-(4-fluorophenyl)acetamido)benzyl)oxy)carbonyl)amino)propanoate (Compound SS42, F-Pnaz-F3Cl-OCH₂CN)

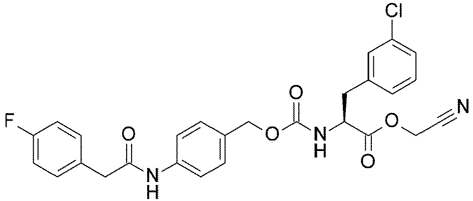

Under nitrogen atmosphere, (S)-3-(3-chlorophenyl)-2-((((4-(2-(4-fluorophenyl)acetamido)benzyl)oxy)carbonyl)amino)propanoic acid (Compound SS41, F-Pnaz-F3Cl-OH) (650 mg, 1.34 mmol) and N-ethyl-isopropylpropan-2-amine (DIPEA) (0.346 g, 2.68 mmol) were dissolved in DCM (28 mL), 2-bromoacetonitrile (640 mg, 5.34 mmol) was added at room temperature, and the mixture was stirred at room temperature for 48 hours. The reaction solution was concentrated and purified by normal phase silica gel column chromatography (ethyl acetate/ petroleum ether) to obtain cyanomethyl (S)-3-(3-chlorophenyl)-2-((((4-(2-(4-fluorophenyl)acetamido)benzyl)oxy)carbonyl)amino)propanoate (Compound SS42, F-Pnaz-F3Cl-OCH₂CN) (330 mg, 47%).
LCMS (ESI) m/z = 546 (M+Na)+
Retention time: 1.13 minutes (analysis condition SMD method 3)

### Synthesis of (2R,3S,4R,5R)-2-((((((2R,3S,4R,5R)-5-(4-amino-2-oxo-pydmidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuan-3-yl(2S)-3-(3-chlorophenyl)-2-((((4-(2-(4-fluorophenyl)acetamido)benzyl)oxy)carbonyl)amino)propanoate (Compound SS39, F-Pnaz-F3Cl-pCpA)

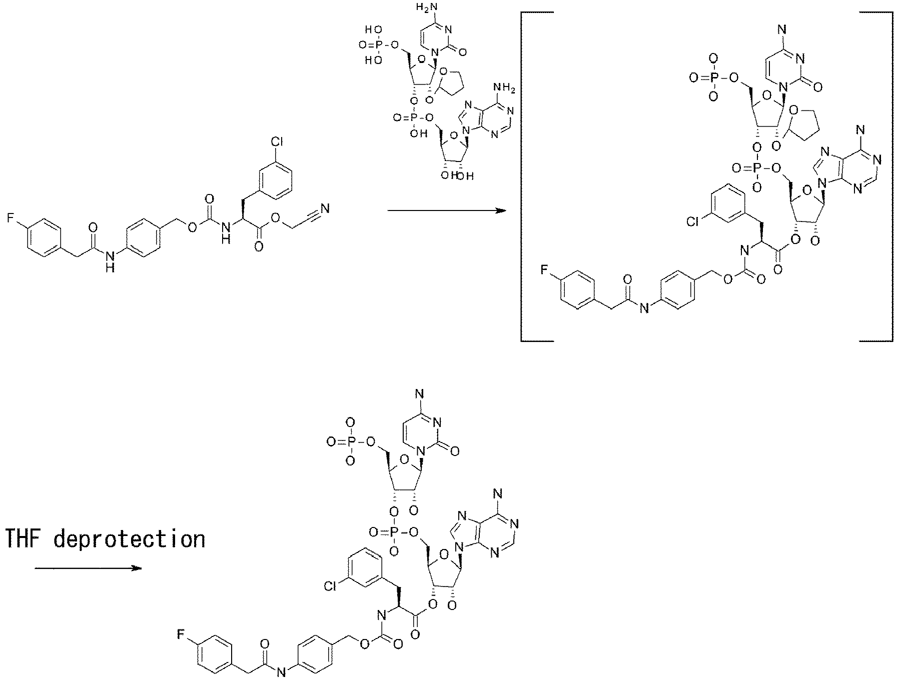

((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)hydroxy)phosphoryl)oxy)-4-((tetrahydrofuran-2-yl)oxy)tetrahydrofuran-2-yl)methyl dihydrogen phosphate (Compound pc01) (552 mg, 0.76 mmol) synthesized by a method described in a literature (Helv. Chim. Acta, 90, 297-310) was dissolved in Buffer A (100 mL), a solution of cyanomethyl (S)-3-(3-chlorophenyl)-2-((((4-(2-(4-fluorophenyl)acetamido)benzyl)oxy)carbonyl)amino)propanoate (Compound SS42, F-Pnaz-F3Cl-OCH₂CN) (200 mg, 0.38 mmol) in acetonitrile (5 mL) was added to it dropwise over 15 minutes or longer using a syringe pump, and this was stirred at room temperature for 30 minutes. Trifluoroacetic acid (2.3 mL) was added to the reaction solution. The reaction solution was freeze-dried, and then purified by reverse-phase silica gel column chromatography (0.05% aqueous trifluoroacetic acid solution/ 0.05% trifluoroacetic acid-acetonitrile) to obtain the title compound (Compound 39, F-Pnaz-F3Cl-pCpA) (25.3 mg, 1%).
LCMS (ESI) m/z = 1117.4 (M-H)-
Retention time: 0.55 minutes (analysis condition SQDFA05_01)

### Synthesis of N-(((4-(2-(4-fluorophenyl)acetamido)benzyl)oxy)carbonyl)-O-isopentyl-L-serine (Compound SS43, F-Pnaz-SiPen-OH)

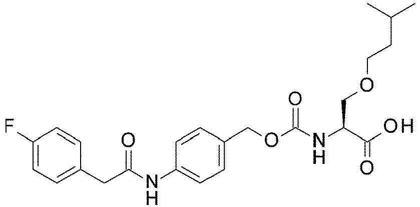

Under nitrogen atmosphere, DMSO (15 mL) and triethylamine (1. 3 mL, 9.42 mmol) were added to a mixture of O-isopentyl-L-serine (H-Ser(iPen)-OH) (1 g, 5.71 mmol) which is described in a patent literature (WO2018225864) and (4-nitrophenyl)-4-(2-(4-fluorophenyl)acetamido)benzyl carbonate (Compound ts11) (2 g, 4.71 mmol) synthesized by a method described in a patent literature (WO2018143145A1) at room temperature. The reaction mixture was stirred at room temperature for 16 hours, and then purified by reverse-phase silica gel column chromatography (0.1% aqueous formic acid solution/ 0.1% formic acid-acetonitrile solution) to obtain N-(((4-(2-(4-fluorophenyl)acetamido)benzyl)oxy)carbonyl)-O-isopentyl-L-serine (Compound SS43, F-Pnaz-SiPen-OH) (1.8 g, 83%).
LCMS (ESI) m/z = 483 (M+Na)+
Retention time: 1.04 minutes (analysis condition SMD method 3)

### Synthesis of cyanomethyl N-(((4-(2₋(4-fluorophenyl)acetamido)benzyl)oxy)carbonyl)-O-isopentyl-L-serinate (Compound SS44, F-Pnaz-SiPen-OCH₂CN)

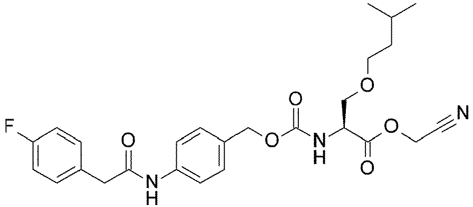

Under nitrogen atmosphere, N-(((4-(2-(4-fluorophenyl)acetamido)benzyl)oxy)carbonyl)-O-isopentyl-L-serine (Compound SS43, F-Pnaz-SiPen-OH) (1.8 g, 3.91 mmol) and N-ethyl-isopropylpropan-2-amine (DIPEA) (1 g, 7.74 mmol) were dissolved in DCM (40 mL), 2-bromoacetonitrile (1.9 g, 15.84 mmol) was added at room temperature, and the mixture was stirred at room temperature for 48 hours. The reaction solution was concentrated and purified by normal phase silica gel column chromatography (ethyl acetate/ petroleum ether) to obtain cyanomethyl_N-(((4-(2-(4-fluorophenyl)acetamido)benzyl)oxy)carbonyl)-O-isopentyl-L-serinate (Compound SS44, F-Pnaz-SiPen-OCH₂CN) (1.6 g, 82%).
LCMS (ESI) m/z = 522 (M+Na)+
Retention time: 1.35 minutes (analysis condition SMD method 4)

### Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuan-3-yl)oxy)(hydroxy)phosphoryl)oxy)methyl)-5-(6-amino-9H-purin-9-yl)-4-hydroxytetrahydrofuran-3-yl N-(((4-(2-(4-fluoro-phenyl)acetamido)benzyl)oxy)carbonyl)-O-isopentyl-L-serinate (Compound SS40, F-Pnaz-SiPen-pCpA)

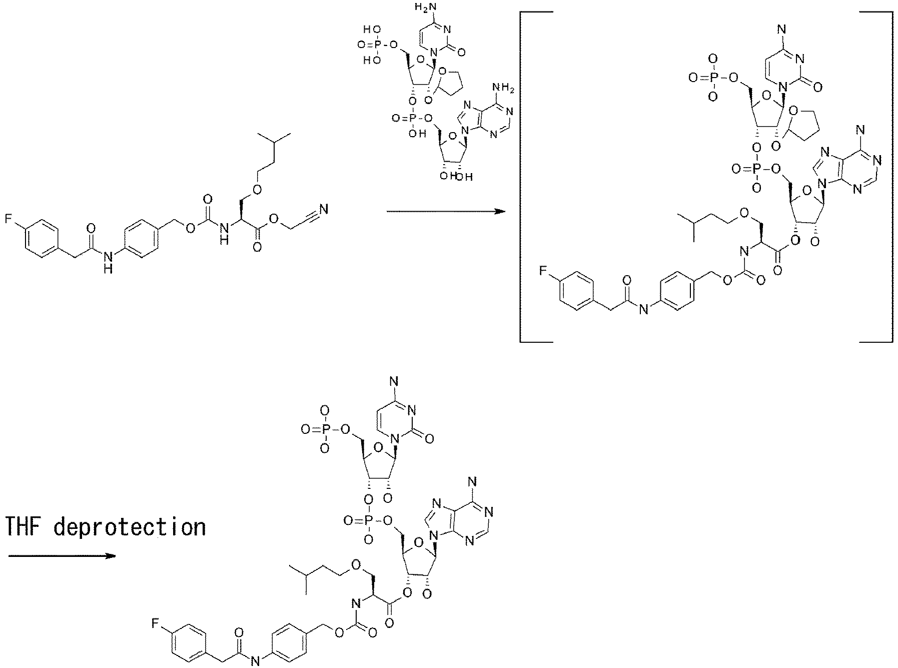

((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogen phosphate (Compound pc01) (400 mg, 0.55 mmol) synthesized by a method described in a literature (Helv. Chim. Acta, 90, 297-310) was dissolved in Buffer A (100 mL), a solution of cyanomethyl_N-(((4-(2-(4-fluorophenyl)acetamido)benzyl)oxy)carbonyl)-O-isopentyl-L-serinate (Compound SS44, F-Pnaz-SiPen-OCH₂CN) (139 mg, 0.28 mmol) in acetonitrile (5 mL) was added to it dropwise over 15 minutes or longer using a syringe pump, and stirred at room temperature for 3 hours. Trifluoroacetic acid (2.3 mL) was added to the reaction solution. The reaction solution was freeze-dried, and then purified by reverse-phase silica gel column chromatography (0.05% aqueous trifluoroacetic acid solution/ 0.05% trifluoroacetic acid-acetonitrile) to obtain the title compound (Compound SS40, F-Pnaz-SiPen-pCpA) (39.5 mg, 3%).
LCMS (ESI) m/z = 1093.5 (M-H)-
Retention time: 0.55 minutes (analysis condition SQDFA05_01)

### Example 6. Synthesis of BdpFL-Phe-pCpA(MT01) Synthesis of (3-(5, 5-difluoro-7,9-dimethyl-5H-4λ⁴,5λ,⁴-dipyrrolo[1,2-c:2', f][1,3,2]diazaborinin-3-yl)propanoyl)-L-phenylalanine (Compound MT02 BdpFL-Phe-OH)

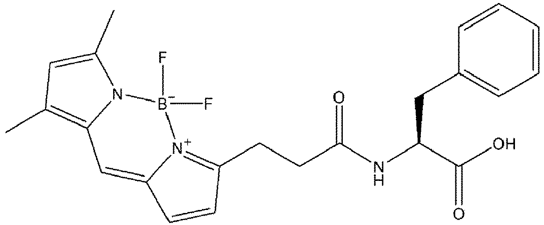

Under nitrogen atmosphere, DIC (0.128 mL, 0.822 mmol) was added to a solution of 3-(2-carboxyethyl)-5,5-difluoro-7,9-dimethyl-5H-5λ⁴-dipyrrolo[1,2-c:2',1'-f][1,3,2]diazaborinin-4-ium (200 mg, 0.685 mmol) and 1-hydroxypyrrolidine-2,5-dione (87 mg, 0.753 mmol) in NMP (4.5 mL) at room temperature, and then the mixture was stirred at 40°C overnight. After returning to room temperature, L-phenylalanine (113 mg, 0.685 mmol) and TEA (0.191 mL, 1.369 mmol) were added to the reaction solution, and stirred at 40°C overnight. The reaction solution was purified by reverse-phase column chromatography (0.1 % FA-MeCN/H2O) to obtain (3-(5, 5-difluoro-7,9-dimethyl-5H-4λ⁴,5λ⁴-dipyrrolo[1,2-c:2',1'-f][1,3,2]diazaborinin-3-yl)propanoyl)-L-phenylalanine (Compound MT02, BdpFL-Phe-OH) (102 mg, 34% yield).
LCMS (ESI) m/z = 438.3 (M-H)-
Retention time: 0.78 minutes (analysis condition SQDFA05_02)

### Synthesis of (3-(5,5-difluoro-7,9-dimethyl-5H-4λ⁴,5λ⁴-dipyrrolo[1,2-c:2',1'-f][1,3,2]diazaborinin-3-yl)propanoyl)-L-phenylalanine cyanomethyl ester (Compound MT03, BdpFL-Phe-OCH₂CN)

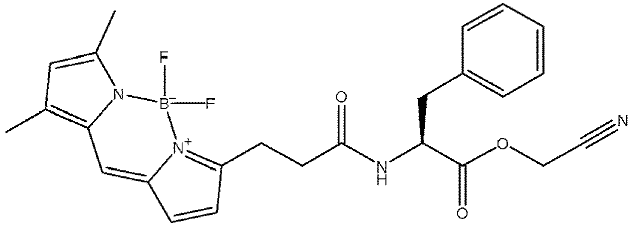

Under nitrogen atmosphere, (3-(5,5-difluoro-7,9-dimethyl-5H-4λ⁴,5λ⁴-dipyrrolo[1,2-c:2',1'-f][1,3,2]diazaborinin-3-yl)propanoyl)-L-phenylalanine (50 mg, 0.114 mmol) and N-ethyl-isopropylpropan-2-amine (DIPEA) (31.0 µL, 0.177 mmol) were dissolved in acetonitrile (500 µL), 2-bromoacetonitrile (12 µL, 0.177 mmol) was added at 0°C, and then the mixture was stirred at 40°C for three hours. The reaction solution was concentrated to obtain (3-(5,5-difluoro-7,9-dimethyl-5H-4λ⁴,5λ⁴-dipyrrolo[1,2-c:2',1'-f][1,3,2]diazaborinin-3-yl)propanoyl)-L-phenylalanine cyanomethyl ester (Compound MT02, BdpFL-Phe-OCH₂CN) as a crude product. The obtained crude product was directly used in the next step.
LCMS (ESI) m/z = 477.3 (M-H)-
Retention time: 0.86 minutes (analysis condition SQDFA05_01)

### Synthesis of 3-(3-(((2S)-1-(((2R,3S,4R,5R)-2-((((((2R,3S,4R,5R)-5-(4-amino-2-oxppydmidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl)oxy)-1-oxo-3-phenylpropan-2-yl)amino)-3-oxopropyl)-5,5-difluoro-7,9-dimethyl-5H-5λ⁴-dipyrrolo[1,2-c:2',1'-f][1,3,2]diazaborinin-4-ium (Compound MT01, BdpFL-Phe-pCpA)

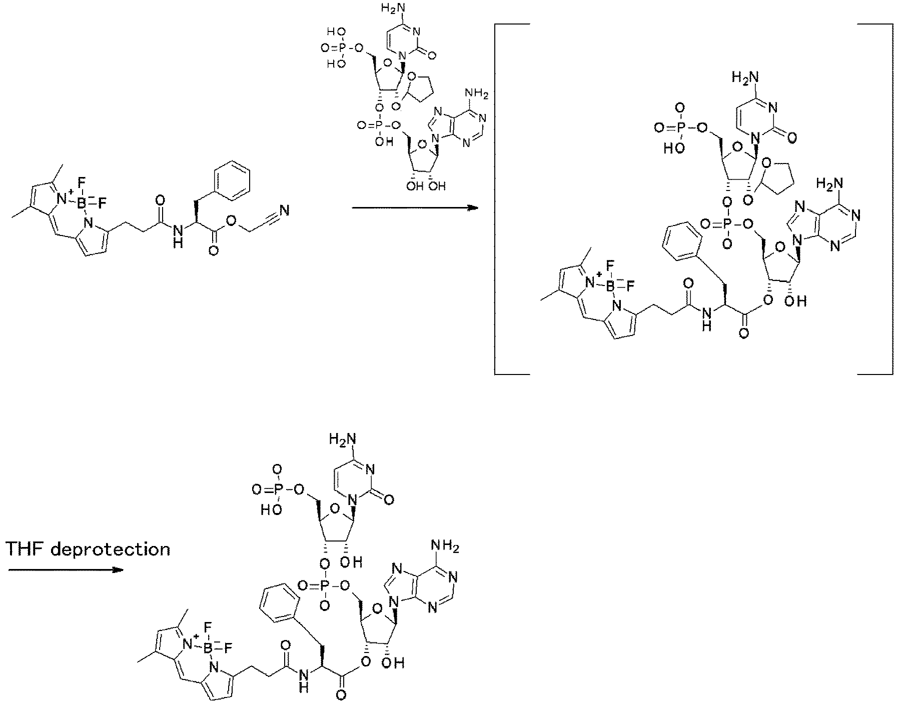

((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogen phosphate (Compound pc01) (33.2 mg, 0.046 mmol) was dissolved in Buffer A (11.3 mL), a solution of (3-(5,5-difluoro-7,9-dimethyl-5H-4λ⁴,5λ⁴-dipyrrolo[1,2-c:2',1'-f][1,3,2]diazaborinin-3-yl)propanoyl)-L-phenylalanine cyanomethyl ester (Compound MT03, BdpFL-Phe-OCH₂CN) (11 mg, 0.023 mmol) in acetonitrile (0.13 mL) was added, and then the mixture was stirred at room temperature for 45 minutes. TFA (0.56 mL) was added to the reaction solution at 0°C and stirred for five minutes, and then stirred at room temperature for ten minutes. The reaction solution was purified by reverse-phase silica gel column chromatography (0.05% TFA-MeCN/H2O) to obtain the title compound (Compound MT01, BdpFL-Phe-pCpA) (2.1 mg, 8.5% yield).
LCMS (ESI) m/z = 1072.5 (M-H)-
Retention time: 0.56 minutes (analysis condition SQDFA05_02)

### Example 7. Synthesis of (2S,3R)-2-((((9H-fluoren-9-yl)methoxy)carbonyl)amino)-3-((tetrahydro-2H-pyran-2-yl)oxy)butanoic acid (Fmoc-Thr(THP)-OH) to be used for peptide synthesis of LCT-12 by a peptide synthesizer

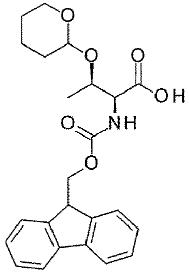

Toluene (50 mL) was added to a mixture of (2S,3R)-2-((((9H-fluoren-9-yl)methoxy)carbonyl)amino)-3-hydroxybutanoic acid monohydrate (monohydrate of Fmoc-Thr-OH purchased from Tokyo Chemical Industry, 5.0 g, 13.9 mmol) and pyridinium p-toluenesulfonate (PPTS, 0.175 g, 0.70 mmol), and by distilling off toluene under reduced pressure, the included water was removed azeotropically. Super-dehydrated tetrahydrofuran (THF, 28 mL) and 3,4-dihydro-2H-pyran (8.8 mL, 97 mmol) were added to the obtained residue, and this was stirred under nitrogen atmosphere at 50°C for four hours. After confirming the disappearance of the starting materials by LCMS (SQDFA05), the mixture was cooled to 25°C, and ethyl acetate (30 mL) was added. Next, saturated aqueous sodium chloride solution (30 mL) was added to wash the organic layer, and the aqueous layer was extracted with ethyl acetate (30 mL). All of the obtained organic layers were combined, and this was further washed twice with saturated aqueous sodium chloride solution (30 mL). The organic layer was dried over sodium sulfate, and the solvent was distilled off under reduced pressure to obtain a crude product (9.3 g).

4.65 g from among the obtained crude product was dissolved in tetrahydrofuran (THF, 30 mL), and then 1.0 M phosphate buffer (30 mL) adjusted to pH8.0 was added. This mixture was stirred at 50°C for four hours. After cooling to 25°C, ethyl acetate (30 mL) was added, and the organic and aqueous layers were separated. Ethyl acetate (30 mL) was added to the aqueous layer for extraction, and then all of the obtained organic layers were combined, and this was washed twice with saturated aqueous sodium chloride solution (30 mL). The organic layer was dried over sodium sulfate, the solvent was distilled off under reduced pressure, and further dried under reduced pressure using a pump at 25°C for 30 minutes.

The obtained residue was dissolved in diethyl ether (50 mL), and then heptane (50 mL) was added. Under controlled reduced pressure (approximately 100 hPa), only diethyl ether was distilled off, and the obtained mixture was filtered to obtain a solid. This washing operation with heptane was repeated twice. The obtained solid was dried under reduced pressure using a pump at 25°C for two hours to obtain the sodium salt of Fmoc-Thr(THP)-OH (2.80 g, 6.26 mmol).

Ethyl acetate (50 mL) and 0.05 M aqueous phosphoric acid solution (140 mL) at pH2.1 were added to the total amount of the obtained sodium salt of Fmoc-Thr(THP)-OH, the mixture was stirred at 25°C for five minutes, and then the organic layer and the aqueous layer were separated. Ethyl acetate (50 mL) was added to the aqueous layer for extraction, and all of the obtained organic layers were mixed, and then washed twice with saturated aqueous sodium chloride solution (50 mL). The organic layer was dried over sodium sulfate, and the solvent was distilled off under reduced pressure. The residue was dried under reduced pressure using a pump at 25°C for two hours, then the obtained solid was dissolved in t-butyl methyl ether (TBME, 50 mL), and the solvent was distilled off under reduced pressure. Furthermore, by drying under reduced pressure using a pump at 25°C for one hour, (2S,3R)-2-((((9H-fluoren-9-yl)methoxy)carbonyl)amino)-3-((tetrahydro-2H-pyran-2-yl)oxy)butanoic acid (Fmoc-Thr(THP)-OH, 2.70 g, 30 mol% of t-butyl methyl ether (TBME) remained) was obtained as a diastereomeric mixture derived from the asymmetric carbon on the THP protecting group. The obtained Fmoc-Thr(THP)-OH was stored in a freezer at -25°C.
LCMS (ESI) m/z = 424.2 (M-H)-
Retention time: 0.84 minutes, 0.85 minutes (analysis condition SQDFA05_01)

### Example 8. Synthesis of a peptide (LCT-12) having BdpFL at the N terminus, which is to be used as a standard for LC/MS

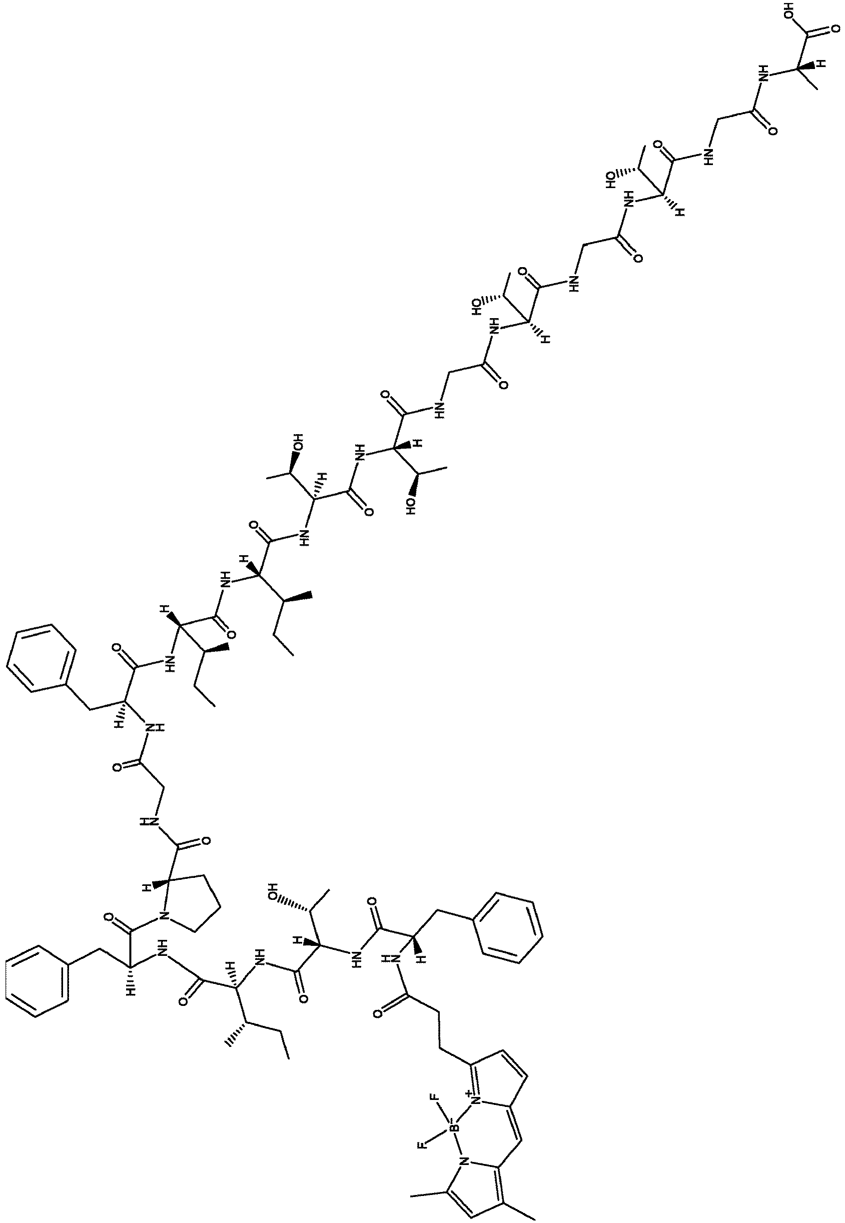

Using 2-chlorotrityl resin bearing Fmoc-Ala-OH (100 mg), and using Fmoc-Gly-OH, Fmoc-Thr(THP)-OH, Fmoc-Ile-OH, Fmoc-Phe-OH, and Fmoc-Pro-OH as Fmoc amino acids, peptide elongation was performed on a peptide synthesizer (abbreviations of amino acids are described separately in this specification). Peptide elongation was performed according to a peptide synthesis method using the Fmoc method (WO2013100132B2). After the peptide elongation, removal of the N-terminal Fmoc group was performed on the peptide synthesizer, and then the resin was washed with DCM.

TFE/DCM (1:1, v/v, 2 mL) was added to the resin and shaken for one hour, then the peptides were cleaved off from the resin. After completion of the reaction, the resin was removed by filtering the solution inside the tube through a column for synthesis, and the resin was washed twice with TFE/DCM (1:1, v/v, 1 mL). All of the extracts were mixed, DMF (2 mL) was added, and then the mixture was concentrated under reduced pressure. The obtained residue was dissolved in NMP (0.5 mL), and one-fourth (125 µL) of it was used in the next reaction. To the peptide solution in NMP, BdpFL succinimide ester (140 µL) adjusted to 76.5 mM was added at room temperature, stirred overnight at 40°C, and then concentrated under reduced pressure. The obtained residue was dissolved in 0.05 M tetramethylammonium hydrogen sulfate in HFIP (1.2 mL, 0.060 mmol) and stirred at room temperature for two hours. The reaction solution was purified by reverse-phase silica gel column chromatography (0.1% FA MeCN/H₂O) to obtain the title compound (LCT-12) (0.3 mg). The amino acid sequence of LCT-12 is shown in SEQ ID NO: 53.
LCMS (ESI) m/z = 1972.9 (M-H)-
Retention time: 0.74 minutes (analysis condition SQDFA05_01)

### Example 9. Production of tRNA-CA by a ligation reaction

By the procedure described below, tRNA5' fragments, pNp (pUp, pLp, or p(Agm)p), and tRNA3' fragments were ligated using a ligation reaction to produce various tRNA-CAs. Chemically synthesized products (Gene Design Co., Ltd.) were used for the tRNA 5' fragments and tRNA 3' fragments. Each tRNA fragment and its full-length sequence, as well as the combinations of the samples used for ligation (Table 4) are shown below.
SEQ ID NO: 54 (FR-1)
   tRNA(Glu)5' RNA sequence
   GUCCCCUUCGUCUAGAGGCCCAGGACACCGCCCU
SEQ ID NO: 55 (FR-2)
   tRNA(Glu)3'ga RNA sequence
   GAACGGCGGUAACAGGGGUUCGAAUCCCCUAGGGGACGC
SEQ ID NO: 56 (UR-1)
   lig-tRNA(Glu)uga-CA RNA sequence 
GUCCCCUUCGUCUAGAGGCCCAGGACACCGCCCUUGAACGGCGGUAACAGGGGUU
CGAAUCCCCUAGGGGACGC
SEQ ID NO: 57 (LR-1)
   tRNA(Glu)Lga-CA RNA sequence 
GUCCCCUUCGUCUAGAGGCCCAGGACACCGCCCULGAACGGCGGUAACAGGGGUU
CGAAUCCCCUAGGGGACGC
SEQ ID NO: 58 (FR-3)
   tRNA(Glu)3'ag RNA sequence
   AGACGGCGGUAACAGGGGUUCGAAUCCCCUAGGGGACGC
SEQ ID NO: 59 (LR-2)
   tRNA(Glu)Lag-CA RNA sequence 
GUCCCCUUCGUCUAGAGGCCCAGGACACCGCCCULAGACGGCGGUAACAGGGGUU
CGAAUCCCCUAGGGGACGC
SEQ ID NO: 60 (FR-4)
   tRNA(Glu)3'ac RNA sequence
   ACACGGCGGUAACAGGGGUUCGAAUCCCCUAGGGGACGC
SEQ ID NO: 61 (LR-3)
   tRNA(Glu)Lac-CA RNA sequence 
GUCCCCUUCGUCUAGAGGCCCAGGACACCGCCCULACACGGCGGUAACAGGGGUU
CGAAUCCCCUAGGGGACGC
SEQ ID NO: 62 (FR-5)
   tRNA(Glu)3'cc RNA sequence
   CCACGGCGGUAACAGGGGUUCGAAUCCCCUAGGGGACGC
SEQ ID NO: 63 (LR-4)
   tRNA(Glu)Lcc-CA RNA sequence 
GUCCCCUUCGUCUAGAGGCCCAGGACACCGCCCULCCACGGCGGUAACAGGGGUU
CGAAUCCCCUAGGGGACGC
SEQ ID NO: 132 (FR-6)
   tRNA(Asp)5' RNA sequence
   GGAGCGGUAGUUCAGUCGGUUAGAAUACCUGCUU
SEQ ID NO: 133 (FR-7)
   tRNA(Asp)3'ag RNA sequence
   AGGUGCAGGGGGUCGCGGGUUCGAGUCCCGUCCGUUCCGC
SEQ ID NO: 134 (LR-5)
   tRNA(Asp)Lag-CA RNA sequence 
GGAGCGGUAGUUCAGUCGGUUAGAAUACCUGCUULAGGUGCAGGGGGUCGCGGG
UUCGAGUCCCGUCCGUUCCGC
SEQ ID NO: 135 (FR-8)
   tRNA(AsnE2)5' RNA sequence
   GGCUCUGUAGUUCAGUCGGUAGAACGGCGGAUU
SEQ ID NO: 136 (FR-9)
   tRNA(AsnE2)3'ag RNA sequence
   AGGUUCCGUAUGUCACUGGUUCGAGUCCAGUCAGAGCCGC
SEQ ID NO: 137 (LR-6)
   tRNA(AsnE2)Lag-CA RNA sequence 
GGCUCUGUAGUUCAGUCGGUAGAACGGCGGAUULAGGUUCCGUAUGUCACUGGU
UCGAGUCCAGUCAGAGCCGC
SEQ ID NO: 139 (FR-10)
   tRNA(Glu)3'cg RNA sequence
   CGACGGCGGUAACAGGGGUUCGAAUCCCCUAGGGGACGC
SEQ ID NO: 140 (LR-7)
   tRNA(Glu)Lcg-CA RNA sequence 
GUCCCCUUCGUCUAGAGGCCCAGGACACCGCCCULCGACGGCGGUAACAGGGGUU
CGAAUCCCCUAGGGGACGC
SEQ ID NO: 141 (FR-11)
   tRNA(Glu)3'au RNA sequence
   AUACGGCGGUAACAGGGGUUCGAAUCCCCUAGGGGACGC
SEQ ID NO: 142 (LR-8)
   tRNA(Glu)Lau-CA RNA sequence 
GUCCCCUUCGUCUAGAGGCCCAGGACACCGCCCULAUACGGCGGUAACAGGGGUU
CGAAUCCCCUAGGGGACGC
SEQ ID NO: 138 (AR-1)
   tRNA(Glu)(Agm)ag-CA RNA sequence

GUCCCCUUCGUCUAGAGGCCCAGGACACCGCCCU(Agm)AGACGGCGGUAACAGGG
GUUCGAAUCCCCUAGGGGACGC

**[Table 4]**

| SEQ ID NO: | tRNA5'fragment | pNp | tRNA3'fragment |
|---|---|---|---|
| UR-1 | FR-1 | pUp | FR-2 |
| LR-1 | FR-1 | pLp | FR-2 |
| LR-2 | FR-1 | pLp | FR-3 |
| LR-3 | FR-1 | pLp | FR-4 |
| LR-4 | FR-1 | pLp | FR-5 |
| LR-5 | FR-6 | pLp | FR-7 |
| LR-6 | FR-8 | pLp | FR-9 |
| LR-7 | FR-1 | pLp | FR-10 |
| LR-8 | FR-1 | pLp | FR-11 |
| AR-1 | FR-1 | p(Agm)p | FR-3 |

A reaction solution composed of 50 mM HEPES-KOH (pH 7.5), 20 mM MgCl₂, 1 mM ATP, 0.125-0.25 mM pNp (pUp, pLp, or p(Agm)p), 25 µM tRNA 5' fragment, 0.6 U/µL T4 RNA ligase (New England Biolabs), and 10% DMSO was left to stand overnight at 15°C to perform a ligation reaction between the tRNA 5' fragment and pNp (pUp, pLp, or p(Agm)p). The ligation product was extracted with phenol-chloroform, and recovered by ethanol precipitation.

To prevent the unreacted tRNA 5' fragment from being carried over to the next ligation reaction, sodium periodate (NaIO₄) was used to cleave the ribose at the 3' end of the tRNA 5' fragment. Specifically, 10 µM ligation product was cleaved by allowing it to stand on ice for 30 minutes in the dark in the presence of 10 mM sodium periodate. After the reaction, one-tenth volume of 100 mM glucose was added, and this was allowed to stand on ice for 30 minutes in the dark to decompose the excess sodium periodate. The reaction product was collected by ethanol precipitation.

After the periodic acid treatment, T4 polynucleotide kinase (T4 PNK) treatment was performed to phosphorylate the 5' end and dephosphorylate the 3' end of the ligation product. The reaction solution composed of the ligation product after 10 µM periodic acid treatment, 50 mM Tris-HCl (pH 8.0), 10 mM MgCl₂, 5 mM DTT, 300 µM ATP, and 0.5 U/µL T4 PNK (TaKaRa) was reacted by allowing it to stand at 37°C for 30 to 60 minutes. The reaction product was extracted with phenol-chloroform and collected by ethanol precipitation.

A ligation reaction was performed between the post-PNK-treatment reaction product and the tRNA 3' fragment. First, a solution composed of 10 µM PNK-treated reaction product, 10 µM tRNA 3' fragment, 50 mM HEPES-KOH (pH 7.5), and 15 mM MgCl₂ was heated at 65°C for seven minutes and then allowed to stand at room temperature for 30 minutes to one hour to anneal the PNK-treated reaction product and the tRNA 3' fragment. Next, T4 PNK treatment was performed to phosphorylate the 5' end of the tRNA 3' fragment. T4 PNK treatment was performed by adding DTT (final concentration of 3.5 mM), ATP (final concentration of 300 µM), and T4 PNK (final concentration of 0.5 U/µL) to the annealed solution, and allowing this to stand at 37°C for 30 minutes. Next, T4 RNA ligase (New England Biolabs) was added at a final concentration of 0.9 U/µL to this solution, and ligation reaction was performed by allowing this mixture to stand at 37°C for 30 to 40 minutes. The ligation product was extracted with phenol-chloroform and collected by ethanol precipitation.

The tRNA-CAs produced by the ligation method were subjected to preparative purification by high-performance reverse-phase chromatography (HPLC) (aqueous solution of 15 mM TEA and 400 mM HFIP/ methanol solution of 15 mM TEA and 400 mM HFIP) and then subjected to denatured urea-10% polyacrylamide electrophoresis, to confirm whether they had the desired length.

### Example 10. Analyses of tRNA fragments cleaved by RNaseTi

Various tRNA-CAs prepared using a ligation reaction were fragmented by RNase and analyzed to confirm whether each of U, L, and (Agm) introduced by pUp, pLp, or p(Agm)p had been introduced to the desired sites.

The combinations of the SEQ ID NO and the sequence of the RNA fragment containing the U, L, or (Agm) introduced by pUp, pLp, or p(Agm)p are shown for each tRNA-CA in Table 5 shown below.

**[Table 5]**

| SEQ ID NO: | Sequnce of the RNA fragment containing U, L, or (Agm) |
|---|---|
| UR-1 | CCCUUGp |
| LR-1 | CCCULGp |
| LR-2 | CCCULAGp |
| LR-3 | CCCULACACGp |
| LR-4 | CCCULCCACGp |
| LR-5 | CUULAGp |
| LR-6 | AUULAGp |
| LR-7 | CCCULCGp |
| LR-8 | CCCULAUACGp |
| AR-1 | CCCU(Agm)AGp |

A reaction solution containing 10 µM tRNA-CA, 5 U/µL RNaseT₁ (Epicentre or ThermoFisher Scientific), and 10 mM ammonium acetate (pH 5.3) was allowed to stand at 37°C for one hour to specifically cleave the RNA at the 3' side of the G base and analyzed the RNA fragment containing U, L, or (Agm) introduced by pUp, pLp, or p(Agm)p.

CCCUUGp
LCMS(ESI) m/z = 944 ((M-2H)/2)-
Retention time: 4.22 minutes (analysis condition LTQTEA/HF1P05_03)

Comparison to the mass chromatogram of the fragment (CCCUGp) expected when pUp is not ligated, confirmed that most of the pUp ligation took place (Fig. 1).

CCCULGp
LCMS(ESI) m/z = 1008 ((M-2H)/2)-
Retention time: 2.34 minutes (analysis condition LTQTEA/HFIP05_03)

Comparison to the mass chromatograms of the fragment (CCCUGp) expected when pUp is not ligated and the fragment (CCCUUGp) expected when uridine is present instead of lysidine, confirmed that most of the pLp ligation took place (Fig. 2).

CCCULAGp
LCMS(ESI) m/z = 1172 ((M-2H)/2)-
Retention time: 3.81 minutes (analysis condition LTQTEA/HFIP05_03)

Comparison to the mass chromatograms of the fragment (CCCUAGp) expected when pLp is not ligated and the fragment (CCCUUAGp) expected when uridine is present instead of lysidine, confirmed that most of the pLp ligation took place (Fig. 3).

CCCULACACGp (SEQ ID NO: 197)
LCMS(ESI) m/z = 1642 ((M-2H)/2)-
Retention time: 5.78 minutes (analysis condition LTQTEA/HFIP05_03)

Comparison to the mass chromatograms of the fragment (CCCUACACGp) expected when pLp is not ligated and the fragment (CCCUUACACGp/ SEQ ID NO: 198) expected when uridine is present instead of lysidine, confirmed that most of the pLp ligation took place (Fig. 4).

CCCULCCACGp (SEQ ID NO: 199)
LCMS(ESI) m/z = 1630 ((M-2H)/2)-
Retention time: 5.64 minutes (analysis condition LTQTEA/HFIP05_03)

Comparison to the mass chromatograms of the fragment (CCCUCCACGp) expected when pLp is not ligated and the fragment (CCCUUCCACGp/ SEQ ID NO: 200) expected when uridine is present instead of lysidine, confirmed that most of the pLp ligation took place (Fig. 5).

CUULAGp
LCMS(ESI) m/z = 1020 ((M-2H)/2)-
Retention time: 3.84 minutes (analysis condition LTQTEA/HFIP05_03)

Since the fragment (CUUAGp) expected when pLp is not ligated and the fragment (UUCAGp) derived from another part of the RNA have the same molecular weight, the unfragmented RNA was analyzed as well.
pGGAGCGGUAGUUCAGUCGGUUAGAAUACCUGCUULAGGUGCAGGGGGUCGCGGG UUCGAGUCCCGUCCGUUCCGC (SEQ ID NO: 134)
LCMS(ESI) m/z = 1109 ((M-22H)/22)-
Retention time: 3.92 minutes (analysis condition LTQTEA/HFIP05_01)

Comparison to the mass chromatograms of the RNA expected when pLp is not ligated and the RNA expected when uridine is present instead of lysidine, confirmed that most of the pLp ligation took place (Fig. 6).

AUULAGp
LCMS(ESI) m/z = 1032 ((M-2H)/2)-
Retention time: 4.16 minutes (analysis condition LTQTEA/HFIP05_03)

Comparison to the mass chromatograms of the fragment (AUUAGp) expected when pLp is not ligated and the fragment (AUUUAGp) expected when uridine is present instead of lysidine, confirmed that most of the pLp ligation took place (Fig. 7).

CCCULCGp
LCMS(ESI) m/z = 1160 ((M-2H)/2)-
Retention time: 4.21 minutes (analysis condition LTQTEA/HFIP05_03)

Comparison to the mass chromatograms of the fragment (CCCUCGp) expected when pLp is not ligated and the fragment (CCCUUCGp) expected when uridine is present instead of lysidine, confirmed that most of the pLp ligation took place (Fig. 8).

CCCULAUACGp (SEQ ID NO: 202)
LCMS(ESI) m/z = 1642 ((M-2H)/2)-
Retention time: 5.95 minutes (analysis condition LTQTEA/HFIP05_03)

Comparison to the mass chromatograms of the fragment (CCCUAUACGp) expected when pLp is not ligated and the fragment (CCCUUAUACGp/ SEQ ID NO: 203) expected when uridine is present instead of lysidine, confirmed that most of the pLp ligation took place (Fig. 9).

CCCU(Agm)AGp
LCMS(ESI) m/z = 1164 ((M-2H)/2)-
Retention time: 4.02 minutes (analysis condition LTQTEA/HFIP05_03)

Comparison to the mass chromatograms of the fragment (CCCUAGp) expected when p(Agm)p is not ligated and the fragment (CCCUUAGp) expected when uridine is present instead of agmatidine, confirmed that most of the p(Agm)p ligation took place (Fig. 10).

### Example 11. Synthesis of aminoacyl tRNAs

From template DNAs (SEQ ID NO: 64 (D-1) to SEQ ID NO: 76 (D-13), SEQ ID NO: 143 (D-26) to SEQ ID NO: 152 (D-35)), tRNAs (SEQ ID NO: 77 (TR-1) to SEQ ID NO: 89 (TR-13), SEQ ID NO: 153 (TR-14) to SEQ ID NO: 162 (TR-23)) were synthesized by in vitro transcription reaction using T7 RNA polymerase, and were purified by RNeasy kit (Qiagen).

Template DNA SEQ ID NO: 64 (D-1)
   DNA sequence: 
GGCGTAATACGACTCACTATAGTCCCCTTCGTCTAGAGGCCCAGGACACCGCCCTAG
AACGGCGGTAACAGGGGTTCGAATCCCCTAGGGGACGC
Template DNA SEQ ID NO: 65 (D-2)
   DNA sequence: 
GGCGTAATACGACTCACTATAGTCCCCTTCGTCTAGAGGCCCAGGACACCGCCCTTG
AACGGCGGTAACAGGGGTTCGAATCCCCTAGGGGACGC
Template DNA SEQ ID NO: 66 (D-3)
   DNA sequence: 
GGCGTAATACGACTCACTATAGTCCCCTTCGTCTAGAGGCCCAGGACACCGCCCTCG
AACGGCGGTAACAGGGGTTCGAATCCCCTAGGGGACGC
Template DNA SEQ ID NO: 67 (D-4)
   DNA sequence: 
GGCGTAATACGACTCACTATAGTCCCCTTCGTCTAGAGGCCCAGGACACCGCCCTAA
GACGGCGGTAACAGGGGTTCGAATCCCCTAGGGGACGC
Template DNA SEQ ID NO: 68 (D-5)
   DNA sequence: 
GGCGTAATACGACTCACTATAGTCCCCTTCGTCTAGAGGCCCAGGACACCGCCCTTA
GACGGCGGTAACAGGGGTTCGAATCCCCTAGGGGACGC
Template DNA SEQ ID NO: 69 (D-6)
   DNA sequence : 
GGCGTAATACGACTCACTATAGTCCCCTTCGTCTAGAGGCCCAGGACACCGCCCTCA
GACGGCGGTAACAGGGGTTCGAATCCCCTAGGGGACGC
Template DNA SEQ ID NO: 70 (D-7)
   DNA sequence: 
GGCGTAATACGACTCACTATAGTCCCCTTCGTCTAGAGGCCCAGGACACCGCCCTAA
CACGGCGGTAACAGGGGTTCGAATCCCCTAGGGGACGC
Template DNA SEQ ID NO: 71 (D-8)
   DNA sequence: 
GGCGTAATACGACTCACTATAGTCCCCTTCGTCTAGAGGCCCAGGACACCGCCCTTA
CACGGCGGTAACAGGGGTTCGAATCCCCTAGGGGACGC
Template DNA SEQ ID NO: 72 (D-9)
   DNA sequence: 
GGCGTAATACGACTCACTATAGTCCCCTTCGTCTAGAGGCCCAGGACACCGCCCTCA
CACGGCGGTAACAGGGGTTCGAATCCCCTAGGGGACGC
Template DNA SEQ ID NO: 73 (D-10)
   DNA sequence: 
GGCGTAATACGACTCACTATAGTCCCCTTCGTCTAGAGGCCCAGGACACCGCCCTGC
CACGGCGGTAACAGGGGTTCGAATCCCCTAGGGGACGC
Template DNA SEQ ID NO: 74 (D-11)
   DNA sequence: 
GGCGTAATACGACTCACTATAGTCCCCTTCGTCTAGAGGCCCAGGACACCGCCCTTC
CACGGCGGTAACAGGGGTTCGAATCCCCTAGGGGACGC
Template DNA SEQ ID NO: 75 (D-12)
   DNA sequence: 
GGCGTAATACGACTCACTATAGTCCCCTTCGTCTAGAGGCCCAGGACACCGCCCTCC
CACGGCGGTAACAGGGGTTCGAATCCCCTAGGGGACGC
Template DNA SEQ ID NO: 76 (D-13)
   DNA sequence: 
GGCGTAATACGACTCACTATAGGCGGGGTGGAGCAGCCTGGTAGCTCGTCGGGCTC
ATAACCCGAAGATCGTCGGTTCAAATCCGGCCCCCGCAAC
Template DNA SEQ ID NO: 143 (D-26)
   DNA sequence: 
GGCGTAATACGACTCACTATAGGAGCGGTAGTTCAGTCGGTTAGAATACCTGCTTaag
GTGCAGGGGGTCGCGGGTTCGAGTCCCGTCCGTTCCGC
Template DNA SEQ ID NO: 144 (D-27)
   DNA sequence: 
GGCGTAATACGACTCACTATAGGAGCGGTAGTTCAGTCGGTTAGAATACCTGCTTTa
gGTGCAGGGGGTCGCGGGTTCGAGTCCCGTCCGTTCCGC
Template DNA SEQ ID NO: 145 (D-28)
   DNA sequence: 
GGCGTAATACGACTCACTATAGGAGCGGTAGTTCAGTCGGTTAGAATACCTGCTTcag
GTGCAGGGGGTCGCGGGTTCGAGTCCCGTCCGTTCCGC
Template DNA SEQ ID NO: 146 (D-29)
   DNA sequence: 
GGCGTAATACGACTCACTATAGGCTCTGTAGTTCAGTCGGTAGAACGGCGGATTaagG
TTCCGTATGTCACTGGTTCGAGTCCAGTCAGAGCCGC
Template DNA SEQ ID NO: 147 (D-30)
   DNA sequence: 
GGCGTAATACGACTCACTATAGGCTCTGTAGTTCAGTCGGTAGAACGGCGGATTtagG
TTCCGTATGTCACTGGTTCGAGTCCAGTCAGAGCCGC
Template DNA SEQ ID NO: 148 (D-31)
   DNA sequence: 
GGCGTAATACGACTCACTATAGGCTCTGTAGTTCAGTCGGTAGAACGGCGGATTcagG
TTCCGTATGTCACTGGTTCGAGTCCAGTCAGAGCCGC
Template DNA SEQ ID NO: 149 (D-32)
   DNA sequence: 
GGCGTAATACGACTCACTATAGTCCCCTTCGTCTAGAGGCCCAGGACACCGCCCTgcg
ACGGCGGTAACAGGGGTTCGAATCCCCTAGGGGACGC
Template DNA SEQ ID NO: 150 (D-33)
   DNA sequence: 
GGCGTAATACGACTCACTATAGTCCCCTTCGTCTAGAGGCCCAGGACACCGCCCTccg
ACGGCGGTAACAGGGGTTCGAATCCCCTAGGGGACGC
Template DNA SEQ ID NO: 151 (D-34)
   DNA sequence: 
GGCGTAATACGACTCACTATAGTCCCCTTCGTCTAGAGGCCCAGGACACCGCCCTaau
ACGGCGGTAACAGGGGTTCGAATCCCCTAGGGGACGC
Template DNA SEQ ID NO: 152 (D-35)
   DNA sequence:

GGCGTAATACGACTCACTATAGTCCCCTTCGTCTAGAGGCCCAGGACACCGCCCTcau
ACGGCGGTAACAGGGGTTCGAATCCCCTAGGGGACGC

tRNA SEQ ID NO: 77 (TR-1)
   tRNA(Glu)aga-CA RNA sequence: 
GUCCCCUUCGUCUAGAGGCCCAGGACACCGCCCUAGAACGGCGGUAACAGGGGUU
CGAAUCCCCUAGGGGACGC
tRNA SEQ ID NO: 78 (TR-2)
   tRNA(Glu)uga-CA RNA sequence: 
GUCCCCUUCGUCUAGAGGCCCAGGACACCGCCCUUGAACGGCGGUAACAGGGGUU
CGAAUCCCCUAGGGGACGC
tRNA SEQ ID NO: 79 (TR-3)
   tRNA(Glu)cga-CA RNA sequence: 
GUCCCCUUCGUCUAGAGGCCCAGGACACCGCCCUCGAACGGCGGUAACAGGGGUU
CGAAUCCCCUAGGGGACGC
tRNA SEQ ID NO: 80 (TR-4)
   tRNA(Glu)aag-CA RNA sequence: 
GUCCCCUUCGUCUAGAGGCCCAGGACACCGCCCUAAGACGGCGGUAACAGGGGUU
CGAAUCCCCUAGGGGACGC
tRNA SEQ ID NO: 81 (TR-5)
   tRNA(Glu)uag-CA RNA sequence: 
GUCCCCUUCGUCUAGAGGCCCAGGACACCGCCCUUAGACGGCGGUAACAGGGGUU
CGAAUCCCCUAGGGGACGC
tRNA SEQ ID NO: 82 (TR-6)
   tRNA(Glu)cag-CA RNA sequence: 
GUCCCCUUCGUCUAGAGGCCCAGGACACCGCCCUCAGACGGCGGUAACAGGGGUU
CGAAUCCCCUAGGGGACGC
tRNA SEQ ID NO: 83 (TR-7)
   tRNA(Glu)aac-CA RNA sequence: 
GUCCCCUUCGUCUAGAGGCCCAGGACACCGCCCUAACACGGCGGUAACAGGGGUU
CGAAUCCCCUAGGGGACGC
tRNA SEQ ID NO: 84 (TR-8)
   tRNA(Glu)uac-CA RNA sequence: 
GUCCCCUUCGUCUAGAGGCCCAGGACACCGCCCUUACACGGCGGUAACAGGGGUU
CGAAUCCCCUAGGGGACGC
tRNA SEQ ID NO: 85 (TR-9)
   tRNA(Glu)cac-CA RNA sequence: GUCCCCUUCGUCUAGAGGCCCAGGACACCGCCCUCACACGGCGGUAACAGGGGUU CGAAUCCCCUAGGGGACGC
tRNA SEQ ID NO: 86 (TR-10)
   tRNA(Glu)gcc-CA RNA sequence: 
GUCCCCUUCGUCUAGAGGCCCAGGACACCGCCCUGCCACGGCGGUAACAGGGGUU
CGAAUCCCCUAGGGGACGC
tRNA SEQ ID NO: 87 (TR-11)
   tRNA(Glu)ucc-CA RNA sequence: 
GUCCCCUUCGUCUAGAGGCCCAGGACACCGCCCUUCCACGGCGGUAACAGGGGUU
CGAAUCCCCUAGGGGACGC
tRNA SEQ ID NO: 88 (TR-12)
   tRNA(Glu)ccc-CA RNA sequence: 
GUCCCCUUCGUCUAGAGGCCCAGGACACCGCCCUCCCACGGCGGUAACAGGGGUU
CGAAUCCCCUAGGGGACGC
tRNA SEQ ID NO: 89 (TR-13)
   tRNA(fMet)cau-CA RNA sequence: 
GGCGGGGUGGAGCAGCCUGGUAGCUCGUCGGGCUCAUAACCCGAAGAUCGUCGGU
UCAAAUCCGGCCCCCGCAA
tRNA SEQ ID NO: 153 (TR-14)
   tRNA(Asp)aag-CA RNA sequence: 
GGAGCGGUAGUUCAGUCGGUUAGAAUACCUGCUUaagGUGCAGGGGGUCGCGGGU
UCGAGUCCCGUCCGUUCCGC
tRNA SEQ ID NO: 154 (TR-15)
   tRNA(Asp)uag-CA RNA sequence: 
GGAGCGGUAGUUCAGUCGGUUAGAAUACCUGCUUuagGUGCAGGGGGUCGCGGGU
UCGAGUCCCGUCCGUUCCGC
tRNA SEQ ID NO: 155 (TR-16)
   tRNA(Asp)cag-CA RNA sequence: 
GGAGCGGUAGUUCAGUCGGUUAGAAUACCUGCUUcagGUGCAGGGGGUCGCGGGU
UCGAGUCCCGUCCGUUCCGC
tRNA SEQ ID NO: 156 (TR-17)
   tRNA(AsnE2)aag-CA RNA sequence: 
GGCUCUGUAGUUCAGUCGGUAGAACGGCGGAUUaagGUUCCGUAUGUCACUGGUU
CGAGUCCAGUCAGAGCCGC
tRNA SEQ ID NO: 157 (TR-18)
   tRNA(AsnE2)uag-CA RNA sequence: GGCUCUGUAGUUCAGUCGGUAGAACGGCGGAUUuagGUUCCGUAUGUCACUGGUU CGAGUCCAGUCAGAGCCGC
tRNA SEQ ID NO: 158 (TR-19)
   tRNA(AsnE2)cag-CA RNA sequence: 
GGCUCUGUAGUUCAGUCGGUAGAACGGCGGAUUcagGUUCCGUAUGUCACUGGUU
CGAGUCCAGUCAGAGCCGC
tRNA SEQ ID NO: 159 (TR-20)
   tRNA(Glu)gcg-CA RNA sequence: 
GUCCCCUUCGUCUAGAGGCCCAGGACACCGCCCUgcgACGGCGGUAACAGGGGUUC
GAAUCCCCUAGGGGACGC
tRNA SEQ ID NO: 160 (TR-21)
   tRNA(Glu)ccg-CA RNA sequence: 
GUCCCCUUCGUCUAGAGGCCCAGGACACCGCCCUccgACGGCGGUAACAGGGGUUC
GAAUCCCCUAGGGGACGC
tRNA SEQ ID NO: 161 (TR-22)
   tRNA(Glu)aau-CA RNA sequence: 
GUCCCCUUCGUCUAGAGGCCCAGGACACCGCCCUaauACGGCGGUAACAGGGGUUC
GAAUCCCCUAGGGGACGC
tRNA SEQ ID NO: 162 (TR-23)
   tRNA(Glu)cau-CA RNA sequence:

GUCCCCUUCGUCUAGAGGCCCAGGACACCGCCCUcauACGGCGGUAACAGGGGUUC
GAAUCCCCUAGGGGACGC

### Preparation of a mixed aminoacyl tRNA solution using aminoacyl pCpA

A reaction solution was prepared by adding Nuclease free water to adjust the solution to 25 µM transcribed tRNA(Glu)aga-CA (SEQ ID NO: 77 (TR-1)), 50 mM HEPES-KOH pH7.5, 20 mM MgCl₂, 1 mM ATP, 0.6 unit/µL T4 RNA ligase (New England Biolabs), and 0.25 mM aminoacylated pCpA (a DMSO solution of Compound TS24 synthesized by a method described in a patent literature (WO2018143145A1)), and ligation reaction was performed at 15°C for 45 minutes. It should be noted that before adding T4 RNA ligase and aminoacylated pCpA, the reaction solution was heated to 95°C for two minutes and then allowed to stand at room temperature for five minutes to refold the tRNA in advance.

To the ligation reaction solution, sodium acetate was added to make a concentration of 0.3 M, and phenol-chloroform extraction was performed to prepare Compound AAtR-1.

Similarly, the transcribed tRNA(Glu)uga-CA (SEQ ID NO: 78 (TR-2)) was ligated to aminoacylated pCpA (SS15) by the method described above. To the ligation reaction solution, sodium acetate was added to make 0.3 M, and phenol-chloroform extraction was performed to prepare Compound AAtR-2.

Similarly, lig-tRNA(Glu)uga-CA (SEQ ID NO: 56 (UR-1)) was ligated to aminoacylated pCpA (SS15) by the method described above. To the ligation reaction solution, sodium acetate was added to make 0.3 M, and phenol-chloroform extraction was performed to prepare Compound AAtR-3.

Similarly, tRNA(Glu)Lga-CA (SEQ ID NO: 57 (LR-1)) was ligated to aminoacylated pCpA (SS15) by the method described above. To the ligation reaction solution, sodium acetate was added to make 0.3 M, and phenol-chloroform extraction was performed to prepare Compound AAtR-4.

Similarly, the transcribed tRNA(Glu)uga-CA (SEQ ID NO: 79 (TR-3)) was ligated to aminoacylated pCpA (ts14; synthesized by a method described in Patent Literature (WO2018143145A1)) by the method described above. To the ligation reaction solution, sodium acetate was added to make 0.3 M, and phenol-chloroform extraction was performed to prepare Compound AAtR-5.

Phenol-chloroform extracts of three compounds: Compound AAtR-1, Compound AAtR-2, and Compound AAtR-5, were mixed in equal amounts, and the mixed aminoacylated tRNA solution (mixed solution of Compound AAtR-1, Compound AAtR-2, and Compound AAtR-5) was subjected to ethanol precipitation for recovery of the Compounds.

Phenol-chloroform extracts of three compounds: Compound AAtR-1, Compound AAtR-3, and Compound AAtR-5, were mixed in equal amounts, and the mixed aminoacylated tRNA solution (mixed solution of Compound AAtR-1, Compound AAtR-3, and Compound AAtR-5) was subjected to ethanol precipitation for recovery of the Compounds.

Phenol-chloroform extracts of three compounds: Compound AAtR-1, Compound AAtR-4, and Compound AAtR-5, were mixed in equal amounts, and the mixed aminoacylated tRNA solution (mixed solution of Compound AAtR-1, Compound AAtR-4, and Compound AAtR-5) was subjected to ethanol precipitation for recovery of the Compounds.

A reaction solution was prepared by adding Nuclease free water to adjust the solution to 25 µM transcribed tRNA(Glu)aag-CA (SEQ ID NO: 80 (TR-4)), 50 mM HEPES-KOH pH7.5, 20 mM MgCl₂, 1 mM ATP, 0.6 unit/µL T4 RNA ligase (New England Biolabs), and 0.25 mM aminoacylated pCpA (a DMSO solution of ts14), and ligation reaction was performed at 15°C for 45 minutes. It should be noted that before adding T4 RNA ligase and aminoacylated pCpA, the reaction solution was heated to 95°C for two minutes and then allowed to stand at room temperature for five minutes to refold the tRNA in advance.

To the ligation reaction solution, sodium acetate was added to make a concentration of 0.3 M, and phenol-chloroform extraction was performed to prepare Compound AAtR-6.

Similarly, the transcribed tRNA(Glu)uag-CA (SEQ ID NO: 81 (TR-5)) was ligated to aminoacylated pCpA (SS14) by the method described above. To the ligation reaction solution, sodium acetate was added to make 0.3 M, and phenol-chloroform extraction was performed to prepare Compound AAtR-7.

Similarly, tRNA(Glu)Lag-CA (SEQ ID NO: 59 (LR-2)) was ligated to aminoacylated pCpA (SS14) by the method described above. To the ligation reaction solution, sodium acetate was added to make 0.3 M, and phenol-chloroform extraction was performed to prepare Compound AAtR-8.

Similarly, tRNA(Glu)cag-CA (SEQ ID NO: 82 (TR-6)) was ligated to aminoacylated pCpA (TS124) by the method described above. To the ligation reaction solution, sodium acetate was added to make 0.3 M, and phenol-chloroform extraction was performed to prepare Compound AAtR-9.

Phenol-chloroform extracts of three compounds: Compound AAtR-6, Compound AAtR-7, and Compound AAtR-9, were mixed in equal amounts, and the mixed aminoacylated tRNA solution (mixed solution of Compound AAtR-6, Compound AAtR-7, and Compound AAtR-9) was subjected to ethanol precipitation for recovery of the Compounds.

Phenol-chloroform extracts of three compounds: Compound AAtR-6, Compound AAtR-8, and Compound AAtR-9, were mixed in equal amounts, and the mixed aminoacylated tRNA solution (mixed solution of Compound AAtR-6, Compound AAtR-8, and Compound AAtR-9) was subjected to ethanol precipitation for recovery of the Compounds.

A reaction solution was prepared by adding Nuclease free water to adjust the solution to 25 µM transcribed tRNA(Glu)aac-CA (SEQ ID NO: 83 (TR-7)), 50 mM HEPES-KOH pH7.5, 20 mM MgCl₂, 1 mM ATP, 0.6 unit/µL T4 RNA ligase (New England Biolabs), and 0.25 mM aminoacylated pCpA (a DMSO solution of ts14), and ligation reaction was performed at 15°C for 45 minutes. It should be noted that before adding T4 RNA ligase and aminoacylated pCpA, the reaction solution was heated to 95°C for two minutes and then left at room temperature for five minutes to refold the tRNA in advance.

To the ligation reaction solution, sodium acetate was added to make a concentration of 0.3 M, and phenol-chloroform extraction was performed to prepare Compound AAtR-10.

Similarly, the transcribed tRNA(Glu)uac-CA (SEQ ID NO: 84 (TR-8)) was ligated to aminoacylated pCpA (SS14) by the method described above. To the ligation reaction solution, sodium acetate was added to make 0.3 M, and phenol-chloroform extraction was performed to prepare Compound AAtR-11.

Similarly, tRNA(Glu)Lac-CA (SEQ ID NO: 61 (LR-3)) was ligated to aminoacylated pCpA (SS14) by the method described above. To the ligation reaction solution, sodium acetate was added to make 0.3 M, and phenol-chloroform extraction was performed to prepare Compound AAtR-12.

Similarly, tRNA(Glu)cac-CA (SEQ ID NO: 85 (TR-9)) was ligated to aminoacylated pCpA (TS24) by the method described above. To the ligation reaction solution, sodium acetate was added to make 0.3 M, and phenol-chloroform extraction was performed to prepare Compound AAtR-13.

Phenol-chloroform extracts of three compounds: Compound AAtR-10, Compound AAtR-11, and Compound AAtR-13, were mixed in equal amounts, and the mixed aminoacylated tRNA solution (mixed solution of Compound AAtR-10, Compound AAtR-11, and Compound AAtR-13) was subjected to ethanol precipitation for recovery of the Compounds.

Phenol-chloroform extracts of three compounds: Compound AAtR-10, Compound AAtR-12, and Compound AAtR-13, were mixed in equal amounts, and the mixed aminoacylated tRNA solution (mixed solution of Compound AAtR-10, Compound AAtR-12, and Compound AAtR-13) was subjected to ethanol precipitation for recovery of the Compounds.

A reaction solution was prepared by adding Nuclease free water to adjust the solution to 25 µM transcribed tRNA(Glu)gcc-CA (SEQ ID NO: 86 (TR-10)), 50 mM HEPES-KOH pH7.5, 20 mM MgCl₂, 1 mM ATP, 0.6 unit/µL T4 RNA ligase (New England Biolabs), and 0.25 mM aminoacylated pCpA (a DMSO solution of TS24), and ligation reaction was performed at 15°C for 45 minutes. It should be noted that before adding T4 RNA ligase and aminoacylated pCpA, the reaction solution was heated to 95°C for two minutes and then left at room temperature for five minutes to refold the tRNA in advance.

To the ligation reaction solution, sodium acetate was added to make a concentration of 0.3 M, and phenol-chloroform extraction was performed to prepare Compound AAtR-14.

Similarly, the transcribed tRNA(Glu)ucc-CA (SEQ ID NO: 87 (TR-11)) was ligated to aminoacylated pCpA (SS14) by the method described above. To the ligation reaction solution, sodium acetate was added to make 0.3 M, and phenol-chloroform extraction was performed to prepare Compound AAtR-15.

Similarly, tRNA(Glu)Lcc-CA (SEQ ID NO: 63 (LR-4)) was ligated to aminoacylated pCpA (SS14) by the method described above. To the ligation reaction solution, sodium acetate was added to make 0.3 M, and phenol-chloroform extraction was performed to prepare Compound AAtR-16.

Similarly, tRNA(Glu)ccc-CA (SEQ ID NO: 88 (TR-12)) was ligated to aminoacylated pCpA (TS16) by the method described above. To the ligation reaction solution, sodium acetate was added to make 0.3 M, and phenol-chloroform extraction was performed to prepare Compound AAtR-17.

Phenol-chloroform extracts of three compounds: Compound AAtR-14, Compound AAtR-15, and Compound AAtR-17, were mixed at a ratio of 1:2:1, and the mixed aminoacylated tRNA solution (mixed solution of Compound AAtR-14, Compound AAtR-15, and Compound AAtR-17) was subjected to ethanol precipitation for recovery of the Compounds.

Phenol-chloroform extracts of three compounds: Compound AAtR-14, Compound AAtR-16, and Compound AAtR-17, were mixed at a ratio of 1:2:1, and the mixed aminoacylated tRNA solution (mixed solution of Compound AAtR-14, Compound AAtR-16, and Compound AAtR-17) was subjected to ethanol precipitation for recovery of the Compounds.

The mixed aminoacylated tRNA solutions were dissolved in 1 mM sodium acetate immediately before addition to the translation mixture.

To prepare Compound AAt-19, a reaction solution was prepared by adding Nuclease free water to adjust the solution to 25 µM transcribed tRNA(Asp)aag-CA (SEQ ID NO: 153 (TR-14)), 50 mM HEPES-KOH pH7.5, 20 mM MgCl₂, 1 mM ATP, 0.6 unit/µL T4 RNA ligase (New England Biolabs), and 0.25 mM aminoacylated pCpA (a DMSO solution of Compound ts14 synthesized by a method described in a patent (WO2018143145A1)), and ligation reaction was performed at 15°C for 45 minutes. It should be noted that before adding T4 RNA ligase and aminoacylated pCpA, the reaction solution was heated to 95°C for two minutes and then left at room temperature for five minutes to refold the tRNA in advance.

Similarly, the transcribed tRNA(Asp)uag-CA (SEQ ID NO: 154 (TR-15)) was ligated to aminoacylated pCpA (SS15) by the method described above to prepare Compound AAtR-20.

Similarly, the transcribed tRNA(Asp)Lag-CA (SEQ ID NO: 134 (TR-5)) was ligated to aminoacylated pCpA (SS15) by the method described above to prepare Compound AAtR-21.

Similarly, the transcribed tRNA(Asp)cag-CA (SEQ ID NO: 155 (TR-16)) was ligated to aminoacylated pCpA (TS24) by the method described above to prepare Compound AAtR-22. After adding 0.3 M sodium acetate and phenol-chloroform solution to each ligated solution, the ligation products were mixed, and the mixture was extracted with phenol-chloroform and collected by ethanol precipitation.

Specifically, 0.3 M sodium acetate and phenol-chloroform solution were added to three ligation products: Compound AAtR-19, Compound AAtR-20, and Compound AAtR-22, and these were mixed in equal amounts. Then the mixture was extracted with phenol-chloroform and collected by ethanol precipitation to prepare a mixed aminoacylated tRNA solution (mixed solution of Compound AAtR-19, Compound AAtR-20, and Compound AAtR-22).

Similarly, 0.3 M sodium acetate and phenol-chloroform solution were added to three ligation products: Compound AAtR-19, Compound AAtR-21, and Compound AAtR-22, and these were mixed in equal amounts. Then the mixture was extracted with phenol-chloroform and collected by ethanol precipitation to prepare a mixed aminoacylated tRNA solution (mixed solution of Compound AAtR-19, Compound AAtR-21, and Compound AAtR-22).

To prepare Compound AAtR-23, a reaction solution was prepared by adding Nuclease free water to adjust the solution to 25 µM transcribed tRNA(AsnE2)aag-CA (SEQ ID NO: 156 (TR-17)), 50 mM HEPES-KOH pH7.5, 20 mM MgCl₂, 1 mM ATP, 0.6 unit/µL T4 RNA ligase (New England Biolabs), and 0.25 mM aminoacylated pCpA (a DMSO solution of Compound ts14 synthesized by a method described in a patent (WO2018143145A1)), and ligation reaction was performed at 15°C for 45 minutes. It should be noted that before adding T4 RNA ligase and aminoacylated pCpA, the reaction solution was heated to 95°C for two minutes and then left at room temperature for five minutes to refold the tRNA in advance.

Similarly, the transcribed tRNA(AsnE2)uag-CA (SEQ ID NO: 157 (TR-18)) was ligated to aminoacylated pCpA (SS15) by the method described above to prepare Compound AAtR-24.

Similarly, the tRNA(AsnE2)Lag-CA (SEQ ID NO: 137 (TR-6)) was ligated to aminoacylated pCpA (SS15) by the method described above to prepare Compound AAtR-25.

Similarly, the transcribed tRNA(AsnE2)cag-CA (SEQ ID NO: 158 (TR-19)) was ligated to aminoacylated pCpA (TS24) by the method described above to prepare Compound AAtR-26.

After adding 0.3 M sodium acetate and phenol-chloroform solution to each ligated solution, the ligation products were mixed, and the mixture was extracted with phenol-chloroform and collected by ethanol precipitation.

Specifically, 0.3 M sodium acetate and phenol-chloroform solution were added to three ligation products: Compound AAtR-23, Compound AAtR-24, and Compound AAtR-26, and these were mixed in equal amounts. Then the mixture was extracted with phenol-chloroform and collected by ethanol precipitation to prepare a mixed aminoacylated tRNA solution (mixed solution of Compound AAtR-23, Compound AAtR-24, and Compound AAtR-26).

Similarly, 0.3 M sodium acetate and phenol-chloroform solution were added to three ligation products: Compound AAtR-23, Compound AAtR-25, and Compound AAtR-26, and these were mixed in equal amounts. Then the mixture was extracted with phenol-chloroform and collected by ethanol precipitation to prepare a mixed aminoacylated tRNA solution (mixed solution of Compound AAtR-23, Compound AAtR-25, and Compound AAtR-26).

To prepare Compound AAtR-6, a reaction solution was prepared by adding Nuclease free water to adjust the solution to 25 (µM transcribed tRNA(Glu)aag-CA (SEQ ID NO: 80 (TR-4)), 50 mM HEPES-KOH pH7.5, 20 mM MgCl₂, 1 mM ATP, 0.6 unit/µL T4 RNA ligase (New England Biolabs), and 0.25 mM aminoacylated pCpA (a DMSO solution of ts14), and ligation reaction was performed at 15°C for 45 minutes. It should be noted that before adding T4 RNA ligase and aminoacylated pCpA, the reaction solution was heated to 95°C for two minutes and then left at room temperature for five minutes to refold the tRNA in advance.

Similarly, the transcribed tRNA(Glu)cag-CA (SEQ ID NO: 82 (TR-6)) was ligated to aminoacylated pCpA (TS24) by the method described above to prepare Compound AAtR-9.

Similarly, tRNA(Glu)uag-CA (SEQ ID NO: 81 (TR-5)) was ligated to aminoacylated pCpA (SS16) by the method described above to prepare Compound AAtR-27.

Similarly, the transcribed tRNA(Glu)Lag-CA (SEQ ID NO: 59 (LR-2)) was ligated to aminoacylated pCpA (SS16) by the method described above to prepare Compound AAtR-28.

Similarly, the transcribed tRNA(Glu)uag-CA (SEQ ID NO: 81 (TR-5)) was ligated to aminoacylated pCpA (SS39) by the method described above to prepare Compound AAtR-29.

Similarly, tRNA(Glu)Lag-CA (SEQ ID NO: 59 (LR-2)) was ligated to aminoacylated pCpA (SS39) by the method described above to prepare Compound AAtR-30.

Similarly, the transcribed tRNA(Glu)uag-CA (SEQ ID NO: 81 (TR-5)) was ligated to aminoacylated pCpA (SS40) by the method described above to prepare Compound AAtR-31.

Similarly, tRNA(Glu)Lag-CA (SEQ ID NO: 59 (LR-2)) was ligated to aminoacylated pCpA (SS40) by the method described above to prepare Compound AAtR-32.

After adding 0.3 M sodium acetate and phenol-chloroform solution to each ligated solution, the ligation products were mixed, and the mixture was extracted with phenol-chloroform and collected by ethanol precipitation.

Specifically, 0.3 M sodium acetate and phenol-chloroform solution were added to three ligation products: Compound AAtR-6, Compound AAtR-27, and Compound AAtR-9, and these were mixed in equal amounts. Then the mixture was extracted with phenol-chloroform and collected by ethanol precipitation to prepare a mixed aminoacylated tRNA solution (mixed solution of Compound AAtR-6, Compound AAtR-28, and Compound AAtR-9).

Similarly, 0.3 M sodium acetate and phenol-chloroform solution were added to three ligation products: Compound AAtR-6, Compound AAtR-28, and Compound AAtR-9, and these were mixed in equal amounts. Then the mixture was extracted with phenol-chloroform and collected by ethanol precipitation to prepare a mixed aminoacylated tRNA solution (mixed solution of Compound AAtR-6, Compound AAtR-28, and Compound AAtR-9).

Similarly, 0.3 M sodium acetate and phenol-chloroform solution were added to three ligation products: Compound AAtR-6, Compound AAtR-29, and Compound AAtR-9, and these were mixed in equal amounts. Then the mixture was extracted with phenol-chloroform and collected by ethanol precipitation to prepare a mixed aminoacylated tRNA solution (mixed solution of Compound AAtR-6, Compound AAtR-29, and Compound AAtR-9).

Similarly, 0.3 M sodium acetate and phenol-chloroform solution were added to three ligation products: Compound AAtR-6, Compound AAtR-30, and Compound AAtR-9, and these were mixed in equal amounts. Then the mixture was extracted with phenol-chloroform and collected by ethanol precipitation to prepare a mixed aminoacylated tRNA solution (mixed solution of Compound AAtR-6, Compound AAtR-30, and Compound AAtR-9).

Similarly, 0.3 M sodium acetate and phenol-chloroform solution were added to three ligation products: Compound AAtR-6, Compound AAtR-31, and Compound AAtR-9, and these were mixed in equal amounts. Then the mixture was extracted with phenol-chloroform and collected by ethanol precipitation to prepare a mixed aminoacylated tRNA solution (mixed solution of Compound AAtR-6, Compound AAtR-31, and Compound AAtR-9).

Similarly, 0.3 M sodium acetate and phenol-chloroform solution were added to three ligation products: Compound AAtR-6, Compound AAtR-32, and Compound AAtR-9, and these were mixed in equal amounts. Then the mixture was extracted with phenol-chloroform and collected by ethanol precipitation to prepare a mixed aminoacylated tRNA solution (mixed solution of Compound AAtR-6, Compound AAtR-32, and Compound AAtR-9).

0.3 M sodium acetate and phenol-chloroform solution were added to the ligation product Compound AAtR-9, and the mixture was extracted with phenol-chloroform and collected by ethanol precipitation to prepare an aminoacylated tRNA.

To prepare Compound AAtR-33, a reaction solution was prepared by adding Nuclease free water to adjust the solution to 25 µM transcribed tRNA(Glu)gcg-CA (SEQ ID NO: 159 (TR-20)), 50 mM HEPES-KOH pH7.5, 20 mM MgCl₂, 1 mM ATP, 0.6 unit/µL T4 RNA ligase (New England Biolabs), and 0.25 mM aminoacylated pCpA (a DMSO solution of Compound TS24 synthesized by a method described in a patent (WO2018143145A1)), and ligation reaction was performed at 15°C for 45 minutes. It should be noted that before adding T4 RNA ligase and aminoacylated pCpA, the reaction solution was heated to 95°C for two minutes and then left at room temperature for five minutes to refold the tRNA in advance.

Similarly, tRNA(Glu)Lcg-CA (SEQ ID NO: 140 (LR-7)) was ligated to aminoacylated pCpA (SS14) by the method described above to prepare Compound AAtR-34.

Similarly, the transcribed tRNA(Glu)ccg-CA (SEQ ID NO: 160 (LR-21)) was ligated to aminoacylated pCpA (ts14) by the method described above to prepare Compound AAtR-35.

Similarly, the transcribed tRNA(Glu)aau-CA (SEQ ID NO: 161 (LR-22)) was ligated to aminoacylated pCpA (ts14) by the method described above to prepare Compound AAtR-36.

Similarly, tRNA(Glu)Lau-CA (SEQ ID NO: 142 (LR-8)) was ligated to aminoacylated pCpA (SS14) by the method described above to prepare Compound AAtR-37.

Similarly, the transcribed tRNA(Glu)cau-CA (SEQ ID NO: 162 (TR-23)) was ligated to aminoacylated pCpA (TS24) by the method described above to prepare Compound AAtR-38.

After adding 0.3 M sodium acetate and phenol-chloroform solution to each ligated solution, the ligation products were mixed, and the mixture was extracted with phenol-chloroform and collected by ethanol precipitation.

Specifically, 0.3 M sodium acetate and phenol-chloroform solution were added to two ligation products: Compound AAtR-33 and Compound AAtR-35, and these were mixed in equal amounts Then the mixture was extracted with phenol-chloroform and collected by ethanol precipitation to prepare a mixed aminoacylated tRNA solution (mixed solution of Compound AAtR-33 and Compound AAtR-35).

Similarly, 0.3 M sodium acetate and phenol-chloroform solution were added to two ligation products: Compound AAtR-36 and Compound AAtR-38, and these were mixed in equal amounts. Then the mixture was extracted with phenol-chloroform and collected by ethanol precipitation to prepare a mixed aminoacylated tRNA solution (mixed solution of Compound AAtR-36 and Compound AAtR-38).

0.3 M sodium acetate and phenol-chloroform solution were added to the ligation product Compound AAtR-37, and the mixture was extracted with phenol-chloroform and collected by ethanol precipitation to prepare an aminoacylated tRNA.

The transcribed tRNA(Glu)aag-CA (SEQ ID NO: 80 (TR-4)) was ligated to aminoacylated pCpA (ts14) by the method described above to prepare Compound AAtR-6.

Similarly, tRNA(Glu)cag-CA (SEQ ID NO: 82 (TR-6)) was ligated to aminoacylated pCpA (TS24) by the method described above to prepare Compound AAtR-9.

After adding 0.3 M sodium acetate and phenol-chloroform solution to each ligated solution, the ligation products were mixed, and the mixture was extracted with phenol-chloroform and collected by ethanol precipitation.

Specifically, 0.3 M sodium acetate and phenol-chloroform solution were added to two ligation products: Compound AAtR-6 and Compound AAtR-9, and these were mixed at a ratio of 1:2. Then the mixture was extracted with phenol-chloroform and collected by ethanol precipitation to prepare a mixed aminoacylated tRNA solution (mixed solution of Compound AAtR-6 and Compound AAtR-9).

The transcribed tRNA(Glu)uag-CA (SEQ ID NO: 81 (TR-5)) was ligated to aminoacylated pCpA (SS15) by the method described above to prepare Compound AAtR-39.

Similarly, tRNA(Glu)(Agm)ag-CA (SEQ ID NO: 138 (AR-1)) was ligated to aminoacylated pCpA (SS15) by the method described above to prepare Compound AAtR-40.

0.3 M sodium acetate and phenol-chloroform solution were added to each ligation reacted solution, then phenol-chloroform extraction and ethanol precipitation were performed to recover.

### Preparation of initiator aminoacyl tRNA using aminoacyl pCpA

A reaction solution was prepared by adding Nuclease free water to adjust the solution to 25 µM transcribed tRNA(fMet)cau-CA (SEQ ID NO: 89 (TR-13)), 50 mM HEPES-KOH pH7.5, 20 mM MgCl₂, 1 mM ATP, 0.6 unit/µL T4 RNA ligase (New England Biolabs), and 0.25 mM aminoacylated pCpA (a DMSO solution of MT01), and ligation reaction was performed at 15°C for 45 minutes. It should be noted that before adding T4 RNA ligase and aminoacylated pCpA, the reaction solution was heated to 95°C for two minutes and then left at room temperature for five minutes to refold the tRNA in advance.

To the ligation reaction solution, sodium acetate was added to make a concentration of 0.3 M, and phenol-chloroform extraction was performed to recover initiator aminoacyl tRNA (Compound AAtR-18) by ethanol precipitation.

The initiator aminoacylated tRNA was dissolved in 1 mM sodium acetate immediately before addition to the translation mixture.

Compound AAtR-1 SEQ ID NO: 90
   dA-tRNA(Glu)aga 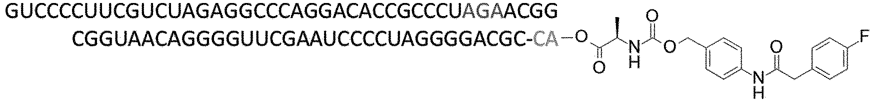
Compound AAtR-2 SEQ ID NO: 91
   SPh2Cl-tRNA(Glu)uga 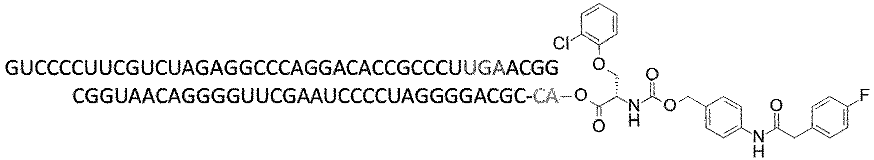
Compound AAtR-3 SEQ ID NO: 92
   SPh2Cl-lig-tRNA(Glu)uga 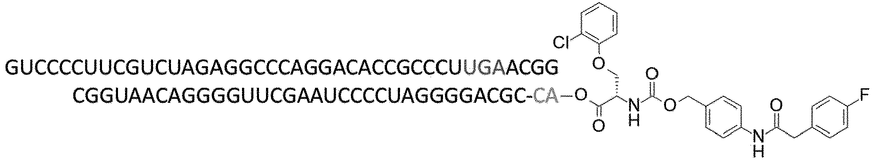
Compound AAtR-4 SEQ ID NO: 93
   SPh2Cl-tRNA(Glu)Lga 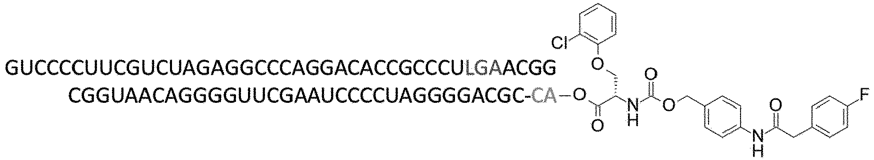
Compound AAtR-5 SEQ ID NO: 94
   nBuG-tRNA(Glu)cga 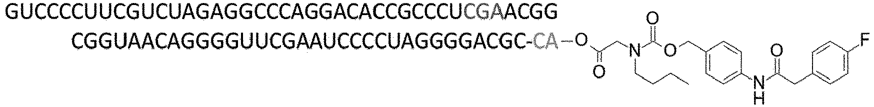
Compound AAtR-6 SEQ ID NO: 95
   nBuG-tRNA(Glu)aag 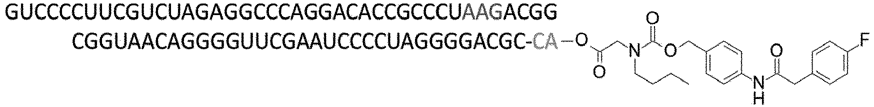
Compound AAtR-7 SEQ ID NO: 96
   Pic2-tRNA(Glu)uag 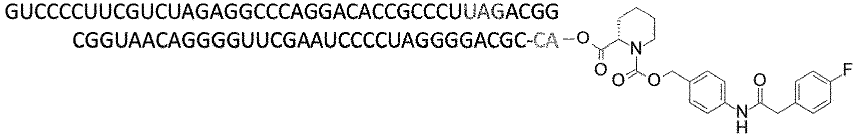
Compound AAtR-8 SEQ ID NO: 97
   Pic2-tRNA(Glu)Lag 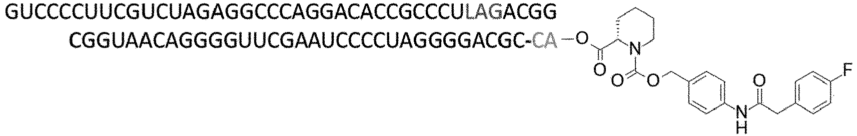
Compound AAtR-9 SEQ ID NO: 98
   dA-tRNA(Glu)cag 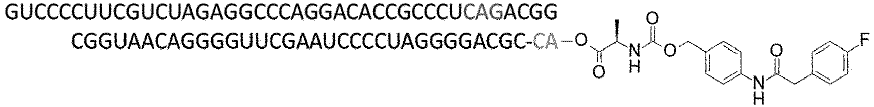
Compound AAtR-10 SEQ ID NO: 99
   nBuG-tRNA(Glu)aac 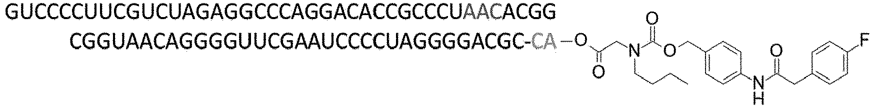
Compound AAtR-11 SEQ ID NO: 100
   Pic2-tRNA(Glu)uac 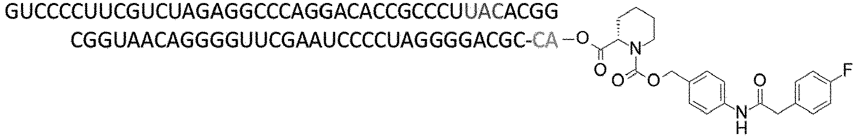
Compound AAtR-12 SEQ ID NO: 101
   Pic2-tRNA(Glu)Lac 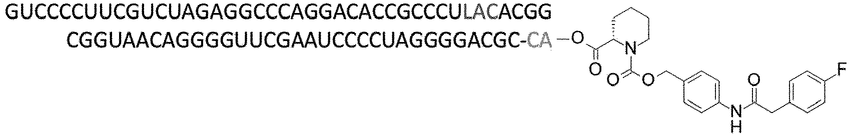
Compound AAtR-13 SEQ ID NO: 102
   dA-tRNA(Glu)cac 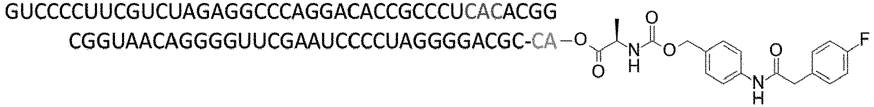
Compound AAtR-14 SEQ ID NO: 103
   dA-tRNA(Glu)gcc 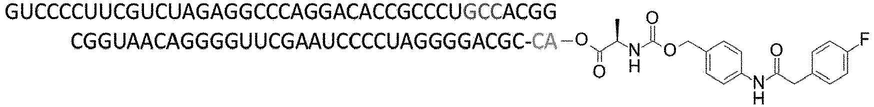
Compound AAtR-15 SEQ ID NO: 104
   Pic2-tRNA(Glu)ucc 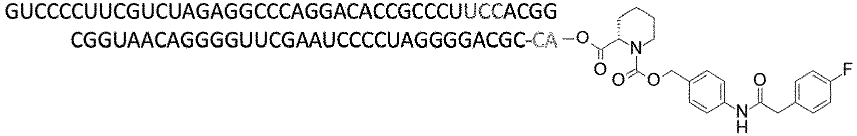
Compound AAtR-16 SEQ ID NO: 105
   Pic2-tRNA(Glu)Lcc 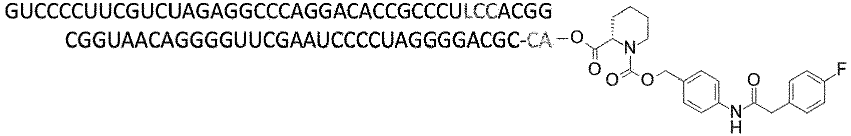
Compound AAtR-17 SEQ ID NO: 106
   MeHph-tRNA(Glu)ccc 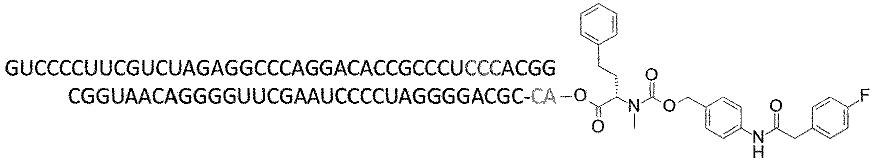
Compound AAtR-18 SEQ ID NO: 107
   BdpFL-Phe-tRNA(fMet)cau 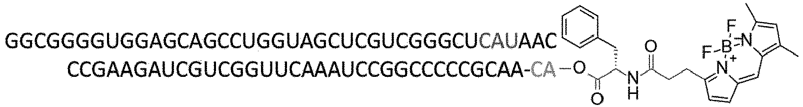
Compound AAtR-19 SEQ ID NO: 175
   nBuG-tRNA(Asp)aag 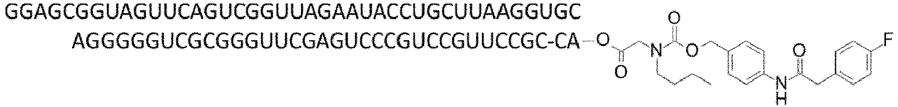
Compound AAtR-20 SEQ ID NO: 176
   SPh2Cl-tRNA(Asp)uag 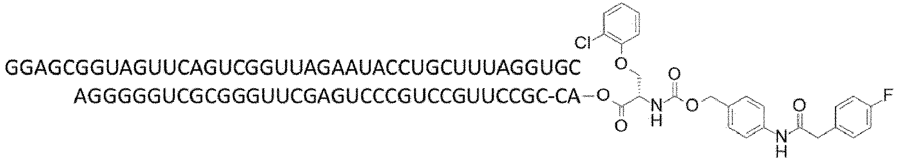
Compound AAtR-21 SEQ ID NO: 177
   SPh2Cl-tRNA(Asp)Lag 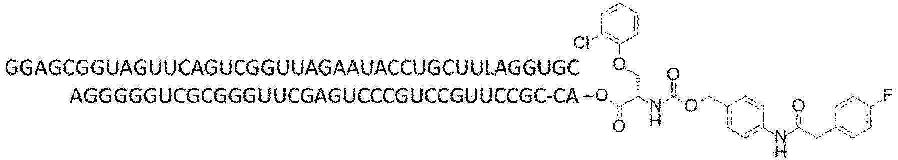
Compound AAtR-22 SEQ ID NO: 178
   dA-tRNA(Asp)cag 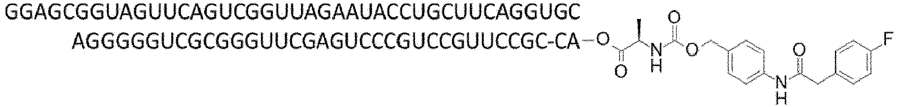
Compound AAtR-23 SEQ ID NO: 179
   nBuG-tRNA(AsnE2)aag 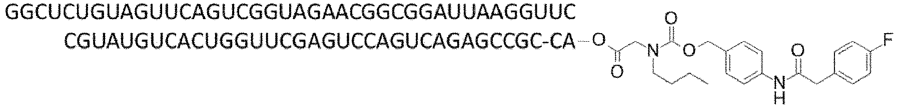
Compound AAtR-24 SEQ ID NO: 180
   SPh2Cl-tRNA(AsnE2)uag 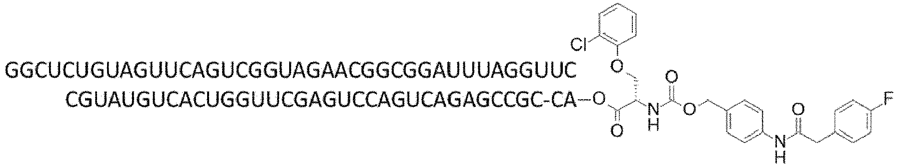
Compound AAtR-25 SEQ ID NO: 181
   SPh2Cl-tRNA(AsnE2)Lag 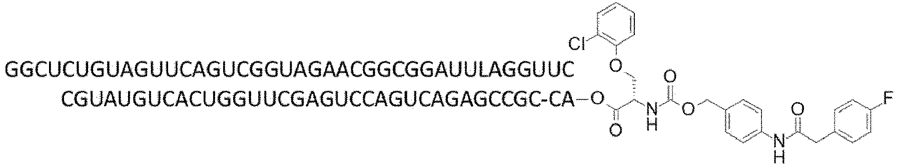
Compound AAtR-26 SEQ ID NO: 182
   dA-tRNA(AsnE2)cag 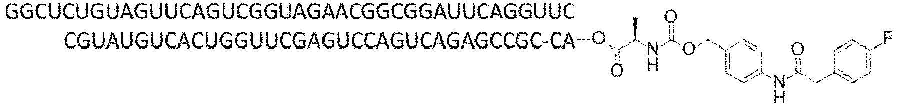
Compound AAtR-27 SEQ ID NO: 183
   MeHph-tRNA(Glu)uag 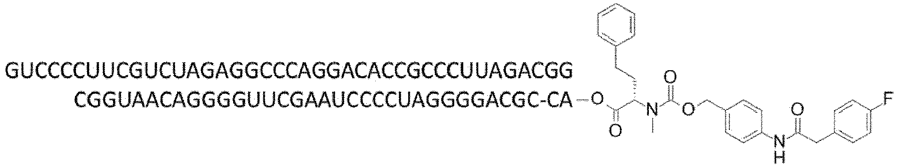
Compound AAtR-28 SEQ ID NO: 184
   MeHph-tRNA(Glu)Lag 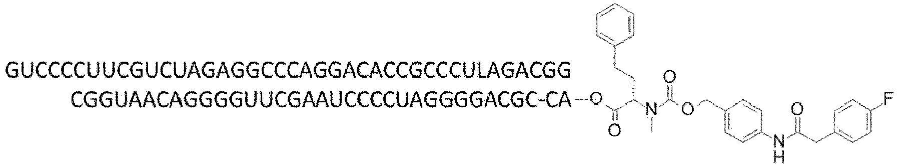
Compound AAtR-29 SEQ ID NO: 185
   F3Cl-tRNA(Glu)uag 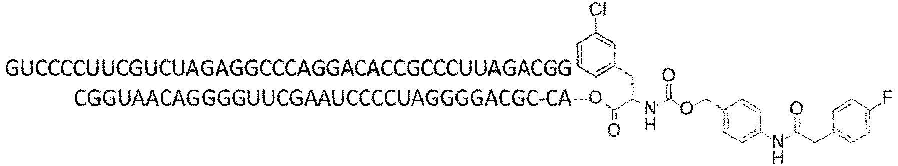
Compound AAtR-30 SEQ ID NO: 186
   F3Cl-tRNA(Glu)Lag 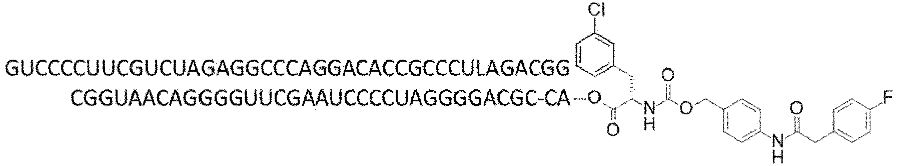
Compound AAtR-31 SEQ ID NO: 187
   SiPen-tRNA(Glu)uag 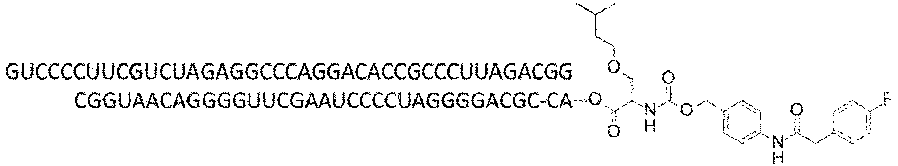
Compound AAtR-32 SEQ ID NO: 188
   SiPen-tRNA(Glu)Lag 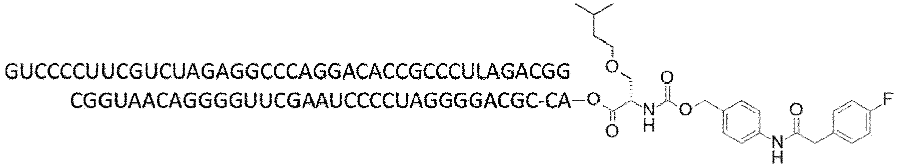
Compound AAtR-33 SEQ ID NO: 189
   dA-tRNA(Glu)gcg 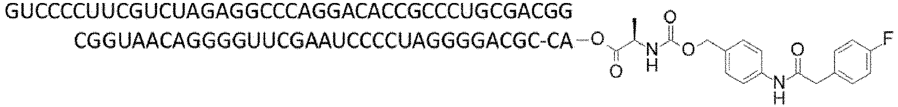
Compound AAtR-34 SEQ ID NO: 190
   Pic2-tRNA(Glu)Lcg 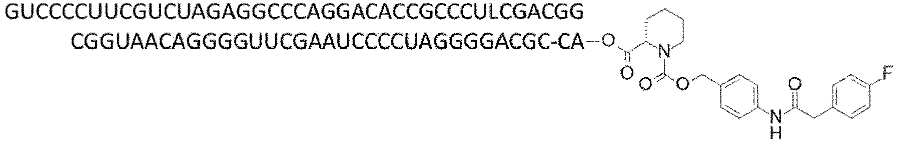
Compound AAtR-35 SEQ ID NO: 191
   nBuG-tRNA(Glu)ccg 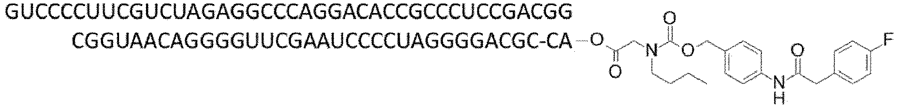
Compound AAtR-36 SEQ ID NO: 192
   nBuG-tRNA(Glu)aau 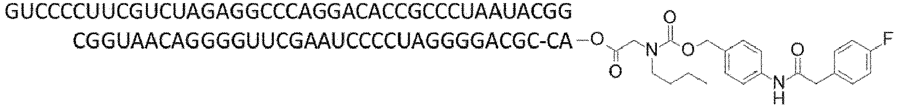
Compound AAtR-37 SEQ ID NO: 193
   Pic2-tRNA(Glu)Lau 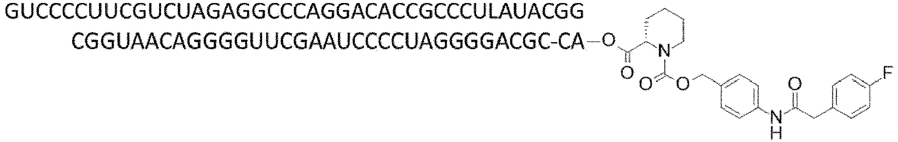
Compound AAtR-38 SEQ ID NO: 194
   dA-tRNA(Glu)cau 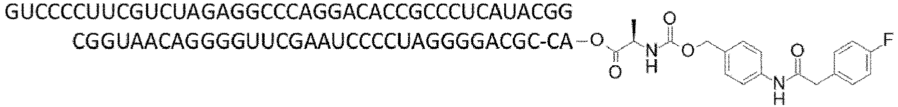
Compound AAtR-39 SEQ ID NO: 195
   SPh2Cl-tRNA(Glu)uag 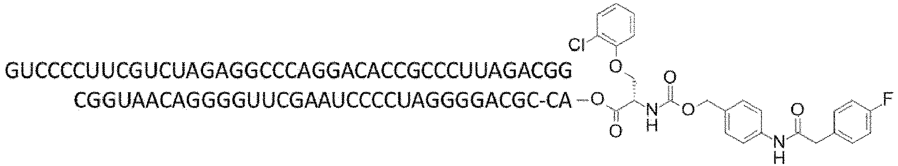
Compound AAtR-40 SEQ ID NO: 196
   SPh2Cl-tRNA(Glu)(Agm)ag

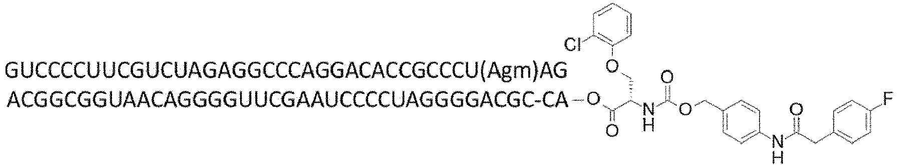

### Example 12. Translational synthesis of peptides

Next, an experiment was performed to confirm the discrimination of three amino acids in one codon box in the presence of three aminoacylated tRNAs. Specifically, template mRNAs containing any one of three codons in the same codon box and having the same sequence for the rest of the sequences (template mRNAs of SEQ ID NO: 120 (mR-1) to SEQ ID NO: 131 (mR-12)) were translated using a mixed aminoacylated tRNA solution not containing a lysidine-modified tRNA (mixed solution of Compound AAtR-1, Compound AAtR-2, and Compound AAtR-5; mixed solution of Compound AAtR-1, Compound AAtR-3, and Compound AAtR-5; mixed solution of Compound AAtR-6, Compound AAtR-7, and Compound AAtR-9; mixed solution of Compound AAtR-10, Compound AAtR-11, and Compound AAtR-13; and mixed solution of Compound AAtR-14, Compound AAtR-15, and Compound AAtR-17) or using a mixed aminoacylated tRNA solution containing a lysidine-modified tRNA (mixed solution of Compound AAtR-1, Compound AAtR-4, and Compound AAtR-5; mixed solution of Compound AAtR-6, Compound AAtR-8, and Compound AAtR-9; mixed solution of Compound AAtR-10, Compound AAtR-12, and Compound AAtR-13; and mixed solution of Compound AAtR-14, Compound AAtR-16, and Compound AAtR-17) to translationally synthesize peptide compounds.

The translation system used was PURE system, a prokaryote-derived reconstituted cell-free protein synthesis system. Specifically, the synthesis was carried out as follows: 1 µM template mRNA (SEQ ID NO: 120 (mR-1), SEQ ID NO: 121 (mR-2), or SEQ ID NO: 122 (mR-3)), a group of natural amino acids encoded in the respective template mRNAs at 0.25 mM respectively, and initiator aminoacylated tRNA (Compound AAtR-18) at 10 µM were added to a translation solution (1 mM GTP, 1 mM ATP, 20 mM phosphocreatine, 50 mM HEPES-KOH pH7.6, 100 mM potassium acetate, 10 mM magnesium acetate, 2 mM spermidine, 1 mM dithiothreitol, 1.5 mg/mL E. coli MRE600 (RNase-negative)-derived tRNA (Roche), 0.26 µM EF-G, 0.24 µM RF2, 0.17 µM RF3, 0.5 µM RRF, 4 µg/mL creatine kinase, 3 µg/mL myokinase, 2 unit/mL inorganic pyrophosphatase, 1.1 µg/mL nucleoside diphosphate kinase, 2.7 µM IF1, 0.4 µM IF2, 1.5 µM IF3, 40 µM EF-Tu, 54 µM EF-Ts, 1 µM EF-P-Lys, 0.4 unit/µL RNasein Ribonuclease inhibitor (Promega, N2111), 1.2 µM ribosome, 0.5 mM PGA, 0.09 µM GlyRS, 0.4 µM IleRS, 0.68 µM PheRS, 0.16 µM ProRS, and 0.09 µM ThrRS), and a mixed aminoacylated tRNA solution (mixed solution of Compound AAtR-1, Compound AAtR-2, and Compound AAtR-5; mixed solution of Compound AAtR-1, Compound AAtR-3, and Compound AAtR-5; or a mixed solution of Compound AAtR-1, Compound AAtR-4, and Compound AAtR-5) was added at 30 µM to the translation reaction mixture, and left at 37°C for one hour.

Cell-free translations were also performed on other sequences (SEQ ID NO: 123 (mR-4), SEQ ID NO: 124 (mR-5), or SEQ ID NO: 125 (mR-6)).

Specifically, the synthesis was carried out as follows: 1 µM template mRNA (SEQ ID NO: 123 (mR-4), SEQ ID NO: 124 (mR-5), or SEQ ID NO: 125 (mR-6)), a group of natural amino acids encoded in the respective template mRNAs at 0.25 mM respectively, and initiator aminoacylated tRNA (Compound AAtR-18) at 10 µM were added to a translation solution (1 mM GTP, 1 mM ATP, 20 mM phosphocreatine, 50 mM HEPES-KOH pH7.6, 100 mM potassium acetate, 10 mM magnesium acetate, 2 mM spermidine, 1 mM dithiothreitol, 1.5 mg/mL E. coli MRE600 (RNase-negative)-derived tRNA (Roche), 0.26 µM EF-G, 0.24 µM RF2, 0.17 µM RF3, 0.5 µM RRF, 4 µg/mL creatine kinase, 3 µg/mL myokinase, 2 unit/mL inorganic pyrophosphatase, 1.1 µg/mL nucleoside diphosphate kinase, 2.7 µM IF1, 0.4 µM IF2, 1.5 µM IF3, 40 µM EF-Tu, 35 µM EF-Ts, 1 µM EF-P-Lys, 0.4 unit/µL RNasein Ribonuclease inhibitor (Promega, N2111), 1.2 µM ribosome, 0.5 mM PGA, 0.09 µM GlyRS, 0.4 µM I1eRS, 0.68 µM PheRS, 0.16 µM ProRS, and 0.09 µM ThrRS), and a mixed aminoacylated tRNA solution (mixed solution of Compound AAtR-6, Compound AAtR-7, and Compound AAtR-9; or mixed solution of Compound AAtR-6, Compound AAtR-8, and Compound AAtR-9) was added at 30 µM to the translation reaction mixture, and left at 37°C for one hour.

Cell-free translations were also performed on other sequences (SEQ ID NO: 126 (mR-7), SEQ ID NO: 127 (mR-8), or SEQ ID NO: 128 (mR-9)).

Specifically, the synthesis was carried out as follows: 1 µM template mRNA (SEQ ID NO: 126 (mR-7), SEQ ID NO: 127 (mR-8), or SEQ ID NO: 128 (mR-9)), a group of natural amino acids encoded in the respective template mRNAs at 0.25 mM respectively, and initiator aminoacylated tRNA (Compound AAtR-18) at 10 µM were added to a translation solution (1 mM GTP, 1 mM ATP, 20 mM phosphocreatine, 50 mM HEPES-KOH pH7.6, 100 mM potassium acetate, 10 mM magnesium acetate, 2 mM spermidine, 1 mM dithiothreitol, 1.5 mg/mL E. coli MRE600 (RNase-negative)-derived tRNA (Roche), 0.26 µM EF-G, 0.24 µM RF2, 0.17 µM RF3, 0.5 µM RRF, 4 µg/mL creatine kinase, 3 µg/mL myokinase, 2 unit/mL inorganic pyrophosphatase, 1.1 µg/mL nucleoside diphosphate kinase, 2.7 µM IF1, 0.4 µM IF2, 1.5 µM IF3, 40 µM EF-Tu, 35 µM EF-Ts, 1 µM EF-P-Lys, 0.4 unit/µL RNasein Ribonuclease inhibitor (Promega, N2111), 1.2 µM ribosome, 0.5 mM PGA, 0.09 µM GlyRS, 0.4 µM I1eRS, 0.68 µM PheRS, 0.16 µM ProRS, and 0.09 µM ThrRS), and a mixed aminoacylated tRNA solution (mixed solution of Compound AAtR-10, Compound AAtR-11, and Compound AAtR-13; or mixed solution of Compound AAtR-10, Compound AAtR-12, and Compound AAtR-13) was added at 30 µM to the translation reaction mixture, and left at 37°C for one hour.

Cell-free translations were also performed on other sequences (SEQ ID NO: 129 (mR-10), SEQ ID NO: 130 (mR-11), or SEQ ID NO: 131 (mR-12)).

Specifically, the synthesis was carried out as follows: 1 µM template mRNA (SEQ ID NO: 129 (mR-10), SEQ ID NO: 130 (mR-11), or SEQ ID NO: 131 (mR-12)), a group of natural amino acids encoded in the respective template mRNAs at 0.25 mM respectively, and initiator aminoacylated tRNA (Compound AAtR-18) at 10 µM were added to a translation solution (1 mM GTP, 1 mM ATP, 20 mM phosphocreatine, 50 mM HEPES-KOH pH7.6, 100 mM potassium acetate, 10 mM magnesium acetate, 2 mM spermidine, 1 mM dithiothreitol, 1.5 mg/mL E. coli MRE600 (RNase-negative)-derived tRNA (Roche), 0.26 µM EF-G, 0.24 µM RF2, 0.17 µM RF3, 0.5 µM RRF, 4 µg/mL creatine kinase, 3 µg/mL myokinase, 2 unit/mL inorganic pyrophosphatase, 1.1 µg/mL nucleoside diphosphate kinase, 2.7 µM IF1, 0.4 µM IF2, 1.5 µM IF3, 40 µM EF-Tu, 54 µM EF-Ts, 1 µM EF-P-Lys, 0.4 unit/µL RNasein Ribonuclease inhibitor (Promega, N2111), 1.2 µM ribosome, 0.5 mM PGA, 0.4 µM I1eRS, 0.04 µM LeuRS, 0.68 µM PheRS, 0.16 µM ProRS, and 0.09 µM ThrRS), and a mixed aminoacylated tRNA solution (mixed solution of Compound AAtR-14, Compound AAtR-15, and Compound AAtR-17; or mixed solution of Compound AAtR-14, Compound AAtR-16, and Compound AAtR-17) was added at 40 µM to the translation reaction mixture, and left at 37°C for one hour.

The template mRNA, the expected translated peptide compound, and the molecular weight (calculated value) of the peptide are shown in Table 6 below.

**[Table 6]**

| Aminoacylated tRNA | Template mRNA sequence | Expected translated peptide compound | m/z [M-H] | R. T. (method1) | R. T. (method2) |
|---|---|---|---|---|---|
| CompoundAA t R-1 | mR - 1 | BdpFL-Phe-TFIIGF-dA-IIPIG | 1681.7 | 2.9 | |
| CompoundAA t R-2 | mR - 2 | BdpFL-Phe-TFIIGF-SPh2Cl-IIPIG | 1807.7 | 3.2 | |
| CompoundAA t R-3 | mR - 2 | BdpFL-Phe-TFIIGF-SPh2Cl-IIPIG | 1807.7 | 3.2 | |
| CompoundAA t R-4 | mR - 2 | BdpFL-Phe-TFIIGF-SPh2Cl-IIPIG | 1807.7 | 3.2 | |
| CompoundAA t R-5 | mR - 3 | BdpFL-Phe-TFIIGF-nBuG-IIPIG | 1723.8 | 3.0 | |
| CompoundAA t R-6 | mR - 4 | BdpFL-Phe-TFIIGF-nBuG-IIPIG | 1723.8 | 3.0 | |
| CompoundAA t R-7 | mR - 5 | BdpFL-Phe-TFIIGF-Pic2-IIPIG | 1721.8 | 3.0 | |
| CompoundAA t R-8 | mR - 5 | BdpFL-Phe-TFIIGF-Pic2-IIPIG | 1721.8 | 3.0 | |
| CompoundAA t R-9 | mR - 6 | BdpFL-Phe-TFIIGF-dA-IIPIG | 1681.8 | 2 8 | |
| CompoundAA t R-10 | mR - 7 | BdpFL-Phe-TFIIGF-nBuG-IIPIG | 1723.8 | 3.0 | |
| CompoundAA t R-11 | mR - 8 | BdpFL-Phe-TFIIGF-Pic2-IIPIG | 1721.8 | 3.0 | |
| CompoundAA t R-12 | mR - 8 | BdpFL-Phe-TFIIGF-Pic2-IIPIG | 1721.8 | 3.0 | |
| CompoundAA t R-13 | mR - 9 | BdpFL-Phe-TFIIGF-dA-IIPIG | 1681.8 | 2.8 | |
| CompoundAA t R-14 | mR - 10 | BdpFL-Phe-TFIILF-dA-IIPIL | 1794.8 | | 3.7 |
| CompoundAA t R-15 | mR - 11 | BdpFL-Phe-TFIILF-Pic2-IIPIL | 1834.8 | | 4.0 |
| CompoundAA t R-16 | mR - 11 | BdpFL-Phe-TFIILF-Pic2-IIPIL | 1834.8 | | 4.0 |
| CompoundAA t R-17 | mR - 12 | BdpFL-Phe-TFIILF-MeHph-IIPIL | 1898.8 | | 4.5 |

Next, by using a tRNA with a body sequence different from the tRNA body sequence of the previous section, an experiment was performed to confirm the discrimination of three amino acids in one codon box in the presence of three aminoacylated tRNAs. Specifically, template mRNAs containing any one of three codons in the same codon box and having the same sequence for the rest of the sequences (template mRNAs of SEQ ID NO: 123 (mR-4), SEQ ID NO: 124 (mR-5), and SEQ ID NO: 125 (mR-6)) were translated using a mixed aminoacylated tRNA solution not containing a lysidine-modified tRNA (mixed solution of Compound AAtR-19, Compound AAtR-20, and Compound AAtR-22; and mixed solution of Compound AAtR-23, Compound AAtR-24, and Compound AAtR-26) or using a mixed aminoacylated tRNA solution containing a lysidine-modified tRNA (mixed solution of Compound AAtR-19, Compound AAtR-21, and Compound AAtR-22; and mixed solution of Compound AAtR-23, Compound AAtR-25, and Compound AAtR-26) to translationally synthesize peptide compounds.

The translation system used was PURE system, a prokaryote-derived reconstituted cell-free protein synthesis system. Specifically, the synthesis was carried out as follows: 1 µM template mRNA (SEQ ID NO: 123 (mR-4), SEQ ID NO: 124 (mR-5), or SEQ ID NO: 125 (mR-6)), a group of natural amino acids encoded in the respective template mRNAs at 0.25 mM respectively, and initiator aminoacylated tRNA (Compound AAtR-18) at 10 µM were added to a translation solution (1 mM GTP, 1 mM ATP, 20 mM phosphocreatine, 50 mM HEPES-KOH pH7.6, 100 mM potassium acetate, 10 mM magnesium acetate, 2 mM spermidine, 1 mM dithiothreitol, 1.5 mg/mL E. coli MRE600 (RNase-negative)-derived tRNA (Roche), 0.26 µM EF-G, 0.24 µM RF2, 0.17 µM RF3, 0.5 µM RRF, 4 µg/mL creatine kinase, 3 µg/mL myokinase, 2 unit/mL inorganic pyrophosphatase, 1.1 µg/mL nucleoside diphosphate kinase, 2.7 µM IF1, 0.4 µM IF2, 1.5 µM IF3, 40 µM EF-Tu, 54 µM EF-Ts, 1 µM EF-P-Lys, 0.4 unit/µL RNasein Ribonuclease inhibitor (Promega, N2111), 1.2 µM ribosome, 0.5 mM PGA, 0.09 µM GlyRS, 0.4 µM IleRS, 0.68 µM PheRS, 0.16 µM ProRS, and 0.09 µM ThrRS), and a mixed aminoacylated tRNA solution (mixed solution of Compound AAtR-19, Compound AAtR-20, and Compound AAtR-22; mixed solution of Compound AAtR-19, Compound AAtR-21, and Compound AAtR-22; mixed solution of Compound AAtR-23, Compound AAtR-24, and Compound AAtR-26; or mixed solution of Compound AAtR-23, Compound AAtR-25, and Compound AAtR-26) was added at 30 µM to the translation reaction mixture, and left at 37°C for one hour.

The template mRNA, the expected translated peptide compound, and the molecular weight (calculated value) of the peptide are shown in Table 7 below.

**[Table 7]**

| Aminoacylated tRNA | Template mRNA sequence | Expected translated peptide compound | m/z [M-H] | R. T. (method1) | R. T. (method2) |
|---|---|---|---|---|---|
| CompoundAA t R-19 | mRNA - 4 | BdpF-TFIIGF-nBuG-IIPIG | 1722.9 | | 2.6 |
| CompoundAA t R-20 | mRNA - 5 | BdpF-TFIIGF-SPh2Cl-IIPIG | 1806.8 | | 3.4 |
| CompoundAA t R-21 | mRNA - 5 | BdpF-TFIIGF-SPh2Cl-IIPIG | 1806.8 | | 3.4 |
| CompoundAA t R-22 | mRNA - 6 | BdpF-TFIIGF-dA-IIPIG | 1680.9 | | 1.9 |
| CompoundAA t R-23 | mRNA - 4 | BdpF-TFIIGF-nBuG-IIPIG | 1722.9 | | 2.6 |
| CompoundAA t R-24 | mRNA - 5 | BdpF-TFIIGF-SPh2Cl-IIPIG | 1806.9 | | 3.4 |
| CompoundAA t R-25 | mRNA - 5 | BdpF-TFIIGF-SPh2C1-IIPIG | 1806.9 | | 3.4 |
| CompoundAA t R-26 | mRNA - 6 | BdpF-TFIIGF-dA-IIPIG | 1680.9 | | 1.9 |

Next, amino acids other than those aminoacylated in the lysidine-modified tRNA in the previous section were aminoacylated in the lysidine-modified tRNA, and a translation experiment was performed to confirm the discrimination of three amino acids in one codon box in the presence of three aminoacylated tRNAs. Specifically, template mRNAs containing any one of three codons in the same codon box and having the same sequence for the rest of the sequences (template mRNAs of SEQ ID NO: 123 (mR-4), SEQ ID NO: 124 (mR-5), and SEQ ID NO: 125 (mR-6)) were translated using a mixed aminoacylated tRNA solution not containing a lysidine-modified tRNA (mixed solution of Compound AAtR-6, Compound AAtR-27, and Compound AAtR-9; mixed solution of Compound AAtR-6, Compound AAtR-29, and Compound AAtR-9; and mixed solution of Compound AAtR-6, Compound AAtR-31, and Compound AAtR-9) or using a mixed aminoacylated tRNA solution containing a lysidine-modified tRNA (mixed solution of Compound AAtR-6, Compound AAtR-28, and Compound AAtR-9; mixed solution of Compound AAtR-6, Compound AAtR-30, and Compound AAtR-9; and mixed solution of Compound AAtR-6, Compound AAtR-32, and Compound AAtR-9) to translationally synthesize peptide compounds.

The translation system used was PURE system, a prokaryote-derived reconstituted cell-free protein synthesis system. Specifically, the synthesis was carried out as follows: 1 µM template mRNA (SEQ ID NO: 123 (mR-4), SEQ ID NO: 124 (mR-5), or SEQ ID NO: 125 (mR-6)), a group of natural amino acids encoded in the respective template mRNAs at 0.25 mM respectively, and initiator aminoacylated tRNA (Compound AAtR-18) at 10 µM were added to a translation solution (1 mM GTP, 1 mM ATP, 20 mM phosphocreatine, 50 mM HEPES-KOH pH7.6, 100 mM potassium acetate, 10 mM magnesium acetate, 2 mM spermidine, 1 mM dithiothreitol, 1.5 mg/mL E. coli MRE600 (RNase-negative)-derived tRNA (Roche), 0.26 µM EF-G, 0.24 µM RF2, 0.17 µM RF3, 0.5 µM RRF, 4 µg/mL creatine kinase, 3 µg/mL myokinase, 2 unit/mL inorganic pyrophosphatase, 1.1 µg/mL nucleoside diphosphate kinase, 2.7 µM IF1, 0.4 µM IF2, 1.5 µM IF3, 40 µM EF-Tu, 49.3 µM EF-Ts, 1 µM EF-P-Lys, 0.4 unit/µL RNasein Ribonuclease inhibitor (Promega, N2111), 1.2 µM ribosome, 0.5 mM PGA, 0.09 µM GlyRS, 0.4 µM IleRS, 0.68 µM PheRS, 0.16 µM ProRS, and 0.09 µM ThrRS), and a mixed aminoacylated tRNA solution (mixed solution of Compound AAtR-6, Compound AAtR-27, and Compound AAtR-9; or mixed solution of Compound AAtR-6, Compound AAtR-28, and Compound AAtR-9) was added at 30 µM to the translation reaction mixture, and left at 37°C for one hour.

Similarly, 30 µM of a mixed solution of Compound AAtR-6, Compound AAtR-29, and Compound AAtR-9 or a mixed solution of Compound AAtR-6, Compound AAtR-30, and Compound AAtR-9, and 10 µM of Compound AAtR-9 were added to the above-described translation reaction mixture containing up to the initiator aminoacylated tRNA, and left at 37°C for one hour.

Similarly, 30 µM of a mixed solution of Compound AAtR-6, Compound AAtR-31, and Compound AAtR-9 or a mixed solution of Compound AAtR-6, Compound AAtR-32, and Compound AAtR-9, and 10 µM of Compound AAtR-9 were added to the above-described translation reaction mixture containing up to the initiator aminoacylated tRNA, and left at 37°C for one hour.

The template mRNA, the expected translated peptide compound, and the molecular weight (calculated value) of the peptide are shown in Table 8 below.

**[Table 8]**

| Aminoacylated tRNA | Template mRNA sequence | Expected translated peptide compound | m/z [M-H] | R. T. (method1) | R. T. (method2) |
|---|---|---|---|---|---|
| Compound A A t R-6 | mRNA - 4 | BdpF-TFIIGF-nBuG-IIPIG | 1722.9 | 3.1 | |
| Compound A A t R-9 | mRNA - 6 | BdpF-TFIIGF-dA-IIPIG | 1680.7 | 2.9 | |
| CompoundAA t R-27 | mRNA - 5 | BdpF-TFIIGF-MeHPh-IIPIG | 1784.9 | 3.1 | |
| CompoundAA t R-28 | mRNA - 5 | BdpF-TFIIGF-MeHPh-IIPIG | 1784.9 | 3.1 | |
| CompoundAA t R-29 | mRNA - 5 | BdpF-TFIIGF-F3Cl-IIPIG | 1791.9 | 3.4 | |
| CompoundAA t R-30 | mRNA - 5 | BdpF-TFIIGF-F3Cl-IIPIG | 1791.9 | 3.4 | |
| CompoundAA t R-31 | mRNA - 5 | BdpF-TFIIGF-SiPen-IIPIG | 1768.0 | 3.3 | |
| CompoundAA t R-32 | mRNA - 5 | BdpF-TFIIGF-SiPen-IIPIG | 1768.0 | 3.3 | |

The codon box of interest was further expanded, and experiments were performed to evaluate the effects of discrimination by lysidine-modified tRNAs.

Specifically, template mRNAs containing any one of three codons in the same codon box and having the same sequence for the rest of the sequences (template mRNAs of SEQ ID NO: 169 (mR-13), SEQ ID NO: 170 (mR-14), and SEQ ID NO: 171 (mR-15), or of SEQ ID NO: 172 (mR-16), SEQ ID NO: 173 (mR-17), and SEQ ID NO: 174 (mR-18)) were translated using a mixed aminoacylated tRNA solution containing a lysidine-modified tRNA and using a mixed aminoacylated tRNA solution not containing a lysidine-modified tRNA to translationally synthesize peptide compounds.

The translation system used was PURE system, a prokaryote-derived reconstituted cell-free protein synthesis system. Specifically, the synthesis was carried out as follows: 1 µM template mRNA (SEQ ID NO: 169 (mR-13), SEQ ID NO: 170 (mR-14), or SEQ ID NO: 171 (mR-15)), a group of natural amino acids encoded in the respective template mRNAs at 0.25 mM respectively, and initiator aminoacylated tRNA (Compound AAtR-18) at 10 µM were added to a translation solution (1 mM GTP, 1 mM ATP, 20 mM phosphocreatine, 50 mM HEPES-KOH pH7.6, 100 mM potassium acetate, 10 mM magnesium acetate, 2 mM spermidine, 1 mM dithiothreitol, 1.5 mg/mL E. coli MRE600 (RNase-negative)-derived tRNA (Roche), 0.26 µM EF-G, 0.24 µM RF2, 0.17 µM RF3, 0.5 µM RRF, 4 µg/mL creatine kinase, 3 µg/mL myokinase, 2 unit/mL inorganic pyrophosphatase, 1.1 µg/mL nucleoside diphosphate kinase, 2.7 µM IF1, 0.4 µM IF2, 1.5 µM IF3, 40 µM EF-Tu, 49.3 µM EF-Ts, 1 µM EF-P-Lys, 0.4 unit/µL RNasein Ribonuclease inhibitor (Promega, N2111), 1.2 µM ribosome, 0.5 mM PGA, 0.09 µM GlyRS, 0.4 µM IleRS, 0.68 µM PheRS, 0.16 µM ProRS, and 0.09 µM ThrRS), and a mixed aminoacylated tRNA solution (mixed solution of Compound AAtR-33 and Compound AAtR-35) was added at 30 µM and an aminoacylated tRNA (Compound AAtR-34) was added at 10 µM to the translation reaction mixture, and left at 37°C for one hour.

Similarly, for the other codon box, the translation system used was PURE system, a prokaryote-derived reconstituted cell-free protein synthesis system. Specifically, the synthesis was carried out as follows: 1 µM template mRNA (SEQ ID NO: 172 (mR-16), SEQ ID NO: 173 (mR-17), or SEQ ID NO: 174 (mR-18)), a group of natural amino acids encoded in the respective template mRNAs at 0.25 mM respectively, and initiator aminoacylated tRNA (Compound AAtR-18) at 10 µM were added to a translation solution (1 mM GTP, 1 mM ATP, 20 mM phosphocreatine, 50 mM HEPES-KOH pH7.6, 100 mM potassium acetate, 10 mM magnesium acetate, 2 mM spermidine, 1 mM dithiothreitol, 1.5 mg/mL E. coli MRE600 (RNase-negative)-derived tRNA (Roche), 0.26 µM EF-G, 0.24 µM RF2, 0.17 µM RF3, 0.5 µM RRF, 4 µg/mL creatine kinase, 3 µg/mL myokinase, 2 unit/mL inorganic pyrophosphatase, 1.1 µg/mL nucleoside diphosphate kinase, 2.7 µM IF1, 0.4 µM IF2, 1.5 µM IF3, 40 µM EF-Tu, 49.3 µM EF-Ts, 1 µM EF-P-Lys, 0.4 unit/µL RNasein Ribonuclease inhibitor (Promega, N2111), 1.2 µM ribosome, 0.5 mM PGA, 0.09 µM GlyRS, 0.4 µM I1eRS, 0.68 µM PheRS, 0.16 µM ProRS, 0.09 µM ThrRS, 0.02 µM ValRS, 2.73 µM AlaRS, 0.04 µM LeuRS, and 0.04 µM SerRS), and a mixed aminoacylated tRNA solution (mixed solution of Compound AAtR-36 and Compound AAtR-38) was added at 30 µM and an aminoacylated tRNA (Compound AAtR-37) was added at 10 µM to the translation reaction mixture, and left at 37°C for one hour.

The template mRNA, the expected translated peptide compound, and the molecular weight (calculated value) of the peptide are shown in Table 9 below.

**[Table 9]**

| Aminoacylated tRNA | Template mRNA sequence | Expected translated peptide compound | m/z [M-H] | R. T. (method1) | R. T. (method2) |
|---|---|---|---|---|---|
| CompoundAA t R-33 | mRNA - 13 | BdpF-TFIIGF-dA-IIPIG | 1680.9 | 2.9 | |
| CompoundAA t R-34 | mRNA - 14 | BdpF-TFIIGF-Pic2-IIPIG | 1721.9 | 3.0 | |
| CompoundAA t R-35 | mRNA - 15 | BdpF-TFIIGF-nBuG-IIPIG | 1724.0 | 3.1 | |
| CompoundAA t R-36 | mRNA - 16 | BdpF-TFLLGF-nBuG-LLPLG | 1724.0 | 2.9 | |
| CompoundAA t R-37 | mRNA - 17 | BdpF-TFLLGF-Pic2-LLPLG | 1721.9 | 3.2 | |
| CompoundAA t R-38 | mRNA - 18 | BdpF-TFLLGF-dA-LLPLG | 1681.9 | 3.0 | |

Next, to confirm the effect of agmatidine modification, experiments were performed to confirm the discrimination of three amino acids in a single codon box in the presence of three prepared aminoacylated tRNAs. Specifically, template mRNAs containing any one of three codons in the same codon box and having the same sequence for the rest of the sequences (template mRNAs of SEQ ID NO: 123 (mR-4), SEQ ID NO: 124 (mR-5), and SEQ ID NO: 125 (mR-6)) were reacted in a translation system to which an amino acylated tRNA (AAtR-39) or a an aminoacylated agmatidine-modified tRNA (AAtR-40) has been added to a mixed aminoacylated tRNA solution (a mixed solution of Compound AAtR-6 and Compound AAtR-9), to translationally synthesize peptide compounds.

The translation system used was PURE system, a prokaryote-derived reconstituted cell-free protein synthesis system. Specifically, the synthesis was carried out as follows: 1 µM template mRNA (SEQ ID NO: 123 (mR-4), SEQ ID NO: 124 (mR-5), or SEQ ID NO: 125 (mR-6)), a group of natural amino acids encoded in the respective template mRNAs at 0.25 mM respectively, and initiator aminoacylated tRNA (Compound AAtR-18) at 10 µM were added to a translation solution (1 mM GTP, 1 mM ATP, 20 mM phosphocreatine, 50 mM HEPES-KOH pH7.6, 100 mM potassium acetate, 10 mM magnesium acetate, 2 mM spermidine, 1 mM dithiothreitol, 1.5 mg/mL E. coli MRE600 (RNase-negative)-derived tRNA (Roche), 0.26 µM EF-G, 0.24 µM RF2, 0.17 µM RF3, 0.5 µM RRF, 4 µg/mL creatine kinase, 3 µg/mL myokinase, 2 unit/mL inorganic pyrophosphatase, 1.1 µg/mL nucleoside diphosphate kinase, 2.7 µM IF1, 0.4 µM IF2, 1.5 µM IF3, 40 µM EF-Tu, 49.3 µM EF-Ts, 1 µM EF-P-Lys, 0.4 unit/µL RNasein Ribonuclease inhibitor (Promega, N2111), 1.2 µM ribosome, 0.5 mM PGA, 0.09 µM GlyRS, 0.4 µM IleRS, 0.68 µM PheRS, 0.16 µM ProRS, and 0.09 µM ThrRS), and a mixed aminoacylated tRNA solution (mixed solution of Compound AAtR-6 and Compound AAtR-9) was added at 30 µM and an aminoacylated tRNA (Compound AAtR-39 or Compound AAtR-40) was added at 20 µM to the translation reaction mixture, and left at 37°C for one hour.

The template mRNA, the expected translated peptide compound, and the molecular weight (calculated value) of the peptide are shown in Table 10 below.

**[Table 10]**

| Aminoacylated tRNA | Template mRNA sequence | Expected translated peptide compound | m/z [M-H] | R. T. (method1) | R. T. (method2) |
|---|---|---|---|---|---|
| Compound AAtR-6 | mRNA - 4 | BdpF-TFIIGF-nBuG-IIPIG | 1724.0 | 3.1 | |
| Compound AAtR-39 | mRNA - 5 | BdpF-TFIIGF-SPh2Cl-IIPIG | 1807.9 | 3.2 | |
| CompoundAAtR-40 | mRNA - 5 | BdpF-TFIIGF-SPh2Cl-IIPIG | 1807.9 | 3.2 | |
| Compound AAtR-9 | mRNA - 6 | BdpF-TFIIGF-dA-IIPIG | 1680.9 | 2.9 | |

From the template DNAs (SEQ ID NO: 108 (D-14) to SEQ ID NO: 119 (D-25), and SEQ ID NO: 163 (D-36) to SEQ ID NO: 168 (D-41)), template mRNAs (SEQ ID NO: 120 (mr-1) to SEQ ID NO: 131 (mr-12), and SEQ ID NO: 169 (mr-13) to SEQ ID NO: 174 (mr-18)) were synthesized by in vitro transcription reaction using RiboMAX Large Scale RNA production System T7 (Promega, P1300), and then purified by RNeasy Mini kit (Qiagen).

Template DNA SEQ ID NO: 108 (D-14)
   DNA sequence: 
GGCGTAATACGACTCACTATAGGGTTAACTTTAAGAAGGAGATATACATATGACTTT
TATTATTGGTTTTTCTATTATTCCGATTGGTTAAGCTTCG
Template DNA SEQ ID NO: 109 (D-15)
   DNA sequence: 
GGCGTAATACGACTCACTATAGGGTTAACTTTAAGAAGGAGATATACATATGACTTT
TATTATTGGTTTTTCAATTATTCCGATTGGTTAAGCTTCG
Template DNA SEQ ID NO: 110 (D-16)
   DNA sequence: 
GGCGTAATACGACTCACTATAGGGTTAACTTTAAGAAGGAGATATACATATGACTTT
TATTATTGGTTTTTCGATTATTCCGATTGGTTAAGCTTCG
Template DNA SEQ ID NO: 111 (D-17)
   DNA sequence: 
GGCGTAATACGACTCACTATAGGGTTAACTTTAAGAAGGAGATATACATATGACTTT
TATTATTGGTTTTCTTATTATTCCGATTGGTTAAGCTTCG
Template DNA SEQ ID NO: 112 (D-18)
   DNA sequence: 
GGCGTAATACGACTCACTATAGGGTTAACTTTAAGAAGGAGATATACATATGACTTT
TATTATTGGTTTTCTAATTATTCCGATTGGTTAAGCTTCG
Template DNA SEQ ID NO: 113 (D-19)
   DNA sequence: 
GGCGTAATACGACTCACTATAGGGTTAACTTTAAGAAGGAGATATACATATGACTTT
TATTATTGGTTTTCTGATTATTCCGATTGGTTAAGCTTCG
Template DNA SEQ ID NO: 114 (D-20)
   DNA sequence: 
GGCGTAATACGACTCACTATAGGGTTAACTTTAAGAAGGAGATATACATATGACTTT
TATTATTGGTTTTGTTATTATTCCGATTGGTTAAGCTTCG
Template DNA SEQ ID NO: 115 (D-21)
   DNA sequence: 
GGCGTAATACGACTCACTATAGGGTTAACTTTAAGAAGGAGATATACATATGACTTT
TATTATTGGTTTTGTAATTATTCCGATTGGTTAAGCTTCG
Template DNA SEQ ID NO: 116 (D-22)
   DNA sequence: 
GGCGTAATACGACTCACTATAGGGTTAACTTTAAGAAGGAGATATACATATGACTTT
TATTATTGGTTTTGTGATTATTCCGATTGGTTAAGCTTCG
Template DNA SEQ ID NO: 117 (D-23)
   DNA sequence: 
GGCGTAATACGACTCACTATAGGGTTAACTTTAAGAAGGAGATATACATATGACTTT
TATTATTCTATTTGGTATTATTCCGATTCTATAAGCTTCG
Template DNA SEQ ID NO: 118 (D-24)
   DNA sequence: 
GGCGTAATACGACTCACTATAGGGTTAACTTTAAGAAGGAGATATACATATGACTTT
TATTATTCTATTTGGAATTATTCCGATTCTATAAGCTTCG
Template DNA SEQ ID NO: 119 (D-25)
   DNA sequence: 
GGCGTAATACGACTCACTATAGGGTTAACTTTAAGAAGGAGATATACATATGACTTT
TATTATTCTATTTGGGATTATTCCGATTCTATAAGCTTCG
Template DNA SEQ ID NO: 163 (D-36)
   DNA sequence: 
GGCGTAATACGACTCACTATAGGGTTAACTTTAAGAAGGAGATATACATATGACTTT
TATTATTGGTTTTCGTATTATTCCGATTGGTTAAGCTTCG
Template DNA SEQ ID NO: 164 (D-37)
   DNA sequence: 
GGCGTAATACGACTCACTATAGGGTTAACTTTAAGAAGGAGATATACATATGACTTT
TATTATTGGTTTTCGAATTATTCCGATTGGTTAAGCTTCG
Template DNA SEQ ID NO: 165 (D-38)
   DNA sequence: 
GGCGTAATACGACTCACTATAGGGTTAACTTTAAGAAGGAGATATACATATGACTTT
TATTATTGGTTTTCGGATTATTCCGATTGGTTAAGCTTCG
Template DNA SEQ ID NO: 166 (D-39)
   DNA sequence: 
GGCGTAATACGACTCACTATAGGGTTAACTTTAAGAAGGAGATATACATATGACTTT
TCTACTAGGTTTTATTCTACTACCGCTAGGTTAAGCTTCG
Template DNA SEQ ID NO: 167 (D-40)
   DNA sequence: 
GGCGTAATACGACTCACTATAGGGTTAACTTTAAGAAGGAGATATACATATGACTTT
TCTACTAGGTTTTATACTACTACCGCTAGGTTAAGCTTCG
Template DNA SEQ ID NO: 168 (D-41)
   DNA sequence: 
GGCGTAATACGACTCACTATAGGGTTAACTTTAAGAAGGAGATATACATATGACTTT
TCTACTAGGTTTTATGCTACTACCGCTAGGTTAAGCTTCG
Template mRNA SEQ ID NO: 120 (mR-1)
   RNA sequence: 
GGGUUAACUUUAAGAAGGAGAUAUACAUAUGACUUUUAUUAUUGGUUUUUCUAU
UAUUCCGAUUGGUUAAGCUUCG
Template mRNA SEQ ID NO: 121 (mR-2)
   RNA sequence: 
GGGUUAACUUUAAGAAGGAGAUAUACAUAUGACUUUUAUUAUUGGUUUUUCAAU
UAUUCCGAUUGGUUAAGCUUCG
Template mRNA SEQ ID NO: 122 (mR-3)
   RNA sequence: 
GGGUUAACUUUAAGAAGGAGAUAUACAUAUGACUUUUAUUAUUGGUUUUUCGAU
UAUUCCGAUUGGUUAAGCUUCG
Template mRNA SEQ ID NO: 123 (mR-4)
   RNA sequence: 
GGGUUAACUUUAAGAAGGAGAUAUACAUAUGACUUUUAUUAUUGGUUUUCUUAU
UAUUCCGAUUGGUUAAGCUUCG
Template mRNA SEQ ID NO: 124 (mR-5)
   RNA sequence: 
GGGUUAACUUUAAGAAGGAGAUAUACAUAUGACUUUUAUUAUUGGUUUUCUAAU
UAUUCCGAUUGGUUAAGCUUCG
Template mRNA SEQ ID NO: 125 (mR-6)
   RNA sequence: 
GGGUUAACUUUAAGAAGGAGAUAUACAUAUGACUUUUAUUAUUGGUUUUCUGAU
UAUUCCGAUUGGUUAAGCUUCG
Template mRNA SEQ ID NO: 126 (mR-7)
   RNA sequence: 
GGGUUAACUUUAAGAAGGAGAUAUACAUAUGACUUUUAUUAUUGGUUUUGUUAU
UAUUCCGAUUGGUUAAGCUUCG
Template mRNA SEQ ID NO: 127 (mR-8)
   RNA sequence: 
GGGUUAACUUUAAGAAGGAGAUAUACAUAUGACUUUUAUUAUUGGUUUUGUAAU
UAUUCCGAUUGGUUAAGCUUCG
Template mRNA SEQ ID NO: 128 (mR-9)
   RNA sequence: 
GGGUUAACUUUAAGAAGGAGAUAUACAUAUGACUUUUAUUAUUGGUUUUGUGAU
UAUUCCGAUUGGUUAAGCUUCG
Template mRNA SEQ ID NO: 129 (mR-10)
   RNA sequence: 
GGGUUAACUUUAAGAAGGAGAUAUACAUAUGACUUUUAUUAUUCUAUUUGGUAU
UAUUCCGAUUCUAUAAGCUUCG
Template mRNA SEQ ID NO: 130 (mR-11)
   RNA sequence: 
GGGUUAACUUUAAGAAGGAGAUAUACAUAUGACUUUUAUUAUUCUAUUUGGAAU
UAUUCCGAUUCUAUAAGCUUCG
Template mRNA SEQ ID NO: 131 (mR-12)
   RNA sequence: 
GGGUUAACUUUAAGAAGGAGAUAUACAUAUGACUUUUAUUAUUCUAUUUGGGAU
UAUUCCGAUUCUAUAAGCUUCG
Template mRNA SEQ ID NO: 169 (mR-13)
   RNA sequence: 
GGGUUAACUUUAAGAAGGAGAUAUACAUAUGACUUUUAUUAUUGGUUUUCGUAU
UAUUCCGAUUGGUUAAGCUUCG
Template mRNA SEQ ID NO: 170 (mR-14)
   RNA sequence: 
GGGUUAACUUUAAGAAGGAGAUAUACAUAUGACUUUUAUUAUUGGUUUUCGAAU
UAUUCCGAUUGGUUAAGCUUCG
Template mRNA SEQ ID NO: 171 (mR-15)
   RNA sequence: 
GGGUUAACUUUAAGAAGGAGAUAUACAUAUGACUUUUAUUAUUGGUUUUCGGAU
UAUUCCGAUUGGUUAAGCUUCG
Template mRNA SEQ ID NO: 172 (mR-16)
   RNA sequence: 
GGGUUAACUUUAAGAAGGAGAUAUACAUAUGACUUUUCUACUAGGUUUUAUUCU
ACUACCGCUAGGUUAAGCUUCG
Template mRNA SEQ ID NO: 173 (mR-17)
   RNA sequence: 
GGGUUAACUUUAAGAAGGAGAUAUACAUAUGACUUUUCUACUAGGUUUUAUACU
ACUACCGCUAGGUUAAGCUUCG
Template mRNA SEQ ID NO: 174 (mR-18)
   RNA sequence:

GGGUUAACUUUAAGAAGGAGAUAUACAUAUGACUUUUCUACUAGGUUUUAUGCU
ACUACCGCUAGGUUAAGCUUCG

### Example 13. Analysis of the translated peptides

The unnatural peptide translation solutions prepared in Example 12 were diluted tenfold, and then analyzed using a LC-FLR-MS system. The amount of translated peptide was evaluated from the analysis data by identifying the retention time of the target translated peptide from the MS data, and quantifying the fluorescence peak at the relevant retention time. In the quantitative evaluation, the LCT12 synthesized in Example 8 was used as a standard to prepare a calibration curve, and the content was calculated by relative quantification. The LC-MS was analyzed according to the conditions shown in Table 11 below by selecting the optimum conditions according to the sample of interest.

**[Table 11]**

| Analysis condition | System | Column | Mobile phase | Gradient (%B) | Flow rate (mL/ min) | Column tempe rature | Fluorometry wave length (Ex/Em) | MS mode |
|---|---|---|---|---|---|---|---|---|
| Method1 | Aquity UPLC-FLR -Xevo G2-XS Tof | waters BEH C18(2.1 x 50mm, ø 1.7um) | A=0.1%FA with H20 | 0-0. 2m in=10% | 0.5 | 40 | 491 nm/ 515 nm | ESI- |
| | | | | 0.2-3.6m in=98% | | | | |
| | | | B=0.1%FA with CH3CN | 3.6-4.0 | | | | |
| | | | | min=10% | | | | |
| Method2 | Aquity UPLC-FLR -Xevo G2-XS Tof | waters BEH C18(2.1 x 100mm, ø 1.7um) | A=0.1%FA with H20 | 0-0.4min=60% | 0.5 | 40 | 491 nm/ 515 nm | ESI- |
| | | | | 0.4-9.0min=98% | | | | |
| | | | B=0.1%FA with CH3CN | 9.0-10.0 | | | | |
| | | | | min=60% | | | | |

As a result of the evaluation, three amino acids were discriminated in a single codon box only under the translation conditions using the mixed aminoacylated tRNA solution containing a lysidine- or agmatidine-modified tRNA. The effect of discriminating lysidine modification was shown for multiple codon boxes (Figs. 11 to 14, Fig. 20, Fig. 21, Tables 12 to 15, Table 21, and Table 22). The effect of discriminating could also be confirmed when the nucleotide sequence of tRNA body was replaced with another sequence (Fig. 15, Fig. 16, Table 16, and Table 17). Also, similar effects could be confirmed when the amino acids linked to tRNA were replaced with others (Figs. 17 to 19 and Tables 18 to 20). It was confirmed that the agmatidine-modified tRNA could also yield the same discrimination effect as the lysidine-modified tRNA (Fig. 22 and Table 23).

**[Table 12]**

| Aminoacylated tRNA | Template mRNA seq. | Translated peptide compound | Amount of translation( µM) |
|---|---|---|---|
| Compound AAtR-1 | mR-1 | BdpFL-Phe-TFIIGF-dA-IIPIG | 0.71 |
| | | BdpFL-Phe-TFIIGF-SPh2Cl-IIPIG | 0.51 |
| | | BdpFL-Phe-TFIIGF-nBuG-IIPIG | 0.04 |
| | mR-2 | BdpFL-Phe-TFIIGF-dA-IIPIG | 0.08 |
| Compound AAtR-2 | | BdpFL-Phe-TFIIGF-SPh2Cl-IIPIG | 0.96 |
| Compound AAtR-5 | | BdpFL-Phe-TFIIGF-nBuG-IIPIG | 0.06 |
| | mR-3 | BdpFL-Phe-TFIIGF-dA-IIPIG | 0.09 |
| | | BdpFL-PheTFIIGF-SPh2Cl-IIPIG | 0.09 |
| | | BdpFL-Phe-TFIIGF-nBuG-IIPIG | 1.13 |
| Compound AAtR-1 | mR-1 | BdpFL-Phe-TFIIGF-dA-IIPIG | 1.19 |
| | | BdpFL-Phe-TFIIGF-SPh2Cl-IIPIG | 0.57 |
| | | BdpFL-Phe-TFIIGF-nBuG-IIPIG | 0.04 |
| | mR-2 | BdpFL-Phe-TFIIGF-dA-IIPIG | 0.03 |
| Compound AAtR-3 | | BdpFL-Phe-TFIIGF-SPh2Cl-IIPIG | 1.47 |
| Compound AAtR-5 | | BdpFL-Phe-TFIIGF-nBuG-IIPIG | 0.03 |
| | mR-3 | BdpFL-Phe-TFIIGF-dA-IIPIG | 0.05 |
| | | BdpFL-Phe-TFIIGF-SPh2Cl-IIPIG | 0.08 |
| | | BdpFL-Phe-TFIIGF-nBuG-IIPIG | 1.47 |
| Compound AAtR-1 | mR-1 | BdpFL-Phe-TFIIGF-dA-IIPIG | 0.77 |
| | | BdpFL-Phe-TFIIGF-SPh2Cl-IIPIG BdpFL-Phe-TFIIGF-SPh2Cl-IIPIG | 0.07 |
| | | BdpFL-Phe-TFIIGF-nBuG-IIPIG | 0.05 |
| | mR-2 | BdpFL-Phe-TFIIGF-dA-IIPIG | 0.08 |
| Compound AAtR-4 | | BdpFL-Phe-TFIIGF-SPh2Cl-IIPIG | 1.17 |
| Compound AAtR-5 | | BdpFL-Phe-TFIIGF-nBuG-IIPIG | 0.07 |
| | mR-3 | BdpFL-Phe-TFIIGF-dA-IIPIG | 0.08 |
| | | BdpFL-Phe-TFIIGF-SPh2Cl-IIPIG | 0.07 |
| | | BdpFL-Phe-TFIIGF-nBuG-IIPIG | 1.05 |

This is a table showing the results of evaluating the effects of the presence or absence of lysidine modification on translation that discriminates three amino acids in a single codon box (evaluated codons: UCU, UCA, and UCG).

**[Table 13]**

| Aminoacylated tRNA | Template mRNA seq. | Translated peptide compound | Amount of translation(uM) |
|---|---|---|---|
| | mR-4 | BdpFL-Phe-TFIIGF-nBuG-IIPIG | 1.22 |
| | | Bdp-FL-Phe-TFIIGF-Pic2-IIPIG | 0.41 |
| | | BdpFL-Phe-TFIIGF-dA-IIPIG | 0.00 |
| Compound AAtR-6 | mR-5 | BdpFL-Phe-TFIIGF-nBuG-IIPIG | 0.00 |
| Compound AAtR-7 | | Bdp-FL-Phe-TFIIGF-Pic2-IIPIG | 1.44 |
| Compound AAtR-9 | | BdpFL-Phe-TFIIGF-dA-IIPIG | 0.02 |
| | mR-6 | BdpFL-Phe-TFIIGF-nBuG-IIPIG | 0.04 |
| | | BdpFL-Phe-TFIIGF-Pic2-IIPIG | 0.40 |
| | | BdpFL-Phe-TFIIGF-dA-IIPIG | 1.44 |
| | mR-4 | BdpFL-Phe-TFIIGF-nBuG-IIPIG | 1.35 |
| | | Bdp-FL-Phe-TFIIGF-Pic2-IIPIG | 0.04 |
| | | BdpFL-Phe-TFIIGF-dA-IIPIG | 0.02 |
| Compound AAtR-6 | mR-5 | BdpFL-Phe-TFIIGF-nBuG-IIPIG | 0,00 |
| Compound AAtR-8 | | Bdp-FL-Phe-TFIIGF-Pic2-IIPIG | 1.39 |
| Compound AAtR-9 | | BdpFL -Phe- TFIIGF-dA-IIPIG | 0.03 |
| | mR-6 | BdpFL-Phe-TFIIGF-nBuG-IIPIG | 0.12 |
| | | Bdp-FL-Phe-TFIIGF-Pic2-IIPIG | 0.05 |
| | | BdpFL-Phe-TFIIGF-dA-IIPIG | 1.66 |

This is a table showing the results of evaluating the effects of the presence or absence of lysidine modification on translation that discriminates three amino acids in a single codon box (evaluated codons: CUU, CUA, and CUG).

**[Table 14]**

| Aminoacylated tRNA | Template mRNA seq. | Translated peptide compound | Amount of translation( wM) |
|---|---|---|---|
| | mR-7 | BdpFL-Phe-TFIIGF-nBuG-IIPIG | 0.76 |
| | | BdpFL-Phe-TFIIGF-Pic2-IIPIG | 0.26 |
| | | BdpFL-Phe-TFIIGF-dA-IIPIG | 0.01 |
| CompoundAAtR-10 | | BdpFL-Phe-TFIIGF-nBuG-IIPIG | 0.00 |
| CompoundAAtR-11 | mR-8 | BdpFL-Phe-TFIIGF-Pic2-IIPIG | 1.19 |
| CompoundAAtR-13 | | BdpFL-Phe-TFIIGF-dA-IIPIG | 0.01 |
| | mR-9 | BdpFL-Phe-TFIIGF-nBuG-IIPIG | 0.00 |
| | | BdpFL-Phe-TFIIGF-Pic2-IIPIG | 0.32 |
| | | BdpFL-Phe-TFIIGF-dA-IIPIG | 0.73 |
| | | BdpFL-Phe-TFIIGF-nBuG-IIPIG | 0.97 |
| | mR-7 | BdpFL-Phe-TFIIGF-Pic2-IIPIG | 0.09 |
| | | BdpFL-Phe-TFIIGF-dA-IIPIG | 0.01 |
| CompoundAAtR-10 | | BdpFL-Phe-TFIIGF-nBuG-IIPIG | 0.00 |
| CompoundAAtR-12 | mR-8 | BdpFL-Phe-TFIIGF-Pic2-IIPIG | 1.32 |
| CompoundAAtR-13 | | BdpFL-Phe-TFIIGF-dA-IIPIG | 0.02 |
| | | BdpFL-Phe-TFIIGF-nBuG-IIPIG | 0.04 |
| | mR-9 | BdpFL-Phe-TFIIGF-Pic2-IIPIG | 0.06 |
| | | BdpFL-Phe-TFIIGF-dA-IIPIG | 1.19 |

This is a table showing the results of evaluating the effects of the presence or absence of lysidine modification on translation that discriminates three amino acids in a single codon box (evaluated codons: GUU, GUA, and GUG).

**[Table 15]**

| Aminoacvlated tRNA | Template mRNA seq. | Translated peptide compound | Amount of translation(µM) |
|---|---|---|---|
| | mR-10 | BdpFL-Phe-TFIILF-dA-IIPIL | 1.10 |
| | | BdpFL-Phe-TFIILF-Pic2-IIPIL | 0.59 |
| | | BdpFL-Phe-TFIILF-MeHph-IIPIL | 0.02 |
| Compound AAtR-14 | mR-11 | BdpFL-Phe-TFIILF-dA-IIPIL | 0.03 |
| CompoundAAtR-15 | | BdpFL-Phe-TFnLF-Pic2-IIPIL | 2.63 |
| CompoundAAtR-17 | | BdpFL-Phe-TFIILF-MeHph-IIPIL | 0.03 |
| | mR-12 | BdpFL-Phe-TFIILF-dA-IIPIL | 0.01 |
| | | BdpFL-Phe-TFIILF-Pic2-IIPIL | 1.30 |
| | | BdpFL-Phe-TFIILF-MeHph-IIPIL | 1.32 |
| | mR-10 | BdpFL-Phe-TFIILF-dA-IIPIL | 1.16 |
| | | BdpFL-Phe-TFIILF-Pic2-IIPIL | 0.06 |
| | | BdpFL-Phe-TFIILF-MeHph-IIPIL | 0.05 |
| CompoundAAtR-14 | mR-11 | BdpFL-Phe-TFIILF-dA-IIPIL | 0.04 |
| CompoundAAtR-16 | | BdpFL-Phe-TFIILF-Pic2-IIPIL | 2.47 |
| CompoundAAtR-17 | | BdpFL-Phe-TFIILF-MeHph-IIPIL | 0.04 |
| | mR-12 | BdpFL-Phe-TFIILF-dA-IIPIL | 0.03 |
| | | BdpFL-Phe-TFIILF-Pic2-IIPIL | 0.03 |
| | | BdpFL-Phe-TFIILF-MeHph-IIPIL | 1.65 |

This is a table showing the results of evaluating the effects of the presence or absence of lysidine modification on translation that discriminates three amino acids in a single codon box (evaluated codons: GGU, GGA, and GGG).

**[Table 16]**

| Aminoacvlated tRNA | Template mRNA seq | Translated peptide compound | Amount of translation (µM) |
|---|---|---|---|
| | mR-4 | BdpFL-Phe-TFIIGF-nBuG-IIPIG | 0.14 |
| | | BdpFL-Phe-TFIIGF-SPh2Cl-IIPIG | 0.04 |
| | | BdpFL-Phe-TFIIGF-dA-IIPIG | 0.00 |
| Compound AAtR-19 | mR-5 | BdpFL-Phe-TFIIGF-nBuG-IIPIG | 0.00 |
| Compound AAtR-20 | | BdpFL-Phe-TFIIGF SPh2Cl-IIPIG | 0.16 |
| Compound AAtR-22 | | BdpFL-Phe-TFIIGF-dA-IIPIG | 0.02 |
| | mR-6 | BdpFL-Phe-TFIIGF-nBuG-IIPIG | 0.00 |
| | | BdpFL-Phe-TFIIGF-SPh2Cl-IIPIG | 0.00 |
| | | BdpFL-Phe-TFIIGF-dA-IIPIG | 0.13 |
| | mR-4 | BdpFL-Phe-TFIIGF-nBuG-IIPIG | 0.16 |
| | | BdpFL-Phe-TFIIGF-SPh2Cl-IIPIG | 0.00 |
| | | BdpFL-Phe-TFIIGF-dA-IIPIG | 0.00 |
| Compound AAtR-19 | mR-5 | BdpFL-Phe-TFIIGF-nBuG-IIPIG | 0.00 |
| Compound AAtR-21 | | BdpFL-Phe-TFIIGF-SPh2Cl-IIPIG | 0.39 |
| Compound AAtR-22 | | BdpFL-Phe-TFIIGF-dA-IIPIG | 0.02 |
| | mR-6 | BdpFL-Phe-TFIIGF-nBuG-IIPIG | 0.00 |
| | | BdpFL-Phe-TFIIGF-SPh2Cl-IIPIG | 0.00 |
| | | BdpFL-Phe-TFIIGF-dA-IIPIG | 0.15 |

This is a table showing the results of evaluating the effects of the presence or absence of lysidine modification on translation that discriminates three amino acids in a single codon box, using the Asp-tRNA body sequence (evaluated codons: CUU, CUA, and CUG).

**[Table 17]**

| Aminoacylated tRNA | Template mRNA seq. | Translated peptide compound | Amount of translation (µM) |
|---|---|---|---|
| | mR- | BdpFL-Phe-TFIIGF-nBuG-IIPIG | 0.18 |
| | | BdpFL-Phe-TFIIGF-SPh2Cl-IIPIG | 0.22 |
| | | BdpFL-Phe-TFIIGF-dA-IIPIG | 0.00 |
| Compound AAtR-23 | mR-5 | BdpFL-Phe-TFIIGF-nBuG-IIPIG | 0.00 |
| Compound AAtR-24 | | BdpFL-Phe-TFIIGF-SPh2Cl-IIPIG | 0.45 |
| Compound AAtR-26 | | BdpFL-Phe-TFIIGF-dA-IIPIG | 0.01 |
| | mR-6 | BdpFL-Phe-TFIIGF-nBuG-IIPIG | 0.00 |
| | | BdpFL-Phe-TFIIGF-SPh2Cl-IIPIG | 0.12 |
| | | BdpFL-Phe-TFIIGF-dA-IIPIG | 0.34 |
| | mR-4 | BdpFL-Phe-TFIIGF-nBuG-IIPIG | 0.26 |
| | | BdpFL-Phe-TFIIGF-SPh2Cl-IIPIG | 0.02 |
| | | BdpFL-Phe-TFIIGF-dA-IIPIG | 0.00 |
| Compound AAtR-23 | mR-5 | BdpFL-Phe-TFIIGF-nBuG-IIPIG | 0.00 |
| Compound AAtR-25 | | BdpFL-Phe-TFIIGF-SPh2Cl-IIPIG | 0.64 |
| Compound AAtR-26 | | BdpFL-Phe-TFIIGF-dA-IIPIG | 0.02 |
| | mR-6 | BdpFL-Phe-TFIIGF-nBuG-IIPIG | 0.00 |
| | | BdpFL-Phe-TFIIGF-SPh2Cl-IIPIG | 0.00 |
| | | BdpFL-Phe-TFIIGF-dA-IIPIG | 0.45 |

This is a table showing the results of evaluating the effects of the presence or absence of lysidine modification on translation that discriminates three amino acids in a single codon box, using the AsnE2-tRNA body sequence (evaluated codons: CUU, CUA, and CUG).

**[Table 18]**

| Aminoacylated tRNA | Template mRNA seq. | Translated peptide compound | Amount of translation(µM) |
|---|---|---|---|
| | mR-4 | BdpFL-Phe-TFIIGF-nBuG-IIPIG | 0.46 |
| | | BdpFL-Phe-TFIIGF-MeHph-IIPIG | 0.17 |
| | | BdpFL-Phe-TFIIGF-dA-IIPIG | 0.01 |
| Compound AAtR-6 | mR-5 | BdpFL-Phe-TFIIGF-nBuG-IIPIG | 0.01 |
| Compound AAtR-27 | | BdpFL-Phe-TFIIGF-MeHph-IIPIG | 1.12 |
| Compound AAtR-9 | | BdpFL-Phe-TFIIGF-dA-IIPIG | 0.06 |
| | mR-6 | BdpFL-Phe-TFIIGF-nBuG-IIPIG | 0.02 |
| | | BdpFL-Phe-TFIIGF-MeHph-IIPIG | 0.12 |
| | | BdpFL-Phe-TFIIGF-dA-IIPIG | 0.79 |
| | mR-4 | BdpFL-Phe-TFnGF-nBuG-IIPIG | 0.52 |
| | | BdpFL-Phe-TFIIGF-MeHph-IIPIG | 0.00 |
| | | BdpFL-Phe-TFIIGF-dA-IIPIG BdpFL-Phe-TFIIGF-dA-IIPIG | 0.01 |
| Compound AAtR-6 | mR-5 | BdpFL-Phe-TFIIGF-nBuG-IIPIG | 0.01 |
| Compound AAtR-28 | | BdpFL-Phe-TFIIGF-MeHph-IIPIG | 1.09 |
| Compound AAtR-9 | | BdpFL-Phe-TFIIGF-dA-IIPIG | 0.08 |
| | mR-6 | BdpFL-Phe-TFIIGF-nBuG-IIPIG | 0.04 |
| | | BdpFL-Phe-TFIIGF-MeHph-IIPIG | 0.01 |
| | | BdpFL-Phe-TFIIGF-dA-IIPIG | 0.92 |

This is a table showing the results of evaluating the effects of the presence or absence of lysidine modification on translation that discriminates three amino acids in a single codon box (evaluated codons: CUU, CUA, and CUG).

**[Table 19]**

| Aminoacylated tRNA | Template mRNA seq. | Translated peptide compound | Amount of translation(µM) |
|---|---|---|---|
| | mR-4 | BdpFL-Phe-TFIIGF-nBuG-IIPIG | 0.43 |
| | | BdpFL-Phe-TFIIGF-F3Cl-IIPIG | |
| | | BdpFL-Phe-TFIIGF-dA-IIPIG | 0.01 |
| Compound AAtR-6 | mR-5 | BdpFL-Phe-TFIIGF-nBuG-IIPIG | 0.01 |
| Compound AAtR-29 | | BdpFL-Phe-TFIIGF-F3Cl-IIPIG | 0.79 |
| Compound AAtR-9 | | BdpFL-Phe-TFIIGF-dA-IIPIG | 0.03 |
| | mR-6 | BdpFL-Phe-TFIIGF-nBuG-IIPIG | 0.04 |
| | | BdpFL-Phe-TFIIGF-F3Cl-IIPIG | 0.14 |
| | | BdpFL-Phe- TFIIGF-dA-IIPIG | 0.74 |
| | mR-4 | BdpFL-Phe-TFIIGF-nBuG-IIPIG | 0.49 |
| | | BdFL-Phe-TFIIGF-F3Cl-IIPIG | 0.02 |
| | | BdpFL-Phe- TFIIGF-dA-IIPIG | 0.02 |
| Compound AAtR-6 | mR-5 | BdFL-Phe-TFIIGF-nBuG-IIPIG | 0.01 |
| Compound AAtR-30 | | BdpFL-Phe-TFIIGF-F3Cl-IIPIG | 0.65 |
| Compound AAtR-9 | | BdpFL-Phe-TFIIGF-dA-IIPIG | 0.05 |
| | mR-6 | BdpFL-Phe-TFIIGF-nBuG-IIPIG | 0.05 |
| | | BdpFL-Phe-TFIIGF-F3Cl-IIPIG | 0.02 |
| | | BdpFL-Phe-TFIIGF-dA-IIPIG | 1.07 |

This is a table showing the results of evaluating the effects of the presence or absence of lysidine modification on translation that discriminates three amino acids in a single codon box (evaluated codons: CUU, CUA, and CUG).

**[Table 20]**

| Aminoacylated tRNA | Template mRNA seq. | Translated peptide compound | Amount of translation(µM) |
|---|---|---|---|
| | mR-4 | BdFL-Phe-TFIIGF-nBuG-IIPIG | 0.35 |
| | | BdpFL-Phe-TFIIGF-SiPen-IIPIG | 0.12 |
| | | BdpFL-Phe-TFIIGF-dA-IIPIG | 0.00 |
| Compound AAtR-6 | mR-5 | BdpFL-Phe-TFIIGF-nBuG-IIPIG | 0.01 |
| Compound AAtR-31 | | BdpFL-Phe-TFIIGF-SiPen-IIPIG | 0.62 |
| Compound AAtR-9 | | BdpFL-Phe-TFIIGF-dA-IIPIG | 0.03 |
| | mR-6 | BdFL-Phe-TFIIGF-nBuG-IIPIG | 0.05 |
| | | BdpFL-Phe-TFIIGF-SiPen-IIPIG | 0.16 |
| | | BdpFL-Phe-TFIIGF-dA-IIPIG | 0.49 |
| | mR-4 | BdpFL-Phe-TFIIGF-nBuG-IIPIG | 0.41 |
| | | BdpFL-Phe-TFIIGF-SiPen-IIPIG | 0.00 |
| | | BdpFL-Phe-TFIIGF-dA-IIPIG | 0.01 |
| Compound AAtR-6 | mR-5 | BdFL-Phe-TFIIGF-nBuG-IIPIG | 0.01 |
| CompoundAAtR-32 | | BdpFL-Phe-TFIIGF-SiPen-IIPIG | 0.64 |
| Compound AAtR-9 | | BdpFL-Phe-TFIIGF-dA-IIPIG | 0.04 |
| | mR-6 | BdpFL-Phe-TFIIGF-nBuG-IIPIG | 0.06 |
| | | BdpFL-Phe-TFIIGF-SiPen-IIPIG- | 0.03 |
| | | BdpFL-Phe-TFIIGF-dA-IIPIG | 0.89 |

This is a table showing the results of evaluating the effects of the presence or absence of lysidine modification on translation that discriminates three amino acids in a single codon box (evaluated codons: CUU, CUA, and CUG).

**[Table 21]**

| Aminoacylated tRNA | Template mRNA seq. | Translated peptide compound | Amount of translation(µM) |
|---|---|---|---|
| | mR-13 | BdpFL-Phe-TFIILF-dA-IIPIL | 0.53 |
| | | BdpFL-Phe-TFIILF-Pic2-IIPIL | 0.01 |
| | | BdpFL-Phe-TFIILF-nBuG-IIPIL | 0.01 |
| Compound AAtR-33 | mR-14 | BdpFL-Phe-TFIILF-dA-IIPIL | 0.04 |
| Compound AAtR-34 | | BdpFL-Phe-TFIILF-Pic2-IIPIL | 0.87 |
| Compound AAtR-35 | | BdpFL-Phe-TFIILF-nBuG-IIPIL | 0.00 |
| | mR-15 | BdpFL-Phe-TFIILF-dA-IIPIL | 0.00 |
| | | BdpFL-Phe-TFIILF-Pic2-IIPIL | 0.01 |
| | | BdpFL-Phe-TFIILF-nBuG-IIPIL | 1.03 |

This is a table showing the results of evaluating the effects of lysidine modification on translation that discriminates three amino acids in a single codon box (evaluated codons: CGU, CGA, and CGG).

**[Table 22]**

| Aminoacylated tRNA | Template mRNA seq. | Translated peptide compound | Amount of translation(µM) |
|---|---|---|---|
| | mR-16 | BdpFL-Phe-TFIILF-nBuG-IIPIL | 0.37 |
| | | BdpFL-Phe-TFIILF-Pic2-IIPIL | 0_{.}03 |
| | | BdpFL-Phe-TFIILF-dA-IIPIL | 0.01 |
| Compound AAtR-36 | mR-17 | BdpFL-Phe-TFIILF-nBuG-IIPIL | 0.00 |
| Compound AAtR-37 | | BdpFL-Phe-TFIILF-Pic2-IIPIL | 0.57 |
| Compound AAtR-38 | | BdpFL-Phe-TFIILF-dA-IIPIL | 0.00 |
| | mR-18 | BdpFL-Phe-TFIILF-nBuG-IIPIL | 0.04 |
| | | BdpFL-Phe-TFIILF-Pic2-IIPIL | 0.00 |
| | | BdpFL-Phe-TFIILF-dA-IIPIL | 1.38 |

This is a table showing the results of evaluating the effects of lysidine modification on translation that discriminates three amino acids in a single codon box (evaluated codons: AUU, AUA, and AUG).

**[Table 23]**

| Aminoacylated tRNA | Template mRNA seq. | Translated peptide compound | Amount of translation(µM) |
|---|---|---|---|
| | mR-4 | BdpFL-Phe-TFIIGF-nBuG-IIPIG | 0.83 |
| | | BdpFL-Phe-TFIIGF-SPh2Cl-IIPIG | 0.78 |
| | | BdpFL-Phe-FIIGF-dA-IIPIG | 0.00 |
| Compound AAtR-6 | mR-5 | BdpFL-Phe-TFIIGF-nBuG-IIPIG | 0.01 |
| Compound AAtR-39 | | BdpFL-Phe-TFIIGF-SPh2Cl-IIPIG | 2,02 |
| Compound AAtR-9 | | BdpFL-Phe-TFIIGF-dA-IIPIG | 0.02 |
| | mR-6 | BdpFL-Phe-TFIIGF-nBuG-IIPIG | 0.06 |
| | | BdpFL-Phe-TFIIGF-SPh2Cl-IIPIG | 0.49 |
| | | BdpFL-Phe-TFIIGF-dA-IIPIG | 1.62 |
| | mR-4 | BdpFL-Phe-TFnGF-nBuG-nPIG | 0.82 |
| | | BdpFL-Phe-TFIIGF-SPh2Cl-IIPIG | 0.19 |
| | | BdpFL-Phe-TFIIGF-dA-IIPIG | 0.03 |
| Compound AAtR-6 | mR-5 | BdpFL-Phe-TFIIGF-nBuG-IIPIG | 0.04 |
| Compound AAtR-40 | | BdpFL-Phe-TFIIGF-SPh2Cl-IIPIG | 1.37 |
| Compound AAtR-9 | | BdpFL-Phe-TFIIGF-dA-IIPIG | 0.34 |
| | mR - | BdpFL-Phe-TFIIGF-nBuG-IIPIG | 0.07 |
| | | BdpFL-Phe-TFIIGF-SPh2Cl-IIPIG | 0.11 |
| | | BdpFL-Phe-TFIIGF-dA-IIPIG | 1.83 |

This is a table showing the results of evaluating the effects of the presence or absence of agmatidine modification on translation that discriminates three amino acids in a single codon box (evaluated codons: CUU, CUA, and CUG).

Although the above-described invention has been described in detail using examples and illustrations for the purpose of facilitating a clear understanding, the descriptions and illustrations herein should not be construed as limiting the scope of the present invention. The disclosures of all patent literature and scientific literature cited herein are hereby expressly incorporated by reference in their entirety.

### [Industrial Applicability]

In one embodiment, the mutated tRNAs of the present disclosure are useful in that they can discriminate the NNA codon and the NNG codon, which are not discriminated according to the natural genetic code table. In one embodiment, the translation systems of the present disclosure are useful in that they can translate more kinds of amino acids (codon expansion) than translation systems that use the natural genetic code table.

## Claims

1. A mutated tRNA produced by engineering a tRNA, wherein the engineering comprises a engineering such that, in its anticodon represented by N₁N₂N₃, the first letter nucleoside N₁ after the engineering is any one of lysidine (k2C), a lysidine derivative, agmatidine (agm2C), and an agmatidine derivative, wherein N₂ and N₃ are arbitrary nucleosides for the second letter and the third letter of the anticodon, respectively.

2. The mutated tRNA of claim 1, wherein N₁ prior to the engineering is cytidine (C), and the engineering from this cytidine (C) to lysidine (k2C) cannot be catalyzed by a lysidine synthetase (tRNA^{Ile}-lysidine synthetase; TilS) having the amino acid sequence of SEQ ID NO: 51.

3. The mutated tRNA of claim 1, wherein N₁ prior to the engineering is cytidine (C), and the engineering from this cytidine (C) to agmatidine (agm2C) cannot be catalyzed by an agmatidine synthetase (tRNA^{Ile}-agmatidine synthetase; TiaS) having the amino acid sequence of SEQ ID NO: 52.

4. The mutated tRNA of any one of claims 1 to 3, comprising an anticodon complementary to a codon represented by M₁M₂A (wherein M₁ and M₂ represent nucleosides for the first and second letters of the codon respectively; each of M₁ and M₂ is selected from any of adenosine (A), guanosine (G), cytidine (C), and uridine (U); and the nucleoside of the third letter is adenosine).

5. The mutated tRNA of claim 4, wherein the anticodon is represented by k2CN₂N₃ or agm2CN₂N₃ (wherein the nucleoside of the first letter of the anticodon is lysidine (k2C) or agmatidine (agm2C), and the nucleoside of the second letter (N₂) and the nucleoside of the third letter (N₃) are complementary to M₂ and M₁, respectively).

6. The mutated tRNA of claim 4 or 5, wherein M₁ and M₂ are selected from codons that constitute a codon box in which a codon with the third letter nucleoside being A and a codon with the third letter nucleoside being G both encode the same amino acid in the natural genetic code table.

7. The mutated tRNA of claim 4 or 5, wherein M₁ and M₂ are selected from codons that constitute a codon box in which a codon with the third letter nucleoside being U, a codon with the third letter nucleoside being C, a codon with the third letter nucleoside being A, and a codon with the third letter nucleoside being G all encode the same amino acid in the natural genetic code table.

8. The mutated tRNA of claim 4 or 5, wherein M₁ and M₂ are selected from the group consisting of the following:
(i) M₁ is uridine (U) and M₂ is cytidine (C);
(ii) M₁ is cytidine (C) and M₂ is uridine (U);
(iii) M₁ is cytidine (C) and M₂ is cytidine (C);
(iv) M₁ is cytidine (C) and M₂ is guanosine (G);
(v) M₁ is adenosine (A) and M₂ is uridine (U);
(vi) M₁ is guanosine (G) and M₂ is uridine (U);
(vii) M₁ is guanosine (G) and M₂ is cytidine (C); and
(viii) M₁ is guanosine (G) and M₂ is guanosine (G).

9. The mutated tRNA of any one of claims 1 to 8, wherein an amino acid or an amino acid analog is attached to the 3' end.

10. A translation system comprising a plurality of different tRNAs, wherein the system comprises the mutated tRNA of any one of claims 1 to 9.

11. The translation system of claim 10, comprising (a) the mutated tRNA of any one of claims 1 to 9, and (b) a tRNA comprising an anticodon complementary to a codon represented by M₁M₂G.

12. The translation system of claim 10 or 11, further comprising (c) a tRNA comprising an anticodon complementary to a codon represented by M₁M₂U or M₁M₂C.

13. The translation system of claim 12, wherein the amino acids or the amino acid analogs attached to the tRNAs of claims 11(a), 11(b), and 12(c) are different from one another.

14. A method for producing a peptide, comprising translating a nucleic acid using the translation system of any one of claims 10 to 13.

15. A nucleic acid-peptide complex comprising a peptide and a nucleic acid encoding the peptide, wherein the nucleic acid encoding the peptide comprises the three codons of either (A) or (B) below:
(A) M₁M₂U, M₁M₂A, and M₁M₂G;
(B) M₁M₂C, M₁M₂A, and M₁M₂G;
and wherein the amino acids corresponding to the three codons are all different on the peptide.


SEQUENCE LISTING

<110>  CHUGAI SEIYAKU KABUSHIKI KAISHA

<120>  A mutated tRNA for codon expansion

<130>  C1-A1833P

<150>  JP 2018-243478
<151>  2018-12-26

<160>  203

<170>  PatentIn version 3.5

<210>  1
<211>  76
<212>  RNA
<213>  Escherichia coli

<400>  1
ggggcuauag cucagcuggg agagcgccug cuuugcacgc aggaggucug cgguucgauc      60

ccgcauagcu ccacca                                                      76


<210>  2
<211>  76
<212>  RNA
<213>  Escherichia coli

<400>  2
ggggcuauag cucagcuggg agagcgcuug cauggcaugc aagaggucag cgguucgauc      60

ccgcuuagcu ccacca                                                      76


<210>  3
<211>  77
<212>  RNA
<213>  Escherichia coli

<400>  3
gcauccguag cucagcugga uagaguacuc ggcuacgaac cgagcggucg gagguucgaa      60

uccucccgga ugcacca                                                     77


<210>  4
<211>  77
<212>  RNA
<213>  Escherichia coli

<400>  4
gcgcccguag cucagcugga uagagcgcug cccuccggag gcagaggucu cagguucgaa      60

uccugucggg cgcgcca                                                     77


<210>  5
<211>  77
<212>  RNA
<213>  Escherichia coli

<400>  5
gcgcccuuag cucaguugga uagagcaacg accuucuaag ucgugggccg cagguucgaa      60

uccugcaggg cgcgcca                                                    77


<210>  6
<211>  75
<212>  RNA
<213>  Escherichia coli

<400>  6
guccucuuag uuaaauggau auaacgagcc ccuccuaagg gcuaauugca gguucgauuc      60

cugcagggga cacca                                                      75


<210>  7
<211>  76
<212>  RNA
<213>  Escherichia coli

<400>  7
uccucuguag uucagucggu agaacggcgg acuguuaauc cguaugucac ugguucgagu      60

ccagucagag gagcca                                                     76


<210>  8
<211>  77
<212>  RNA
<213>  Escherichia coli

<400>  8
ggagcgguag uucagucggu uagaauaccu gccugucacg cagggggucg cggguucgag      60

ucccguccgu uccgcca                                                    77


<210>  9
<211>  74
<212>  RNA
<213>  Escherichia coli

<400>  9
ggcgcguuaa caaagcgguu auguagcgga uugcaaaucc gucuaguccg guucgacucc      60

ggaacgcgcc ucca                                                       74


<210>  10
<211>  77
<212>  RNA
<213>  Escherichia coli

<400>  10
cgcggggugg agcagccugg uagcucgucg ggcucauaac ccgaaggucg ucgguucaaa      60

uccggccccc gcaacca                                                    77


<210>  11

<211>  77
<212>  RNA
<213>  Escherichia coli

<400>  11
cgcggggugg agcagccugg uagcucgucg ggcucauaac ccgaagaucg ucgguucaaa      60

uccggccccc gcaacca                                                     77


<210>  12
<211>  75
<212>  RNA
<213>  Escherichia coli

<400>  12
   ugggguaucg ccaagcggua aggcaccggu uuuugauacc ggcauucccu gguucgaauc      60
   cagguacccc agcca                                                       75


<210>  13
<211>  75
<212>  RNA
<213>  Escherichia coli

<400>  13
   ugggguaucg ccaagcggua aggcaccgga uucugauucc ggcauuccga gguucgaauc      60
   cucguacccc agcca                                                       75


<210>  14
<211>  76
<212>  RNA
<213>  Escherichia coli

<400>  14
guccccuucg ucuagaggcc caggacaccg cccuuucacg gcgguaacag ggguucgaau      60

ccccuagggg acgcca                                                      76


<210>  15
<211>  74
<212>  RNA
<213>  Escherichia coli

<400>  15
gcgggcguag uucaauggua gaacgagagc uucccaagcu cuauacgagg guucgauucc      60

cuucgcccgc ucca                                                        74


<210>  16
<211>  75
<212>  RNA
<213>  Escherichia coli

<400>  16
gcgggcaucg uauaauggcu auuaccucag ccuuccaagc ugaugaugcg gguucgauuc      60

ccgcugcccg cucca                                                       75

<210>  17
<211>  76
<212>  RNA
<213>  Escherichia coli

<400>  17
gcgggaauag cucaguuggu agagcacgac cuugccaagg ucggggucgc gaguucgagu      60

cucguuuccc gcucca                                                      76


<210>  18
<211>  76
<212>  RNA
<213>  Escherichia coli

<400>  18
guggcuauag cucaguuggu agagcccugg auugugauuc caguugucgu ggguucgaau      60

cccauuagcc acccca                                                      76


<210>  19
<211>  77
<212>  RNA
<213>  Escherichia coli

<400>  19
aggcuuguag cucagguggu uagagcgcac cccugauaag ggugaggucg gugguucaag      60

uccacucagg ccuacca                                                     77


<210>  20
<211>  76
<212>  RNA
<213>  Escherichia coli

<400>  20
ggccccuuag cucagugguu agagcaggcg acucauaauc gcuuggucgc ugguucaagu      60

ccagcagggg ccacca                                                      76


<210>  21
<211>  87
<212>  RNA
<213>  Escherichia coli

<400>  21
   gcgaaggugg cggaauuggu agacgcgcua gcuucaggug uuaguguucu uacggacgug      60
   gggguucaag uccccccccu cgcacca                                           87


<210>  22
<211>  87
<212>  RNA
<213>  Escherichia coli

<400>  22

gccgaggugg uggaauuggu agacacgcua ccuugaggug guagugccca auagggcuua 60

cggguucaag ucccguccuc gguacca 87


<210> 23
<211> 85
<212> RNA
<213> Escherichia coli

<400> 23
gcgggagugg cgaaauuggu agacgcacca gauuuagguu cuggcgccgc aaggugugcg 60

aguucaaguc ucgccucccg cacca 85


<210> 24
<211> 87
<212> RNA
<213> Escherichia coli

<400> 24
gcccggaugg uggaaucggu agacacaagg gauuuaaaau cccucggcgu ucgcgcugug 60

cggguucaag ucccgcuccg gguacca 87


<210> 25
<211> 85
<212> RNA
<213> Escherichia coli

<400> 25
gccgaagugg cgaaaucggu agacgcaguu gauucaaaau caaccguaga aauacgugcc 60

gguucgaguc cggccuucgg cacca 85


<210> 26
<211> 76
<212> RNA
<213> Escherichia coli

<400> 26
gggucguuag cucaguuggu agagcaguug acuuuuaauc aauuggucgc agguucgaau 60

ccugcacgac ccacca 76


<210> 27
<211> 77
<212> RNA
<213> Escherichia coli

<400> 27
ggcuacguag cucaguuggu uagagcacau cacucauaau gaugggguca cagguucgaa 60

ucccgucgua gccacca 77


<210> 28
<211> 76
<212> RNA

<213>  Escherichia coli

<400>  28
gcccggauag cucagucggu agagcagggg auugaaaauc cccguguccu ugguucgauu      60

ccgaguccgg gcacca                                                      76


<210>  29
<211>  77
<212>  RNA
<213>  Escherichia coli

<400>  29
cggugauugg cgcagccugg uagcgcacuu cguucgggac gaaggggucg gagguucgaa      60

uccucuauca ccgacca                                                     77


<210>  30
<211>  77
<212>  RNA
<213>  Escherichia coli

<400>  30
cggcacguag cgcagccugg uagcgcaccg ucauggggug ucgggggucg gagguucaaa      60

uccucucgug ccgacca                                                     77


<210>  31
<211>  77
<212>  RNA
<213>  Escherichia coli

<400>  31
cggcgaguag cgcagcuugg uagcgcaacu gguuugggac cagugggucg gagguucgaa      60

uccucucucg ccgacca                                                     77


<210>  32
<211>  95
<212>  RNA
<213>  Escherichia coli

<400>  32
ggaagaucgu cgucuccggu gaggcggcug gacuucaaau ccaguugggg ccgccagcgg      60

ucccgggcag guucgacucc ugugaucuuc cgcca                                 95


<210>  33
<211>  88
<212>  RNA
<213>  Escherichia coli

<400>  33
ggaagugugg ccgagcgguu gaaggcaccg gucuugaaaa ccggcgaccc gaaaggguuc      60

cagaguucga aucucugcgc uuccgcca                                         88

<210>  34
<211>  90
<212>  RNA
<213>  Escherichia coli

<400>  34
ggagagaugc cggagcggcu gaacggaccg gucucgaaaa ccggaguagg ggcaacucua      60

ccggggguuc aaaucccccu cucuccgcca                                        90


<210>  35
<211>  93
<212>  RNA
<213>  Escherichia coli

<400>  35
ggugaggugg ccgagaggcu gaaggcgcuc cccugcuaag ggaguaugcg gucaaaagcu      60

gcauccgggg uucgaauccc cgccucaccg cca                                   93


<210>  36
<211>  88
<212>  RNA
<213>  Escherichia coli

<400>  36
ggugaggugu ccgaguggcu gaaggagcac gccuggaaag uguguauacg gcaacguauc      60

gggguucga aucccccccu caccgcca                                          88


<210>  37
<211>  76
<212>  RNA
<213>  Escherichia coli

<400>  37
gcugauaugg cucaguuggu agagcgcacc cuugguaagg gugagguccc caguucgacu      60

cuggguauca gcacca                                                      76


<210>  38
<211>  76
<212>  RNA
<213>  Escherichia coli

<400>  38
gccgauauag cucaguuggu agagcagcgc auucguaaug cgaaggucgu agguucgacu      60

ccuauuaucg gcacca                                                      76


<210>  39
<211>  76
<212>  RNA
<213>  Escherichia coli

<400>  39
gcugauauag cucaguuggu agagcgcacc cuugguaagg gugaggucgg caguucgaau      60

cugccuauca gcacca                                                    76


<210>  40
<211>  76
<212>  RNA
<213>  Escherichia coli

<400>  40
gccgacuuag cucaguaggu agagcaacug acuuguaauc aguaggucac caguucgauu      60

ccgguagucg gcacca                                                    76


<210>  41
<211>  76
<212>  RNA
<213>  Escherichia coli

<400>  41
aggggcguag uucaauuggu agagcaccgg ucuccaaaac cggguguugg gaguucgagu      60

cucuccgccc cugcca                                                    76


<210>  42
<211>  85
<212>  RNA
<213>  Escherichia coli

<400>  42
   ggugggguuc ccgagcggcc aaagggagca gacuguaaau cugccgucau cgacuucgaa      60
   gguucgaauc cuucccccac cacca                                          85


<210>  43
<211>  85
<212>  RNA
<213>  Escherichia coli

<400>  43
   ggugggguuc ccgagcggcc aaagggagca gacuguaaau cugccgucac agacuucgaa      60
   gguucgaauc cuucccccac cacca                                          85


<210>  44
<211>  76
<212>  RNA
<213>  Escherichia coli

<400>  44
gggugauuag cucagcuggg agagcaccuc ccuuacaagg agggggucgg cgguucgauc      60

ccgucaucac ccacca                                                    76


<210>  45
<211>  77
<212>  RNA
<213>  Escherichia coli

<400>  45
gcguccguag cucaguuggu uagagcacca ccuugacaug gugggggucg gugguucgag      60

uccacucgga cgcacca                                                     77


<210>  46
<211>  77
<212>  RNA
<213>  Escherichia coli

<400>  46
gcguucauag cucaguuggu uagagcacca ccuugacaug gugggggucg uugguucgag      60

uccaauugaa cgcacca                                                     77


<210>  47
<211>  72
<212>  RNA
<213>  Desulfitobacterium hafniense

<400>  47
   ggggggugga ucgaauagau cacacggacu cuaaauucgu gcaggcgggu gaaacucccg      60
   uacuccccgc ca                                                          72


<210>  48
<211>  72
<212>  RNA
<213>  Methanosarcina mazei

<400>  48
ggaaaccuga ucauguagau cgaauggacu cuaaauccgu ucagccgggu uagauucccg      60

ggguuuccgc ca                                                          72


<210>  49
<211>  76
<212>  RNA
<213>  Escherichia coli


<220>
<221>  misc_feature
<222>  (32)..(38)
<223>  n is a, c, g, or u

<400>  49
ggcucuguag uucagucggu agaacggcgg annnnnnnuc cguaugucac ugguucgagu      60

ccagucagag ccgcca                                                      76


<210>  50
<211>  77
<212>  RNA
<213>  Escherichia coli


<220>

<221>  misc_feature
<222>  (33)..(39)
<223>  n is a, c, g, or u

<400>  50
gggugauugg cgcagccugg uagcgcacuu cgnnnnnnnc gaagggguca gggguucgaa      60

uccccuauca cccgcca                                                     77


<210>  51
<211>  432
<212>  PRT
<213>  Escherichia coli

<400>  51

Met Thr Leu Thr Leu Asn Arg Gln Leu Leu Thr Ser Arg Gln Ile Leu
1               5                   10                  15

Val Ala Phe Ser Gly Gly Leu Asp Ser Thr Val Leu Leu His Gln Leu
            20                  25                  30

Val Gln Trp Arg Thr Glu Asn Pro Gly Val Ala Leu Arg Ala Ile His
        35                  40                  45

Val His His Gly Leu Ser Ala Asn Ala Asp Ala Trp Val Thr His Cys
    50                  55                  60

Glu Asn Val Cys Gln Gln Trp Gln Val Pro Leu Val Val Glu Arg Val
65                  70                  75                  80

Gln Leu Ala Gln Glu Gly Leu Gly Ile Glu Ala Gln Ala Arg Gln Ala
                85                  90                  95

Arg Tyr Gln Ala Phe Ala Arg Thr Leu Leu Pro Gly Glu Val Leu Val
            100                 105                 110

Thr Ala Gln His Leu Asp Asp Gln Cys Glu Thr Phe Leu Leu Ala Leu
        115                 120                 125

Lys Arg Gly Ser Gly Pro Ala Gly Leu Ser Ala Met Ala Glu Val Ser
    130                 135                 140

Glu Phe Ala Gly Thr Arg Leu Ile Arg Pro Leu Leu Ala Arg Thr Arg
145                 150                 155                 160

Gly Glu Leu Val Gln Trp Ala Arg Gln Tyr Asp Leu Arg Trp Ile Glu
                165                 170                 175

Asp Glu Ser Asn Gln Asp Asp Ser Tyr Asp Arg Asn Phe Leu Arg Leu
            180                 185                 190

Arg Val Val Pro Leu Leu Gln Gln Arg Trp Pro His Phe Ala Glu Ala
        195                 200                 205

Thr Ala Arg Ser Ala Ala Leu Cys Ala Glu Gln Glu Ser Leu Leu Asp
    210                 215                 220

Glu Leu Leu Ala Asp Asp Leu Ala His Cys Gln Ser Pro Gln Gly Thr
225                 230                 235                 240

Leu Gln Ile Val Pro Met Leu Ala Met Ser Asp Ala Arg Arg Ala Ala
                245                 250                 255

Ile Ile Arg Arg Trp Leu Ala Gly Gln Asn Ala Pro Met Pro Ser Arg
            260                 265                 270

Asp Ala Leu Val Arg Ile Trp Gln Glu Val Ala Leu Ala Arg Glu Asp
        275                 280                 285

Ala Ser Pro Cys Leu Arg Leu Gly Ala Phe Glu Ile Arg Arg Tyr Gln
    290                 295                 300

Ser Gln Leu Trp Trp Ile Lys Ser Val Thr Gly Gln Ser Glu Asn Ile
305                 310                 315                 320

Val Pro Trp Gln Thr Trp Leu Gln Pro Leu Glu Leu Pro Ala Gly Leu
                325                 330                 335

Gly Ser Val Gln Leu Asn Ala Gly Gly Asp Ile Arg Pro Pro Arg Ala
            340                 345                 350

Asp Glu Ala Val Ser Val Arg Phe Lys Ala Pro Gly Leu Leu His Ile
        355                 360                 365

Val Gly Arg Asn Gly Gly Arg Lys Leu Lys Lys Ile Trp Gln Glu Leu
    370                 375                 380

Gly Val Pro Pro Trp Leu Arg Asp Thr Thr Pro Leu Leu Phe Tyr Gly
385                 390                 395                 400

Glu Thr Leu Ile Ala Ala Ala Gly Val Phe Val Thr Gln Glu Gly Val
                405                 410                 415

Ala Glu Gly Glu Asn Gly Val Ser Phe Val Trp Gln Lys Thr Leu Ser
            420                 425                 430

<210> 52

<211>  428
<212>  PRT
<213>  Methanosarcina acetivorans

<400>  52

Met Ile Ile Gly Ile Asp Asp Thr Asp Ser Asn Glu Gly Met Cys Thr
1               5                   10                  15

Thr Tyr Leu Gly Ala Leu Leu Leu Glu Glu Leu Gln Glu Tyr Gly Thr
            20                  25                  30

Val Glu Thr Leu Pro Leu Leu Val Arg Leu Asn Pro Thr Ile Pro Tyr
        35                  40                  45

Lys Thr Arg Gly Asn Ala Ala Ile Ala Leu Lys Leu Lys Thr Asp Cys
    50                  55                  60

Pro Glu Lys Ile Ile Ala His Val Thr Ser Arg Ile Glu Glu Phe Ala
65                  70                  75                  80

Arg Met Glu Cys Glu Lys Thr Asn Pro Gly Ala Val Phe Ile Gln Glu
                85                  90                  95

Lys Asp Tyr Arg Ser Leu Lys Pro Ile Leu Leu Ser Phe Leu Glu Lys
            100                 105                 110

Ala Val Lys Asp Val Ile Glu Ile Glu Thr Ala Lys His Leu Ile Ser
        115                 120                 125

Glu Leu Gly Ile Ser Ser Lys Ser Phe Lys Asn Gly Arg Gly Leu Ile
    130                 135                 140

Gly Ala Leu Ala Ala Cys Gly Ala Met Leu Asn Pro Glu Lys Trp Asp
145                 150                 155                 160

Cys Thr Phe Glu His Leu Ala Tyr Arg Gln Lys Lys Lys Trp Gly Ser
                165                 170                 175

Pro Arg Asp Val Asn Lys Asp Ser Phe Phe Glu Ala Asp Arg Gln Thr
            180                 185                 190

Tyr Pro Gly Thr Trp Asp Thr Val Asp Leu Ala Asn Arg Leu Val Val
        195                 200                 205

Cys Val Pro His Ser Ala Asp Pro Val Leu Phe Gly Ile Arg Gly Glu
    210                 215                 220

Ser Pro Glu Leu Val Ser Lys Ala Ala Ser Leu Ile Arg Ser Glu Pro

225                 230                 235                 240

Val Glu Arg Phe Ala Val Tyr Arg Thr Asn Gln Gly Thr Asp Met His
                245                 250                 255

Leu Leu Pro Ala Ala Ser Ile Ser Glu Ile Arg Asp Met His Ser Tyr
            260                 265                 270

Arg Phe Glu Gly Thr Val Ser Ala Val Pro Lys Thr Ile Glu Gly Gly
        275                 280                 285

His Val Ile Phe Ala Val Arg Asp Gly Lys Gly Asp Glu Ile Asp Cys
    290                 295                 300

Ala Ala Phe Glu Pro Thr Lys Asn Phe Arg Val Leu Ala Arg Arg Leu
305                 310                 315                 320

Leu Leu Gly Asp Gln Ile Phe Leu Ser Gly Ser Val Thr Ser Gly Thr
                325                 330                 335

Leu Asn Ile Glu Lys Met Gln Val Lys Glu Leu Val Leu Leu Tyr Arg
            340                 345                 350

Glu Glu Asn Pro Lys Cys Pro Glu Cys Gly Lys His Met Lys Ser Ala
        355                 360                 365

Gly Gln Gly Gln Gly Phe Arg Cys Lys Lys Cys Gly Thr Arg Ala Ser
    370                 375                 380

Ser Lys Ile Arg Cys Glu Ala Glu Arg Asp Leu Glu Pro Gly Leu Tyr
385                 390                 395                 400

Glu Val Pro Pro Cys Ala Arg Arg His Leu Ala Lys Pro Leu Ala Arg
                405                 410                 415

Glu Arg Asp Gln Asn Ile Arg Ile His Pro Ser Arg
            420                 425


<210>  53
<211>  17
<212>  PRT
<213>  Artificial Sequence

<220>
<223>  LCT12

<400>  53

Phe Thr Ile Phe Pro Gly Phe Ile Ile Thr Thr Gly Thr Gly Thr Gly
1               5                   10                  15

Ala

<210>  54
<211>  34
<212>  RNA
<213>  Artificial Sequence

<220>
<223>  FR-1

<400>  54
guccccuucg ucuagaggcc caggacaccg cccu                                    34


<210>  55
<211>  39
<212>  RNA
<213>  Artificial Sequence

<220>
<223>  FR-2

<400>  55
   gaacggcggu aacagggguu cgaauccccu aggggacgc                        39

<210>  56
<211>  74
<212>  RNA
<213>  Artificial Sequence

<220>
<223>  UR-1

<400>  56
guccccuucg ucuagaggcc caggacaccg cccuugaacg gcgguaacag ggguucgaau        60

ccccuagggg acgc                                                          74


<210>  57
<211>  74
<212>  RNA
<213>  Artificial Sequence

<220>
<223>  LR-1



<220>
<221>  misc_feature
<222>  (35)..(35)
<223>  n = lysidine (2-lysylcytidine)

<400>  57
guccccuucg ucuagaggcc caggacaccg cccungaacg gcgguaacag ggguucgaau        60

ccccuagggg acgc                                                          74

<210>  58
<211>  39
<212>  RNA
<213>  Artificial Sequence

<220>
<223>  FR-3

<400>  58
   agacggcggu aacagggguu cgaauccccu aggggacgc                                    39

<210>  59
<211>  74
<212>  RNA
<213>  Artificial Sequence

<220>
<223>  LR-2

<220>
<221>  misc_feature
<222>  (35)..(35)
<223>  n = lysidine (2-lysylcytidine)

<400>  59
guccccuucg ucuagaggcc caggacaccg cccunagacg gcgguaacag ggguucgaau        60

ccccuagggg acgc                                                          74

<210>  60
<211>  39
<212>  RNA
<213>  Artificial Sequence

<220>
<223>  FR-4

<400>  60
   acacggcggu aacagggguu cgaauccccu aggggacgc                                    39

<210>  61
<211>  74
<212>  RNA
<213>  Artificial Sequence

<220>
<223>  LR-3

<220>
<221>  misc_feature
<222>  (35)..(35)
<223>  n = lysidine (2-lysylcytidine)

<400>  61
guccccuucg ucuagaggcc caggacaccg cccunacacg gcgguaacag ggguucgaau        60

ccccuagggg acgc                                                          74

<210>  62
<211>  39
<212>  RNA
<213>  Artificial Sequence

<220>
<223>  FR-5

<400>  62
ccacggcggu aacagggguu cgaauccccu aggggacgc                                39


<210>  63
<211>  74
<212>  RNA
<213>  Artificial Sequence

<220>
<223>  LR-4


<220>
<221>  misc_feature
<222>  (35)..(35)
<223>  n = lysidine (2-lysylcytidine)

<400>  63
guccccuucg ucuagaggcc caggacaccg cccunccacg gcgguaacag ggguucgaau        60

ccccuagggg acgc                                                          74


<210>  64
<211>  95
<212>  DNA
<213>  Artificial Sequence

<220>
<223>  D-1

<400>  64
ggcgtaatac gactcactat agtccccttc gtctagaggc ccaggacacc gccctagaac        60

ggcggtaaca ggggttcgaa tcccctaggg gacgc                                   95


<210>  65
<211>  95
<212>  DNA
<213>  Artificial Sequence

<220>
<223>  D-2

<400>  65
ggcgtaatac gactcactat agtccccttc gtctagaggc ccaggacacc gcccttgaac        60

ggcggtaaca ggggttcgaa tcccctaggg gacgc                                   95


<210>  66

<211>  95
<212>  DNA
<213>  Artificial Sequence

<220>
<223>  D-3

<400>  66
ggcgtaatac gactcactat agtccccttc gtctagaggc ccaggacacc gccctcgaac      60

ggcggtaaca ggggttcgaa tcccctaggg gacgc                                 95


<210>  67
<211>  95
<212>  DNA
<213>  Artificial Sequence

<220>
<223>  D-4

<400>  67
ggcgtaatac gactcactat agtccccttc gtctagaggc ccaggacacc gccctaagac      60

ggcggtaaca ggggttcgaa tcccctaggg gacgc                                 95


<210>  68
<211>  95
<212>  DNA
<213>  Artificial Sequence

<220>
<223>  D-5

<400>  68
ggcgtaatac gactcactat agtccccttc gtctagaggc ccaggacacc gcccttagac      60

ggcggtaaca ggggttcgaa tcccctaggg gacgc                                 95


<210>  69
<211>  95
<212>  DNA
<213>  Artificial Sequence

<220>
<223>  D-6

<400>  69
ggcgtaatac gactcactat agtccccttc gtctagaggc ccaggacacc gccctcagac      60

ggcggtaaca ggggttcgaa tcccctaggg gacgc                                 95


<210>  70
<211>  95
<212>  DNA
<213>  Artificial Sequence

<220>
<223>  D-7

<400>  70
ggcgtaatac gactcactat agtccccttc gtctagaggc ccaggacacc gccctaacac        60

ggcggtaaca ggggttcgaa tcccctaggg gacgc                                   95


<210>  71
<211>  95
<212>  DNA
<213>  Artificial Sequence

<220>
<223>  D-8

<400>  71
ggcgtaatac gactcactat agtccccttc gtctagaggc ccaggacacc gcccttacac        60

ggcggtaaca ggggttcgaa tcccctaggg gacgc                                   95


<210>  72
<211>  95
<212>  DNA
<213>  Artificial Sequence

<220>
<223>  D-9

<400>  72
ggcgtaatac gactcactat agtccccttc gtctagaggc ccaggacacc gccctcacac        60

ggcggtaaca ggggttcgaa tcccctaggg gacgc                                   95


<210>  73
<211>  95
<212>  DNA
<213>  Artificial Sequence

<220>
<223>  D-10

<400>  73
ggcgtaatac gactcactat agtccccttc gtctagaggc ccaggacacc gccctgccac        60

ggcggtaaca ggggttcgaa tcccctaggg gacgc                                   95


<210>  74
<211>  95
<212>  DNA
<213>  Artificial Sequence

<220>
<223>  D-11

<400>  74
ggcgtaatac gactcactat agtccccttc gtctagaggc ccaggacacc gcccttccac        60

ggcggtaaca ggggttcgaa tcccctaggg gacgc                                   95


<210>  75

<211>  95
<212>  DNA
<213>  Artificial Sequence

<220>
<223>  D-12

<400>  75
ggcgtaatac gactcactat agtccccttc gtctagaggc ccaggacacc gccctcccac      60

ggcggtaaca ggggttcgaa tcccctaggg gacgc                                 95


<210>  76
<211>  96
<212>  DNA
<213>  Artificial Sequence

<220>
<223>  D-13

<400>  76
ggcgtaatac gactcactat aggcggggtg gagcagcctg gtagctcgtc gggctcataa      60

cccgaagatc gtcggttcaa atccggcccc cgcaac                                96


<210>  77
<211>  74
<212>  RNA
<213>  Artificial Sequence

<220>
<223>  TR-1

<400>  77
   guccccuucg ucuagaggcc caggacaccg cccuagaacg gcgguaacag ggguucgaau      60
   ccccuagggg acgc                                                        74


<210>  78
<211>  74
<212>  RNA
<213>  Artificial Sequence

<220>
<223>  TR-2

<400>  78
   guccccuucg ucuagaggcc caggacaccg cccuugaacg gcgguaacag ggguucgaau      60
   ccccuagggg acgc                                                        74


<210>  79
<211>  74
<212>  RNA
<213>  Artificial Sequence

<220>
<223>  TR-3

<400>  79
guccccuucg ucuagaggcc caggacaccg cccucgaacg gcgguaacag ggguucgaau      60

ccccuagggg acgc                                                        74


<210>  80
<211>  74
<212>  RNA
<213>  Artificial Sequence

<220>
<223>  TR-4

<400>  80
guccccuucg ucuagaggcc caggacaccg cccuaagacg gcgguaacag ggguucgaau      60

ccccuagggg acgc                                                        74


<210>  81
<211>  74
<212>  RNA
<213>  Artificial Sequence

<220>
<223>  TR-5

<400>  81
guccccuucg ucuagaggcc caggacaccg cccuuagacg gcgguaacag ggguucgaau      60

ccccuagggg acgc                                                        74


<210>  82
<211>  74
<212>  RNA
<213>  Artificial Sequence

<220>
<223>  TR-6

<400>  82
guccccuucg ucuagaggcc caggacaccg cccucagacg gcgguaacag ggguucgaau      60

ccccuagggg acgc                                                        74


<210>  83
<211>  74
<212>  RNA
<213>  Artificial Sequence

<220>
<223>  TR-7

<400>  83
guccccuucg ucuagaggcc caggacaccg cccuaacacg gcgguaacag ggguucgaau      60

ccccuagggg acgc                                                        74


<210>  84

<211>  74
<212>  RNA
<213>  Artificial Sequence

<220>
<223>  TR-8

<400>  84
guccccuucg ucuagaggcc caggacaccg cccuuacacg gcgguaacag ggguucgaau      60

ccccuagggg acgc                                                       74


<210>  85
<211>  74
<212>  RNA
<213>  Artificial Sequence

<220>
<223>  TR-9

<400>  85
guccccuucg ucuagaggcc caggacaccg cccucacacg gcgguaacag gguucgaau      60

ccccuagggg acgc                                                       74


<210>  86
<211>  74
<212>  RNA
<213>  Artificial Sequence

<220>
<223>  TR-10

<400>  86
guccccuucg ucuagaggcc caggacaccg cccugccacg gcgguaacag gguucgaau      60

ccccuagggg acgc                                                       74


<210>  87
<211>  74
<212>  RNA
<213>  Artificial Sequence

<220>
<223>  TR-11

<400>  87
guccccuucg ucuagaggcc caggacaccg cccuuccacg gcgguaacag gguucgaau      60

ccccuagggg acgc                                                       74


<210>  88
<211>  74
<212>  RNA
<213>  Artificial Sequence

<220>
<223>  TR-12

<400>  88
guccccuucg ucuagaggcc caggacaccg cccucccacg gcgguaacag ggguucgaau      60

ccccuagggg acgc                                                        74


<210>  89
<211>  74
<212>  RNA
<213>  Artificial Sequence

<220>
<223>  TR-13

<400>  89
ggcggggugg agcagccugg uagcucgucg ggcucauaac ccgaagaucg ucgguucaaa      60

uccggccccc gcaa                                                        74


<210>  90
<211>  76
<212>  RNA
<213>  Artificial Sequence

<220>
<223>  AAtR-1

<400>  90
guccccuucg ucuagaggcc caggacaccg cccuagaacg gcgguaacag ggguucgaau      60

ccccuagggg acgcca                                                      76


<210>  91
<211>  76
<212>  RNA
<213>  Artificial Sequence

<220>
<223>  AAtR-2

<400>  91
guccccuucg ucuagaggcc caggacaccg cccuugaacg gcgguaacag ggguucgaau      60

ccccuagggg acgcca                                                      76


<210>  92
<211>  76
<212>  RNA
<213>  Artificial Sequence

<220>
<223>  AAtR-3

<400>  92
guccccuucg ucuagaggcc caggacaccg cccuugaacg gcgguaacag ggguucgaau      60

ccccuagggg acgcca                                                      76


<210>  93

<211>  76
<212>  RNA
<213>  Artificial Sequence

<220>
<223>  AAtR-4


<220>
<221>  misc_feature
<222>  (35)..(35)
<223>  n = lysidine (2-lysylcytidine)

<400>  93
guccccuucg ucuagaggcc caggacaccg cccungaacg gcgguaacag ggguucgaau      60

ccccuagggg acgcca                                                      76


<210>  94
<211>  76
<212>  RNA
<213>  Artificial Sequence

<220>
<223>  AAtR-5

<400>  94
guccccuucg ucuagaggcc caggacaccg cccucgaacg gcgguaacag ggguucgaau      60

ccccuagggg acgcca                                                      76


<210>  95
<211>  76
<212>  RNA
<213>  Artificial Sequence

<220>
<223>  AAtR-6

<400>  95
guccccuucg ucuagaggcc caggacaccg cccuaagacg gcgguaacag ggguucgaau      60

ccccuagggg acgcca                                                      76


<210>  96
<211>  76
<212>  RNA
<213>  Artificial Sequence

<220>
<223>  AAtR-7

<400>  96
guccccuucg ucuagaggcc caggacaccg cccuuagacg gcgguaacag ggguucgaau      60

ccccuagggg acgcca                                                      76


<210>  97
<211>  76

<212>  RNA
<213>  Artificial Sequence

<220>
<223>  AAtR-8



<220>
<221>  misc_feature
<222>  (35)..(35)
<223>  n = lysidine (2-lysylcytidine)

<400>  97
guccccuucg ucuagaggcc caggacaccg cccunagacg gcgguaacag ggguucgaau      60

ccccuagggg acgcca                                                      76


<210>  98
<211>  76
<212>  RNA
<213>  Artificial Sequence

<220>
<223>  AAtR-9

<400>  98
guccccuucg ucuagaggcc caggacaccg cccucagacg gcgguaacag ggguucgaau      60

ccccuagggg acgcca                                                      76


<210>  99
<211>  76
<212>  RNA
<213>  Artificial Sequence

<220>
<223>  AAtR-10

<400>  99
guccccuucg ucuagaggcc caggacaccg cccuaacacg gcgguaacag ggguucgaau      60

ccccuagggg acgcca                                                      76


<210>  100
<211>  76
<212>  RNA
<213>  Artificial Sequence

<220>
<223>  AAtR-11

<400>  100
guccccuucg ucuagaggcc caggacaccg cccuuacacg gcgguaacag ggguucgaau      60

ccccuagggg acgcca                                                      76


<210>  101
<211>  76
<212>  RNA

<213>  Artificial Sequence

<220>
<223>  AAtR-12


<220>
<221>  misc_feature
<222>  (35)..(35)
<223>  n = lysidine (2-lysylcytidine)

<400>  101
guccccuucg ucuagaggcc caggacaccg cccunacacg gcgguaacag ggguucgaau      60

ccccuagggg acgcca                                                      76


<210>  102
<211>  76
<212>  RNA
<213>  Artificial Sequence

<220>
<223>  AAtR-13

<400>  102
guccccuucg ucuagaggcc caggacaccg cccucacacg gcgguaacag ggguucgaau      60

ccccuagggg acgcca                                                      76


<210>  103
<211>  76
<212>  RNA
<213>  Artificial Sequence

<220>
<223>  AAtR-14

<400>  103
guccccuucg ucuagaggcc caggacaccg cccugccacg gcgguaacag ggguucgaau      60

ccccuagggg acgcca                                                      76


<210>  104
<211>  76
<212>  RNA
<213>  Artificial Sequence

<220>
<223>  AAtR-15

<400>  104
guccccuucg ucuagaggcc caggacaccg cccuuccacg gcgguaacag ggguucgaau      60

ccccuagggg acgcca                                                      76


<210>  105
<211>  76
<212>  RNA
<213>  Artificial Sequence

<220>
<223>  AAtR-16


<220>
<221>  misc_feature
<222>  (35)..(35)
<223>  n = lysidine (2-lysylcytidine)

<400>  105
guccccuucg ucuagaggcc caggacaccg cccunccacg gcgguaacag ggguucgaau      60

ccccuagggg acgcca                                                      76


<210>  106
<211>  76
<212>  RNA
<213>  Artificial Sequence

<220>
<223>  AAtR-17

<400>  106
guccccuucg ucuagaggcc caggacaccg cccucccacg gcgguaacag ggguucgaau      60

ccccuagggg acgcca                                                      76


<210>  107
<211>  76
<212>  RNA
<213>  Artificial Sequence

<220>
<223>  AAtR-18

<400>  107
ggcggggugg agcagccugg uagcucgucg ggcucauaac ccgaagaucg ucgguucaaa      60

uccggccccc gcaaca                                                      76


<210>  108
<211>  97
<212>  DNA
<213>  Artificial Sequence

<220>
<223>  D-14

<400>  108
ggcgtaatac gactcactat agggttaact ttaagaagga gatatacata tgacttttat      60

tattggtttt tctattattc cgattggtta agcttcg                               97


<210>  109
<211>  97
<212>  DNA
<213>  Artificial Sequence

<220>
<223>  D-15

<400>  109
ggcgtaatac gactcactat agggttaact ttaagaagga gatatacata tgacttttat      60

tattggtttt tcaattattc cgattggtta agcttcg                               97


<210>  110
<211>  97
<212>  DNA
<213>  Artificial Sequence

<220>
<223>  D-16

<400>  110
ggcgtaatac gactcactat agggttaact ttaagaagga gatatacata tgacttttat      60

tattggtttt tcgattattc cgattggtta agcttcg                               97


<210>  111
<211>  97
<212>  DNA
<213>  Artificial Sequence

<220>
<223>  D-17

<400>  111
ggcgtaatac gactcactat agggttaact ttaagaagga gatatacata tgacttttat      60

tattggtttt cttattattc cgattggtta agcttcg                               97


<210>  112
<211>  97
<212>  DNA
<213>  Artificial Sequence

<220>
<223>  D-18

<400>  112
ggcgtaatac gactcactat agggttaact ttaagaagga gatatacata tgacttttat      60

tattggtttt ctaattattc cgattggtta agcttcg                               97


<210>  113
<211>  97
<212>  DNA
<213>  Artificial Sequence

<220>
<223>  D-19

<400>  113
ggcgtaatac gactcactat agggttaact ttaagaagga gatatacata tgacttttat      60

tattggtttt ctgattattc cgattggtta agcttcg                               97

<210>  114
<211>  97
<212>  DNA
<213>  Artificial Sequence

<220>
<223>  D-20

<400>  114
ggcgtaatac gactcactat agggttaact ttaagaagga gatatacata tgacttttat      60

tattggtttt gttattattc cgattggtta agcttcg                               97


<210>  115
<211>  97
<212>  DNA
<213>  Artificial Sequence

<220>
<223>  D-21

<400>  115
ggcgtaatac gactcactat agggttaact ttaagaagga gatatacata tgacttttat      60

tattggtttt gtaattattc cgattggtta agcttcg                               97


<210>  116
<211>  97
<212>  DNA
<213>  Artificial Sequence

<220>
<223>  D-22

<400>  116
ggcgtaatac gactcactat agggttaact ttaagaagga gatatacata tgacttttat      60

tattggtttt gtgattattc cgattggtta agcttcg                               97


<210>  117
<211>  97
<212>  DNA
<213>  Artificial Sequence

<220>
<223>  D-23

<400>  117
ggcgtaatac gactcactat agggttaact ttaagaagga gatatacata tgacttttat      60

tattctattt ggtattattc cgattctata agcttcg                               97


<210>  118
<211>  97
<212>  DNA
<213>  Artificial Sequence

<220>
<223>  D-24

<400>  118
ggcgtaatac gactcactat agggttaact ttaagaagga gatatacata tgacttttat      60

tattctattt ggaattattc cgattctata agcttcg                              97


<210>  119
<211>  97
<212>  DNA
<213>  Artificial Sequence

<220>
<223>  D-25

<400>  119
ggcgtaatac gactcactat agggttaact ttaagaagga gatatacata tgacttttat      60

tattctattt gggattattc cgattctata agcttcg                              97


<210>  120
<211>  76
<212>  RNA
<213>  Artificial Sequence

<220>
<223>  mR-1

<400>  120
ggguuaacuu uaagaaggag auauacauau gacuuuuauu auugguuuuu cuauuauucc      60

gauugguuaa gcuucg                                                     76


<210>  121
<211>  76
<212>  RNA
<213>  Artificial Sequence

<220>
<223>  mR-2

<400>  121
ggguuaacuu uaagaaggag auauacauau gacuuuuauu auugguuuuu caauuauucc      60

gauugguuaa gcuucg                                                     76


<210>  122
<211>  76
<212>  RNA
<213>  Artificial Sequence

<220>
<223>  mR-3

<400>  122
ggguuaacuu uaagaaggag auauacauau gacuuuuauu auugguuuuu cgauuauucc      60

gauugguuaa gcuucg                                                     76

<210>  123
<211>  76
<212>  RNA
<213>  Artificial Sequence

<220>
<223>  mR-4

<400>  123
ggguuaacuu uaagaaggag auauacauau gacuuuuauu auugguuuuc uuauuauucc      60

gauugguuaa gcuucg                                                      76


<210>  124
<211>  76
<212>  RNA
<213>  Artificial Sequence

<220>
<223>  mR-5

<400>  124
ggguuaacuu uaagaaggag auauacauau gacuuuuauu auugguuuuc uaauuauucc      60

gauugguuaa gcuucg                                                      76


<210>  125
<211>  76
<212>  RNA
<213>  Artificial Sequence

<220>
<223>  mR-6

<400>  125
ggguuaacuu uaagaaggag auauacauau gacuuuuauu auugguuuuc ugauuauucc      60

gauugguuaa gcuucg                                                      76


<210>  126
<211>  76
<212>  RNA
<213>  Artificial Sequence

<220>
<223>  mR-7

<400>  126
ggguuaacuu uaagaaggag auauacauau gacuuuuauu auugguuuug uuauuauucc      60

gauugguuaa gcuucg                                                      76


<210>  127
<211>  76
<212>  RNA
<213>  Artificial Sequence

<220>
<223>  mR-8

<400>  127
ggguuaacuu uaagaaggag auauacauau gacuuuuauu auugguuuug uaauuauucc      60

gauugguuaa gcuucg                                                      76


<210>  128
<211>  76
<212>  RNA
<213>  Artificial Sequence

<220>
<223>  mR-9

<400>  128
ggguuaacuu uaagaaggag auauacauau gacuuuuauu auugguuuug ugauuauucc      60

gauugguuaa gcuucg                                                      76


<210>  129
<211>  76
<212>  RNA
<213>  Artificial Sequence

<220>
<223>  mR-10

<400>  129
ggguuaacuu uaagaaggag auauacauau gacuuuuauu auucuauuug guauuauucc      60

gauucuauaa gcuucg                                                      76


<210>  130
<211>  76
<212>  RNA
<213>  Artificial Sequence

<220>
<223>  mR-11

<400>  130
ggguuaacuu uaagaaggag auauacauau gacuuuuauu auucuauuug gaauuauucc      60

gauucuauaa gcuucg                                                      76


<210>  131
<211>  76
<212>  RNA
<213>  Artificial Sequence

<220>
<223>  mR-12

<400>  131
ggguuaacuu uaagaaggag auauacauau gacuuuuauu auucuauuug ggauuauucc      60

gauucuauaa gcuucg                                                      76

<210>  132
<211>  34
<212>  RNA
<213>  Artificial Sequence

<220>
<223>  FR-6

<400>  132
ggagcgguag uucagucggu uagaauaccu gcuu                                    34


<210>  133
<211>  40
<212>  RNA
<213>  Artificial Sequence

<220>
<223>  FR-7

<400>  133
aggugcaggg ggucgcgggu ucgagucccg uccguuccgc                              40


<210>  134
<211>  75
<212>  RNA
<213>  Artificial Sequence

<220>
<223>  LR-5


<220>
<221>  misc_feature
<222>  (35)..(35)
<223>  n = lysidine (2-lysylcytidine)

<400>  134
ggagcgguag uucagucggu uagaauaccu gcuunaggug cagggggucg cggguucgag        60

ucccguccgu uccgc                                                         75


<210>  135
<211>  33
<212>  RNA
<213>  Artificial Sequence

<220>
<223>  FR-8

<400>  135
ggcucuguag uucagucggu agaacggcgg auu                                     33


<210>  136
<211>  40
<212>  RNA
<213>  Artificial Sequence

<220>
<223>  FR-9

<400>  136
agguuccgua ugucacuggu ucgaguccag ucagagccgc                              40


<210>  137
<211>  74
<212>  RNA
<213>  Artificial Sequence

<220>
<223>  LR-6


<220>
<221>  misc_feature
<222>  (34)..(34)
<223>  n = lysidine (2-lysylcytidine)

<400>  137
ggcucuguag uucagucggu agaacggcgg auunagguuc cguaugucac ugguucgagu        60

ccagucagag ccgc                                                          74


<210>  138
<211>  74
<212>  RNA
<213>  Artificial Sequence

<220>
<223>  AR-1


<220>
<221>  misc_feature
<222>  (35)..(35)
<223>  n = agmatidine (2-agmatinylcytidine)

<400>  138
guccccuucg ucuagaggcc caggacaccg cccunagacg gcgguaacag ggguucgaau        60

ccccuagggg acgc                                                          74


<210>  139
<211>  39
<212>  RNA
<213>  Artificial Sequence

<220>
<223>  FR-10

<400>  139
cgacggcggu aacagggguu cgaauccccu aggggacgc                               39


<210>  140
<211>  74
<212>  RNA
<213>  Artificial Sequence

<220>
<223>  LR-7

<220>
<221>  misc_feature
<222>  (35)..(35)
<223>  n = lysidine (2-lysylcytidine)

<400>  140
guccccuucg ucuagaggcc caggacaccg cccuncgacg gcgguaacag ggguucgaau      60

ccccuagggg acgc                                                        74

<210>  141
<211>  39
<212>  RNA
<213>  Artificial Sequence

<220>
<223>  FR-11

<400>  141
   auacggcggu aacagggguu cgaauccccu aggggacgc                            39

<210>  142
<211>  74
<212>  RNA
<213>  Artificial Sequence

<220>
<223>  LR-8

<220>
<221>  misc_feature
<222>  (35)..(35)
<223>  n = lysidine (2-lysylcytidine)

<400>  142
guccccuucg ucuagaggcc caggacaccg cccunauacg gcgguaacag ggguucgaau      60

ccccuagggg acgc                                                        74

<210>  143
<211>  96
<212>  DNA
<213>  Artificial Sequence

<220>
<223>  D-26

<400>  143
ggcgtaatac gactcactat aggagcggta gttcagtcgg ttagaatacc tgcttaaggt      60

gcagggggtc gcgggttcga gtcccgtccg ttccgc                                96

<210>  144

<211>  96
<212>  DNA
<213>  Artificial Sequence

<220>
<223>  D-27

<400>  144
ggcgtaatac gactcactat aggagcggta gttcagtcgg ttagaatacc tgctttaggt      60

gcagggggtc gcgggttcga gtcccgtccg ttccgc                                96


<210>  145
<211>  96
<212>  DNA
<213>  Artificial Sequence

<220>
<223>  D-28

<400>  145
ggcgtaatac gactcactat aggagcggta gttcagtcgg ttagaatacc tgcttcaggt      60

gcagggggtc gcgggttcga gtcccgtccg ttccgc                                96


<210>  146
<211>  95
<212>  DNA
<213>  Artificial Sequence

<220>
<223>  D-29

<400>  146
ggcgtaatac gactcactat aggctctgta gttcagtcgg tagaacggcg gattaaggtt      60

ccgtatgtca ctggttcgag tccagtcaga gccgc                                 95


<210>  147
<211>  95
<212>  DNA
<213>  Artificial Sequence

<220>
<223>  D-30

<400>  147
ggcgtaatac gactcactat aggctctgta gttcagtcgg tagaacggcg gatttaggtt      60

ccgtatgtca ctggttcgag tccagtcaga gccgc                                 95


<210>  148
<211>  95
<212>  DNA
<213>  Artificial Sequence

<220>
<223>  D-31

<400>  148
ggcgtaatac gactcactat aggctctgta gttcagtcgg tagaacggcg gattcaggtt      60

ccgtatgtca ctggttcgag tccagtcaga gccgc                                 95


<210>  149
<211>  95
<212>  DNA
<213>  Artificial Sequence

<220>
<223>  D-32

<400>  149
ggcgtaatac gactcactat agtccccttc gtctagaggc ccaggacacc gccctgcgac      60

ggcggtaaca ggggttcgaa tcccctaggg gacgc                                 95


<210>  150
<211>  95
<212>  DNA
<213>  Artificial Sequence

<220>
<223>  D-33

<400>  150
ggcgtaatac gactcactat agtccccttc gtctagaggc ccaggacacc gccctccgac      60

ggcggtaaca ggggttcgaa tcccctaggg gacgc                                 95


<210>  151
<211>  95
<212>  DNA
<213>  Artificial Sequence

<220>
<223>  D-34

<400>  151
ggcgtaatac gactcactat agtccccttc gtctagaggc ccaggacacc gccctaauac      60

ggcggtaaca ggggttcgaa tcccctaggg gacgc                                 95


<210>  152
<211>  95
<212>  DNA
<213>  Artificial Sequence

<220>
<223>  D-35

<400>  152
ggcgtaatac gactcactat agtccccttc gtctagaggc ccaggacacc gccctcauac      60

ggcggtaaca ggggttcgaa tcccctaggg gacgc                                 95


<210>  153

<211>  75
<212>  RNA
<213>  Artificial Sequence

<220>
<223>  TR-14

<400>  153
ggagcgguag uucagucggu uagaauaccu gcuuaaggug cagggggucg cggguucgag      60

ucccguccgu uccgc                                                      75


<210>  154
<211>  75
<212>  RNA
<213>  Artificial Sequence

<220>
<223>  TR-15

<400>  154
ggagcgguag uucagucggu uagaauaccu gcuuuaggug cagggggucg cggguucgag      60

ucccguccgu uccgc                                                      75


<210>  155
<211>  75
<212>  RNA
<213>  Artificial Sequence

<220>
<223>  TR-16

<400>  155
ggagcgguag uucagucggu uagaauaccu gcuucaggug cagggggucg cggguucgag      60

ucccguccgu uccgc                                                      75


<210>  156
<211>  74
<212>  RNA
<213>  Artificial Sequence

<220>
<223>  TR-17

<400>  156
ggcucuguag uucagucggu agaacggcgg auuaagguuc cguaugucac ugguucgagu      60

ccagucagag ccgc                                                       74


<210>  157
<211>  74
<212>  RNA
<213>  Artificial Sequence

<220>
<223>  TR-18

<400>  157
ggcucuguag uucagucggu agaacggcgg auuuagguuc cguaugucac ugguucgagu      60

ccagucagag ccgc                                                        74


<210>  158
<211>  74
<212>  RNA
<213>  Artificial Sequence

<220>
<223>  TR-19

<400>  158
ggcucuguag uucagucggu agaacggcgg auucagguuc cguaugucac ugguucgagu      60

ccagucagag ccgc                                                        74


<210>  159
<211>  74
<212>  RNA
<213>  Artificial Sequence

<220>
<223>  TR-20

<400>  159
guccccuucg ucuagaggcc caggacaccg cccuacgacg gcgguaacag ggguucgaau      60

ccccuagggg acgc                                                        74


<210>  160
<211>  74
<212>  RNA
<213>  Artificial Sequence

<220>
<223>  TR-21

<400>  160
guccccuucg ucuagaggcc caggacaccg cccuccgacg gcgguaacag ggguucgaau      60

ccccuagggg acgc                                                        74


<210>  161
<211>  74
<212>  RNA
<213>  Artificial Sequence

<220>
<223>  TR-22

<400>  161
guccccuucg ucuagaggcc caggacaccg cccuaauacg gcgguaacag ggguucgaau      60

ccccuagggg acgc                                                        74


<210>  162

<211>  74
<212>  RNA
<213>  Artificial Sequence

<220>
<223>  TR-23

<400>  162
guccccuucg ucuagaggcc caggacaccg cccucauacg gcgguaacag ggguucgaau      60

ccccuagggg acgc                                                        74


<210>  163
<211>  97
<212>  DNA
<213>  Artificial Sequence

<220>
<223>  D-36

<400>  163
ggcgtaatac gactcactat agggttaact ttaagaagga gatatacata tgacttttat      60

tattggtttt cgtattattc cgattggtta agcttcg                               97


<210>  164
<211>  97
<212>  DNA
<213>  Artificial Sequence

<220>
<223>  D-37

<400>  164
ggcgtaatac gactcactat agggttaact ttaagaagga gatatacata tgacttttat      60

tattggtttt cgaattattc cgattggtta agcttcg                               97


<210>  165
<211>  97
<212>  DNA
<213>  Artificial Sequence

<220>
<223>  D-38

<400>  165
ggcgtaatac gactcactat agggttaact ttaagaagga gatatacata tgacttttat      60

tattggtttt cggattattc cgattggtta agcttcg                               97


<210>  166
<211>  97
<212>  DNA
<213>  Artificial Sequence

<220>
<223>  D-39

<400>  166
ggcgtaatac gactcactat agggttaact ttaagaagga gatatacata tgacttttct      60

actaggtttt attctactac cgctaggtta agcttcg                               97


<210>  167
<211>  97
<212>  DNA
<213>  Artificial Sequence

<220>
<223>  D-40

<400>  167
ggcgtaatac gactcactat agggttaact ttaagaagga gatatacata tgacttttct      60

actaggtttt atactactac cgctaggtta agcttcg                               97


<210>  168
<211>  97
<212>  DNA
<213>  Artificial Sequence

<220>
<223>  D-41

<400>  168
ggcgtaatac gactcactat agggttaact ttaagaagga gatatacata tgacttttct      60

actaggtttt atgctactac cgctaggtta agcttcg                               97


<210>  169
<211>  76
<212>  RNA
<213>  Artificial Sequence

<220>
<223>  mR-13

<400>  169
ggguuaacuu uaagaaggag auauacauau gacuuuuauu auugguuuuc guauuauucc      60

gauugguuaa gcuucg                                                      76


<210>  170
<211>  76
<212>  RNA
<213>  Artificial Sequence

<220>
<223>  mR-14

<400>  170
ggguuaacuu uaagaaggag auauacauau gacuuuuauu auugguuuuc gaauuauucc      60

gauugguuaa gcuucg                                                      76


<210>  171

<211>  76
<212>  RNA
<213>  Artificial Sequence

<220>
<223>  mR-15

<400>  171
ggguuaacuu uaagaaggag auauacauau gacuuuuauu auugguuuuc ggauuauucc      60

gauugguuaa gcuucg                                                      76


<210>  172
<211>  76
<212>  RNA
<213>  Artificial Sequence

<220>
<223>  mR-16

<400>  172
ggguuaacuu uaagaaggag auauacauau gacuuuucua cuagguuuua uucuacuacc      60

gcuagguuaa gcuucg                                                      76


<210>  173
<211>  76
<212>  RNA
<213>  Artificial Sequence

<220>
<223>  mR-17

<400>  173
ggguuaacuu uaagaaggag auauacauau gacuuuucua cuagguuuua uacuacuacc      60

gcuagguuaa gcuucg                                                      76


<210>  174
<211>  76
<212>  RNA
<213>  Artificial Sequence

<220>
<223>  mR-18

<400>  174
ggguuaacuu uaagaaggag auauacauau gacuuuucua cuagguuuua ugcuacuacc      60

gcuagguuaa gcuucg                                                      76


<210>  175
<211>  77
<212>  RNA
<213>  Artificial Sequence

<220>
<223>  AAtR-19

<400>  175
ggagcgguag uucagucggu uagaauaccu gcuuaaggug cagggggucg cggguucgag      60

ucccguccgu uccgcca                                                      77


<210>  176
<211>  77
<212>  RNA
<213>  Artificial Sequence

<220>
<223>  AAtR-20

<400>  176
ggagcgguag uucagucggu uagaauaccu gcuuuaggug cagggggucg cggguucgag      60

ucccguccgu uccgcca                                                      77


<210>  177
<211>  77
<212>  RNA
<213>  Artificial Sequence

<220>
<223>  AAtR-21


<220>
<221>  misc_feature
<222>  (35)..(35)
<223>  n = lysidine (2-lysylcytidine)

<400>  177
ggagcgguag uucagucggu uagaauaccu gcuunaggug cagggggucg cggguucgag      60

ucccguccgu uccgcca                                                      77


<210>  178
<211>  77
<212>  RNA
<213>  Artificial Sequence

<220>
<223>  AAtR-22

<400>  178
ggagcgguag uucagucggu uagaauaccu gcuucaggug cagggggucg cggguucgag      60

ucccguccgu uccgcca                                                      77


<210>  179
<211>  76
<212>  RNA
<213>  Artificial Sequence

<220>
<223>  AAtR-23

<400>  179

ggcucuguag uucagucggu agaacggcgg auuaagguuc cguaugucac ugguucgagu 60

ccagucagag ccgcca 76

<210> 180
<211> 76
<212> RNA
<213> Artificial Sequence

<220>
<223> AAtR-24

<400> 180
ggcucuguag uucagucggu agaacggcgg auuuagguuc cguaugucac ugguucgagu 60

ccagucagag ccgcca 76

<210> 181
<211> 76
<212> RNA
<213> Artificial Sequence

<220>
<223> AAtR-25

<220>
<221> misc_feature
<222> (34)..(34)
<223> n = lysidine (2-lysylcytidine)

<400> 181
ggcucuguag uucagucggu agaacggcgg auunagguuc cguaugucac ugguucgagu 60

ccagucagag ccgcca 76

<210> 182
<211> 76
<212> RNA
<213> Artificial Sequence

<220>
<223> AAtR-26

<400> 182
ggcucuguag uucagucggu agaacggcgg auucagguuc cguaugucac ugguucgagu 60

ccagucagag ccgcca 76

<210> 183
<211> 76
<212> RNA
<213> Artificial Sequence

<220>
<223> AAtR-27

<400> 183
   guccccuucg ucuagaggcc caggacaccg cccuuagacg gcgguaacag ggguucgaau 60
   ccccuagggg acgcca                                                    76

<210>  184
<211>  76
<212>  RNA
<213>  Artificial Sequence

<220>
<223>  AAtR-28

<220>
<221>  misc_feature
<222>  (35)..(35)
<223>  n = lysidine (2-lysylcytidine)

<400>  184
guccccuucg ucuagaggcc caggacaccg cccunagacg gcgguaacag ggguucgaau      60

ccccuagggg acgcca                                                    76

<210>  185
<211>  76
<212>  RNA
<213>  Artificial Sequence

<220>
<223>  AAtR-29

<400>  185
guccccuucg ucuagaggcc caggacaccg cccuuagacg gcgguaacag ggguucgaau      60

ccccuagggg acgcca                                                    76

<210>  186
<211>  76
<212>  RNA
<213>  Artificial Sequence

<220>
<223>  AAtR-30

<220>
<221>  misc_feature
<222>  (35)..(35)
<223>  n = lysidine (2-lysylcytidine)

<400>  186
guccccuucg ucuagaggcc caggacaccg cccunagacg gcgguaacag ggguucgaau      60

ccccuagggg acgcca                                                    76

<210>  187
<211>  76
<212>  RNA
<213>  Artificial Sequence

<220>
<223>  AAtR-31

<400>  187
guccccuucg ucuagaggcc caggacaccg cccuuagacg gcgguaacag ggguucgaau      60

ccccuagggg acgcca                                                     76


<210>  188
<211>  76
<212>  RNA
<213>  Artificial Sequence

<220>
<223>  AAtR-32


<220>
<221>  misc_feature
<222>  (35)..(35)
<223>  n = lysidine (2-lysylcytidine)

<400>  188
guccccuucg ucuagaggcc caggacaccg cccunagacg gcgguaacag gguucgaau      60

ccccuagggg acgcca                                                     76


<210>  189
<211>  76
<212>  RNA
<213>  Artificial Sequence

<220>
<223>  AAtR-33

<400>  189
guccccuucg ucuagaggcc caggacaccg cccugcgacg gcgguaacag gguucgaau      60

ccccuagggg acgcca                                                     76


<210>  190
<211>  76
<212>  RNA
<213>  Artificial Sequence

<220>
<223>  AAtR-34


<220>
<221>  misc_feature
<222>  (35)..(35)
<223>  n = lysidine (2-lysylcytidine)

<400>  190
guccccuucg ucuagaggcc caggacaccg cccuncgacg gcgguaacag gguucgaau      60

ccccuagggg acgcca                                                     76

<210>  191
<211>  76
<212>  RNA
<213>  Artificial Sequence

<220>
<223>  AAtR-35

<400>  191
guccccuucg ucuagaggcc caggacaccg cccuccgacg gcgguaacag ggguucgaau      60

ccccuagggg acgcca                                                      76


<210>  192
<211>  76
<212>  RNA
<213>  Artificial Sequence

<220>
<223>  AAtR-36

<400>  192
guccccuucg ucuagaggcc caggacaccg cccuaauacg gcgguaacag ggguucgaau      60

ccccuagggg acgcca                                                      76


<210>  193
<211>  76
<212>  RNA
<213>  Artificial Sequence

<220>
<223>  AAtR-37


<220>
<221>  misc_feature
<222>  (35)..(35)
<223>  n = lysidine (2-lysylcytidine)

<400>  193
guccccuucg ucuagaggcc caggacaccg cccunauacg gcgguaacag ggguucgaau      60

ccccuagggg acgcca                                                      76


<210>  194
<211>  76
<212>  RNA
<213>  Artificial Sequence

<220>
<223>  AAtR-38

<400>  194
guccccuucg ucuagaggcc caggacaccg cccucauacg gcgguaacag ggguucgaau      60

ccccuagggg acgcca                                                      76


<210>  195

<211>  76
<212>  RNA
<213>  Artificial Sequence

<220>
<223>  AAtR-39

<400>  195
guccccuucg ucuagaggcc caggacaccg cccuuagacg gcgguaacag ggguucgaau      60

ccccuagggg acgcca                                                      76


<210>  196
<211>  76
<212>  RNA
<213>  Artificial Sequence

<220>
<223>  AAtR-40



<220>
<221>  misc_feature
<222>  (35)..(35)
<223>  n = agmatidine (2-agmatinylcytidine)

<400>  196
guccccuucg ucuagaggcc caggacaccg cccunagacg gcgguaacag ggguucgaau      60

ccccuagggg acgcca                                                      76


<210>  197
<211>  10
<212>  RNA
<213>  Artificial Sequence

<220>
<223>  tRNA fragment



<220>
<221>  misc_feature
<222>  (5)..(5)
<223>  n = lysidine (2-lysylcytidine)

<400>  197
cccunacacg                                                             10


<210>  198
<211>  10
<212>  RNA
<213>  Artificial Sequence

<220>
<223>  tRNA fragment

<400>  198
cccuuacacg                                                             10

<210>  199
<211>  10
<212>  RNA
<213>  Artificial Sequence

<220>
<223>  tRNA fragment


<220>
<221>  misc_feature
<222>  (5)..(5)
<223>  n = lysidine (2-lysylcytidine)

<400>  199
cccunccacg                                                              10


<210>  200
<211>  10
<212>  RNA
<213>  Artificial Sequence

<220>
<223>  tRNA fragment

<400>  200
cccuuccacg                                                              10


<210>  201
<211>  74
<212>  RNA
<213>  Artificial Sequence

<220>
<223>  FR6 + FR7

<400>  201
ggagcgguag uucagucggu uagaauaccu gcuuaggugc agggggucgc ggguucgagu        60

cccguccguu ccgc                                                         74


<210>  202
<211>  10
<212>  RNA
<213>  Artificial Sequence

<220>
<223>  tRNA fragment


<220>
<221>  misc_feature
<222>  (5)..(5)
<223>  n = lysidine (2-lysylcytidine)

<400>  202
cccunauacg                                                              10


<210>  203

<211>  10
<212>  RNA
<213>  Artificial Sequence

<220>
<223>  tRNA fragment

<400>  203
cccuuauacg                                                              10